# EUROPEAN PATENT SPECIFICATION

(11) **EP 3 615 566 B1**
(45) Date of publication and mention of the grant of the patent: **20.12.2023**
(21) Application number: 18724726.7
(22) Date of filing: 27.04.2018
(51) Int. Cl.: C07K 14/725, C07K 16/00

(54) **MULTISPECIFIC MOLECULES COMPRISING A NON-IMMUNOGLOBULIN HETERODIMERIZATION DOMAIN AND USES THEREOF**
MULTISPEZIFISCHE MOLEKÜLE MIT EINEM NICHT-IMMUNOGLOBULIN-HETERODIMERISAZIONSBEREICH
MOLÉCULE MULTISPÉCIFIQUE AVEC UN DOMAIN DE HÉTÉRODIMÉRISATION NON-IMMUNOGLOBULINAIRE

(30) Priority: 28.04.2017 US 201762491633 P
(43) Date of publication of application: 04.03.2020
(60) Divisional application: 23206219.0
(73) Proprietor: Marengo Therapeutics, Inc., Cambridge, MA 02139 (US)
(72) Inventor: LOEW, Andreas, Boston,MA 02118 (US); VASH, Brian, Edward, Cambridge, MA 02142 (US); MAIOCCO, Stephanie, J., Cambridge, MA 02139 (US)
(74) Representative: Brand Murray Fuller LLP
(86) International application number: PCT/US2018/029951
(87) International publication number: WO 2018/201047

(56) References cited:
- WO-A1-2016/087416
- WO-A1-2016/115274
- WO-A1-2017/055391
- WO-A1-2017/165464
- WO-A1-2018/057955
- WO-A2-2012/131555
- SKEGRO D. ET AL.: "Immunoglobulin domain interface exchange as a platform technology for the generation of Fc heterodimers and bispecific antibodies", J. BIOL. CHEM., vol. 292, no. 23, 27 April 2017 (2017-04-27), pages 9745-9759, XP002782441,
- ORTIZ-SANCHEZ E. ET AL: "Antibody-cytokine fusion proteins: applications in cancer therapy", EXPERT OPIN. BIOL. THER., vol. 8, no. 5, 13 August 2015 (2015-08-13) , pages 609-632, XP002782442,
- SHPILBERG O. & JACKISH C.: "Subcutaneous administration of rituximab (MabThera) and trastuzumab (Herceptin) using hyaluronidase", BRITISH J. CANCER, vol. 109, 3 September 2013 (2013-09-03), pages 1556-1561, XP002782443,

## Description

### SEQUENCE LISTING

The instant application contains a Sequence Listing which has been submitted electronically in ASCII format. Said ASCII copy, created on April 26, 2018, is named E2070-7006WO_SL.txt and is 308,533 bytes in size.

### BACKGROUND

Multispecific molecules that include a non-immunoglobulin heterodimerization domain (e.g., a T cell receptor (TCR) constant domain), methods of making said molecules (e.g., in a single cell), and methods of using the same, are disclosed. Relevant prior art is: WO2012/131555, and Skegro (2017); JBC 292(23):9745-9759.

### SUMMARY OF THE INVENTION

The invention is defined in the appended claims. The disclosure relates, *inter alia,* to novel multispecific molecules that include a non-immunoglobulin heterodimerization domain (e.g., a naturally occurring heterodimerization domain (e.g., a T cell receptor (TCR) constant domain)). Without being bound by theory, the multispecific molecules disclosed herein are expected to provide correctly assembled multispecific molecules (e.g., bispecific antibodies) retaining a natural immunoglobulin like structure, comprising a non-immunoglobulin heterodimerization domain (e.g., a T cell receptor (TCR) constant domain). Accordingly, provided herein are, *inter alia,* multispecific molecules (e.g., multispecific (e.g., bispecific) antibody molecules) that include the aforementioned non-immunoglobulin heterodimerization domain, nucleic acids encoding the same, methods of producing the aforementioned molecules (e.g., in a single cell), and methods of treating a cancer using the aforementioned molecules.

In a first aspect, disclosed herein is a multispecific (e.g., bispecific) molecule (e.g., an isolated multispecific molecule), comprising at least two non-contiguous polypeptide chains comprising:
(a) a first antigen binding moiety (ABM) (e.g., a first antibody molecule);
(b) a second ABM (e.g., a second antibody molecule) that does not bind to the same epitope as the first ABM, and
(c) a heterodimerization domain comprising:
   - a first polypeptide chain comprising an immunoglobulin CH2 domain connected, optionally via a linker, to a TCRα constant domain or fragment thereof; and
   - a second polypeptide chain comprising an immunoglobulin CH2 domain connected, optionally via a linker, to a TCRβ constant domain or fragment thereof, wherein:
      (i) the first ABM is connected, optionally via a linker, to the first polypeptide
      (ii) the second ABM is connected, optionally via a linker, to the second polypeptide
      (iii) the TCRα constant domain or fragment thereof comprises or consists of at least 20, 30, 40, 50, 60, 70, 80, 90 or 100 contiguous amino acids of SEQ ID NO: 158 or at least 20, 30, 40, 50, 60, 70 or 80 contiguous amino acids of SEQ ID NO: 1; and/or
      (iv) the TCRβ constant domain or fragment thereof comprises or consists of at least 20, 30, 40, 50, 60, 70, 80, 90, 100, 110, 120, 130, 140, 150, 160 or 170 contiguous amino acids of SEQ ID NO: 159 or at least 20, 30, 40, 50, 60, 70, 80, 90, 100, 110, 120 or 130 contiguous amino acids of SEQ ID NO: 2.

The first polypeptide chain comprises an immunoglobulin CH2 domain (e.g., an IgG1, IgG2, or IgG4 CH2 domain) connected to the TCRα constant domain, optionally via a linker, and the second polypeptide chain comprises an immunoglobulin CH2 domain (e.g., an IgG1, IgG2, or IgG4 CH2 domain) connected to the TCRβ constant domain, optionally via a linker.

In a second aspect, the invention relates to an isolated nucleic acid molecule encoding the multispecific molecule of the first aspect.

In a third aspect, the invention relates to a vector comprising the nucleic acid molecule of the second aspect.

In a fourth aspect, the invention relates to a host cell comprising the nucleic acid molecule of the second aspect or the vector of the third aspect.

In some embodiments, the multispecific molecule may be constructed as follows:
(i) the first ABM is connected to the first polypeptide chain, optionally via a linker; and the second ABM is connected to the second polypeptide chain, optionally via a linker;
(ii) wherein:
   the first polypeptide chain comprises the immunoglobulin CH2 domain (e.g., an IgG1, IgG2, or IgG4 CH2 domain) connected, optionally via a linker, to the N-terminus or the C-terminus of the TCRα constant domain or fragment thereof, and/or
   the second polypeptide chain comprises the immunoglobulin CH2 domain (e.g., an IgG1, IgG2, or IgG4 CH2 domain) connected, optionally via a linker, to the N-terminus or the C-terminus of the TCRβ constant domain; and/or
(iii) optionally wherein:
   (a) the first polypeptide chain of the heterodimerization domain does not comprise an immunoglobulin CH3 domain or a portion thereof,
   (b) the second polypeptide chain of the heterodimerization domain does not comprise an immunoglobulin CH3 domain or a portion thereof
   (c) neither the first polypeptide chain nor the second polypeptide chain of the heterodimerization domain contains an immunoglobulin CH3 domain or a portion thereof;
optionally, wherein the linker comprises or consists of the amino acid sequence of any one of SEQIDNOs: 101-110.

In the multispecific molecule of the present invention, (i) the first ABM may be connected to the N-terminus of the first polypeptide chain, optionally via a linker; and/or (ii) the second ABM may be connected to the N-terminus of the second polypeptide chain, optionally via a linker. In the multispecific molecule of the present invention, (i) the first ABM may be connected to the C-terminus of the first polypeptide chain, optionally via a linker; and/or (ii) the second ABM may be connected to the C-terminus of the second polypeptide chain, optionally via a linker. In the multispecific molecule of the present invention, (i) the first polypeptide chain may comprise an immunoglobulin CH2 domain (e.g., an IgG1, IgG2, or IgG4 CH2 domain) connected to the TCRα constant domain, optionally via a linker, and/or (ii) the second polypeptide chain may comprise an immunoglobulin CH2 domain (e.g., an IgG1, IgG2, or IgG4 CH2 domain) connected to the TCRβ constant domain, optionally via a linker. In the multispecific molecule of the present invention, (i) the first polypeptide chain may comprise an immunoglobulin CH2 domain (e.g., an IgG1, IgG2, or IgG4 CH2 domain) connected to the N-terminus of the TCRα constant domain, optionally via a linker, and/or (ii) the second polypeptide chain may comprise an immunoglobulin CH2 domain (e.g., an IgG1, IgG2, or IgG4 CH2 domain) connected to the N-terminus of the TCRβ constant domain, optionally via a linker. In the multispecific molecule of the present invention, (i) the first polypeptide chain may comprise an immunoglobulin CH2 domain (e.g., an IgG1, IgG2, or IgG4 CH2 domain) connected to the C-terminus of the TCRα constant domain, optionally via a linker, and/or (ii) the second polypeptide chain may comprise an immunoglobulin CH2 domain (e.g., an IgG1, IgG2, or IgG4 CH2 domain) connected to the C-terminus of the TCRβ constant domain, optionally via a linker. In the multispecific molecule of the present invention, the linker may comprise or consist of the amino acid sequence of any of SEQ ID NOs: 101-110. The first and the second polypeptide chain of the heterodimerization domain may not contain any portion of an immunoglobulin CH3 domain capable of stable self-association (i.e., the first polypeptide chain does not contain any portion of a CH3 domain capable of stable association with the CH3 domain of the second polypeptide chain).

In some embodiments, the TCRα constant domain comprises or consists of the amino acid sequence of SEQ ID NO: 158, or a sequence having at least 75, 80, 85, 90, or 99% identity thereof and/or the TCRβ constant domain comprises or consists of the amino acid sequence of SEQ ID NO: 159, or a sequence having at least 75, 80, 85, 90, or 99% identity thereof, optionally wherein the TCRα constant domain comprises or consists of the amino acid sequence of SEQ ID NO: 158 and/or the TCRβ constant domain comprises or consists of the amino acid sequence of SEQ ID NO: 159.

The TCRα domain may have 1 or more (e.g., 1, 2, 3, 4, 5, 5, 6, 7, 8, 9, 10, or more) amino acid modifications (e.g., substitutions, additions, or deletions) from SEQ ID NO: 158; and/or the TCRβ domain has 1 or more (e.g., 1, 2, 3, 4, 5, 5, 6, 7, 8, 9, 10, or more) amino acid modifications (e.g., substitutions, additions, or deletions) from SEQ ID NO: 159. The TCRα domain may have no more than 10 (e.g., 10, 9, 8, 7, 6, 5, 4, 3, 2, or 1) amino acid modifications (e.g., substitutions, additions, or deletions) from SEQ ID NO: 158; and/or the TCRβ domain has no more than 10 (e.g., 10, 9, 8, 7, 6, 5, 4, 3, 2, or 1) amino acid modifications (e.g., substitutions, additions, or deletions) from SEQ ID NO: 159.

In some embodiments, the TCRα constant domain comprises or consists of the amino acid sequence of SEQ ID NO: 1, or a sequence having at least 75, 80, 85, 90, or 99% identity thereof and/or the TCRβ constant domain comprises or consists of the amino acid sequence of SEQ ID NO: 2, or a sequence having at least 75, 80, 85, 90, or 99% identity thereof, optionally wherein the TCRα constant domain comprises or consists of the amino acid sequence of SEQ ID NO: 1 and/or the TCRβ constant domain comprises or consists of the amino acid sequence of SEQ ID NO: 2.

The TCRα domain may have 1 or more (e.g., 1, 2, 3, 4, 5, 5, 6, 7, 8, 9, 10, or more) amino acid modifications (e.g., substitutions, additions, or deletions) from SEQ ID NO: 1; and/or the TCRβ domain has 1 or more (e.g., 1, 2, 3, 4, 5, 5, 6, 7, 8, 9, 10, or more) amino acid modifications (e.g., substitutions, additions, or deletions) from SEQ ID NO: 2. The TCRα domain may have no more than 10 (e.g., 10, 9, 8, 7, 6, 5, 4, 3, 2, or 1) amino acid modifications (e.g., substitutions, additions, or deletions) from SEQ ID NO: 1; and/or the TCRβ domain has no more than 10 (e.g., 10, 9, 8, 7, 6, 5, 4, 3, 2, or 1) amino acid modifications (e.g., substitutions, additions, or deletions) from SEQ ID NO: 2.

The TCRα domain may comprise or consist of at least 5, 10, 20, 30, 40, 50, 60, 70, 80, 90, or 100 contiguous amino acids of SEQ ID NO: 158. In some embodiments, the TCRα domain comprises or consists of at least 30, 40, 50, 60, 70, 80, 90, or 100 contiguous amino acids of SEQ ID NO: 158. The TCRα domain may comprise or consist of at least 5, 10, 20, 30, 40, 50, 60, 70, or 80 contiguous amino acids of SEQ ID NO: 1. In some embodiments, the TCRα domain comprises or consists of at least 30, 40, 50, 60, 70, or 80 contiguous amino acids of SEQ ID NO: 1.

The TCRα domain may have 1 or more (e.g., 1, 2, 3, 4, 5, 5, 6, 7, 8, 9, 10, or more) amino acid modifications (e.g., substitutions, additions, or deletions) from SEQ ID NO: 1. The TCRα domain may have no more than 10 (e.g., 10, 9, 8, 7, 6, 5, 4, 3, 2, or 1) amino acid modifications (e.g., substitutions, additions, or deletions) from SEQ ID NO: 1. The TCRβ domain comprises or consists of at least 5, 10, 20, 30, 40, 50, 60, 70, 80, 90, 100, 110, 120, 130, 140, 150, 160, or 170 contiguous amino acids of SEQ ID NO: 159. In some embodiments, the TCRβ domain comprises or consists of at least 30, 40, 50, 60, 70, 80, 90, 100, 110, 120, 130, 140, 150, 160, or 170 contiguous amino acids of SEQ ID NO: 159.

The TCRβ domain may comprise or consist of at least 5, 10, 20, 30, 40, 50, 60, 70, 80, 90, 100, 110, 120, or 130 contiguous amino acids of SEQ ID NO: 2. In some embodiments, the TCRβ domain comprises or consists of at least 30, 40, 50, 60, 70, 80, 90, 100, 110, 120, or 130 contiguous amino acids of SEQ ID NO: 2.

The TCRβ may have 1 or more (e.g., 1, 2, 3, 4, 5, 5, 6, 7, 8, 9, 10, or more) amino acid modifications (e.g., substitutions, additions, or deletions) from SEQ ID NO: 2. The TCRβ domain may have no more than 10 (e.g., 10, 9, 8, 7, 6, 5, 4, 3, 2, or 1) amino acid modifications (e.g., substitutions, additions, or deletions) from SEQ ID NO: 2.

The TCRα constant domain may comprise a functional fragment of the amino acid sequence of SEQ ID NO: 158 (e.g., a fragment capable of forming a stable association with a TCRβ constant domain, e.g., the TCRα constant domain comprises amino acids 1-140, 1-130, 1-120, 1-110, 1-100, 1-93, 1-90, 1-85, 1-80, 1-70, 10-100, 10-90, or 10-70 of SEQ ID NO: 158 (or a sequence with no more than 5 (e.g., 5, 4, 3, 2, or 1) amino acid modifications from amino acids 1-140, 1-130, 1-120, 1-110, 1-100, 1-93, 1-90, 1-85, 1-80, 1-70, 10-100, 10-90, or 10-70 of SEQ ID NO: 158)); and/or the TCRβ constant domain comprises a functional fragment of the amino acid sequence of SEQ ID NO: 159 (e.g., a fragment capable of forming a stable association with a TCRβ constant domain, e.g., the TCRβ constant domain comprises amino acids 1-170, 1-160, 1-150, 1-140, 1-130, 1-120, 1-110, 10-150, 10-140, 10-130, or 10-120 of SEQ ID NO: 159 (or a sequence with no more than 10 (e.g., 10, 9, 8, 7, 6, 5, 4, 3, 2, or 1) amino acid modifications from amino acids 1-170, 1-160, 1-150, 1-140, 1-130, 1-120, 1-110, 10-150, 10-140, 10-130, or 10-120 of SEQ ID NO: 159)).

In some embodiments, the TCRα constant domain or fragment thereof comprises amino acids 1-85 or 1-93 of SEQ ID NO: 158 (or a sequence with no more than 5 (e.g., 5, 4, 3, 2, or 1) amino acid modifications from amino acids 1-85 or 1-93 of SEQ ID NO: 158); and/or the TCRβ constant domain or fragment thereof comprises amino acids 1-130 of SEQ ID NO: 159 (or a sequence with no more than 10 (e.g., 10, 9, 8, 7, 6, 5, 4, 3, 2, or 1) amino acid modifications from amino acids 1-130 of SEQ ID NO: 159).

The TCRα constant domain may comprise a cysteine amino acid substitution relative to a naturally-existing TCRα constant domain (e.g., SEQ ID NO: 158) (e.g., the TCRα constant domain comprises a T49C substitution, numbered according to SEQ ID NO: 158) and/or the TCRβ constant domain comprises a cysteine amino acid substitution relative to a naturally-existing TCRβ constant domain (e.g., SEQ ID NO: 159) (e.g., the TCRβ constant domain comprises a S57C, numbered according to SEQ ID NO: 159). In some embodiments the TCRα constant domain or fragment thereof comprises a T49C substitution, numbered according to SEQ ID NO: 158; and/or the TCRβ constant domain or fragment thereof comprises a S57C substitution, numbered according to SEQ ID NO: 159.

The multispecific molecule comprises at least two non-contiguous polypeptide chains. In some embodiments, the first ABM comprises a first antibody molecule (e.g., a first antibody molecule comprising a first heavy and first light chain), and the second ABM comprises a second antibody molecule (e.g., a second antibody molecule comprising a second heavy and second light chain).

The heterodimerization domain may promote correct pairing of the first and second heavy chains, e.g., as measured by a method described herein (e.g., as measure by mass spectrometry), e.g., as measured by a method described in Example 3, e.g., the first heavy chain is more likely (e.g., 10, 20, 30, or 40-fold more likely) to form a heterodimer with the second heavy chain in the presence of the heterodimerization domain, than in the absence of the heterodimerization. The first antibody molecule and the second antibody molecule may be, independently, a full antibody (e.g., an antibody that includes at least one, and preferably two, complete heavy chains, and at least one, and preferably two, complete light chains), or an antigen-binding fragment (e.g., a Fab, F(ab')2, Fv, a scFv, a single domain antibody, or a diabody (dAb)). In some embodiments, the first antibody molecule comprises a kappa light chain constant region, or a fragment thereof, and the second antibody molecule comprises a lambda light chain constant region, or a fragment thereof. In some embodiments, the first antibody molecule comprises a lambda light chain constant region, or a fragment thereof, and the second antibody molecule comprises a kappa light chain constant region, or a fragment thereof. The first antibody molecule and the second antibody molecule may have a common light chain variable region.

The first or second ABM may comprise a tumor-targeting moiety. In some embodiments, the first or second ABM comprises an immune cell engager, or a binding moiety to a cytokine. The first ABM may comprise a first tumor-targeting moiety, and the second ABM may comprise a second tumor-targeting moiety. The first ABM may comprise a first immune cell engager, and the second ABM may comprise a second immune cell engager. The first ABM may comprise a tumor-targeting moiety, and the second ABM may comprise an immune cell engager. The first ABM may comprise an immune cell engager, and the second ABM may comprise a tumor-targeting moiety.

In one embodiment, provided herein is a multispecific molecule comprising:
(a) a first polypeptide chain having the following configuration from N-terminus to C-terminus: a first portion of a first antigen binding moiety (ABM) (e.g., wherein the first ABM comprises a VH-CH1 of a first Fab molecule, that binds to an antigen, e.g., a cancer antigen, connected, optionally via a linker to, a first subunit of a heterodimerization domain (e.g., an immunoglobulin CH2 connected, optionally via a linker to, a TCRα constant domain);
(b) a second polypeptide chain having the following configuration from N-terminus to C-terminus: a first portion of a second ABM (e.g., wherein the second ABM comprises a VH-CH1 of a second Fab molecule, that binds to an antigen, e.g., a cancer antigen, connected, optionally via a linker to, a second subunit of a heterodimerization domain (e.g., an immunoglobulin CH2 connected, optionally via a linker to, a TCRβ constant domain);
(c) a third polypeptide having the following configuration from N-terminus to C-terminus: a second portion of the first ABM (e.g., a VL-CL of the first Fab, where the VL is of kappa subtype and binds to an antigen, e.g., a cancer antigen (e.g., the same cancer antigen bound by the VH-CH1 of the first Fab molecule); and
(d) a fourth polypeptide having the following configuration from N-terminus to C-terminus: a second portion of the second antigen domain (e.g. a VL-CL of the second Fab, where the VL is of lambda subtype and binds to an antigen, e.g., a cancer antigen, (e.g., the same cancer antigen bound by the VH-CH1 of the second Fab molecule).

A multispecific molecule of the invention may comprise:
(a) a first polypeptide chain having the following configuration from N-terminus to C-terminus: a first portion of a first antigen binding moiety (ABM) (e.g., wherein the first ABM comprises a VH-CH1 of a first Fab molecule, that binds to an antigen, e.g., a cancer antigen, connected, optionally via a linker to, a first subunit of a heterodimerization domain (e.g., an immunoglobulin CH2 connected, optionally via a linker to, a TCRα constant domain);
(b) a second polypeptide chain having the following configuration from N-terminus to C-terminus: a first portion of a second ABM (e.g., wherein the second ABM comprises a VH-CH1 of a second Fab molecule, that binds to an antigen, e.g., a cancer antigen, connected, optionally via a linker to, a second subunit of a heterodimerization domain (e.g., an immunoglobulin CH2 connected, optionally via a linker to, a TCRβ constant domain);
(c) a third polypeptide having the following configuration from N-terminus to C-terminus: a second portion of the first ABM (e.g., a VL-CL of the first Fab, where the VL is of lambda subtype and binds to an antigen, e.g., a cancer antigen (e.g., the same cancer antigen bound by the VH-CH1 of the first Fab molecule); and
(d) a fourth polypeptide having the following configuration from N-terminus to C-terminus: a second portion of the second antigen domain (e.g. a VL-CL of the second Fab, where the VL is of kappa subtype and binds to an antigen, e.g., a cancer antigen, (e.g., the same cancer antigen bound by the VH-CH1 of the second Fab molecule).

A multispecific molecule of the invention may comprise:
(a) a first polypeptide chain having the following configuration from N-terminus to C-terminus: a first portion of a first antigen binding moiety (ABM) (e.g., wherein the first ABM comprises a VH-CH1 of a first Fab molecule, that binds to an antigen, e.g., a cancer antigen, connected, optionally via a linker to, a first subunit of a heterodimerization domain (e.g., a TCRα variable domain connected a TCRα constant domain);
(b) a second polypeptide chain having the following configuration from N-terminus to C-terminus: a first portion of a second ABM (e.g., wherein the second ABM comprises a VH-CH1 of a second Fab molecule, that binds to an antigen, e.g., a cancer antigen, connected, optionally via a linker to, a second subunit of a heterodimerization domain (e.g., TCRβ variable domain connected to a TCRβ constant domain);
(c) a third polypeptide having the following configuration from N-terminus to C-terminus: a second portion of the first ABM (e.g., a VL-CL of the first Fab, where the VL is of kappa subtype and binds to an antigen, e.g., a cancer antigen (e.g., the same cancer antigen bound by the VH-CH1 of the first Fab molecule); and
(d) a fourth polypeptide having the following configuration from N-terminus to C-terminus: a second portion of the second antigen domain (e.g. a VL-CL of the second Fab, where the VL is of lambda subtype and binds to an antigen, e.g., a cancer antigen, (e.g., the same cancer antigen bound by the VH-CH1 of the second Fab molecule).

A multispecific molecule of the invention may comprise:
(a) a first polypeptide chain having the following configuration from N-terminus to C-terminus: a first portion of a first antigen binding moiety (ABM) (e.g., wherein the first ABM comprises a VH-CH1 of a first Fab molecule, that binds to an antigen, e.g., a cancer antigen, connected, optionally via a linker to, a first subunit of a heterodimerization domain (e.g., a TCRα variable domain connected a TCRα constant domain);
(b) a second polypeptide chain having the following configuration from N-terminus to C-terminus: a first portion of a second ABM (e.g., wherein the second ABM comprises a VH-CH1 of a second Fab molecule, that binds to an antigen, e.g., a cancer antigen, connected, optionally via a linker to, a second subunit of a heterodimerization domain (e.g., TCRβ variable domain connected to a TCRβ constant domain);
(c) a third polypeptide having the following configuration from N-terminus to C-terminus: a second portion of the first ABM (e.g., a VL-CL of the first Fab, where the VL is of lambda subtype and binds to an antigen, e.g., a cancer antigen (e.g., the same cancer antigen bound by the VH-CH1 of the first Fab molecule); and
(d) a fourth polypeptide having the following configuration from N-terminus to C-terminus: a second portion of the second antigen domain (e.g. a VL-CL of the second Fab, where the VL is of kappa subtype and binds to an antigen, e.g., a cancer antigen, (e.g., the same cancer antigen bound by the VH-CH1 of the second Fab molecule).

A multispecific molecule of the invention may comprise:
(a) a first polypeptide comprising, from N-terminus to C-terminus, a first VH, a first CH1, a first CH2, and a TCRα constant domain,
(b) a second polypeptide comprising, from N-terminus to C-terminus, a second VH, a second CH1, a second CH2, and a TCRβ constant domain,
(c) a third polypeptide comprising, from N-terminus to C-terminus, a first VL (e.g., a VL of kappa subtype), and a kappa CL, and
(d) a fourth polypeptide comprising, from N-terminus to C-terminus, a second VL (e.g., a VL of lambda subtype), and a lambda CL, wherein:
   (i) the first and the third polypeptides form a first antigen binding moiety (ABM) that binds a first antigen,
   (ii) the second and the fourth polypeptides form a second ABM that binds a second antigen, and
   (iii) the first and the second polypeptides form a heterodimer, optionally wherein:
      the TCRα constant domain comprises the amino acid sequence of SEQ ID NO: 1 (or a sequence having at least 75, 80, 85, 90, or 99% identity thereof), and/or the TCRβ constant domain comprises the amino acid sequence of SEQ ID NO: 2 (or a sequence having at least 75, 80, 85, 90, or 99% identity thereof).

A multispecific molecule of the invention may comprise:
(a) a first polypeptide comprising, from N-terminus to C-terminus, a first VH, a first CH1, a first CH2, and a TCRα constant domain,
(b) a second polypeptide comprising, from N-terminus to C-terminus, a second VH, a second CH1, a second CH2, and a TCRβ constant domain,
(c) a third polypeptide comprising, from N-terminus to C-terminus, a first VL (e.g., a VL of lambda subtype), and a lambda CL, and
(d) a fourth polypeptide comprising, from N-terminus to C-terminus, a second VL (e.g., a VL of kappa subtype), and a kappa CL, wherein:
   (i) the first and the third polypeptides form a first antigen binding moiety (ABM) that binds a first antigen,
   (ii) the second and the fourth polypeptides form a second ABM that binds a second antigen, and
   (iii) the first and the second polypeptides form a heterodimer, optionally wherein:
      the TCRα constant domain comprises the amino acid sequence of SEQ ID NO: 1 (or a sequence having at least 75, 80, 85, 90, or 99% identity thereof), and/or the TCRβ constant domain comprises the amino acid sequence of SEQ ID NO: 2 (or a sequence having at least 75, 80, 85, 90, or 99% identity thereof).

A multispecific molecule of the invention may comprise a multispecific molecule disclosed in WO2018/057955.

A multispecific molecule of the invention may comprise a multispecific molecule disclosed in the subsection titled "Lambda/Kappa Formats" provided herein.

The multispecific molecule may comprise:
(i) an antigen binding moiety (ABM) comprising:
   a first heavy chain comprising a first heavy chain variable region and a first heavy chain constant region, and
   a lambda light chain comprising a lambda variable region and a lambda constant region, and
(ii) an ABM comprising:
   a second heavy chain comprising a second heavy chain variable region and a second heavy chain constant region, and
   a kappa light chain comprising a kappa variable region and a kappa constant region,

In a multispecific molecule of the invention, (i) the first heavy chain variable region may have at least 75, 80, 85, 90, 95, 98, or 100% sequence identity with a first heavy chain germline sequence selected from SEQ ID Nos: 187, 193, 194, 196, and 205, the lambda variable region may have at least 75, 80, 85, 90, 95, 98, or 100% sequence identity with a lambda light chain germline sequence selected from SEQ ID Nos: 209, 210, and 211, the second heavy chain variable region may have at least 75, 80, 85, 90, 95, 98, or 100% sequence identity with a second heavy chain germline sequence selected from SEQ ID Nos: 191, 192, 193, and 213, and/or (iv) the kappa variable region may have at least 75, 80, 85, 90, 95, 98, or 100% sequence identity with a kappa light chain germline sequence selected from SEQ ID Nos: 200, 201, 204, and 205; and/or b) the first heavy chain germline sequence, the lambda light chain germline sequence, the second heavy chain germline sequence, and the kappa light chain germline sequence may be selected from SEQ ID NO: 196, 211, 191, and 200; SEQ ID Nos: 194, 210, 213, and 201; SEQ ID Nos: 193, 209, 192, and 205; SEQ ID Nos: 205, 211, 192, and 205; SEQID Nos: 187, 211, 192 and 205; SEQ ID Nos: 205, 211, 193, and 204; SEQ ID Nos: 194, 210, 193, and 204; and SEQ ID Nos: 205, 211, 192, and 205.

In a multispecific molecule of the invention, (i) the first heavy chain constant region may not comprise a mutation that promotes the preferential pairing of the first heavy chain and the lambda light chain (e.g., the first heavy chain constant region is a naturally existing heavy chain constant region), or the lambda constant region may not comprise a mutation that promotes the preferential pairing of the first heavy chain and the lambda light chain (e.g., the lambda constant region is a naturally existing lambda constant region), and (ii) the second heavy chain constant region may not comprise a mutation that promotes the preferential pairing of the second heavy chain and the kappa light chain (e.g., the second heavy chain constant region is a naturally existing heavy chain constant region), or the kappa constant region may not comprise a mutation that promotes the preferential pairing of the second heavy chain and the kappa light chain (e.g., the kappa constant region is a naturally existing kappa constant region).

In a multispecific molecule of the invention, (i) the first heavy chain may preferentially bind to the lambda light chain over the kappa light chain, (ii) the lambda light chain may preferentially bind to the first heavy chain over the second heavy chain, (iii) the second heavy chain may preferentially bins to the kappa light chain over the lambda light chain, and/or (iv) the kappa light chain may preferentially bind to the second heavy chain over the first heavy chain.

Disclosed herein is a multispecific molecule (e.g., an isolated multispecific molecule), comprising (i) a first antigen binding moiety (ABM) (e.g., an antibody molecule); (ii) a second ABM, wherein the first ABM and the second ABM do not bind the same antigen; and (iii) a constant region (e.g., a heavy chain constant region of IgG1, IgG2, and IgG4) comprising a first and a second polypeptide chain, wherein (a) the first polypeptide chain comprises a first non-immunoglobulin domain, and (b) the second polypeptide chain comprises a second non-immunoglobulin domain, wherein the first and the second non-immunoglobulin domains are capable of forming a stable association, and wherein neither the first nor second polypeptide chain contains a CH3 domain (e.g., any portion of a CH3 domain, any portion of a CH3 domain capable of stable self-association).

The disclosure provides, multispecific molecules (e.g., an isolated multispecific molecule), comprising (i) a first antigen binding moiety (ABM) (e.g., an antibody molecule); (ii) a second ABM, wherein the first and second ABMs do not bind the same antigen, and (iii) a heterodimerization domain comprising a first and a second polypeptide chain, wherein the first polypeptide chain comprises a TCRα constant domain (or a functional fragment thereof, e.g., a fragment capable of forming stable association with a TCRβ constant domain), and the first polypeptide chain comprises a TCRβ constant domain (or a functional fragment thereof, e.g., a fragment capable of forming stable association with a TCRα constant domain).

The first polypeptide chain comprises an immunoglobulin CH2 domain (e.g., an IgG1, IgG2, or IgG4 CH2 domain) connected to (optionally via a linker) the TCRα constant domain, and the second polypeptide chain comprises an immunoglobulin CH2 domain (e.g., an IgG1, IgG2, or IgG4 CH2 domain) connected to (optionally via a linker) the TCRβ constant domain. In some embodiments, neither the first nor the second polypeptide chain of the heterodimerization domain contains an immunoglobulin CH3 domain (e.g., any portion of a CH3 domain). In some embodiments, neither the first nor the second polypeptide chain of the heterodimerization domain contains any portion of an immunoglobulin CH3 domain capable of stable self-association (i.e., the first polypeptide chain does not contain an any portion of a CH3 domain capable of stable association with the CH3 domain of the second polypeptide).

The first polypeptide chain may comprise a TCRα variable domain connected to the TCRα constant domain, and the second polypeptide chain comprises a TCRβ variable domain connected to the TCRβ constant domain. The first or the second polypeptide chain of the heterodimerization domain may not contain more than 50, 25, 10, or 5 amino acids of an immunoglobulin CH2 and/or no more than 50, 25, 10, or 5 amino acids of an immunoglobulin CH3. The first or the second polypeptide chain of the heterodimerization domain may not contain an immunoglobulin CH2 and/or CH3 domain (e.g., any portion of a CH2 and/or CH3 domain).

The TCRα constant domain may comprise or consist of the amino acid sequence of SEQ ID NO: 158 and/or the TCRβ constant domain comprises or consists of the amino acid sequence of SEQ ID NO: 159. The TCRα domain may have 1 or more (e.g., 1, 2, 3, 4, 5, 5, 6, 7, 8, 9, 10, or more) amino acid substitutions; and/or the TCRβ domain may have 1 or more (e.g., 1, 2, 3, 4, 5, 5, 6, 7, 8, 9, 10, or more) amino acid substitutions. The TCRα domain may have no more than 10 (e.g., 10, 9, 8, 7, 6, 5, 4, 3, 2, or 1) amino acid substitutions; and/or the TCRβ domain may have no more than 10 (e.g., 10, 9, 8, 7, 6, 5, 4, 3, 2, or 1) amino acid substitutions.

The TCRα constant domain may comprise or consist of the amino acid sequence of SEQ ID NO: 1 and/or the TCRβ constant domain comprises or consists of the amino acid sequence of SEQ ID NO: 2. The TCRα domain may have 1 or more (e.g., 1, 2, 3, 4, 5, 5, 6, 7, 8, 9, 10, or more) amino acid substitutions; and/or the TCRβ domain may have 1 or more (e.g., 1, 2, 3, 4, 5, 5, 6, 7, 8, 9, 10, or more) amino acid substitutions. The TCRα domain may have no more than 10 (e.g., 10, 9, 8, 7, 6, 5, 4, 3, 2, or 1) amino acid substitutions; and/or the TCRβ domain may have no more than 10 (e.g., 10, 9, 8, 7, 6, 5, 4, 3, 2, or 1) amino acid substitutions.

The TCRα domain may comprise or consist of at least 20, 30, 40, 50, 60, 70, 80, 90, or 100 contiguous amino acids of SEQ ID NO: 158. In some embodiments, the TCRα domain comprises or consists of at least 20, 30, 40, 50, 60, 70, or 80 contiguous amino acids of SEQ ID NO: 1. The TCRα may have 1 or more (e.g., 1, 2, 3, 4, 5, 5, 6, 7, 8, 9, 10, or more) amino acid substitutions. The TCRα domain may have no more than 10 (e.g., 10, 9, 8, 7, 6, 5, 4, 3, 2, or 1) amino acid substitutions.

The TCRβ domain may comprise or consist of at least 20, 30, 40, 50, 60, 70, 80, 90, 100, 110, 120, 130, 140, 150, 160, or 170 contiguous amino acids of SEQ ID NO: 159. The TCRβ domain may comprise or consist of at least 20, 30, 40, 50, 60, 70, 80, 90, 100, 110, 120, or 130 contiguous amino acids of SEQ ID NO: 2. The TCRβ may have 1 or more (e.g., 1, 2, 3, 4, 5, 5, 6, 7, 8, 9, 10, or more) amino acid substitutions.

The TCRα constant domain may comprise a functional fragment of the amino acid sequence of SEQ ID NO: 158 (e.g., a fragment capable of forming a stable association with a TCRβ constant domain, (e.g., the TCRα constant domain comprises amino acids 1-140 (e.g., 1-130, 1-120, 1-110, 1-100, 1-90, 1-80 (e.g., 1-85), 1-70, 10-100, 10-90, 10-70) of SEQ ID NO: 158); and/or the TCRβ constant domain may comprise a functional fragment of the amino acid sequence of SEQ ID NO: 159 (e.g., a fragment capable of forming a stable association with a TCRβ constant domain (e.g., the TCRβ constant domain comprises amino acids 1-170 (e.g., 1-170, 1-160, 1-150, 1-140, 1-130, 1-120, 1-110, 10-150, 10-40, 10-130, 10-120) of SEQ ID NO: 159)).

The TCRα constant domain may comprise amino acids 1-85of SEQ ID NO: 158; and/or the TCRβ constant domain may comprise amino acids 1-130 of SEQ ID NO: 159.

The TCRα constant domain may comprise a cysteine amino acid substitution relative to SEQ ID NO: 1 or SEQ ID NO: 158 and and/or the TCRβ constant domain may comprise a cysteine amino acid substitution relative to SEQ ID NO: 2 or SEQ ID NO: 159.

The first ABM may be a first antibody molecule comprising a first heavy and first light chain, and the second ABM may be a second antibody molecule comprising a second heavy and second light chain. The heterodimerization domain may promote correct pairing of the first and second heavy chains (e.g., as measure by mass spectrometry).

The TCRα and TCRβ variable domains may bind HSA. The TCRα and TCRβ variable domains may bind protein A or protein G. The TCRα and TCRβ variable domains may bind a tumor antigen (e.g., as described herein).

The first ABM may be a tumor targeting moiety that binds to a cancer antigen; and the second ABM may be an immune cell engager. The immune cell engager may be a natural killer (NK) cell engager, a B cell engager, a dendritic cell engager, or a macrophage cell engager. The multispecific molecule further may comprise a first cytokine molecule. The multispecific molecule may further comprise a first stromal modifying molecule.

The tumor-targeting moiety may comprise an antibody molecule, a receptor molecule (e.g., a receptor, a receptor fragment or functional variant thereof), or a ligand molecule (e.g., a ligand, a ligand fragment or functional variant thereof), or a combination thereof, that binds to the cancer antigen. The tumor-targeting moiety may bind to a cancer antigen present on a hematological cancer, a solid cancer, a metastatic cancer, or a combination thereof. The cancer antigen may be a tumor antigen, a stromal antigen, or a hematological antigen.

The tumor antigen may be present on a solid tumor (e.g., is a solid tumor antigen). The tumor, e.g., solid tumor, may be chosen from one or more of pancreatic (e.g., pancreatic adenocarcinoma), breast, colorectal, lung (e.g., small or non-small cell lung cancer), skin, ovarian, or liver cancer. In some embodiments, the tumor, e.g., solid tumor, antigen is chosen from: PDL1, mesothelin, CD47, gangloside 2 (GD2), prostate stem cell antigen (PSCA), prostate specific membrane antigen (PMSA), prostate-specific antigen (PSA), carcinoembryonic antigen (CEA), Ron Kinase, c-Met, Immature laminin receptor, TAG-72, BING-4, Calciumactivated chloride channel 2, Cyclin-B1, 9D7, Ep-CAM, EphA3, Her2/neu, Telomerase, SAP-1, Survivin, NY-ESO-1/LAGE-1, FRAME, SSX-2, Melan-A/MART-1, Gp100/pmel17, Tyrosinase, TRP-1/-2, MC1R, β-catenin, BRCA1/2, CDK4, CML66, Fibronectin, p53, Ras, TGF-B receptor, AFP, ETA, MAGE, MUC-1, CA-125, BAGE, GAGE, NY-ESO-1, β-catenin, CDK4, CDC27, CD47, α actinin-4, TRP1/gp75, TRP2, gp100, Melan-A/MART1, gangliosides, WT1, EphA3, Epidermal growth factor receptor (EGFR), CD20, MART-2, MART-1, MUC1, MUC2, MUM1, MUM2, MUM3, NA88-1, NPM, OA1, OGT, RCC, RUI1, RUI2, SAGE, TRG, TRP1, TSTA, Folate receptor alpha, L1-CAM, CAIX, EGFRvIII, gpA33, GD3, GM2, VEGFR, Intergrins, Carbohydrates (Le), IGF1R, EPHA3, TRAILR1, TRAILR2, or RANKL, optionally, wherein the Integrins are Integrin alphaVbeta3, or Integrin alpha5Beta1. The solid tumor antigen may be chosen from: PDL1, Mesothelin, GD2, PMSA, CEA, Ron Kinase, or c-Met.

The multispecific molecule may comprise two or three antibody molecules to two or three cancer antigens chosen from mesothelin, PDL1, HER3, IGF1R, FAP, CD123 or CD47.

In some embodiments, the stromal antigen is chosen from fibroblast activating protease (FAP), TGF-beta, hyaluronic acid, collagen, e.g., collagen IV, tenascin C, or tenascin W. In some embodiments, the hematological antigen is chosen from CD19, CD33, CD47, CD123, CD20, CD99, CD30, BCMA, CD38, CD22, SLAMF7, or NY-ESO1.

The immune cell engager may comprise an NK cell engager that mediates binding to, and/or activation of, an NK cell. In some embodiments, the NK cell engager is chosen from an antibody molecule, e.g., an antigen binding domain, or ligand that binds to (e.g., activates): NKp30, NKp40, NKp44, NKp46, NKG2D, DNAM1, DAP10, CD16 (e.g., CD16a, CD16b, or both), CRTAM, CD27, PSGL1, CD96, CD100 (SEMA4D), NKp80, CD244 (also known as SLAMF4 or 2B4), SLAMF6, SLAMF7, KIR2DS2, KIR2DS4, KIR3DS1, KIR2DS3, KIR2DS5, KIR2DS1, CD94, NKG2C, NKG2E, or CD160. The NK cell engager may be an antibody molecule, e.g., an antigen binding domain. The NK cell engager may be a ligand. The NK cell engager may be a ligand of NKp44, NKp46, DAP10, or CD16. The immune cell engager may mediate binding to, or activation of, one or more of a B cell, a macrophage, and/or a dendritic cell.

In some embodiments, the immune cell engager comprises a B cell, macrophage, and/or dendritic cell engager chosen from one or more of CD40 ligand (CD40L) or a CD70 ligand; an antibody molecule that binds to CD40 or CD70; an antibody molecule to OX40; an OX40 ligand (OX40L); an agonist of a Toll-like receptor (e.g., a TLR4, e.g., a constitutively active TLR4 (caTLR4) or a TLR9 agonist); a 41BB; a CD2 agonist; a CD47; or a STING agonist, or a combination thereof. In some embodiments, the B cell engager is a CD40L, an OX40L, or a CD70 ligand, or an antibody molecule that binds to OX40, CD40 or CD70. In some embodiments, the macrophage cell engager is a CD2 agonist; a CD40L; an OX40L; an antibody molecule that binds to OX40, CD40 or CD70; an agonist of a Toll-like receptor (TLR)(e.g., a TLR4, e.g., a constitutively active TLR4 (caTLR4) or a TLR9 agonist); CD47; or a STING agonist. In some embodiments, the dendritic cell engager is a CD2 agonist, an OX40 antibody, an OX40L, 41BB agonist, a Toll-like receptor agonist or a fragment thereof (e.g., a TLR4, e.g., a constitutively active TLR4 (caTLR4)), CD47 agonist, or a STING agonist. The STING agonist may comprise a cyclic dinucleotide, e.g., a cyclic di-GMP (cdGMP), a cyclic di-AMP (cdAMP), or a combination thereof, optionally with 2',5' or 3',5' phosphate linkages.

In some embodiments, the cytokine molecule is chosen from interleukin-2 (IL-2), interleukin-7 (IL-7), interleukin-12 (IL-12), interleukin-15 (IL-15), interleukin-18 (IL-18), interleukin-21 (IL-21), or interferon gamma, or a fragment or variant thereof, or a combination of any of the aforesaid cytokines. The cytokine molecule may be a monomer or a dimer. The cytokine molecule may further comprise a receptor dimerizing domain, e.g., an IL15Ralpha dimerizing domain. The cytokine molecule (e.g., IL-15) and the receptor dimerizing domain (e.g., an IL15Ralpha dimerizing domain) may not be covalently linked, e.g., are non-covalently associated.

The stromal modifying moiety may comprise an enzyme molecule that degrades a tumor stroma or extracellular matrix (ECM). The enzyme molecule may be chosen from a hyaluronidase molecule, a collagenase molecule, a chondroitinase molecule, a matrix metalloproteinase molecule (e.g., macrophage metalloelastase), or a variant (e.g., a fragment) of any of the aforesaid. The hyaluronidase molecule may be chosen from HYAL1, HYAL2, or PH-20/SPAM1, or a variant thereof (e.g., a truncated form thereof). The multispecific molecule may further comprise a third ABM(e.g., is a trispecific or trifunctional molecule).

The multispecific molecule may further comprise a fourth ABM (e.g., is a tetraspecific or tetrafunctional molecule).

The multispecific molecule may further comprise a second cytokine (the same or different than the first cytokine). The multispecific molecule may comprise (i) one tumor-targeting moiety; (ii) two immune cell engagers (e.g., same or different immune cell engagers); and (iii) one cytokine molecule. The multispecific molecule may comprise (i) two tumor-targeting moieties (e.g., same or different targeting moieties); (ii) one immune cell engager; and (iii) one cytokine molecule. The multispecific molecule may comprise (i) one tumor-targeting moiety; (ii) one immune cell engager; and (iii) two cytokine molecules (e.g., same or different cytokine molecules).

The multispecific molecule comprises at least two non-contiguous polypeptide chains.

The first and/or the second ABM may comprise an antibody molecule or functional fragment thereof. The first ABM antibody molecule and the second ABM antibody molecule may be, independently, a full antibody (e.g., an antibody that includes at least one, and preferably two, complete heavy chains, and at least one, and preferably two, complete light chains), or an antigen-binding fragment (e.g., a Fab, F(ab')2, Fv, a scFv, a single domain antibody, or a diabody (dAb)).

In some embodiments, the first ABM antibody molecule comprises a kappa light chain constant region, or a fragment thereof, and the second ABM antibody molecule comprises a lambda light chain constant region, or a fragment thereof (or vice versa).

The first ABM antibody molecule and the second ABM antibody molecule may have a common light chain variable region.

A multispecific antibody molecule of the invention may (e.g., an isolated multispecific antibody), comprise (i) a first antibody molecule; (ii) a second antibody molecule, wherein the first and second antibody molecules do not bind the same antigen, and an Fc domain consisting of two subunits, wherein each subunit comprises a CH2 and a CH3 domain, wherein (a) the CH3 domain of the first subunit is replaced (e.g., entirely replaced) with at least a portion of a TCRα constant domain (or a functional fragment thereof, e.g., a fragment capable of forming stable association with a TCRβ constant domain) and the CH3 domain of the second subunit is replaced with at least a portion of a TCRβ constant domain (or a functional fragment thereof, e.g., a fragment capable of forming stable association with a TCRα constant domain); or (b) the CH2 domain of the first subunit is replaced with a TCRα variable domain and the CH3 domain of the first subunit is replaced with at least a portion of a TCRα constant domain (or a functional fragment thereof, e.g., a fragment capable of forming stable association with a TCRβ constant domain); and the CH2 domain of the second subunit is replaced with a TCRβ variable domain and the CH3 domain of the first subunit is replaced with at least a portion of a TCRα constant domain (or a functional fragment thereof, e.g., a fragment capable of forming stable association with a TCRα constant domain).

A multispecific molecule of the invention may comprise
(a) a first polypeptide chain having the following configuration from N-terminus to C-terminus: a first portion of a first antigen binding moiety (ABM) (e.g., wherein the first ABM comprises a VH-CH1 of a first Fab molecule, that binds to, e.g., a cancer antigen, connected, optionally via a linker to, a first subunit of a heterodimerization domain (e.g., an immunoglobulin CH2 connected to a TCRα constant domain); (b) a second polypeptide chain having the following configuration from N-terminus to C-terminus: a first portion of a second ABM (e.g., wherein the second ABM comprises a VH-CH1 of a second Fab molecule, that binds to, e.g., a cancer antigen, connected, optionally via a linker to, a second subunit of a heterodimerization domain (e.g., an immunoglobulin CH2 connected to a TCRβ constant domain); (c) a third polypeptide having the following configuration from N-terminus to C-terminus: a second portion of the first ABM (e.g., a VL-CL of the first Fab, where the VL is of kappa subtype and binds to, e.g., a cancer antigen (e.g., the same cancer antigen bound by the VH-CH1 of the first Fab molecule); (d) a fourth polypeptide having the following configuration from N-terminus to C-terminus: a second portion of the second antigen domain (e.g. a VL-CL of the second Fab, where the VL is of lambda subtype and binds to, e.g., a cancer antigen, (e.g., the same cancer antigen bound by the VH-CH1 of the second Fab molecule).

A multispecific molecule of the invention may comprise (a) a first polypeptide chain having the following configuration from N-terminus to C-terminus: a first portion of a first antigen binding moiety (ABM) (e.g., wherein the first ABM comprises a VH-CH1 of a first Fab molecule, that binds to, e.g., a cancer antigen, connected, optionally via a linker to, a first subunit of a heterodimerization domain (e.g., a TCRα variable domain connected to a TCRα constant domain); (b) a second polypeptide chain having the following configuration from N-terminus to C-terminus: a first portion of a second ABM (e.g., wherein the second ABM comprises a VH-CH1 of a second Fab molecule, that binds to, e.g., a cancer antigen, connected, optionally via a linker to, a second subunit of a heterodimerization domain (e.g., TCRβ variable domain connected to a TCRβ constant domain); (c) a third polypeptide having the following configuration from N-terminus to C-terminus: a second portion of the first ABM (e.g., a VL-CL of the first Fab, where the VL is of kappa subtype and binds to, e.g., a cancer antigen (e.g., the same cancer antigen bound by the VH-CH1 of the first Fab molecule); (d) a fourth polypeptide having the following configuration from N-terminus to C-terminus: a second portion of the second antigen domain (e.g. a VL-CL of the second Fab, where the VL is of lambda subtype and binds to, e.g., a cancer antigen, (e.g., the same cancer antigen bound by the VH-CH1 of the second Fab molecule).

In an aspect, the invention provides isolated nucleic acid molecules encoding the multispecific molecule described herein.

In an aspect, the invention provides, isolated nucleic acid molecules, which comprise a nucleotide sequence encoding any of the multispecific molecules described herein, or a nucleotide sequence substantially homologous thereto (e.g., at least 95% to 99.9% identical thereto).

In an aspect, the invention provides, vectors, e.g., an expression vector, comprising one or more of the nucleic acid molecules described herein.

The invention provides, host cells comprising a nucleic acid molecule or a vector described herein.

The invention provides, pharmaceutical compositions comprising the multispecific molecule described herein and a pharmaceutically acceptable carrier, excipient, or stabilizer.

The invention provides, methods of making, e.g., producing, a multispecific molecule described herein, comprising culturing a host cell described herein, under suitable conditions, e.g., conditions suitable for gene expression and/or heterodimerization.

The invention provides, methods of making, e.g., producing, the multispecific molecule (e.g., multispecific antibody molecule) described herein, comprising (a) generating a first antibody (e.g., a human antibody) comprising (i) a first heavy chain comprising a CH2 domain connected (optionally via a linker) to a first non-immunoglobulin dimerization domain (e.g., a TCRα constant domain) and (ii) a first light chain (e.g., a kappa light chain); (b) generating a second antibody (e.g., a human antibody) comprising a second heavy chain comprising a CH2 domain connected (optionally via a linker) to a second non-immunoglobulin dimerization domain (e.g., a TCRβ constant domain) and (ii) a second light chain (e.g., a lambda light chain), wherein the first and the second non-immunoglobulin dimerization domains are not the same; (c) transfecting a cell (or cells) with a nucleic acid encoding the amino acid sequence of the first antibody and the second antibody; (d) culturing the cell (or cells) under suitable conditions, e.g., conditions suitable for gene expression; (e) purifying the antibody (e.g., using Protein A); (f) optionally determining the presence of the first and second heavy chain (e.g. via gel electrophoresis under reducing conditions); and (g) optionally determining the presence of correctly paired first and second heavy chains with the first and the second light chains, respectively (e.g., via mass spectrometry).

The invention provides, methods of manufacturing a multispecific molecule described herein, comprising purifying the multispecific molecule using a Protein A column.

The invention provides, methods of manufacturing a multispecific molecule described herein, comprising purifying the multispecific molecule using a Protein G column

In an aspect, the invention provides, a multispecific molecule for use in a method of treating a cancer, comprising administering to a subject in need thereof a multispecific molecule described herein, wherein the multispecific antibody is administered in an amount effective to treat the cancer.

The cancer may be a solid tumor cancer, or a metastatic lesion. The solid tumor cancer may be one or more of pancreatic (e.g., pancreatic adenocarcinoma), breast, colorectal, lung (e.g., small or non-small cell lung cancer), skin, ovarian, or liver cancer. The cancer may be a hematological cancer. The method may further comprise administering a second therapeutic treatment. The second therapeutic treatment may comprise a therapeutic agent (e.g., a chemotherapeutic agent, a biologic agent, hormonal therapy), radiation, or surgery. The therapeutic agent may be selected from: a chemotherapeutic agent, or a biologic agent.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In the case of conflict, the present specification, including definitions, will control. In addition, the materials, methods, and examples are illustrative only and are not intended to be limiting.

Other features and advantages of the invention will be apparent from the following detailed description and claims.

### BRIEF DESCRIPTION OF THE DRAWINGS

**FIGs. 1A** and **1B** show topology maps of 5HY9 and 4WW1 respectively. 5HY9 is the structure of a KiH Fc showing the domain linkage between CH2 and CH3, which is used to form the heterodimer. 4WW1 is the structure of TCR showing the linkage between the variable domain and constant domain, which is used to form the heterodimer.
**FIGs. 2A** and **2B** show the overall alignment of the knobs into holes CH3 domain with the TCR constant domain.
**FIGs. 3A** and **3B** are flattened topology views, showing the CH3 knob/TCRα constant domain (SEQ ID NOs. 169 and 168, respectively) and the CH3 hole/TCRβ constant domain (SEQ ID NOs. 171 and 170, respectively) topology alignments based on FIGs. 2A and 2B.

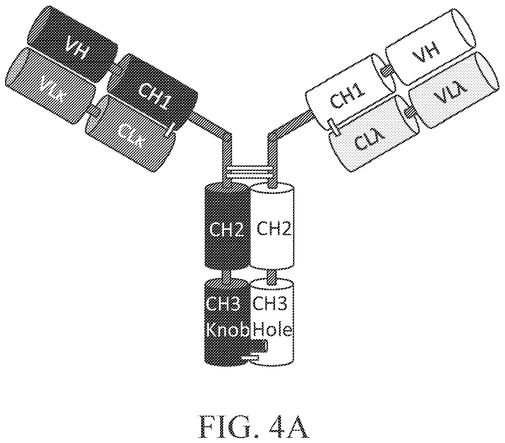

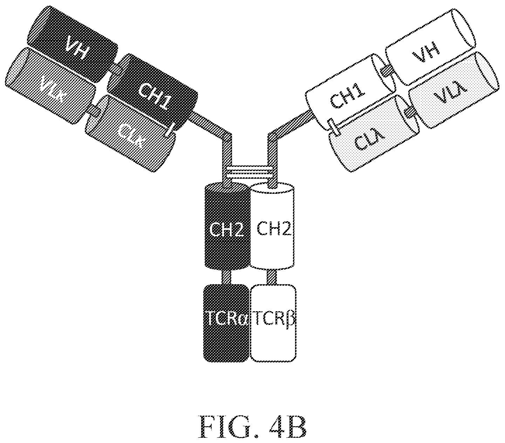

**FIGs. 4A** **and** **4B** depict schematic representations of multispecific molecules of the present disclosure. FIG. 4A shows a multispecific molecule comprising two heavy chains, one kappa light chain, and one lambda light chain. The two heavy chains comprise knob-into-hole mutations in the CH3 domains. Multispecific molecule 1 tested in Example 2 has the configuration shown in FIG. 4A. FIG. 4B shows a multispecific molecule where the CH3 domains of the two heavy chains are replaced by a TCRα constant domain and a TCRβ constant domain, respectively. Multispecific molecule 2 tested in Example 3 has the configuration shown in FIG. 4B.
**FIG. 5****.** Gel of reduced samples of multispecific molecule 1 following kappa/lambda select analysis. Lane 1 is the load, lane 2 is the flow-through from the KappaSelect column, lane 3 is the elution from the KappaSelect column, lane 4 is the flow-through from the LambdaFabSelect column, and lane 5 is the elution from the LambdaFabSelect column.
**FIG. 6****.** Gel of multispecific molecule 2.
**FIG. 7****.** Size exclusion chromatogram of multispecific molecule 2.
**FIG. 8****.** Gel of non-reduced samples of multispecific molecule 2 following kappa/lambda select analysis. Lane 1 is the load, lane 2 is the flow-through from the KappaSelect column, lane 3 is the elution from the KappaSelect column, lane 4 is the flow-through from the LambdaFabSelect column, and lane 5 is the elution from the LambdaFabSelect column.

### DETAILED DESCRIPTION OF THE INVENTION

The technical disclosure set out below may in some respects go beyond the scope of the claims. Elements of the disclosure which do not fall within the scope of the claims are provided for information.

Disclosed herein are multispecific molecules (also referred to herein as "multifunctional molecules") (e.g., bispecific molecules, e.g., bispecific antibodies) that include non-immunoglobulin dimerization domains (e.g., naturally occurring dimerization domains, e.g., TCR α and β constant domains). Without being bound by theory, the multispecific molecules disclosed herein are expected to provide stable correctly assembled multispecific molecules (e.g., bispecific antibodies) retaining a natural immunoglobulin like structure, comprising a non-immunoglobulin heterodimerization domain (e.g., TCR α and β constant domains). Accordingly, provided herein are, *inter alia,* multispecific molecules (e.g., multispecific antibody molecules) that include the aforesaid non-immunoglobulin dimerization domains, nucleic acids encoding the same, methods of producing the aforesaid molecules (e.g., in a single cell), and methods of treating a cancer using the aforesaid molecules.

### Similarity of Fe and TCR dimerization domains

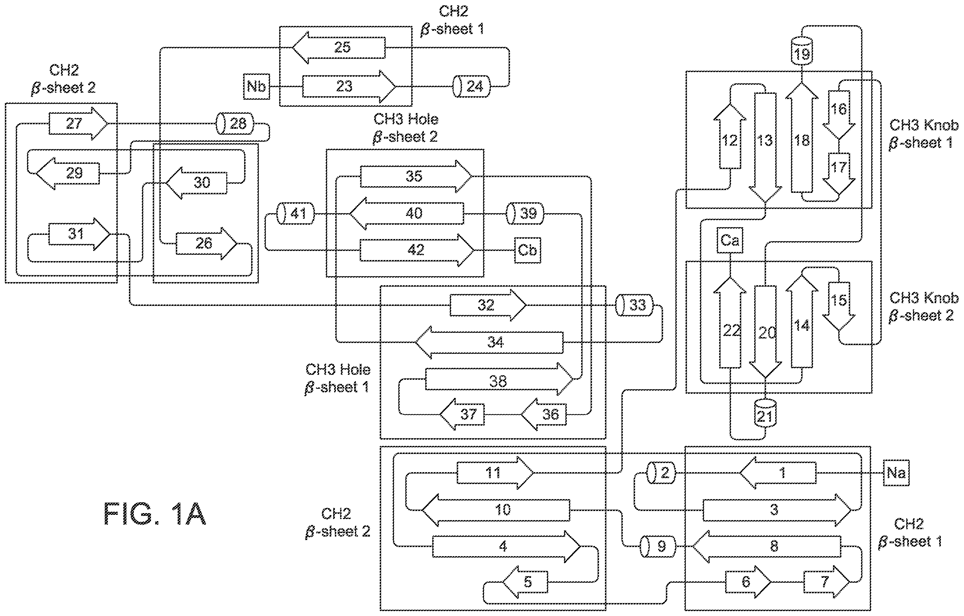

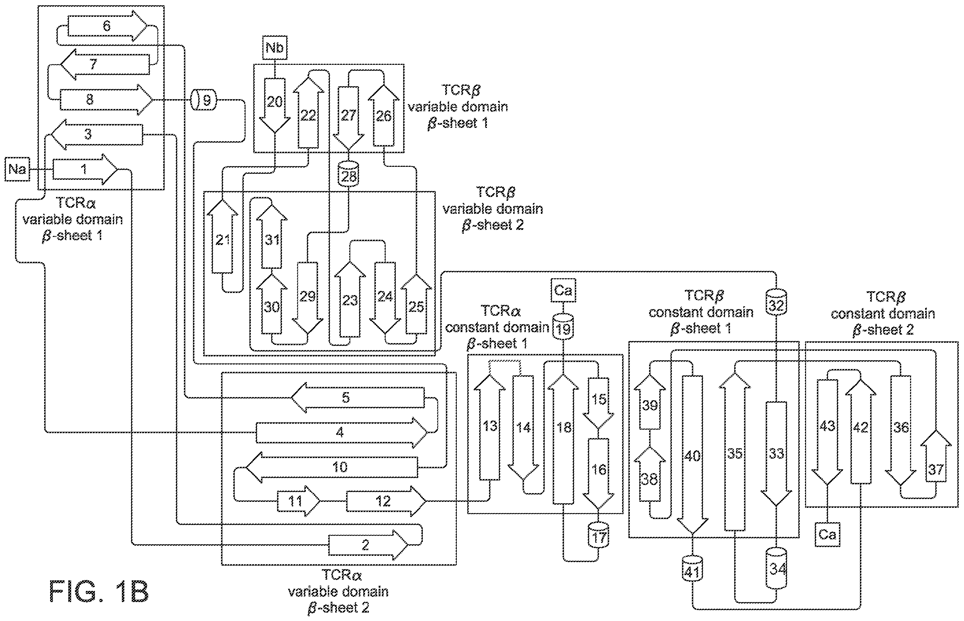

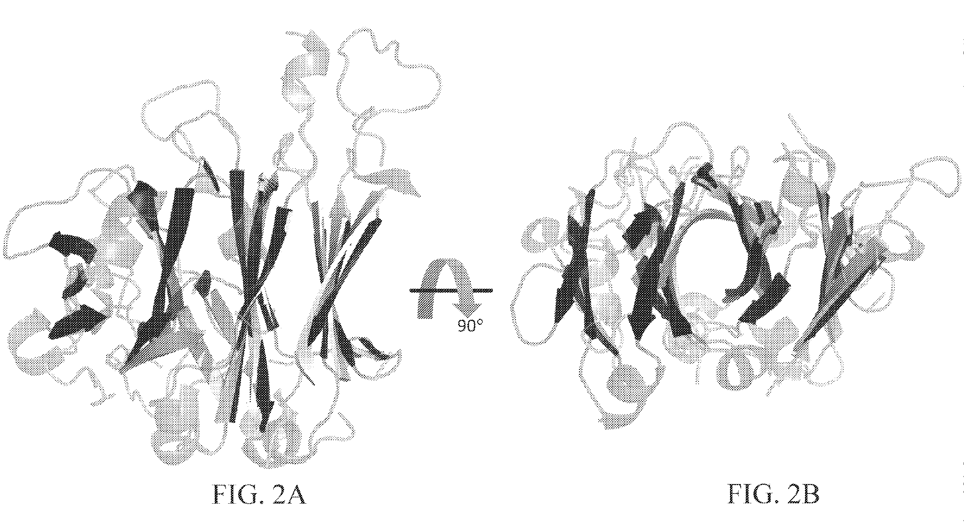

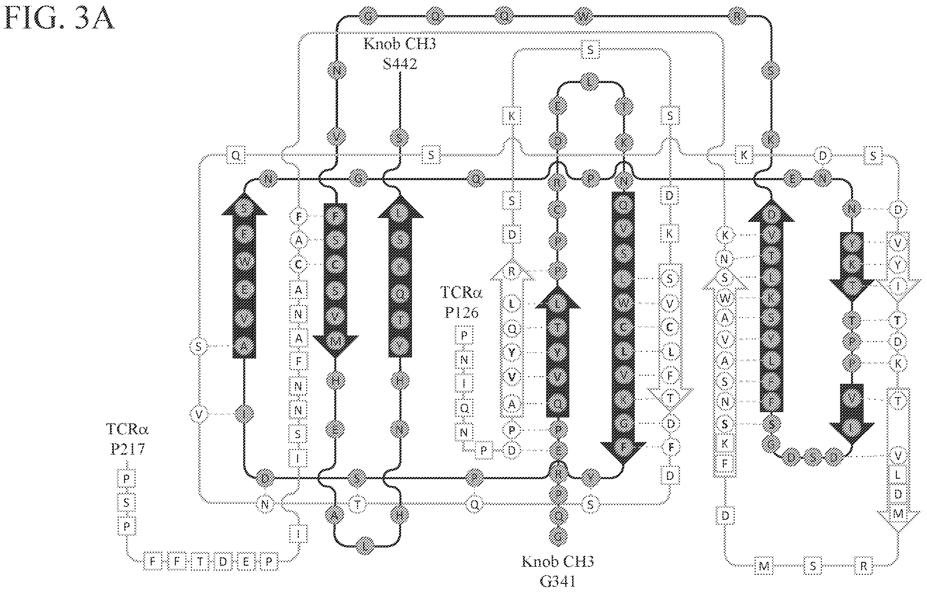

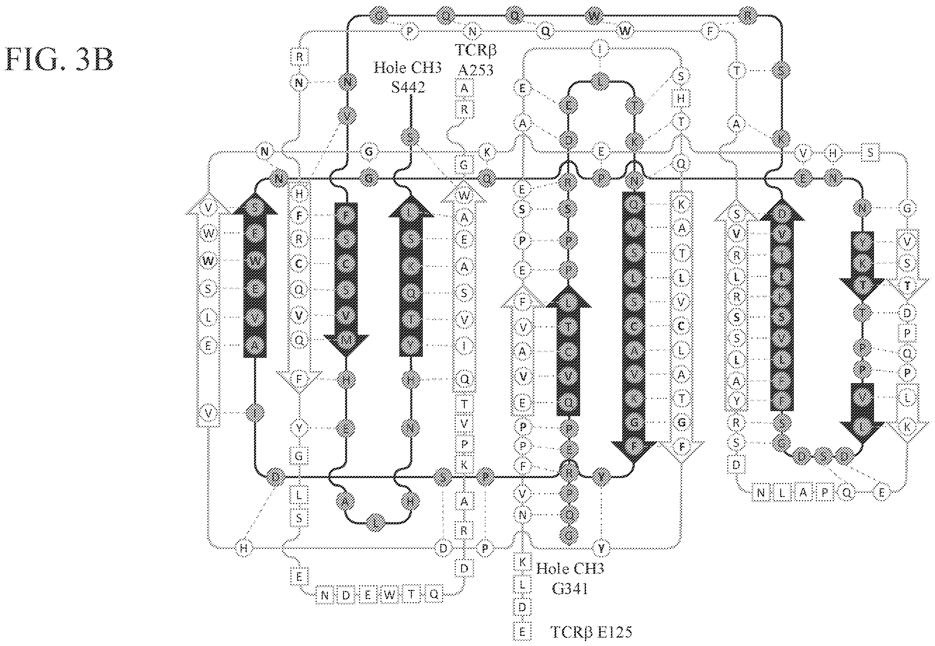

The overall structure of the α/β T-cell receptor (TCR) is similar to that of the IgG Fab region with an overall alignment of Cα carbons with a RMSD of 6 Å² vs. 13 Å² for the Fc region. Although the relationship between the CH2 and CH3 domains of the Fc region is dissimilar to the relationship between the TCR constant and variable domains, as elucidated herein the constant domain of the TCR interacts to form a heterodimer in a fashion comparable to that of the knobs into holes CH3 domains. Superposition of the CH3 domains from the structure of the knobs into holes Fc domain, RCSB code 5HY9, with the constant domains of the α/β TCR, RCSB code 4WW1, results in a RMSD of 2.6 Å² for all Cα carbons and 0.5 Å² for those in the β-strands forming the core of the IgG fold. **FIGs. 1A** and **1B** show topology maps (A. Stivala, M. Wybrow, A. Wirth, J. Whisstock and P. Stuckey 2011 Automatic generation of protein structure cartoons with Pro-origami Bioinformatics 27(23):3315-3316) of 5HY9 and 4WW1 respectively, while **FIGs. 2A** and **2B** show the overall alignment of the knobs into holes CH3 domains with the TCR constant domains. The fold for both CH3 domains in 5HY9 is classified by SCOP as b.1.1.2, C1 set domains or antibody constant domain like (Murzin A. G., Brenner S. E., Hubbard T., Chothia C. (1995). SCOP: a structural classification of proteins database for the investigation of sequences and structures. J. Mol. Biol. 247, 536-540.), with the domain defined by 7 β-strands forming 2 sheets which are connected via a disulfide. The TCRβ constant domain is also classified as b.1.1.2 while TCRα constant domain is classified as b.1.1.0, not a true immunoglobulin family due to one of the β-sheets missing. **FIGs. 3A** and **3B** show the CH3 knob/TCRα constant domain and the CH3 hole/TCRβ constant domain topology alignments based on FIGs. 2A and 2B. Without wishing to be bound by theory, IgG CH3 domain and TCR constant domain are similar in structure and relative dimer formation.
The constant domain of the α/β TCR can substitute for the CH3 domain in the Fc region to make immunoglobulin heavy chain heterodimers.

### Certain terms are defined below.

As used herein a "tumor-targeting moiety," refers to a binding agent that recognizes or associates with, e.g., binds to, a target in a cancer cell. The tumor-targeting moiety can be an antibody molecule, a receptor molecule (e.g., a full length receptor, receptor fragment, or fusion thereof (e.g., a receptor-Fc fusion)), or a ligand molecule (e.g., a full length ligand, ligand fragment, or fusion thereof (e.g., a ligand-Fc fusion)) that binds to the cancer antigen (e.g., the tumor and/or the stromal antigen). The tumor-targeting moiety may specifically bind to the target tumor, e.g., binds preferentially to the target tumor. For example, when the tumor-targeting moiety is an antibody molecule, it binds to the cancer antigen (e.g., the tumor antigen and/or the stromal antigen) with a dissociation constant of less than about 10 nM, and more typically, 10 - 100 pM.

As used herein, an "immune cell engager" refers to one or more binding specificities that bind and/or activate an immune cell, e.g., a cell involved in an immune response. In embodiments, the immune cell is chosen from an NK cell, a B cell, a dendritic cell, and/or the macrophage cell. The immune cell engager can be an antibody molecule, a receptor molecule (e.g., a full length receptor, receptor fragment, or fusion thereof), or a ligand molecule (e.g., a full length ligand, ligand fragment, or fusion thereof) that binds to the immune cell antigen (e.g., the NK cell antigen, the B cell antigen, the dendritic cell antigen, and/or the macrophage cell antigen). The immune cell engager may specifically bind to the target immune cell, e.g., binds preferentially to the target immune cell. For example, when the immune cell engager is an antibody molecule, it binds to the immune cell antigen (e.g., the NK cell antigen, the B cell antigen, the dendritic cell antigen, and/or the macrophage cell antigen) with a dissociation constant of less than about 10 nM, and more typically, 10 - 100 pM.

As used herein, a "cytokine molecule" refers to full length, a fragment or a variant of a cytokine; a cytokine further comprising a receptor domain, e.g., a cytokine receptor dimerizing domain; or an agonist of a cytokine receptor, e.g., an antibody molecule (e.g., an agonistic antibody) to a cytokine receptor, that elicits at least one activity of a naturally-occurring cytokine. The cytokine molecule may be chosen from interleukin-2 (IL-2), interleukin-12 (IL-12), interleukin-15 (IL-15), interleukin-18 (IL-18), interleukin-21 (IL-21), or interferon gamma, or a fragment or variant thereof, or a combination of any of the aforesaid cytokines. The cytokine molecule can be a monomer or a dimer. The cytokine molecule may further include a cytokine receptor dimerizing domain. The cytokine molecule may be an agonist of a cytokine receptor, e.g., an antibody molecule (e.g., an agonistic antibody) to a cytokine receptor chosen from an IL-15Ra or IL-21R.

As used herein, the term "molecule" as used in, e.g., antibody molecule, cytokine molecule, receptor molecule, includes full-length, naturally-occurring molecules, as well as variants, e.g., functional variants (e.g., truncations, fragments, mutated (e.g., substantially similar sequences) or derivatized form thereof), so long as at least one function and/or activity of the unmodified (e.g., naturally-occurring) molecule remains.

The term "functional variant" refers to polypeptides that have a substantially identical amino acid sequence to the naturally-occurring sequence, or are encoded by a substantially identical nucleotide sequence, and are capable of having one or more activities of the naturally-occurring sequence.

As used herein, the articles "a" and "an" refer to one or more than one, *e.g.,* to at least one, of the grammatical object of the article. The use of the words "a" or "an" when used in conjunction with the term "comprising" herein may mean "one," but it is also consistent with the meaning of "one or more," "at least one," and "one or more than one."

As used herein, "about" and "approximately" generally mean an acceptable degree of error for the quantity measured given the nature or precision of the measurements. Exemplary degrees of error are within 20 percent (%), typically, within 10%, and more typically, within 5% of a given range of values.

"Antibody molecule" as used herein refers to a protein, e.g., an immunoglobulin chain or fragment thereof, comprising at least one immunoglobulin variable domain sequence. An antibody molecule includes e.g., antibodies (e.g., full-length antibodies) and antibody fragments. An antibody molecule may comprise an antigen binding or functional fragment of a full length antibody, or a full length immunoglobulin chain. For example, a full-length antibody is an immunoglobulin (Ig) molecule (e.g., an IgG antibody) that is naturally occurring or formed by normal immunoglobulin gene fragment recombinatorial processes). An antibody molecule may refer to an immunologically active, antigen-binding portion of an immunoglobulin molecule, such as an antibody fragment. An antibody fragment, e.g., functional fragment, is a portion of an antibody, e.g., Fab, Fab', F(ab')₂, F(ab)₂, variable fragment (Fv), domain antibody (dAb), or single chain variable fragment (scFv). A functional antibody fragment binds to the same antigen as that recognized by the intact (e.g., full-length) antibody. The terms "antibody fragment" or "functional fragment" also include isolated fragments consisting of the variable regions, such as the "Fv" fragments consisting of the variable regions of the heavy and light chains or recombinant single chain polypeptide molecules in which light and heavy variable regions are connected by a peptide linker ("scFv proteins"). An antibody fragment may not include portions of antibodies without antigen binding activity, such as Fc fragments or single amino acid residues. Exemplary antibody molecules include full length antibodies and antibody fragments, e.g., dAb (domain antibody), single chain, Fab, Fab', and F(ab')₂ fragments, and single chain variable fragments (scFvs).

As used herein, a "CH2 domain" refers to an immunoglobulin CH2 domain, e.g., an IgG1, an IgG2, an IgG3, an IgG4 CH2 domain, or any fragment thereof.

As used herein, a "CH3 domain" refers to an immunoglobulin CH3 domain, e.g., an IgG1, an IgG2, an IgG3, an IgG4 CH3 domain, or any fragment thereof.

As used herein, an "immunoglobulin variable domain sequence" refers to an amino acid sequence which can form the structure of an immunoglobulin variable domain. For example, the sequence may include all or part of the amino acid sequence of a naturally-occurring variable domain. For example, the sequence may or may not include one, two, or more N- or C-terminal amino acids, or may include other alterations that are compatible with formation of the protein structure.

An antibody molecule may be monospecific, e.g., it comprises binding specificity for a single epitope. An antibody molecule may be multispecific, e.g., it comprises a plurality of immunoglobulin variable domain sequences, where a first immunoglobulin variable domain sequence has binding specificity for a first epitope and a second immunoglobulin variable domain sequence has binding specificity for a second epitope. An antibody molecule may be a bispecific antibody molecule. "Bispecific antibody molecule" as used herein refers to an antibody molecule that has specificity for more than one (e.g., two, three, four, or more) epitope.

"Antigen" (Ag) as used herein refers to a molecule that can provoke an immune response, e.g., involving activation of certain immune cells and/or antibody generation. The terms "antigen" and "epitope" are used synonymously herein. Any macromolecule, including almost all proteins or peptides, can be an antigen. Antigens can also be derived from genomic recombinant or DNA. For example, any DNA comprising a nucleotide sequence or a partial nucleotide sequence that encodes a protein capable of eliciting an immune response encodes an "antigen." An antigen may not need to be encoded solely by a full length nucleotide sequence of a gene, nor does an antigen need to be encoded by a gene at all. An antigen may be synthesized or can be derived from a biological sample, e.g., a tissue sample, a tumor sample, a cell, or a fluid with other biological components. As used herein, a "tumor antigen" or interchangeably, a "cancer antigen" includes any molecule present on, or associated with, a cancer, e.g., a cancer cell or a tumor microenvironment that can provoke an immune response. As used, herein an "immune cell antigen" includes any molecule present on, or associated with, an immune cell that can provoke an immune response.

The "antigen-binding site" or "binding portion" of an antibody molecule refers to the part of an antibody molecule, e.g., an immunoglobulin (Ig) molecule that participates in antigen binding. The antigen binding site may be formed by amino acid residues of the variable (V) regions of the heavy (H) and light (L) chains. Three highly divergent stretches within the variable regions of the heavy and light chains, referred to as hypervariable regions, are disposed between more conserved flanking stretches called "framework regions," (FRs). FRs are amino acid sequences that are naturally found between, and adjacent to, hypervariable regions in immunoglobulins. In an antibody molecule, the three hypervariable regions of a light chain and the three hypervariable regions of a heavy chain may be disposed relative to each other in three dimensional space to form an antigen-binding surface, which is complementary to the three-dimensional surface of a bound antigen. The three hypervariable regions of each of the heavy and light chains are referred to as "complementarity-determining regions," or "CDRs." The framework region and CDRs have been defined and described, e.g., in Kabat, E.A., et al. (1991) Sequences of Proteins of Immunological Interest, Fifth Edition, U.S. Department of Health and Human Services, NIH Publication No. 91-3242, and Chothia, C. et al. (1987) J. Mol. Biol. 196:901-917. Each variable chain (e.g., variable heavy chain and variable light chain) is typically made up of three CDRs and four FRs, arranged from amino-terminus to carboxy-terminus in the amino acid order: FR1, CDR1, FR2, CDR2, FR3, CDR3, and FR4.

As used herein, the term a "TCRα constant domain" refers to a portion of a TCR that is encoded by the TRAC gene, or a fragment or variant thereof. The term a "TCRβ constant domain" refers to a portion of a TCR that is encoded by the TRBC1 gene or TRBC2 gene, or a fragment or variant thereof.

"Cancer" as used herein can encompass all types of oncogenic processes and/or cancerous growths. Cancer may include primary tumors as well as metastatic tissues or malignantly transformed cells, tissues, or organs. Cancer may encompass all histopathologies and stages, e.g., stages of invasiveness/severity, of a cancer. Cancer may include relapsed and/or resistant cancer. The terms "cancer" and "tumor" can be used interchangeably. For example, both terms encompass solid and liquid tumors. As used herein, the term "cancer" or "tumor" includes premalignant, as well as malignant cancers and tumors.

As used herein, an "immune cell" refers to any of various cells that function in the immune system, e.g., to protect against agents of infection and foreign matter. This term may include leukocytes, e.g., neutrophils, eosinophils, basophils, lymphocytes, and monocytes. Innate leukocytes include phagocytes (e.g., macrophages, neutrophils, and dendritic cells), mast cells, eosinophils, basophils, and natural killer cells. Innate leukocytes identify and eliminate pathogens, either by attacking larger pathogens through contact or by engulfing and then killing microorganisms, and are mediators in the activation of an adaptive immune response. The cells of the adaptive immune system are special types of leukocytes, called lymphocytes. B cells and T cells are important types of lymphocytes and are derived from hematopoietic stem cells in the bone marrow. B cells are involved in the humoral immune response, whereas T cells are involved in cell-mediated immune response. The term "immune cell" includes immune effector cells.

"Immune effector cell," as that term is used herein, refers to a cell that is involved in an immune response, e.g., in the promotion of an immune effector response. Examples of immune effector cells include, but are not limited to, T cells, e.g., alpha/beta T cells and gamma/delta T cells, B cells, natural killer (NK) cells, natural killer T (NK T) cells, and mast cells.

The term "effector function" or "effector response" refers to a specialized function of a cell. Effector function of a T cell, for example, may be cytolytic activity or helper activity including the secretion of cytokines.

The compositions and methods of the present invention encompass polypeptides and nucleic acids having the sequences specified, or sequences substantially identical or similar thereto, *e.g*., sequences at least 85%, 90%, 95% identical or higher to the sequence specified. In the context of an amino acid sequence, the term "substantially identical" is used herein to refer to a first amino acid that contains a sufficient or minimum number of amino acid residues that are i) identical to, or ii) conservative substitutions of aligned amino acid residues in a second amino acid sequence such that the first and second amino acid sequences can have a common structural domain and/or common functional activity. For example, amino acid sequences that contain a common structural domain having at least about 85%, 90%. 91%, 92%, 93%, 94%, 95%, 96%, 97%, 98% or 99% identity to a reference sequence, *e.g*., a sequence provided herein.

In the context of nucleotide sequence, the term "substantially identical" is used herein to refer to a first nucleic acid sequence that contains a sufficient or minimum number of nucleotides that are identical to aligned nucleotides in a second nucleic acid sequence such that the first and second nucleotide sequences encode a polypeptide having common functional activity, or encode a common structural polypeptide domain or a common functional polypeptide activity. For example, nucleotide sequences having at least about 85%, 90%, 91%, 92%, 93%, 94%, 95%, 96%, 97%, 98% or 99% identity to a reference sequence, *e.g.*, a sequence provided herein.

Calculations of homology or sequence identity between sequences (the terms are used interchangeably herein) are performed as follows.

To determine the percent identity of two amino acid sequences, or of two nucleic acid sequences, the sequences are aligned for optimal comparison purposes (*e.g*., gaps can be introduced in one or both of a first and a second amino acid or nucleic acid sequence for optimal alignment and non-homologous sequences can be disregarded for comparison purposes). The length of a reference sequence aligned for comparison purposes may be at least 30%, preferably at least 40%, more preferably at least 50%, 60%, and even more preferably at least 70%, 80%, 90%, 100% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology").

The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. The percent identity between two amino acid sequences may be determined using the Needleman and Wunsch ((1970) J. Mol. Biol. 48:444-453 ) algorithm which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6. The percent identity between two nucleotide sequences may be determined using the GAP program in the GCG software package (available at http://www.gcg.com), using a NWSgapdna.CMP matrix and a gap weight of 40, 50, 60, 70, or 80 and a length weight of 1, 2, 3, 4, 5, or 6. A particularly preferred set of parameters (and the one that should be used unless otherwise specified) are a Blossum 62 scoring matrix with a gap penalty of 12, a gap extend penalty of 4, and a frameshift gap penalty of 5.

The percent identity between two amino acid or nucleotide sequences can be determined using the algorithm of E. Meyers and W. Miller ((1989) CABIOS, 4:11-17) which has been incorporated into the ALIGN program (version 2.0), using a PAM120 weight residue table, a gap length penalty of 12 and a gap penalty of 4.

The nucleic acid and protein sequences described herein can be used as a "query sequence" to perform a search against public databases to, for example, identify other family members or related sequences. Such searches can be performed using the NBLAST and XBLAST programs (version 2.0) of Altschul, et al. (1990) J. Mol. Biol. 215:403-10. BLAST nucleotide searches can be performed with the NBLAST program, score = 100, wordlength = 12 to obtain nucleotide sequences homologous to a nucleic acid molecules of the invention. BLAST protein searches can be performed with the XBLAST program, score = 50, wordlength = 3 to obtain amino acid sequences homologous to protein molecules of the invention. To obtain gapped alignments for comparison purposes, Gapped BLAST can be utilized as described in Altschul et al., (1997) Nucleic Acids Res. 25:3389-3402. When utilizing BLAST and Gapped BLAST programs, the default parameters of the respective programs (*e.g*., XBLAST and NBLAST) can be used. See http://www.ncbi.nlm.nih.gov.

It is understood that the molecules of the present invention may have additional conservative or non-essential amino acid substitutions, which do not have a substantial effect on their functions.

The term "amino acid" is intended to embrace all molecules, whether natural or synthetic, which include both an amino functionality and an acid functionality and capable of being included in a polymer of naturally-occurring amino acids. Exemplary amino acids include naturally-occurring amino acids; analogs, derivatives and congeners thereof; amino acid analogs having variant side chains; and all stereoisomers of any of any of the foregoing. As used herein the term "amino acid" includes both the D- or L- optical isomers and peptidomimetics.

A "conservative amino acid substitution" is one in which the amino acid residue is replaced with an amino acid residue having a similar side chain. Families of amino acid residues having similar side chains have been defined in the art. These families include amino acids with basic side chains (*e.g.,* lysine, arginine, histidine), acidic side chains (*e.g.,* aspartic acid, glutamic acid), uncharged polar side chains (*e.g*., glycine, asparagine, glutamine, serine, threonine, tyrosine, cysteine), nonpolar side chains (*e.g*., alanine, valine, leucine, isoleucine, proline, phenylalanine, methionine, tryptophan), beta-branched side chains (*e.g*., threonine, valine, isoleucine) and aromatic side chains (*e.g*., tyrosine, phenylalanine, tryptophan, histidine).

The terms "polypeptide", "peptide" and "protein" (if single chain) are used interchangeably herein to refer to polymers of amino acids of any length. The polymer may be linear or branched, it may comprise modified amino acids, and it may be interrupted by nonamino acids. The terms also encompass an amino acid polymer that has been modified; for example, disulfide bond formation, glycosylation, lipidation, acetylation, phosphorylation, or any other manipulation, such as conjugation with a labeling component. The polypeptide can be isolated from natural sources, can be a produced by recombinant techniques from a eukaryotic or prokaryotic host, or can be a product of synthetic procedures.

The terms "nucleic acid," "nucleic acid sequence," "nucleotide sequence," or "polynucleotide sequence," and "polynucleotide" are used interchangeably. They refer to a polymeric form of nucleotides of any length, either deoxyribonucleotides or ribonucleotides, or analogs thereof. The polynucleotide may be either single-stranded or double-stranded, and if single-stranded may be the coding strand or non-coding (antisense) strand. A polynucleotide may comprise modified nucleotides, such as methylated nucleotides and nucleotide analogs. The sequence of nucleotides may be interrupted by non-nucleotide components. A polynucleotide may be further modified after polymerization, such as by conjugation with a labeling component. The nucleic acid may be a recombinant polynucleotide, or a polynucleotide of genomic, cDNA, semisynthetic, or synthetic origin which either does not occur in nature or is linked to another polynucleotide in a non-natural arrangement.

The term "isolated," as used herein, refers to material that is removed from its original or native environment (*e.g*., the natural environment if it is naturally occurring). For example, a naturally-occurring polynucleotide or polypeptide present in a living animal is not isolated, but the same polynucleotide or polypeptide, separated by human intervention from some or all of the co-existing materials in the natural system, is isolated. Such polynucleotides could be part of a vector and/or such polynucleotides or polypeptides could be part of a composition, and still be isolated in that such vector or composition is not part of the environment in which it is found in nature.

Various aspects of the invention are described in further detail below. Additional definitions are set out throughout the specification.

### Antibody Molecules

The antibody molecule may bind to a cancer antigen, e.g., a tumor antigen or a stromal antigen. The cancer antigen may be, *e.g.,* a mammalian, e.g., a human, cancer antigen. The antibody molecule may bind to an immune cell antigen, *e.g*., a mammalian, e.g., a human, immune cell antigen. For example, the antibody molecule binds specifically to an epitope, *e.g.,* linear or conformational epitope, on the cancer antigen or the immune cell antigen.

An antibody molecule may be a monospecific antibody molecule and binds a single epitope. *E.g*., a monospecific antibody molecule having a plurality of immunoglobulin variable domain sequences, each of which binds the same epitope.

An antibody molecule may be a multispecific antibody molecule, *e.g.,* it comprises a plurality of immunoglobulin variable domains sequences, wherein a first immunoglobulin variable domain sequence of the plurality has binding specificity for a first epitope and a second immunoglobulin variable domain sequence of the plurality has binding specificity for a second epitope. The first and second epitopes may be on the same antigen, *e.g*., the same protein (or subunit of a multimeric protein). The first and second epitopes may overlap. The first and second epitopes may not overlap. The first and second epitopes may be on different antigens, *e.g.,* the different proteins (or different subunits of a multimeric protein). A multispecific antibody molecule may comprise a third, fourth or fifth immunoglobulin variable domain. A multispecific antibody molecule may be a bispecific antibody molecule, a trispecific antibody molecule, or a tetraspecific antibody molecule.

A multispecific antibody molecule may be a bispecific antibody molecule. A bispecific antibody has specificity for no more than two antigens. A bispecific antibody molecule is characterized by a first immunoglobulin variable domain sequence which has binding specificity for a first epitope and a second immunoglobulin variable domain sequence that has binding specificity for a second epitope. The first and second epitopes may be on the same antigen, *e.g.,* the same protein (or subunit of a multimeric protein). The first and second epitopes may overlap. The first and second epitopes may not overlap. The first and second epitopes may be on different antigens, *e.g*., the different proteins (or different subunits of a multimeric protein). A bispecific antibody molecule may comprise a heavy chain variable domain sequence and a light chain variable domain sequence which have binding specificity for a first epitope and a heavy chain variable domain sequence and a light chain variable domain sequence which have binding specificity for a second epitope. A bispecific antibody molecule may comprise a half antibody having binding specificity for a first epitope and a half antibody having binding specificity for a second epitope. A bispecific antibody molecule may comprise a half antibody, or fragment thereof, having binding specificity for a first epitope and a half antibody, or fragment thereof, having binding specificity for a second epitope. A bispecific antibody molecule may comprise a scFv or a Fab, or fragment thereof, have binding specificity for a first epitope and a scFv or a Fab, or fragment thereof, have binding specificity for a second epitope.

An antibody molecule may comprise a diabody, and a single-chain molecule, as well as an antigen-binding fragment of an antibody (*e.g.*, Fab, F(ab')₂, and Fv). For example, an antibody molecule can include a heavy (H) chain variable domain sequence (abbreviated herein as VH), and a light (L) chain variable domain sequence (abbreviated herein as VL). An antibody molecule may comprise or consist of a heavy chain and a light chain (referred to herein as a half antibody. In another example, an antibody molecule includes two heavy (H) chain variable domain sequences and two light (L) chain variable domain sequence, thereby forming two antigen binding sites, such as Fab, Fab', F(ab')₂, Fc, Fd, Fd', Fv, single chain antibodies (scFv for example), single variable domain antibodies, diabodies (Dab) (bivalent and bispecific), and chimeric (*e.g*., humanized) antibodies, which may be produced by the modification of whole antibodies or those synthesized de novo using recombinant DNA technologies. These functional antibody fragments retain the ability to selectively bind with their respective antigen or receptor. Antibodies and antibody fragments can be from any class of antibodies including, but not limited to, IgG, IgA, IgM, IgD, and IgE, and from any subclass (*e.g*., IgG1, IgG2, IgG3, and IgG4) of antibodies. The a preparation of antibody molecules can be monoclonal or polyclonal. An antibody molecule can also be a human, humanized, CDR-grafted, or *in vitro* generated antibody. The antibody can have a heavy chain constant region chosen from, *e.g.,* IgG1, IgG2, IgG3, or IgG4. The antibody can also have a light chain chosen from, *e.g.*, kappa or lambda. The term "immunoglobulin" (Ig) is used interchangeably with the term "antibody" herein.

Examples of antigen-binding fragments of an antibody molecule include: (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a diabody (dAb) fragment, which consists of a VH domain; (vi) a camelid or camelized variable domain; (vii) a single chain Fv (scFv), *see e.g.,* Bird et al. (1988) Science 242:423-426; and Huston et al. (1988) Proc. Natl. Acad. Sci. USA 85:5879-5883); (viii) a single domain antibody. These antibody fragments are obtained using conventional techniques known to those with skill in the art, and the fragments are screened for utility in the same manner as are intact antibodies.

Antibody molecules include intact molecules as well as functional fragments thereof. Constant regions of the antibody molecules can be altered, *e.g*., mutated, to modify the properties of the antibody (*e.g.*, to increase or decrease one or more of: Fc receptor binding, antibody glycosylation, the number of cysteine residues, effector cell function, or complement function).

Antibody molecules can also be single domain antibodies. Single domain antibodies can include antibodies whose complementary determining regions are part of a single domain polypeptide. Examples include, but are not limited to, heavy chain antibodies, antibodies naturally devoid of light chains, single domain antibodies derived from conventional 4-chain antibodies, engineered antibodies and single domain scaffolds other than those derived from antibodies. Single domain antibodies may be any of the art, or any future single domain antibodies. Single domain antibodies may be derived from any species including, but not limited to mouse, human, camel, llama, fish, shark, goat, rabbit, and bovine. According to another aspect of the invention, a single domain antibody is a naturally occurring single domain antibody known as heavy chain antibody devoid of light chains. Such single domain antibodies are disclosed in WO 9404678, for example. For clarity reasons, this variable domain derived from a heavy chain antibody naturally devoid of light chain is known herein as a VHH or nanobody to distinguish it from the conventional VH of four chain immunoglobulins. Such a VHH molecule can be derived from antibodies raised in *Camelidae* species, for example in camel, llama, dromedary, alpaca and guanaco. Other species besides *Camelidae* may produce heavy chain antibodies naturally devoid of light chain; such VHHs are within the scope of the invention.

The VH and VL regions can be subdivided into regions of hypervariability, termed "complementarity determining regions" (CDR), interspersed with regions that are more conserved, termed "framework regions" (FR or FW).

The extent of the framework region and CDRs has been precisely defined by a number of methods (*see,* Kabat, E. A., et al. (1991) Sequences of Proteins of Immunological Interest, Fifth Edition, U.S. Department of Health and Human Services, NIH Publication No. 91-3242; Chothia, C. et al. (1987) J. Mol. Biol. 196:901-917; and the AbM definition used by Oxford Molecular's AbM antibody modeling software. *See,* generally, *e.g.,* Protein Sequence and Structure Analysis of Antibody Variable Domains. In: Antibody Engineering Lab Manual (Ed.: Duebel, S. and Kontermann, R., Springer-Verlag, Heidelberg).

The terms "complementarity determining region," and "CDR," as used herein refer to the sequences of amino acids within antibody variable regions which confer antigen specificity and binding affinity. In general, there are three CDRs in each heavy chain variable region (HCDR1, HCDR2, HCDR3) and three CDRs in each light chain variable region (LCDR1, LCDR2, LCDR3).

The precise amino acid sequence boundaries of a given CDR can be determined using any of a number of known schemes, including those described by Kabat et al. (1991), "Sequences of Proteins of Immunological Interest," 5th Ed. Public Health Service, National Institutes of Health, Bethesda, MD ("Kabat" numbering scheme), Al-Lazikani et al., (1997) JMB 273,927-948 ("Chothia" numbering scheme). As used herein, the CDRs defined according the "Chothia" number scheme are also sometimes referred to as "hypervariable loops."

For example, under Kabat, the CDR amino acid residues in the heavy chain variable domain (VH) are numbered 31-35 (HCDR1), 50-65 (HCDR2), and 95-102 (HCDR3); and the CDR amino acid residues in the light chain variable domain (VL) are numbered 24-34 (LCDR1), 50-56 (LCDR2), and 89-97 (LCDR3). Under Chothia, the CDR amino acids in the VH are numbered 26-32 (HCDR1), 52-56 (HCDR2), and 95-102 (HCDR3); and the amino acid residues in VL are numbered 26-32 (LCDR1), 50-52 (LCDR2), and 91-96 (LCDR3).

Each VH and VL typically includes three CDRs and four FRs, arranged from amino-terminus to carboxy-terminus in the following order: FR1, CDR1, FR2, CDR2, FR3, CDR3, FR4.

The antibody molecule can be a polyclonal or a monoclonal antibody.

The terms "monoclonal antibody" or "monoclonal antibody composition" as used herein refer to a preparation of antibody molecules of single molecular composition. A monoclonal antibody composition displays a single binding specificity and affinity for a particular epitope. A monoclonal antibody can be made by hybridoma technology or by methods that do not use hybridoma technology (*e.g.*, recombinant methods).

The antibody can be recombinantly produced, *e.g.,* produced by phage display or by combinatorial methods.

Phage display and combinatorial methods for generating antibodies are known in the art (as described in, *e.g.,* Ladner et al. U.S. Patent No. 5,223,409; Kang et al. International Publication No. WO 92/18619; Dower et al. International Publication No. WO 91/17271; Winter et al. International Publication WO 92/20791; Markland et al. International Publication No. WO 92/15679; Breitling et al. International Publication WO 93/01288; McCafferty et al. International Publication No. WO 92/01047; Garrard et al. International Publication No. WO 92/09690; Ladner et al. International Publication No. WO 90/02809; Fuchs et al. (1991) Bio/Technology 9:1370-1372; Hay et al. (1992) Hum Antibod Hybridomas 3:81-85; Huse et al. (1989) Science 246:1275-1281; Griffths et al. (1993) EMBO J 12:725-734; Hawkins et al. (1992) J Mol Biol 226:889-896; Clackson et al. (1991) Nature 352:624-628; Gram et al. (1992) PNAS 89:3576-3580; Garrad etal. (1991) Bio/Technology 9:1373-1377; Hoogenboom et al. (1991) Nuc Acid Res 19:4133-4137; and Barbas et al. (1991) PNAS 88:7978-7982).

The antibody may be a fully human antibody (*e.g*., an antibody made in a mouse which has been genetically engineered to produce an antibody from a human immunoglobulin sequence), or a non-human antibody, *e.g.,* a rodent (mouse or rat), goat, primate *(e.g.,* monkey), camel antibody. Preferably, the non-human antibody is a rodent (mouse or rat antibody). Methods of producing rodent antibodies are known in the art.

Human monoclonal antibodies can be generated using transgenic mice carrying the human immunoglobulin genes rather than the mouse system. Splenocytes from these transgenic mice immunized with the antigen of interest are used to produce hybridomas that secrete human mAbs with specific affinities for epitopes from a human protein (see, *e.g.,* Wood et al. International Application WO 91/00906, Kucherlapati et al. PCT publication WO 91/10741; Lonberg et al. International Application WO 92/03918; Kay et al. International Application 92/03917; Lonberg, N. et al. 1994 Nature 368:856-859; Green, L.L. et al. 1994 Nature Genet. 7:13-21; Morrison, S.L. et al. 1994 Proc. Natl. Acad. Sci. USA 81:6851-6855; Bruggeman et al. 1993 Year Immunol 7:33-40; Tuaillon et al. 1993 PNAS 90:3720-3724; Bruggeman et al. 1991 Eur J Immunol 21:1323-1326).

An antibody molecule can be one in which the variable region, or a portion thereof, *e.g.,* the CDRs, are generated in a non-human organism, *e.g*., a rat or mouse. Chimeric, CDR-grafted, and humanized antibodies are within the invention. Antibody molecules generated in a non-human organism, *e.g.,* a rat or mouse, and then modified, *e.g.,* in the variable framework or constant region, to decrease antigenicity in a human are within the invention.

An "effectively human" protein is a protein that does substantially not evoke a neutralizing antibody response, *e.g.,* the human anti-murine antibody (HAMA) response. HAMA can be problematic in a number of circumstances, *e.g.,* if the antibody molecule is administered repeatedly, *e.g.,* in treatment of a chronic or recurrent disease condition. A HAMA response can make repeated antibody administration potentially ineffective because of an increased antibody clearance from the serum (*see, e.g.,* Saleh et al., Cancer Immunol. Immunother., 32:180-190 (1990)) and also because of potential allergic reactions (*see, e.g.,* LoBuglio et al., Hybridoma, 5:5117-5123 (1986)).

Chimeric antibodies can be produced by recombinant DNA techniques known in the art (see Robinson et al., International Patent Publication PCT/US86/02269; Akira, et al., European Patent Application 184,187; Taniguchi, M, European Patent Application 171,496; Morrison et al., European Patent Application 173,494; Neuberger et al., International Application WO 86/01533; Cabilly et al. U.S. Patent No. 4,816,567; Cabilly et al., European Patent Application 125,023; Better et al. (1988 Science 240:1041-1043); Liu et al. (1987) PNAS 84:3439-3443; Liu et al., 1987, J. Immunol. 139:3521-3526; Sun et al. (1987) PNAS 84:214-218; Nishimura et al., 1987, Cane. Res. 47:999-1005; Wood et al. (1985) Nature 314:446-449; and Shaw et al., 1988, J. Natl Cancer Inst. 80:1553-1559).

A humanized or CDR-grafted antibody will have at least one or two but generally all three recipient CDRs (of heavy and or light immuoglobulin chains) replaced with a donor CDR. The antibody may be replaced with at least a portion of a non-human CDR or only some of the CDRs may be replaced with non-human CDRs. It is only necessary to replace the number of CDRs required for binding to the antigen. Preferably, the donor will be a rodent antibody, e.g., a rat or mouse antibody, and the recipient will be a human framework or a human consensus framework. Typically, the immunoglobulin providing the CDRs is called the "donor" and the immunoglobulin providing the framework is called the "acceptor." In one embodiment, the donor immunoglobulin is a non-human (*e.g.,* rodent). The acceptor framework is a naturally-occurring *(e.g.,* a human) framework or a consensus framework, or a sequence about 85% or higher, preferably 90%, 95%, 99% or higher identical thereto.

As used herein, the term "consensus sequence" refers to the sequence formed from the most frequently occurring amino acids (or nucleotides) in a family of related sequences (See *e.g.,* Winnaker, From Genes to Clones (Verlagsgesellschaft, Weinheim, Germany 1987). In a family of proteins, each position in the consensus sequence is occupied by the amino acid occurring most frequently at that position in the family. If two amino acids occur equally frequently, either can be included in the consensus sequence. A "consensus framework" refers to the framework region in the consensus immunoglobulin sequence.

An antibody molecule can be humanized by methods known in the art (*see e.g.,* Morrison, S. L., 1985, Science 229:1202-1207, by Oi et al., 1986, BioTechniques 4:214, and by Queen et al. US 5,585,089, US 5,693,761 and US 5,693,762).

Humanized or CDR-grafted antibody molecules can be produced by CDR-grafting or CDR substitution, wherein one, two, or all CDRs of an immunoglobulin chain can be replaced. *See e.g.,* U.S. Patent 5,225,539; Jones et al. 1986 Nature 321:552-525; Verhoeyan etal. 1988 Science 239:1534; Beidler et al. 1988 J. Immunol. 141:4053-4060; Winter US 5,225,539. Winter describes a CDR-grafting method which may be used to prepare the humanized antibodies of the present invention (UK Patent Application GB 2188638A, filed on March 26, 1987; Winter US 5,225,539).

Also within the scope of the invention are humanized antibody molecules in which specific amino acids have been substituted, deleted or added. Criteria for selecting amino acids from the donor are described in US 5,585,089, *e.g.,* columns 12-16 of US 5,585,089, *e.g.,* columns 12-16 of US 5,585,089. Other techniques for humanizing antibodies are described in Padlan et al. EP 519596 A1, published on December 23, 1992.

The antibody molecule can be a single chain antibody. A single-chain antibody (scFV) may be engineered (see, for example, Colcher, D. et al. (1999) Ann N YAcad Sci 880:263-80; and Reiter, Y. (1996) Clin Cancer Res 2:245-52). The single chain antibody can be dimerized or multimerized to generate multivalent antibodies having specificities for different epitopes of the same target protein.

The antibody molecule may have a heavy chain constant region chosen from, *e.g.,* the heavy chain constant regions of IgG1, IgG2, IgG3, IgG4, IgM, IgA1, IgA2, IgD, and IgE; particularly, chosen from, *e.g.,* the *(e.g.,* human) heavy chain constant regions of IgG1, IgG2, IgG3, and IgG4. The antibody molecule may have a light chain constant region chosen from, *e.g.,* the (*e.g.*, human) light chain constant regions of kappa or lambda. The constant region can be altered, *e.g.,* mutated, to modify the properties of the antibody *(e.g.,* to increase or decrease one or more of: Fc receptor binding, antibody glycosylation, the number of cysteine residues, effector cell function, and/or complement function). The antibody may have: effector function; and can fix complement. The antibody may not; recruit effector cells; or fix complement. The antibody may have reduced or no ability to bind an Fc receptor. For example, it is a isotype or subtype, fragment or other mutant, which does not support binding to an Fc receptor, *e.g.,* it has a mutagenized or deleted Fc receptor binding region.

Methods for altering an antibody constant region are known in the art. Antibodies with altered function, *e.g*. altered affinity for an effector ligand, such as FcR on a cell, or the C1 component of complement can be produced by replacing at least one amino acid residue in the constant portion of the antibody with a different residue (*see e.g.,* EP 388,151 A1, U.S. Pat. No. 5,624,821 and U.S. Pat. No. 5,648,260). Similar type of alterations could be described which if applied to the murine, or other species immunoglobulin would reduce or eliminate these functions.

An antibody molecule can be derivatized or linked to another functional molecule (*e.g*., another peptide or protein). As used herein, a "derivatized" antibody molecule is one that has been modified. Methods of derivatization include but are not limited to the addition of a fluorescent moiety, a radionucleotide, a toxin, an enzyme or an affinity ligand such as biotin. Accordingly, the antibody molecules of the invention are intended to include derivatized and otherwise modified forms of the antibodies described herein, including immunoadhesion molecules. For example, an antibody molecule can be functionally linked (by chemical coupling, genetic fusion, noncovalent association or otherwise) to one or more other molecular entities, such as another antibody (*e.g*., a bispecific antibody or a diabody), a detectable agent, a cytotoxic agent, a pharmaceutical agent, and/or a protein or peptide that can mediate association of the antibody or antibody portion with another molecule (such as a streptavidin core region or a polyhistidine tag).

One type of derivatized antibody molecule is produced by crosslinking two or more antibodies (of the same type or of different types, *e.g.,* to create bispecific antibodies). Suitable crosslinkers include those that are heterobifunctional, having two distinctly reactive groups separated by an appropriate spacer (*e.g*., m-maleimidobenzoyl-N-hydroxysuccinimide ester) or homobifunctional (*e.g*., disuccinimidyl suberate). Such linkers are available from Pierce Chemical Company, Rockford, Ill.

### Non-immunoglobulin Heterodimerization Domains

Non-immunoglobulin heterodimerization domains described herein include, e.g., TCRα constant domain and TCRβ constant domain.

### TCRα Constant Domain

The TCRα domain may comprise the WT human TCRα constant domain having the following amino acid sequence (human WT full length TCRα constant domain):
Uniprot Reference: P01848

PNIQNPDPAVYQLRDSKSSDKSVCLFTDFDSQTNVSQSKDSDVYITDKTVLDMRSMDFKSNSAVAWSNKS
DFACANAFNNSIIPEDTFFPSPESSCDVKLVEKSFETDTNLNFQNLSVIGFRILLLKVAGFNLLMTLRLW
SS (SEQ ID NO: 158).

The TCRα domain may comprise a fragment of SEQ ID NO: 158.

The TCRα domain may comprise or consist of amino acids 1-85 of SEQ ID NO: 158.

The TCRα domain may comprise or consist of the following amino acid sequence:

PDIQNPDPAVYQLRDSKSSDKSVCLFTDFDSQTNVSQSKDSDVYITDKCVLDMRSMDFKSNSAVAWSNKS
DFACANAFNNSIIPEDTFFPSPE (SEQ ID NO: 1).

The TCRα domain may comprise or consist of at least 20, 30, 40, 50, 60, 70, 80, 90, or 100 contiguous amino acids of SEQ ID NO: 158.

The TCRα domain comprises or consists of at least 5, 10, 20, 30, 40, 50, 60, 70, or 80 contiguous amino acids of SEQ ID NO: 1.

The TCRα domain may comprise or consist of at least 20, 30, 40, 50, 60, 70, 80, 90, or 100 contiguous amino acids of SEQ ID NO: 158, with no more than 10, 9, 8, 7, 6, 5, 4, 3, 2, or 1 amino acid substitutions. The TCRα domain may comprise or consist of at least 20, 30, 40, 50, 60, 70, or 80 contiguous amino acids of SEQ ID NO: 1, with no more than 10, 9, 8, 7, 6, 5, 4, 3, 2, or 1 amino acid substitutions.

The TCRα domain may comprise or consist of at least 20, 30, 40, 50, 60, 70, 80, 90, or 100 contiguous amino acids of SEQ ID NO: 158, with 1, 2, 3, 4, 5, 5, 6, 7, 8, 9, 10, or more amino acid substitutions. The TCRα domain may comprise or consist of at least 20, 30, 40, 50, 60, 70, 80, 90, or 100 contiguous amino acids of SEQ ID NO: 1, with 1, 2, 3, 4, 5, 5, 6, 7, 8, 9, 10, or more amino acid substitutions.

The TCRβ domain may comprise the WT human TCRβ constant domain having the following amino acid sequence (human WT full length TCRβ constant domain):
Uniprot Reference: P01850

EDLNKVFPPEVAVFEPSEAEISHTQKATLVCLATGFFPDHVELSWWVNGKEVHSGVSTDP
QPLKEQPALNDSRYCLSSRLRVSATFWQNPRNHFRCQVQFYGLSENDEWTQDRAKPVTQI
VSAEAWGRADCGFTSVSYQQGVLSATILYEILLGKATLYAVLVSALVLMAMVKRKDF (SEQ ID
NO: 159).

The TCRβ domain may comprise a fragment of SEQ ID NO: 159.

The TCRβ domain may comprise or consist of amino acids 1-130 of SEQ ID NO: 159.

The TCRβ constant domain may comprise or consist of the following amino acid sequence:

EDLNKVFPPEVALFEPSEAEISHTQKATLVCLATGFYPDHVELSWWVNGKEVHSGVCTDPQPLKEQPALN
DSRYALSSRLRVSATFWQDPRNHFRCQVQFYGLSEADEWTQARAKPVTQIVSAEAWGRAD (SEQ ID NO:
2).

The TCRβ domain may comprise or consist of at least 20, 30, 40, 50, 60, 70, 80, 90, 100, 110, 120, 130, 140, 150, 160, or 170 contiguous amino acids of SEQ ID NO: 159.

The TCRβ domain may comprise or consist of at least 20, 30, 40, 50, 60, 70, 80, 90, 100, 110, 120, or 130 contiguous amino acids of SEQ ID NO: 2.

The TCRβ domain may comprise or consist of at least 20, 30, 40, 50, 60, 70, 80, 90, 100, 110, 120, 130, 140, 150, 160, or 170 contiguous amino acids of SEQ ID NO: 159, with no more than 10, 9, 8, 7, 6, 5, 4, 3, 2, or 1 amino acid substitutions. The TCRβ domain may comprise or consist of at least 20, 30, 40, 50, 60, 70, 80, 90, 100, 110, 120, or 130 contiguous amino acids of SEQ ID NO: 2, with no more than 10, 9, 8, 7, 6, 5, 4, 3, 2, or 1 amino acid substitutions.

The TCRβ domain comprises or consists of at least 20, 30, 40, 50, 60, 70, 80, 90, 100, 110, 120, 130, 140, 150, 160, or 170 contiguous amino acids of SEQ ID NO: 159, with 1, 2, 3, 4, 5, 5, 6, 7, 8, 9, 10, or more amino acid substitutions. The TCRβ domain may comprise or consist of at least 20, 30, 40, 50, 60, 70, 80, 90, 100, 110, 120, or 130 contiguous amino acids of SEQ ID NO: 2, with 1, 2, 3, 4, 5, 5, 6, 7, 8, 9, 10, or more amino acid substitutions.

The multispecific molecules may include a portion immunoglobulin constant region (e.g., an Fc region) (e.g., CH2 domain of an Fc). Exemplary Fc regions can be chosen from the heavy chain constant regions of IgG1, IgG2, IgG3 or IgG4; more particularly, the CH2 heavy chain constant region of human IgG1, IgG2, IgG3, or IgG4.

The immunoglobulin chain constant region (e.g., CH2 of the Fc region) may be altered, e.g., mutated, to increase or decrease one or more of: Fc receptor binding, antibody glycosylation, the number of cysteine residues, effector cell function, or complement function.

A linker may be present between the CH2 and TCRα and TCRβ domain.

The TCRα and/or β constant domain may be altered, e.g., mutated, to increase or decrease dimerization. For example, dimerization of the chain is enhanced by introducing a cysteine residue in the TCRα and TCRβ domains creating an engineered disulfide, such that a greater ratio of heteromultimer to homomultimer forms, e.g., relative to a non-engineered interface.

The multispecific molecule may include a half-life extender, e.g., a human serum albumin or an antibody molecule to human serum albumin.

### Multispecific Molecules

### Exemplary Formats

Multispecific molecules, e.g., antibody molecules, comprise more than one antigen-binding site, where different sites are specific for different antigens. Multispecific antibody molecules may bind more than one (e.g., two or more) epitopes on the same antigen. Multispecific antibody molecules may comprise an antigen-binding site specific for a target cell (e.g., cancer cell) and a different antigen-binding site specific for an immune effector cell. The multispecific antibody molecule may be a bispecific antibody molecule. Bispecific antibody molecules can be classified into five different structural groups: (i) bispecific immunoglobulin G (BsIgG); (ii) IgG appended with an additional antigen-binding moiety; (iii) bispecific antibody fragments; (iv) bispecific fusion proteins; and (v) bispecific antibody conjugates.

BsIgG is a format that is monovalent for each antigen. Exemplary BsIgG formats include but are not limited to crossMab, DAF (two-in-one), DAF (four-in-one), DutaMab, DT-IgG, , , charge pair, Fab-arm exchange, , triomab, LUZ-Y, Fcab, κλ-body, orthogonal Fab. *See* Spiess et al. Mol. Immunol. 67(2015):95-106. Exemplary BsIgGs include catumaxomab (Fresenius Biotech, Trion Pharma, Neopharm), which contains an anti-CD3 arm and an anti-EpCAM arm; and ertumaxomab (Neovii Biotech, Fresenius Biotech), which targets CD3 and HER2BsIgG can be produced by separate expression of the component antibodies in different host cells and subsequent purification/assembly into a BsIgG. BsIgG can also be produced by expression of the component antibodies in a single host cell. BsIgG can be purified using affinity chromatography, e.g., using protein A and sequential pH elution.

IgG appended with an additional antigen-binding moiety is another format of bispecific antibody molecules. For example, monospecific IgG can be engineered to have bispecificity by appending an additional antigen-binding unit onto the monospecific IgG, e.g., at the N- or C-terminus of either the heavy or light chain. Exemplary additional antigen-binding units include single domain antibodies (e.g., variable heavy chain or variable light chain), engineered protein scaffolds, and paired antibody variable domains (e.g., single chain variable fragments or variable fragments). *See Id.* Examples of appended IgG formats include dual variable domain IgG (DVD-Ig), IgG(H)-scFv, scFv-(H)IgG, IgG(L)-scFv, scFv-(L)IgG, IgG(L,H)-Fv, IgG(H)-V, V(H)-IgG, IgG(L)-V, V(L)-IgG, KIH IgG-scFab, 2scFv-IgG, IgG-2scFv, scFv4-Ig, zybody, and DVI-IgG (four-in-one). *See* Spiess et al. Mol. Immunol. 67(2015):95-106. An example of an IgG-scFv is MM-141 (Merrimack Pharmaceuticals), which binds IGF-1R and HER3. Examples of DVD-Ig include ABT-981 (AbbVie), which binds IL-1α and IL-1β; and ABT-122 (AbbVie), which binds TNF and IL-17A.

Bispecific antibody fragments (BsAb) are a format of bispecific antibody molecules that lack some or all of the antibody constant domains. For example, some BsAb lack an Fc region. Bispecific antibody fragments may include heavy and light chain regions that are connected by a peptide linker that permits efficient expression of the BsAb in a single host cell. Exemplary bispecific antibody fragments include but are not limited to nanobody, nanobody-HAS, BiTE, Diabody, DART, TandAb, scDiabody, scDiabody-non-immunoglobulin heterodimerization domain (e.g., TCR constant domain α/β), Diabody-non-immunoglobulin heterodimerization domain (e.g., TCR constant domain α/β), triple body, miniantibody, minibody, TriBi minibody, scFv-non-immunoglobulin heterodimerization domain (e.g., TCR constant domain α/β) KIH, Fab-scFv, scFv-CH-CL-scFv, F(ab')2, F(ab')2-scFv2, scFv-KIH, Fab-scFv-Fc, tetravalent HCAb, scDiabody-Fc, Diabody-Fc, tandem scFv-Fc, and intrabody. *See Id.* For example, the BiTE format comprises tandem scFvs, where the component scFvs bind to CD3 on T cells and a surface antigen on cancer cells

Bispecific fusion proteins include antibody fragments linked to other proteins, e.g., to add additional specificity and/or functionality. An example of a bispecific fusion protein is an immTAC, which comprises an anti-CD3 scFv linked to an affinity-matured T-cell receptor that recognizes HLA-presented peptides. The dock-and-lock (DNL) method may be used to generate bispecific antibody molecules with higher valency. Also, fusions to albumin binding proteins or human serum albumin can be extend the serum half-life of antibody fragments. *See Id.*

Chemical conjugation, e.g., chemical conjugation of antibodies and/or antibody fragments, may be used to create BsAb molecules. *See Id.* An exemplary bispecific antibody conjugate includes the CovX-body format, in which a low molecular weight drug is conjugated site-specifically to a single reactive lysine in each Fab arm or an antibody or fragment thereof. The conjugation may improve the serum half-life of the low molecular weight drug. An exemplary CovX-body is CVX-241 (NCT01004822), which comprises an antibody conjugated to two short peptides inhibiting either VEGF or Ang2. *See Id.*

The antibody molecules can be produced by recombinant expression, e.g., of at least one or more component, in a host system. Exemplary host systems include eukaryotic cells (e.g., mammalian cells, e.g., CHO cells, or insect cells, e.g., SF9 or S2 cells) and prokaryotic cells (e.g., *E. coli*)*.* Bispecific antibody molecules can be produced by separate expression of the components in different host cells and subsequent purification/assembly. Alternatively, the antibody molecules can be produced by expression of the components in a single host cell. Purification of bispecific antibody molecules can be performed by various methods such as affinity chromatography, e.g., using protein A and sequential pH elution. Affinity tags may be used for purification, e.g., histidine-containing tag, myc tag, or streptavidin tag.

### CDR-grafted scaffolds

The antibody molecule may be a CDR-grafted scaffold domain. The scaffold domain may be based on a fibronectin domain, e.g., fibronectin type III domain. The overall fold of the fibronectin type III (Fn3) domain is closely related to that of the smallest functional antibody fragment, the variable domain of the antibody heavy chain. There are three loops at the end of Fn3; the positions of BC, DE and FG loops approximately correspond to those of CDR1, 2 and 3 of the VH domain of an antibody. Fn3 does not have disulfide bonds; and therefore Fn3 is stable under reducing conditions, unlike antibodies and their fragments (see, e.g., WO 98/56915; WO 01/64942; WO 00/34784). An Fn3 domain can be modified (e.g., using CDRs or hypervariable loops described herein) or varied, e.g., to select domains that bind to an antigen/marker/cell described herein.

A scaffold domain, e.g., a folded domain, is based on an antibody, e.g., a "minibody" scaffold may be created by deleting three beta strands from a heavy chain variable domain of a monoclonal antibody (see, e.g., Tramontano et al., 1994, J Mol. Recognit. 7:9; and Martin et al., 1994, EMBO J. 13:5303-5309). The "minibody" can be used to present two hypervariable loops. The scaffold domain may be a V-like domain (see, e.g., Coia et al. WO 99/45110) or a domain derived from tendamistatin, which is a 74 residue, six-strand beta sheet sandwich held together by two disulfide bonds (see, e.g., McConnell and Hoess, 1995, J Mol. Biol. 250:460). For example, the loops of tendamistatin can be modified (e.g., using CDRs or hypervariable loops) or varied, e.g., to select domains that bind to a marker/antigen/cell described herein. Another exemplary scaffold domain is a beta-sandwich structure derived from the extracellular domain of CTLA-4 (see, e.g., WO 00/60070).

Other exemplary scaffold domains include but are not limited to T-cell receptors; MHC proteins; extracellular domains (e.g., fibronectin Type III repeats, EGF repeats); protease inhibitors (e.g., Kunitz domains, ecotin, BPTI, and so forth); TPR repeats; trifoil structures; zinc finger domains; DNA-binding proteins; particularly monomeric DNA binding proteins; RNA binding proteins; enzymes, e.g., proteases (particularly inactivated proteases), RNase; chaperones, e.g., thioredoxin, and heat shock proteins; and intracellular signaling domains (such as SH2 and SH3 domains). See, e.g., US 20040009530 and US 7,501,121.

A scaffold domain may be evaluated and chosen, e.g., by one or more of the following criteria: (1) amino acid sequence, (2) sequences of several homologous domains, (3) 3-dimensional structure, and/or (4) stability data over a range of pH, temperature, salinity, organic solvent, oxidant concentration. The scaffold domain may be a small, stable protein domain, e.g., a protein of less than 100, 70, 50, 40 or 30 amino acids. The domain may include one or more disulfide bonds or may chelate a metal, e.g., zinc.

Exemplary structures of the multifunctional molecules defined herein are described below. Exemplary structures are further described in: Weidle U et al. (2013) The Intriguing Options of Multispecific Antibody Formats for Treatment of Cancer. Cancer Genomics & Proteomics 10: 1-18 (2013); and Spiess C et al. (2015) Alternative molecular formats and therapeutic applications for bispecific antibodies. Molecular Immunology 67: 95-106).

### Antibody-Based Fusions

A variety of formats can be generated which contain additional binding entities attached to the N or C terminus of antibodies. These fusions with single chain or disulfide stabilized Fvs or Fabs result in the generation of tetravalent molecules with bivalent binding specificity for each antigen. Combinations of scFvs and scFabs with IgGs enable the production of molecules which can recognize three or more different antigens.

### Antibody-Fab Fusion

Antibody-Fab fusions are bispecific antibodies comprising a traditional antibody to a first target and a Fab to a second target fused to the C terminus of the antibody heavy chain. Commonly the antibody and the Fab will have a common light chain. Antibody fusions can be produced by *(1)* engineering the DNA sequence of the target fusion, and *(2)* transfecting the target DNA into a suitable host cell to express the fusion protein. It seems like the antibody-scFv fusion may be linked by a (Gly)-Ser linker between the C-terminus of the non-immunoglobulin heterodimerization domain and the N-terminus of the scFv, as described by Coloma, J. et al. (1997) Nature Biotech 15:159.

### Antibody-scFv Fusion

*Antibody-scFv Fusions* are bispecific antibodies comprising a traditional antibody and a scFv of unique specificity fused to the C terminus of the antibody heavy chain. The scFv can be fused to the C terminus through the Heavy Chain of the scFv either directly or through a linker peptide. Antibody fusions can be produced by *(1)* engineering the DNA sequence of the target fusion, and *(2)* transfecting the target DNA into a suitable host cell to express the fusion protein. It seems like the antibody-scFv fusion may be linked by a (Gly)-Ser linker between the C-terminus of the non-immunoglobulin heterodimerization domain and the N-terminus of the scFv, as described by Coloma, J. et al. (1997) Nature Biotech 15:159.

### Lambda/Kappa Formats

Multispecific molecules (*e.g*., multispecific antibody molecules) that include the lambda light chain polypeptide and a kappa light chain polypeptides, can be used to allow for heterodimerization (e.g., correct pairing of the light chains). Methods for generating bispecific antibody molecules comprising the lambda light chain polypeptide and a kappa light chain polypeptides are disclosed in USSN 62/399,319 filed on September 23, 2016 and WO2018/057955.

The multispecific molecule may be a multispecific antibody molecule, e.g., an antibody molecule comprising two binding specificities, e.g., a bispecific antibody molecule. The multispecific antibody molecule includes:
a lambda light chain polypeptide 1 (LLCP1) specific for a first epitope;
a heavy chain polypeptide 1 (HCP1) specific for the first epitope;
a kappa light chain polypeptide 2 (KLCP2) specific for a second epitope; and
a heavy chain polypeptide 2 (HCP2) specific for the second epitope.

"Lambda light chain polypeptide 1 (LLCP1)", as that term is used herein, refers to a polypeptide comprising sufficient light chain (LC) sequence, such that when combined with a cognate heavy chain variable region, can mediate specific binding to its epitope and complex with an HCP1. LLCP1 and "Lambda light chain polypeptide (LLCP)" are used interchangeably. It may comprise all or a fragment of a CH1 region. An LLCP1 may comprise LC-CDR1, LC-CDR2, LC-CDR3, FR1, FR2, FR3, FR4, and CH1, or sufficient sequence therefrom to mediate specific binding of its epitope and complex with an HCP1. LLCP1, together with its HCP1, provide specificity for a first epitope (while KLCP2, together with its HCP2, provide specificity for a second epitope). As described elsewhere herein, LLCP1 has a higher affinity for HCP1 than for HCP2.

"Kappa light chain polypeptide 2 (KLCP2)", as that term is used herein, refers to a polypeptide comprising sufficient light chain (LC) sequence, such that when combined with a cognate heavy chain variable region, can mediate specific binding to its epitope and complex with an HCP2. KLCP2 and "Kappa light chain polypeptide (KLCP)" are used interchangeably. It may comprise all or a fragment of a CH1 region. A KLCP2 may comprise LC-CDR1, LC-CDR2, LC-CDR3, FR1, FR2, FR3, FR4, and CH1, or sufficient sequence therefrom to mediate specific binding of its epitope and complex with an HCP2. KLCP2, together with its HCP2, provide specificity for a second epitope (while LLCP1, together with its HCP1, provide specificity for a first epitope).

"Heavy chain polypeptide 1 (HCP1)", as that term is used herein, refers to a polypeptide comprising sufficient heavy chain (HC) sequence, e.g., HC variable region sequence, such that when combined with a cognate LLCP1, can mediate specific binding to its epitope and complex with an HCP1. It may comprise all or a fragment of a CH1region. It may comprise all or a fragment of a CH2 and/or CH3 region. An HCP1 may comprise HC-CDR1, HC-CDR2, HC-CDR3, FR1, FR2, FR3, FR4, CH1, CH2, and CH3, or sufficient sequence therefrom to: (i) mediate specific binding of its epitope and complex with an LLCP1, (ii) to complex preferentially, as described herein to LLCP1 as opposed to KLCP2; and (iii) to complex preferentially, as described herein, to an HCP2, as opposed to another molecule of HCP1. HCP1, together with its LLCP1, provide specificity for a first epitope (while KLCP2, together with its HCP2, provide specificity for a second epitope).

"Heavy chain polypeptide 2 (HCP2)", as that term is used herein, refers to a polypeptide comprising sufficient heavy chain (HC) sequence, e.g., HC variable region sequence, such that when combined with a cognate LLCP1, can mediate specific binding to its epitope and complex with an HCP1. It may comprise all or a fragment of a CH1 region. It may comprise all or a fragment of a CH2 and/or CH3 region. An HCP1 may comprise HC-CDR1, HC-CDR2, HC-CDR3, FR1, FR2, FR3, FR4, CH1, CH2, and CH3, or sufficient sequence therefrom to: (i) mediate specific binding of its epitope and complex with an KLCP2, (ii) to complex preferentially, as described herein to KLCP2 as opposed to LLCP1; and (iii) to complex preferentially, as described herein, to an HCP1, as opposed to another molecule of HCP2. HCP2, together with its KLCP2, provide specificity for a second epitope (while LLCP1, together with its HCP1, provide specificity for a first epitope).

As used herein, preferential pairing of a heavy chain polypeptide and a light chain polypeptide refers to the condition, where the heavy chain polypeptide and the light chain polypeptide preferentially bind to each other, over an unrelated heavy chain polypeptide, or an unrelated light chain polypeptide. The heavy chain polypeptide may bind to the light chain polypeptide with a higher affinity than when the heavy chain polypeptide binds to an unrelated light chain polypeptide. The light chain polypeptide may bind to the heavy chain polypeptide with a higher affinity than when the light chain polypeptide binds to an unrelated heavy chain polypeptide.

As used here, a percent binding between a first heavy chain polypeptide and a first light chain polypeptide in the presence of a competing polypeptide (e.g., a second heavy chain polypeptide or a second light chain polypeptide) refers to the amount of binding between the first heavy chain polypeptide and the first light chain polypeptide in the presence of the competing polypeptide, relative to the amount of binding between the first heavy chain polypeptide and the first light chain polypeptide in the absence of any competing polypeptide (the latter was set to 100%). The percent binding may be measured when the first heavy chain polypeptide, the first light chain polypeptide, and the competing polypeptide are present at 1:1:1. The percent binding may be measured when the first heavy chain polypeptide, the first light chain polypeptide, and the competing polypeptide are present at 1:1:1, wherein the competing polypeptide is a second light chain polypeptide. The percent binding may be measured by an assay described herein, e.g., the NanoBiT assay described in WO2018/057955.

The multispecific antibody molecule disclosed herein:
LLCP1 may have a higher affinity for HCP1 than for HCP2; and/or
KLCP2 may have a higher affinity for HCP2 than for HCP1.

The affinity of LLCP1 for HCP1 may be sufficiently greater than its affinity for HCP2, such that under preselected conditions, e.g., in aqueous buffer, e.g., at pH 7, in saline, e.g., at pH 7, or under physiological conditions, at least 75%, 80, 90, 95, 98, 99, 99.5, or 99.9 % of the multispecific antibody molecule molecules have a LLCP1complexed, or interfaced with, a HCP 1.

The multispecific antibody molecule disclosed herein:
the HCP1 may have a greater affinity for HCP2, than for a second molecule of HCP1;
   and/or
the HCP2 may have a greater affinity for HCP1, than for a second molecule of HCP2.

The affinity of HCP1 for HCP2 may be sufficiently greater than its affinity for a second molecule of HCP1, such that under preselected conditions, e.g., in aqueous buffer, e.g., at pH 7, in saline, e.g., at pH 7, or under physiological conditions, at least 75%, 80, 90, 95, 98, 99 99.5 or 99.9 % of the multispecific antibody molecule molecules have a HCP1 complexed, or interfaced with, a HCP2.

Disclosed herein is a method for making, or producing, a multispecific antibody molecule. The method includes:
(i) providing a first heavy chain polypeptide (e.g., a heavy chain polypeptide comprising one, two, three or all of a first heavy chain variable region (first VH), a first CH1, a first heavy chain constant region (e.g., a first CH2, a first CH3, or both));
(ii) providing a second heavy chain polypeptide (e.g., a heavy chain polypeptide comprising one, two, three or all of a second heavy chain variable region (second VH), a second CH1, a second heavy chain constant region (e.g., a second CH2, a second CH3, or both));
(iii) providing a lambda chain polypeptide (e.g., a lambda light variable region (VLλ), a lambda light constant chain (VLλ), or both) that preferentially associates with the first heavy chain polypeptide (e.g., the first VH); and
(iv) providing a kappa chain polypeptide (e.g., a lambda light variable region (VLκ), a lambda light constant chain (VLκ), or both) that preferentially associates with the second heavy chain polypeptide (e.g., the second VH),
under conditions where (i)-(iv) associate.

The first and second heavy chain polypeptides may form an Fc interface that enhances heterodimerization.
(i)-(iv) (e.g., nucleic acid encoding (i)-(iv)) may be introduced in a single cell, e.g., a single mammalian cell, e.g., a CHO cell. (i)-(iv) may be expressed in the cell.
(i)-(iv) (e.g., nucleic acid encoding (i)-(iv)) may be introduced in different cells, e.g., different mammalian cells, e.g., two or more CHO cell. (i)-(iv) may be expressed in the cells.

The method may further comprise purifying a cell-expressed antibody molecule, e.g., using a lambda- and/or- kappa-specific purification, e.g., affinity chromatography.

The method may further comprise evaluating the cell-expressed multispecific antibody molecule. For example, the purified cell-expressed multispecific antibody molecule can be analyzed by techniques known in the art, include mass spectrometry. The purified cell-expressed antibody molecule may be cleaved, e.g., digested with papain to yield the Fab moieties and evaluated using mass spectrometry.

The method may produce correctly paired kappa/lambda multispecific, e.g., bispecific, antibody molecules in a high yield, e.g., at least 75%, 80, 90, 95, 98, 99 99.5 or 99.9 %.

The multispecific, e.g., a bispecific, antibody molecule may include:
(i) a first heavy chain polypeptide (HCP1) (e.g., a heavy chain polypeptide comprising one, two, three or all of a first heavy chain variable region (first VH), a first CH1, a first heavy chain constant region (e.g., a first CH2, a first CH3, or both)), e.g., wherein the HCP1 binds to a first epitope;
(ii) a second heavy chain polypeptide (HCP2) (e.g., a heavy chain polypeptide comprising one, two, three or all of a second heavy chain variable region (second VH), a second CH1, a second heavy chain constant region (e.g., a second CH2, a second CH3, or both)), e.g., wherein the HCP2 binds to a second epitope;
(iii) a lambda light chain polypeptide (LLCP1) (e.g., a lambda light variable region (VL1), a lambda light constant chain (VLl), or both) that preferentially associates with the first heavy chain polypeptide (e.g., the first VH), e.g., wherein the LLCP1 binds to a first epitope; and
(iv) a kappa light chain polypeptide (KLCP2) (e.g., a lambda light variable region (VLk), a lambda light constant chain (VLk), or both) that preferentially associates with the second heavy chain polypeptide (e.g., the second VH), e.g., wherein the KLCP2 binds to a second epitope.

The first and second heavy chain polypeptides may form an Fc interface that enhances heterodimerization. The multispecific antibody molecule may have a first binding specificity that includes a hybrid VLl-CLl heterodimerized to a first heavy chain variable region connected to the Fc constant, CH2-CH3 domain (having a knob modification) and a second binding specificity that includes a hybrid VLk-CLk heterodimerized to a second heavy chain variable region connected to the Fc constant, CH2-CH3 domain (having a hole modification).

Disclosed herein is a multispecific antibody molecule, e.g., an antibody molecule comprising two binding specificities, e.g., a bispecific antibody molecule. The multispecific antibody molecule comprises:
i) a first antigen-binding domain that binds to a first antigen, wherein the first antigen-binding domain comprises:
   a) a first heavy chain polypeptide (HCP1) comprising: a first heavy chain variable region sequence (HCVRS) sufficient that, when paired with i)b) allows the first antigen-binding domain to bind to the first antigen; and
   b) a lambda light chain polypeptide (LLCP) comprising: a lambda light chain variable region sequence (LLCVRS) sufficient that, when paired with i)a) allows the first antigen-binding domain to bind to the first antigen; and
ii) a second antigen-binding domain that binds to a second antigen, wherein the second antigen-binding domain comprises:
   a) a second heavy chain polypeptide (HCP2) comprising: a second heavy chain variable region sequence (HCVRS) sufficient that, when paired with ii)b) allows the second antigen-binding domain to bind to the second antigen; and
   b) a kappa light chain polypeptide (KLCP) comprising: a kappa light chain variable region sequence (KLCVRS) sufficient that, when paired with ii)a) allows the second antigen-binding domain to bind to the second antigen.

In one embodiment, 1) the first HCVRS has at least 75, 80, 85, 90, 95, 98, or 100% sequence identity with a first heavy chain germline sequence selected from column 2 of Table 9; 2) the LLCVRS has at least 75, 80, 85, 90, 95, 98, or 100% sequence identity with a lambda light chain germline sequence selected from column 3 of Table 9; 3) the second HCVRS has at least 75, 80, 85, 90, 95, 98, or 100% sequence identity with a second heavy chain germline sequence selected from column 4 of Table 9; or 4) the KLCVRS has at least 75, 80, 85, 90, 95, 98, or 100% sequence identity with a kappa light chain germline sequence selected from column 5 of Table 9.

The first HCVRS may have at least 75, 80, 85, 90, 95, 98, or 100% sequence identity with a first heavy chain germline sequence selected from column 2 of Table 9. The first heavy chain germline sequence and the lambda light chain germline sequence may be selected from a single row of Table 9. The first heavy chain germline sequence, the lambda light chain germline sequence, and the kappa light chain germline sequence may be selected from a single row of Table 9. The second heavy chain germline sequence and the kappa light chain germline sequence may be selected from a single row of Table 9. The second heavy chain germline sequence, the kappa light chain germline sequence, and the lambda light chain germline sequence may be selected from a single row of Table 9. At least two (e.g., two, three, or all) of the following: the first heavy chain germline sequence, the lambda light chain germline sequence, the second heavy chain germline sequence, and the kappa light chain germline sequence, may be selected from a single row of Table 9.

The LLCVRS may have at least 75, 80, 85, 90, 95, 98, or 100% sequence identity with a lambda light chain germline sequence selected from column 3 of Table 9. The first heavy chain germline sequence and the lambda light chain germline sequence are selected from a single row of Table 9. The first heavy chain germline sequence, the lambda light chain germline sequence, and the kappa light chain germline sequence may be selected from a single row of Table 9. The second heavy chain germline sequence and the kappa light chain germline sequence may be selected from a single row of Table 9. The second heavy chain germline sequence, the kappa light chain germline sequence, and the lambda light chain germline sequence may be selected from a single row of Table 9. At least two (e.g., two, three, or all) of the following: the first heavy chain germline sequence, the lambda light chain germline sequence, the second heavy chain germline sequence, and the kappa light chain germline sequence, may be selected from a single row of Table 9.

The second HCVRS may have at least 75, 80, 85, 90, 95, 98, or 100% sequence identity with a second heavy chain germline sequence selected from column 4 of Table 9. The first heavy chain germline sequence and the lambda light chain germline sequence may be selected from a single row of Table 9. The first heavy chain germline sequence, the lambda light chain germline sequence, and the kappa light chain germline sequence may be selected from a single row of Table 9. The second heavy chain germline sequence and the kappa light chain germline sequence may be selected from a single row of Table 9. The second heavy chain germline sequence, the kappa light chain germline sequence, and the lambda light chain germline sequence may be selected from a single row of Table 9At least two (e.g., two, three, or all) of the following: the first heavy chain germline sequence, the lambda light chain germline sequence, the second heavy chain germline sequence, and the kappa light chain germline sequence, may be selected from a single row of Table 9.

The KLCVRS may have at least 75, 80, 85, 90, 95, 98, or 100% sequence identity with a kappa light chain germline sequence selected from column 5 of Table 9. The first heavy chain germline sequence and the lambda light chain germline sequence may be selected from a single row of Table 9. The first heavy chain germline sequence, the lambda light chain germline sequence, and the kappa light chain germline sequence may be selected from a single row of Table 9. The second heavy chain germline sequence and the kappa light chain germline sequence may be selected from a single row of Table 9. The second heavy chain germline sequence, the kappa light chain germline sequence, and the lambda light chain germline sequence may be selected from a single row of Table 9. At least two (e.g., two, three, or all) of the following: the first heavy chain germline sequence, the lambda light chain germline sequence, the second heavy chain germline sequence, and the kappa light chain germline sequence, may be selected from a single row of Table 9.

The first heavy chain germline sequence and the lambda light chain germline sequence may be selected from a single row of Table 9. The first heavy chain germline sequence and the second heavy chain germline sequence may be selected from a single row of Table 9. The first heavy chain germline sequence and the kappa light chain germline sequence may be selected from a single row of Table 9. The lambda light chain germline sequence and the second heavy chain germline sequence may be selected from a single row of Table 9. The lambda light chain germline sequence and the kappa light chain germline sequence may be selected from a single row of Table 9. The second heavy chain germline sequence and the kappa light chain germline sequence may be selected from a single row of Table 9. The first heavy chain germline sequence, the lambda light chain germline sequence, and the second heavy chain germline sequence may be selected from a single row of Table 9. The first heavy chain germline sequence, the lambda light chain germline sequence, and the kappa light chain germline sequence may be selected from a single row of Table 9. The first heavy chain germline sequence, the second heavy chain germline sequence, and the kappa light chain germline sequence may be selected from a single row of Table 9. The lambda light chain germline sequence, the second heavy chain germline sequence, and the kappa light chain germline sequence may be selected from a single row of Table 9. The first heavy chain germline sequence, the lambda light chain germline sequence, the second heavy chain germline sequence, and the kappa light chain germline sequence may be selected from a single row of Table 9.

The multispecific antibody molecule may further comprise an accessory moiety, wherein the accessory moiety has a property chosen from:
1) the accessory moiety has a molecular weight of at least 10, 20, 30, 40, 50, 60, 70, 80, 90, or 100 kDa;
2) the accessory moiety comprises a polypeptide having at least 30, 40, 50, 60, 70, 80, 90, or 100 amino acid residues;
3) the accessory moiety comprises a polypeptide having the ability to modulate the activity of an immune cell, e.g., a T cell, a B cell, an antigen presenting cell (APC), or an NK cell; or
4) the accessory moiety is chosen from one or more of an immune cell engager (e.g., a CD40 agonist, e.g., a CD40L polypeptide or an agonistic anti-CD40 antibody molecule, or a PD-1 binding moiety, e.g., a PD-1 binding sequence of PDL-1 or an anti-PD-1 antibody molecule), a cytokine molecule (e.g. an IL-2 molecule), a cytokine antagonist (e.g., a TGF-β antagonist), an enzyme, a toxin, or a labeling agent.

Disclosed herein is a multispecific antibody molecule comprising:
i) a first antigen-binding domain that binds to a first antigen, wherein the first antigen-binding domain comprises:
   a) a first heavy chain polypeptide (HCP1) comprising: a first heavy chain variable region sequence (HCVRS) sufficient that, when paired with i)b) allows the first antigen-binding domain to bind to the first antigen; and
   b) a lambda light chain polypeptide (LLCP) comprising: a lambda light chain variable region sequence (LLCVRS) sufficient that, when paired with i)a) allows the first antigen-binding domain to bind to the first antigen; and
ii) a second antigen-binding domain that binds to a second antigen, wherein the second antigen-binding domain comprises:
   a) a second heavy chain polypeptide (HCP2) comprising: a second heavy chain variable region sequence (HCVRS) sufficient that, when paired with ii)b) allows the second antigen-binding domain to bind to the second antigen; and
   b) a kappa light chain polypeptide (KLCP) comprising: a kappa light chain variable region sequence (KLCVRS) sufficient that, when paired with ii)a) allows the second antigen-binding domain to bind to the second antigen, wherein:
      the multispecific antibody molecule further comprises an accessory moiety, wherein the accessory moiety has a property chosen from:
      1) the accessory moiety has a molecular weight of at least 10, 20, 30, 40, 50, 60, 70, 80, 90, or 100 kDa;
      2) the accessory moiety comprises a polypeptide having at least 30, 40, 50, 60, 70, 80, 90, or 100 amino acid residues;
      3) the accessory moiety comprises a polypeptide having the ability to modulate the activity of an immune cell, e.g., a T cell, a B cell, an antigen presenting cell (APC), or an NK cell; or
      4) the accessory moiety is chosen from one or more of an immune cell engager (e.g., a CD40 agonist, e.g., a CD40L polypeptide or an agonistic anti-CD40 antibody molecule, or a PD-1 binding moiety, e.g., a PD-1 binding sequence of PDL-1 or an anti-PD-1 antibody molecule), a cytokine molecule (e.g. an IL-2 molecule), a cytokine antagonist (e.g., a TGF-β antagonist), an enzyme, a toxin, or a labeling agent.

The accessory moiety may have a molecular weight of at least 10, 20, 30, 40, 50, 60, 70, 80, 90, or 100 kDa. The accessory moiety may comprise a polypeptide having at least 30, 40, 50, 60, 70, 80, 90, or 100 amino acid residues. The accessory moiety may comprise a polypeptide having the ability to modulate the activity of an immune cell, e.g., a T cell, a B cell, an antigen presenting cell (APC), or an NK cell. The accessory moiety may be chosen from one or more of an immune cell engager (e.g., a CD40 agonist, e.g., a CD40L polypeptide or an agonistic anti-CD40 antibody molecule, or a PD-1 binding moiety, e.g., a PD-1 binding sequence of PDL-1 or an anti-PD-1 antibody molecule), a cytokine molecule (e.g. an IL-2 molecule), a cytokine antagonist (e.g., a TGF-β antagonist), an enzyme, a toxin, or a labeling agent.

The accessory moiety may be fused to the polypeptide of a , b, c, or d of the multispecific antibody molecule. The accessory moiety may be fused to any of the following: the HCP1, first HCVRS, LLCP, LLCVRS, HCP2, second HCVRS, KLCP, or KLCVRS of the multispecific antibody molecule, e.g., the C-terminus or N-terminus of HCP1, first HCVRS, LLCP, LLCVRS, HCP2, second HCVRS, KLCP, or KLCVRS of the multispecific antibody molecule. The accessory moiety may be fused to the HCP1. The accessory moiety may be fused to the first HCVRS (e.g., the C-terminus or N-terminus of the first HCVRS). The accessory moiety may be fused to the LLCP (e.g., the C-terminus or N-terminus of the LLCP). The accessory moiety may be fused to the LLCVRS (e.g., the C-terminus or N-terminus of the LLCVRS). The accessory moiety may be fused to the HCP2 (e.g., the C-terminus or N-terminus of the HCP2). The accessory moiety may be fused to the second HCVRS (e.g., the C-terminus or N-terminus of the second HCVRS). The accessory moiety may be fused to the KLCP (e.g., the C-terminus or N-terminus of the KLCP). The accessory moiety may be fused to the KLCVRS (e.g., the C-terminus or N-terminus of the KLCVRS). The HCP1 may comprise a first heavy chain constant region sequence (HCCRS) (e.g., CH1, CH2, and CH3 sequences), wherein the accessory moiety is fused to the first HCCRS, e.g., the C-terminus of the first HCCRS. The HCP2 may comprise a second heavy chain constant region sequence (HCCRS) (e.g., CH1, CH2, and CH3 sequences), wherein the accessory moiety is fused to the second HCCRS, e.g., the C-terminus of the second HCCRS. The LLCP may comprise a lambda light chain constant region sequence (LLCCRS), wherein the accessory moiety is fused to the LLCCRS, e.g., the C-terminus of the LLCCRS. The KLCP may comprise a kappa light chain constant region sequence (KLCCRS), wherein the accessory moiety is fused to the KLCCRS, e.g., the C-terminus of the KLCCRS.

The multispecific antibody molecule may comprise one or more (e.g., two, three, four, five, or more) accessory molecule. The multispecific antibody molecule may comprise a first accessory moiety and a second accessory moiety, wherein the first or second accessory moiety has a property chosen from:
1) the first or second accessory moiety has a molecular weight of at least 10, 20, 30, 40, 50, 60, 70, 80, 90, or 100 kDa;
2) the first or second accessory moiety comprises a polypeptide having at least 30, 40, 50, 60, 70, 80, 90, or 100 amino acid residues;
3) the first or second accessory moiety comprises a polypeptide having the ability to modulate the active of an immune cell, e.g., a T cell, a B cell, an antigen presenting cell (APC), or an NK cell; or
4) the first or second accessory moiety is chosen from one or more of an immune cell engager (e.g., a CD40 agonist, e.g., a CD40L polypeptide or an agonistic anti-CD40 antibody molecule, or a PD-1 binding moiety, e.g., a PD-1 binding sequence of PDL-1 or an anti-PD-1 antibody molecule), a cytokine molecule (e.g. an IL-2 molecule), a cytokine antagonist (e.g., a TGF-β antagonist), an enzyme, a toxin, or a labeling agent.

The first and second accessory moieties may be the same. The first and second accessory moieties may be different. The first accessory moiety may be fused to the HCP1 or HCP2, e.g., the C-terminus of the HCP1 or HCP2; and the second accessory moiety may be fused to the LLCP or KLCP, e.g., the C-terminus of the LLCP or KLCP. The first accessory moiety may be fused to the HCP1, e.g., the C-terminus of the HCP1; and the second accessory moiety may be fused to the LLCP, e.g., the C-terminus of the LLCP. The first accessory moiety may be fused to the HCP1, e.g., the C-terminus of the HCP1; and the second accessory moiety may be fused to the KLCP, e.g., the C-terminus of the KLCP. The first accessory moiety may be fused to the HCP2, e.g., the C-terminus of the HCP2; and the second accessory moiety may be fused to the LLCP, e.g., the C-terminus of the LLCP. The first accessory moiety may be fused to the HCP2, e.g., the C-terminus of the HCP2; and the second accessory moiety may be fused to the KLCP, e.g., the C-terminus of the KLCP. In one embodiment, i) the first accessory moiety is fused to the KLCP, e.g., the C-terminus of the KLCP; and ii) the second accessory moiety is fused to the LLCP, e.g., the C-terminus of the LLCP. In one embodiment, i) the first accessory moiety is fused to the LLCP, e.g., the C-terminus of the LLCP; and ii) the second accessory moiety is fused to the KLCP, e.g., the C-terminus of the KLCP. In one embodiment, i) the HCP1 comprises a first heavy chain constant region sequence (HCCRS) (e.g., CH1, CH2, and CH3 sequences), wherein the first accessory moiety is fused to the first HCCRS, e.g., the C-terminus of the first HCCRS; and ii) the LLCP comprises a lambda light chain constant region sequence (LLCCRS), wherein the second accessory moiety is fused to the LLCCRS, e.g., the C-terminus of the LLCCRS. In one embodiment, i) the HCP2 comprises a second heavy chain constant region sequence (HCCRS) (e.g., CH1, CH2, and CH3 sequences), wherein the accessory moiety is fused to the second HCCRS, e.g., the C-terminus of the second HCCRS; and ii) the KLCP comprises a kappa light chain constant region sequence (KLCCRS), wherein the accessory moiety is fused to the KLCCRS, e.g., the C-terminus of the KLCCRS. In one embodiment, i) the HCP1 comprises a first heavy chain constant region sequence (HCCRS) (e.g., CH1, CH2, and CH3 sequences), wherein the first accessory moiety is fused to the first HCCRS, e.g., the C-terminus of the first HCCRS; and ii) the KLCP comprises a kappa light chain constant region sequence (KLCCRS), wherein the accessory moiety is fused to the KLCCRS, e.g., the C-terminus of the KLCCRS. In one embodiment, i) the HCP2 comprises a second heavy chain constant region sequence (HCCRS) (e.g., CH1, CH2, and CH3 sequences), wherein the accessory moiety is fused to the second HCCRS, e.g., the C-terminus of the second HCCRS; and ii) the LLCP comprises a lambda light chain constant region sequence (LLCCRS), wherein the second accessory moiety is fused to the LLCCRS, e.g., the C-terminus of the LLCCRS.

The multispecific antibody molecule may comprise:
i)a) the HCP1 comprises a first heavy chain constant region sequence (HCCRS) (e.g., a first CH1 sequence),
i)b) the LLCP comprises a lambda light chain constant region sequence (LLCCRS),
ii)a) the HCP2 comprises a second heavy chain constant region sequence (HCCRS) (e.g., a second CH1 sequence), and
ii)b) the KLCP comprises a kappa light chain constant region sequence (KLCCRS), wherein:
   1) the multispecific antibody molecule does not comprise a mutation in the first HCCRS (e.g., a mutation relative to a naturally existing heavy chain constant region sequence) that promotes the preferential pairing of the HCP1 and the LLCP, compared with pairing of the HCP1 and the LLCP without the mutation, or the multispecific antibody molecule does not comprise a mutation in the LLCCRS (e.g., a mutation relative to a naturally existing lambda light chain constant region sequence) that promotes the preferential pairing of the HCP1 and the LLCP, compared with pairing of the HCP1 and the LLCP without the mutation; and
   2) the multispecific antibody molecule does not comprise a mutation in the second HCCRS (e.g., a mutation relative to a naturally existing heavy chain constant region sequence) that promotes the preferential pairing of the HCP2 and the KLCP, compared with pairing of the HCP2 and the KLCP without the mutation, or the multispecific antibody molecule does not comprise a mutation in the KLCCRS (e.g., a mutation relative to a naturally existing kappa light chain constant region sequence) that promotes the preferential pairing of the HCP2 and the KLCP, compared with pairing of the HCP2 and the KLCP without the mutation.

Disclosed herein is a multispecific antibody comprising:
i) a first antigen-binding domain that binds to a first antigen, wherein the first antigen-binding domain comprises:
   a) a first heavy chain polypeptide (HCP1) comprising: a first heavy chain variable region sequence (HCVRS) sufficient that, when paired with i)b) allows the first antigen-binding domain to bind to the first antigen; and a first heavy chain constant region sequence (HCCRS) (e.g., a first CH1 sequence), and
   b) a lambda light chain polypeptide (LLCP) comprising: a lambda light chain variable region sequence (LLCVRS) sufficient that, when paired with i)a) allows the first antigen-binding domain to bind to the first antigen; and a lambda light chain constant region sequence (LLCCRS), and
ii) a second antigen-binding domain that binds to a second antigen, wherein the second antigen-binding domain comprises:
   a) a second heavy chain polypeptide (HCP2) comprising: a second heavy chain variable region sequence (HCVRS) sufficient that, when paired with ii)b) allows the second antigen-binding domain to bind to the second antigen; and a second heavy chain constant region sequence (HCCRS) (e.g., a second CH1 sequence) and
   b) a kappa light chain polypeptide (KLCP) comprising: a kappa light chain variable region sequence (KLCVRS) sufficient that, when paired with ii)a) allows the second antigen-binding domain to bind to the second antigen; and a kappa light chain constant region sequence (KLCCRS), wherein:
      1) the multispecific antibody molecule does not comprise a mutation in the first HCCRS (e.g., a mutation relative to a naturally existing heavy chain constant region sequence) that promotes the preferential pairing of the HCP1 and the LLCP, compared with pairing of the HCP1 and the LLCP without the mutation, or the multispecific antibody molecule does not comprise a mutation in the LLCCRS (e.g., a mutation relative to a naturally existing lambda light chain constant region sequence) that promotes the preferential pairing of the HCP1 and the LLCP, compared with pairing of the HCP1 and the LLCP without the mutation; and
      2) the multispecific antibody molecule does not comprise a mutation in the second HCCRS (e.g., a mutation relative to a naturally existing heavy chain constant region sequence) that promotes the preferential pairing of the HCP2 and the KLCP, compared with pairing of the HCP2 and the KLCP without the mutation, or the multispecific antibody molecule does not comprise a mutation in the KLCCRS (e.g., a mutation relative to a naturally existing kappa light chain constant region sequence) that promotes the preferential pairing of the HCP2 and the KLCP, compared with pairing of the HCP2 and the KLCP without the mutation.

The multispecific antibody molecule may not comprise a mutation in the first HCCRS (e.g., a mutation relative to a naturally existing heavy chain constant region sequence) that promotes the preferential pairing of the HCP1 and the LLCP, compared with pairing of the HCP1 and the LLCP without the mutation; and
the multispecific antibody molecule may not comprise a mutation in the second HCCRS (e.g., a mutation relative to a naturally existing heavy chain constant region sequence) that promotes the preferential pairing of the HCP2 and the KLCP, compared with pairing of the HCP2 and the KLCP without the mutation.

The multispecific antibody molecule may not comprise a mutation in the first HCCRS (e.g., a mutation relative to a naturally existing heavy chain constant region sequence) that promotes the preferential pairing of the HCP1 and the LLCP, compared with pairing of the HCP1 and the LLCP without the mutation; and
the multispecific antibody molecule may not comprise a mutation in the KLCCRS (e.g., a mutation relative to a naturally existing kappa light chain constant region sequence) that promotes the preferential pairing of the HCP2 and the KLCP, compared with pairing of the HCP2 and the KLCP without the mutation.

The multispecific antibody molecule may not comprise a mutation in the LLCCRS (e.g., a mutation relative to a naturally existing lambda light chain constant region sequence) that promotes the preferential pairing of the HCP1 and the LLCP, compared with pairing of the HCP1 and the LLCP without the mutation; and
the multispecific antibody molecule may not comprise a mutation in the second HCCRS (e.g., a mutation relative to a naturally existing heavy chain constant region sequence) that promotes the preferential pairing of the HCP2 and the KLCP, compared with pairing of the HCP2 and the KLCP without the mutation.

The multispecific antibody molecule may not comprise a mutation in the LLCCRS (e.g., a mutation relative to a naturally existing lambda light chain constant region sequence) that promotes the preferential pairing of the HCP1 and the LLCP, compared with pairing of the HCP1 and the LLCP without the mutation; and
the multispecific antibody molecule may not comprise a mutation in the KLCCRS (e.g., a mutation relative to a naturally existing kappa light chain constant region sequence) that promotes the preferential pairing of the HCP2 and the KLCP, compared with pairing of the HCP2 and the KLCP without the mutation.

The multispecific antibody molecule may not comprise a mutation in the first HCCRS (e.g., a mutation relative to a naturally existing heavy chain constant region sequence) that promotes the preferential pairing of the HCP1 and the LLCP, compared with pairing of the HCP1 and the LLCP without the mutation, and the multispecific antibody molecule does not comprise a mutation in the LLCCRS (e.g., a mutation relative to a naturally existing lambda light chain constant region sequence) that promotes the preferential pairing of the HCP1 and the LLCP, compared with pairing of the HCP1 and the LLCP without the mutation; and
the multispecific antibody molecule may not comprise a mutation in the second HCCRS (e.g., a mutation relative to a naturally existing heavy chain constant region sequence) that promotes the preferential pairing of the HCP2 and the KLCP, compared with pairing of the HCP2 and the KLCP without the mutation, or the multispecific antibody molecule does not comprise a mutation in the KLCCRS (e.g., a mutation relative to a naturally existing kappa light chain constant region sequence) that promotes the preferential pairing of the HCP2 and the KLCP, compared with pairing of the HCP2 and the KLCP without the mutation.

The multispecific antibody molecule may not comprise a mutation in the first HCCRS (e.g., a mutation relative to a naturally existing heavy chain constant region sequence) that promotes the preferential pairing of the HCP1 and the LLCP, compared with pairing of the HCP1 and the LLCP without the mutation, or the multispecific antibody molecule does not comprise a mutation in the LLCCRS (e.g., a mutation relative to a naturally existing lambda light chain constant region sequence) that promotes the preferential pairing of the HCP1 and the LLCP, compared with pairing of the HCP1 and the LLCP without the mutation; and
the multispecific antibody molecule may not comprise a mutation in the second HCCRS (e.g., a mutation relative to a naturally existing heavy chain constant region sequence) that promotes the preferential pairing of the HCP2 and the KLCP, compared with pairing of the HCP2 and the KLCP without the mutation, and the multispecific antibody molecule does not comprise a mutation in the KLCCRS (e.g., a mutation relative to a naturally existing kappa light chain constant region sequence) that promotes the preferential pairing of the HCP2 and the KLCP, compared with pairing of the HCP2 and the KLCP without the mutation.

The multispecific antibody molecule may not comprise a mutation in the first HCCRS (e.g., a mutation relative to a naturally existing heavy chain constant region sequence) that promotes the preferential pairing of the HCP1 and the LLCP, compared with pairing of the HCP1 and the LLCP without the mutation, and the multispecific antibody molecule does not comprise a mutation in the LLCCRS (e.g., a mutation relative to a naturally existing lambda light chain constant region sequence) that promotes the preferential pairing of the HCP1 and the LLCP, compared with pairing of the HCP1 and the LLCP without the mutation; and
the multispecific antibody molecule may not comprise a mutation in the second HCCRS (e.g., a mutation relative to a naturally existing heavy chain constant region sequence) that promotes the preferential pairing of the HCP2 and the KLCP, compared with pairing of the HCP2 and the KLCP without the mutation, and the multispecific antibody molecule does not comprise a mutation in the KLCCRS (e.g., a mutation relative to a naturally existing kappa light chain constant region sequence) that promotes the preferential pairing of the HCP2 and the KLCP, compared with pairing of the HCP2 and the KLCP without the mutation.

The multispecific antibody molecule may not comprise a mutation in the first HCCRS (e.g., a mutation relative to a naturally existing heavy chain constant region sequence) that increases the preferential pairing of the HCP1 and the LLCP by at least 1.5, 2, 3, 4, 5, 6, 7, 8, 9, 10, 20, 30, 40, or 50 folds, compared with pairing of the HCP1 and the LLCP without the mutation. The multispecific antibody molecule may not comprise a mutation in the LLCCRS (e.g., a mutation relative to a naturally existing lambda light chain constant region sequence) that increases the preferential pairing of the HCP1 and the LLCP by at least 1.5, 2, 3, 4, 5, 6, 7, 8, 9, 10, 20, 30, 40, or 50 folds, compared with pairing of the HCP1 and the LLCP without the mutation.

The multispecific antibody molecule may not comprise a mutation in the second HCCRS (e.g., a mutation relative to a naturally existing heavy chain constant region sequence) that increases the preferential pairing of the HCP2 and the KLCP by at least 1.5, 2, 3, 4, 5, 6, 7, 8, 9, 10, 20, 30, 40, or 50 folds, compared with pairing of the HCP2 and the KLCP without the mutation. The multispecific antibody molecule may not comprise a mutation in the KLCCRS (e.g., a mutation relative to a naturally existing kappa light chain constant region sequence) that increases the preferential pairing of the HCP2 and the KLCP by at least 1.5, 2, 3, 4, 5, 6, 7, 8, 9, 10, 20, 30, 40, or 50 folds, compared with pairing of the HCP2 and the KLCP without the mutation.

The multispecific antibody molecule may comprise:
i)a) the HCP1 comprises a first heavy chain constant region sequence (HCCRS) (e.g., a first CH1 sequence),
i)b) the LLCP comprises a lambda light chain constant region sequence (LLCCRS),
ii)a) the HCP2 comprises a second heavy chain constant region sequence (HCCRS) (e.g., a second CH1 sequence), and
ii)b) the KLCP comprises a kappa light chain constant region sequence (KLCCRS), wherein:
   1) the multispecific antibody molecule does not comprise a mutation in the first HCCRS (e.g., a mutation relative to a naturally existing heavy chain constant region sequence), or the multispecific antibody molecule does not comprise a mutation in the LLCCRS (e.g., a mutation relative to a naturally existing lambda light chain constant region sequence); and
   2) the multispecific antibody molecule does not comprise a mutation in the second HCCRS (e.g., a mutation relative to a naturally existing heavy chain constant region sequence), or the multispecific antibody molecule does not comprise a mutation in the KLCCRS (e.g., a mutation relative to a naturally existing kappa light chain constant region sequence).

Disclosed herein is a multispecific antibody comprising:
i) a first antigen-binding domain that binds to a first antigen, wherein the first antigen-binding domain comprises:
   a) a first heavy chain polypeptide (HCP1) comprising: a first heavy chain variable region sequence (HCVRS) sufficient that, when paired with i)b) allows the first antigen-binding domain to bind to the first antigen; and a first heavy chain constant region sequence (HCCRS) (e.g., a first CH1 sequence), and
   b) a lambda light chain polypeptide (LLCP) comprising: a lambda light chain variable region sequence (LLCVRS) sufficient that, when paired with i)a) allows the first antigen-binding domain to bind to the first antigen; and a lambda light chain constant region sequence (LLCCRS), and
ii) a second antigen-binding domain that binds to a second antigen, wherein the second antigen-binding domain comprises:
   a) a second heavy chain polypeptide (HCP2) comprising: a second heavy chain variable region sequence (HCVRS) sufficient that, when paired with ii)b) allows the second antigen-binding domain to bind to the second antigen; and a second heavy chain constant region sequence (HCCRS) (e.g., a second CH1 sequence) and
   b) a kappa light chain polypeptide (KLCP) comprising: a kappa light chain variable region sequence (KLCVRS) sufficient that, when paired with ii)a) allows the second antigen-binding domain to bind to the second antigen; and a kappa light chain constant region sequence (KLCCRS), wherein:
      1) the multispecific antibody molecule does not comprise a mutation in the first HCCRS (e.g., a mutation relative to a naturally existing heavy chain constant region sequence), or the multispecific antibody molecule does not comprise a mutation in the LLCCRS (e.g., a mutation relative to a naturally existing lambda light chain constant region sequence); and
      2) the multispecific antibody molecule does not comprise a mutation in the second HCCRS (e.g., a mutation relative to a naturally existing heavy chain constant region sequence), or the multispecific antibody molecule does not comprise a mutation in the KLCCRS (e.g., a mutation relative to a naturally existing kappa light chain constant region sequence).

The multispecific antibody molecule may not comprise a mutation in the first HCCRS (e.g., a mutation relative to a naturally existing heavy chain constant region sequence), and the multispecific antibody molecule may not comprise a mutation in the second HCCRS (e.g., a mutation relative to a naturally existing heavy chain constant region sequence).

The multispecific antibody molecule may not comprise a mutation in the first HCCRS (e.g., a mutation relative to a naturally existing heavy chain constant region sequence), and the multispecific antibody molecule may not comprise a mutation in the KLCCRS (e.g., a mutation relative to a naturally existing kappa light chain constant region sequence).

The multispecific antibody molecule may not comprise a mutation in the LLCCRS (e.g., a mutation relative to a naturally existing lambda light chain constant region sequence), and the multispecific antibody molecule may not comprise a mutation in the second HCCRS (e.g., a mutation relative to a naturally existing heavy chain constant region sequence).

The multispecific antibody molecule may not comprise a mutation in the LLCCRS (e.g., a mutation relative to a naturally existing lambda light chain constant region sequence), and the multispecific antibody molecule may not comprise a mutation in the KLCCRS (e.g., a mutation relative to a naturally existing kappa light chain constant region sequence).

The multispecific antibody molecule may not comprise a mutation in the first HCCRS (e.g., a mutation relative to a naturally existing heavy chain constant region sequence), and the multispecific antibody molecule may not comprise a mutation in the LLCCRS (e.g., a mutation relative to a naturally existing lambda light chain constant region sequence); and the multispecific antibody molecule may not comprise a mutation in the second HCCRS (e.g., a mutation relative to a naturally existing heavy chain constant region sequence), or the multispecific antibody molecule may not comprise a mutation in the KLCCRS (e.g., a mutation relative to a naturally existing kappa light chain constant region sequence).

The multispecific antibody molecule may not comprise a mutation in the first HCCRS (e.g., a mutation relative to a naturally existing heavy chain constant region sequence), or the multispecific antibody molecule may not comprise a mutation in the LLCCRS (e.g., a mutation relative to a naturally existing lambda light chain constant region sequence); and the multispecific antibody molecule may not comprise a mutation in the second HCCRS (e.g., a mutation relative to a naturally existing heavy chain constant region sequence), and the multispecific antibody molecule may not comprise a mutation in the KLCCRS (e.g., a mutation relative to a naturally existing kappa light chain constant region sequence).

The multispecific antibody molecule may not comprise a mutation in the first HCCRS (e.g., a mutation relative to a naturally existing heavy chain constant region sequence), and the multispecific antibody molecule may not comprise a mutation in the LLCCRS (e.g., a mutation relative to a naturally existing lambda light chain constant region sequence); and the multispecific antibody molecule may not comprise a mutation in the second HCCRS (e.g., a mutation relative to a naturally existing heavy chain constant region sequence), and the multispecific antibody molecule may not comprise a mutation in the KLCCRS (e.g., a mutation relative to a naturally existing kappa light chain constant region sequence).

The multispecific antibody molecule may not comprise a mutation in any of the following: the first HCCRS, the LLCCRS, the second HCCRS, and the KLCCRS (e.g., a mutation relative to a naturally existing heavy chain constant region sequence, a naturally existing lambda light chain constant region sequence, or a naturally existing kappa light chain constant region sequence).

The multispecific antibody molecule may not comprise a mutation disclosed in WO2017059551.

The multispecific antibody molecule may comprise:
i)a) the HCP1 comprises a first heavy chain constant region sequence (HCCRS) (e.g., a first CH1 sequence),
i)b) the LLCP comprises a lambda light chain constant region sequence (LLCCRS),
ii)a) the HCP2 comprises a second heavy chain constant region sequence (HCCRS) (e.g., a second CH1 sequence) and
ii)b) the KLCP comprises a kappa light chain constant region sequence (KLCCRS), wherein:
   1) the first HCCRS comprises a naturally existing heavy chain constant region sequence, or the LLCCRS comprises a naturally existing lambda light chain constant region sequence; and
   2) the second HCCRS comprises a naturally existing heavy chain constant region sequence, or the KLCCRS comprises a naturally existing kappa light chain constant region sequence.

Disclosed herein is a multispecific antibody molecule comprising:
i) a first antigen-binding domain that binds to a first antigen, wherein the first antigen-binding domain comprises:
   a) a first heavy chain polypeptide (HCP1) comprising: a first heavy chain variable region sequence (HCVRS) sufficient that, when paired with i)b) allows the first antigen-binding domain to bind to the first antigen; and a first heavy chain constant region sequence (HCCRS) (e.g., a first CH1 sequence), and
   b) a lambda light chain polypeptide (LLCP) comprising: a lambda light chain variable region sequence (LLCVRS) sufficient that, when paired with i)a) allows the first antigen-binding domain to bind to the first antigen; and a lambda light chain constant region sequence (LLCCRS), and
ii) a second antigen-binding domain that binds to a second antigen, wherein the second antigen-binding domain comprises:
   a) a second heavy chain polypeptide (HCP2) comprising: a second heavy chain variable region sequence (HCVRS) sufficient that, when paired with ii)b) allows the second antigen-binding domain to bind to the second antigen; and a second heavy chain constant region sequence (HCCRS) (e.g., a second CH1 sequence) and
   b) a kappa light chain polypeptide (KLCP) comprising: a kappa light chain variable region sequence (KLCVRS) sufficient that, when paired with ii)a) allows the second antigen-binding domain to bind to the second antigen; and a kappa light chain constant region sequence (KLCCRS), wherein:
      1) the first HCCRS comprises a naturally existing heavy chain constant region sequence, or the LLCCRS comprises a naturally existing lambda light chain constant region sequence; and
      2) the second HCCRS comprises a naturally existing heavy chain constant region sequence, or the KLCCRS comprises a naturally existing kappa light chain constant region sequence.

The first HCCRS may comprise a naturally existing heavy chain constant region sequence; and the second HCCRS may comprise a naturally existing heavy chain constant region sequence. The first HCCRS may comprise a naturally existing heavy chain constant region sequence; and the KLCCRS may comprise a naturally existing kappa light chain constant region sequence. The LLCCRS may comprise a naturally existing lambda light chain constant region sequence; and the second HCCRS may comprise a naturally existing heavy chain constant region sequence. The LLCCRS may comprise a naturally existing lambda light chain constant region sequence; and the KLCCRS may comprise a naturally existing kappa light chain constant region sequence. The first HCCRS may comprise a naturally existing heavy chain constant region sequence, and the LLCCRS may comprise a naturally existing lambda light chain constant region sequence; and the second HCCRS may comprise a naturally existing heavy chain constant region sequence, or the KLCCRS may comprise a naturally existing kappa light chain constant region sequence. The first HCCRS may comprise a naturally existing heavy chain constant region sequence, or the LLCCRS may comprise a naturally existing lambda light chain constant region sequence; and the second HCCRS may comprise a naturally existing heavy chain constant region sequence, and the KLCCRS may comprise a naturally existing kappa light chain constant region sequence.

The first HCCRS may comprise a naturally existing heavy chain constant region sequence, the LLCCRS may comprise a naturally existing lambda light chain constant region sequence, the second HCCRS may comprise a naturally existing heavy chain constant region sequence, and the KLCCRS comprise a naturally existing kappa light chain constant region sequence.

The HCP1 may preferentially bind to the LLCP over the KLCP. The LLCP may preferentially bind to the HCP1 over the HCP2. The HCP2 may preferentially bind to the KLCP over the LLCP. The KLCP may preferentially bind to the HCP2 over the HCP1. The HCP1 may have a higher affinity, e.g., a substantially higher affinity, for the LLCP than for the KLCP (e.g., the KD for the binding between the HCP1 and the LLCP is no more than 50%, 40%, 30%, 20%, 10%, 1%, 0.1%, or 0.01% of the KD for the binding between the HCP1 and the KLCP). The LLCP may have a higher affinity, e.g., a substantially higher affinity, for the HCP1 than for the HCP2 (e.g., the KD for the binding between the LLCP and the HCP1 is no more than 50%, 40%, 30%, 20%, 10%, 1%, 0.1%, or 0.01% of the KD for the binding between the LLCP and the first HCP2). The HCP2 may have a higher affinity, e.g., a substantially higher affinity, for the KLCP than for the LLCP (e.g., the KD for the binding between the HCP2 and the KLCP is no more than 50%, 40%, 30%, 20%, 10%, 1%, 0.1 %, or 0.01% of the KD for the binding between the HCP2 and the LLCP). The KLCP may have a higher affinity, e.g., a substantially higher affinity, for the HCP2 than for the HCP1 (e.g., the KD for the binding between the KLCP and the HCP2 is no more than 50%, 40%, 30%, 20%, 10%, 1%, 0.1%, or 0.01% of the KD for the binding between the KLCP and the HCP1).

The percent binding between the HCP1 and the LLCP in the presence of the KLCP may be at least 75, 80, 90, 95, 98, 99, or 99.5%. When the HCP1, LLCP, and KLCP are present at 1:1:1, the percent binding between the HCP1 and the LLCP in the presence of the KLCP may be at least 75, 80, 90, 95, 98, 99, or 99.5% (setting the binding between the HCP1 and the LLCP in the absence of any competing peptide to 100%, and the binding between the HCP1 and the LLCP in the presence of LLCP to 50%). The percent binding may be measured by an assay described herein, e.g., the NanoBiT assay described in WO2018/057955.

The percent binding between the HCP1 and the LLCP in the presence of the HCP2 may be at least 75, 80, 90, 95, 98, 99, or 99.5%. When HCP1, LLCP, and HCP2 are present at 1:1:1, the percent binding between the HCP1 and the LLCP in the presence of the HCP2 may be at least 75, 80, 90, 95, 98, 99, or 99.5% (setting the binding between the HCP1 and the LLCP in the absence of any competing peptide to 100%, and the binding between the HCP1 and the LLCP in the presence of HCP1 to 50%). The percent binding may be measured by an assay described herein, e.g., the NanoBiT assay described in WO2018/057955.

The percent binding between the HCP2 and the KLCP in the presence of the LLCP may be at least 75, 80, 90, 95, 98, 99, or 99.5%. When HCP2, KLCP, and LLCP are present at 1:1:1, the percent binding between the HCP2 and the KLCP in the presence of the LLCP may be at least 75, 80, 90, 95, 98, 99, or 99.5% (setting the binding between the HCP2 and the KLCP in the absence of any competing peptide to 100%, and the binding between the HCP2 and the KLCP in the presence of KLCP to 50%). The percent binding may be measured by an assay described herein, e.g., the NanoBiT assay described in WO2018/057955.

The percent binding between the HCP2 and the KLCP in the presence of the HCP1 may be at least 75, 80, 90, 95, 98, 99, or 99.5%. When HCP2, KLCP, and HCP1 are present at 1:1:1, the percent binding between the HCP2 and the KLCP in the presence of the HCP1 may be at least 75, 80, 90, 95, 98, 99, or 99.5% (setting the binding between the HCP2 and the KLCP in the absence of any competing peptide to 100%, and the binding between the HCP2 and the KLCP in the presence of HCP2 to 50%). The percent binding may be measured by an assay described herein, e.g., the NanoBiT assay described in WO2018/057955.

When the HCP1, LLCP, HCP2, and KLCP are present under preselected conditions, e.g., in aqueous buffer, e.g., at pH 7, in saline, e.g., at pH 7, or under physiological conditions: at least 70, 75, 80, 90, 95, 98, 99, 99.5, or 99.9 % of the HCP1 may be complexed, or interfaced with, the LLCP. When the HCP1, LLCP, HCP2, and KLCP are present under preselected conditions, e.g., in aqueous buffer, e.g., at pH 7, in saline, e.g., at pH 7, or under physiological conditions: at least 70, 75, 80, 90, 95, 98, 99, 99.5, or 99.9 % of the LLCP may be complexed, or interfaced with, the HCP1. When the HCP1, LLCP, HCP2, and KLCP are present under preselected conditions, e.g., in aqueous buffer, e.g., at pH 7, in saline, e.g., at pH 7, or under physiological conditions: at least 70, 75, 80, 90, 95, 98, 99, 99.5, or 99.9 % of the HCP2 may be complexed, or interfaced with, the KLCP. When the HCP1, LLCP, HCP2, and KLCP are present under preselected conditions, e.g., in aqueous buffer, e.g., at pH 7, in saline, e.g., at pH 7, or under physiological conditions: at least 70, 75, 80, 90, 95, 98, 99, 99.5, or 99.9 % of the KLCP may be complexed, or interfaced with, the HCP2.

The multispecific antibody molecule may comprise:
i)a) the HCP1 comprises a first heavy chain constant region sequence (HCCRS) (e.g., a first CH1 sequence),
i)b) the LLCP comprises a lambda light chain constant region sequence (LLCCRS),
ii)a) the HCP2 comprises a second heavy chain constant region sequence (HCCRS) (e.g., a second CH1 sequence) and
ii)b) the KLCP comprises a kappa light chain constant region sequence (KLCCRS), wherein:
   1) the first HCCRS is complexed, or interfaced with, LLCCRS, and
   2) the second HCCRS is complexed, or interfaced with, KLCCRS.

The HCP1 may be complexed, or interfaced with, the HCP2. The HCP1 may have a greater affinity, e.g., a substantially greater affinity, for HCP2, than for a second molecule of HCP1. The HCP2 may have a greater affinity, e.g., a substantially greater affinity, for HCP1, than for a second molecule of HCP2. The HCP1 may comprise a sequence element that increases the ratio of HCP1-HCP2: HCP1-HCP1 pairings, compared to the ratio that would be seen in the absence of the sequence element, e.g., where a naturally occurring sequence replaces the sequence element. The HCP2 may comprise a sequence element that increases the ratio of HCP1-HCP2: HCP2-HCP2 pairings, compared to the ratio that would be seen in the absence of the sequence element, e.g., where a naturally occurring sequence replaces the sequence element. The sequence element may not be a naturally occurring constant region sequence. The sequence element may be disposed in CH3. One or both of HCP1 and HCP2 may be selected to minimize self-dimerization (e.g., HCP1-HCP1) as opposed to heterodimerization (e.g., HCP2-HCP2). HCP1 and HCP2 may be members of a paired protuberance/cavity, e.g., knob and hole pair. HCP1-HCP2 paring may be promoted by an electrostatic interaction. HCP1-HCP2 paring may be promoted by strand exchange. HCP1 and HCP2 may not be members of a paired protuberance/cavity, e.g., knob and hole pair. The HCP1 may comprise a first heavy chain constant region sequence (HCCRS), wherein the first HCCRS does not comprise a mutation (e.g., a mutation relative to a naturally existing heavy chain constant region sequence). The HCP2 may comprise a second heavy chain constant region sequence (HCCRS), wherein the second HCCRS does not comprise a mutation (e.g., a mutation relative to a naturally existing heavy chain constant region sequence). The HCP1 may comprise a first heavy chain constant region sequence (HCCRS), wherein the first HCCRS does not comprise a mutation (e.g., a mutation relative to a naturally existing heavy chain constant region sequence); and the HCP2 may comprises a second heavy chain constant region sequence (HCCRS), wherein the second HCCRS does not comprise a mutation (e.g., a mutation relative to a naturally existing heavy chain constant region sequence). The HCP1 may comprise a first CH2 domain sequence and a first CH3 domain sequence, wherein the first CH2 domain sequence and the first CH3 domain sequence do not comprise a mutation (e.g., a mutation relative to a naturally existing CH2 domain sequence or a naturally existing CH3 domain sequence). The HCP2 may comprise a second CH2 domain sequence and a second CH3 domain sequence, wherein the second CH2 domain sequence and the second CH3 domain sequence do not comprise a mutation (e.g., a mutation relative to a naturally existing CH2 domain sequence or a naturally existing CH3 domain sequence). The HCP1 may comprise a first CH2 domain sequence and a first CH3 domain sequence, wherein the first CH2 domain sequence and the first CH3 domain sequence do not comprise a mutation (e.g., a mutation relative to a naturally existing CH2 domain sequence or a naturally existing CH3 domain sequence); and the HCP2 may comprise a second CH2 domain sequence and a second CH3 domain sequence, wherein the second CH2 domain sequence and the second CH3 domain sequence do not comprise a mutation (e.g., a mutation relative to a naturally existing CH2 domain sequence or a naturally existing CH3 domain sequence).

The HCP1 may be derived from an antibody arising, either in vivo or in vitro, as a lambda antibody. The HCP2 may be derived from an antibody arising, either in vivo or in vitro, as a kappa antibody.

The HCP1 and LLCP may comprise amino acid sequences selected from Table 8 (e.g., as paired in Table 8), or functional variant or fragment thereof (e.g., HCP1 comprises VH, CH1, and/or CH2 from an amino acid sequence selected from Table 8, and LLCP comprises VL, and/or CL from an amino acid sequence selected from Table 8). The HCP2 and KLCP may comprise amino acid sequences selected from Table 8 (e.g., as paired in Table 8), or functional variant or fragment thereof (e.g., HCP2 comprises VH, CH1, and/or CH2 from an amino acid sequence selected from Table 8, and KLCP comprises VL, and/or CL from an amino acid sequence selected from Table 8). The HCP1, LLCP, HCP2, and KLCP may comprise amino acid sequences selected from Table 8 (e.g., a single row of Table 8), or functional variant or fragment thereof (e.g., HCP1 and HCP2 comprise VH, CH1, and/or CH2 from amino acid sequences selected from Table 8, and LLCP and KLCP comprise VL, and/or CL from amino acid sequences selected from Table 8).

The first or second antigen may be a tumor antigen, e.g., a pancreatic, lung, or colorectal tumor antigen. The first or second antigen may be chosen from: PD-L1, HER3, TROP2, mesothelin, IGF-1R, or CA19-9. The first or second antigen may be chosen from: PD-L1, HER3, TROP2, VEGF-A, EGFR, MUC1, DLL4, or HGF. The first or second antigen may be chosen from: PD-L1, HER3, TROP2, VEGF-A, EGFR, MUC1, MAGE-A3, gpA33, NY-ESO-1, ANG2, RSPO3, HER2, CEACAM5, or CEA. The first or second antigen may be an antigen of an immune effector cell, e.g., a T cell, an NK cell, or a myeloid cell. The first or second antigen may be chosen from: CD3, PD-1, LAG-3, TIM-3, CTLA-4, VISTA, TIGIT, PD-L1, B7-H3, 4-1BB, or ICOS. The first antigen may be a tumor antigen, e.g., mesothelin, and the second antigen may be an antigen chosen from NKP30, PD-L1, CD3, NKG2D, CD47, 4-1BB, or NKP46; or the second antigen may be a tumor antigen, e.g., mesothelin, and the first antigen may be an antigen chosen from NKP30, PD-L1, CD3, NKG2D, CD47, 4-1BB, or NKP46. The first antigen may be IGF1R and the second antigen may be HER3, or the second antigen may be IGF1R and the first antigen may be HER3. The first antigen may be mesothelin and the second antigen may be PD-L1, or the second antigen may be mesothelin and the first antigen may be PD-L1. The first antigen may be CTLA4 and the second antigen may be IL12β, or the second antigen may be CTLA4 and the first antigen may be IL12β. The first antigen may be CTLA4 and the second antigen may be TRAILR2, or the second antigen may be CTLA4 and the first antigen may be TRAILR2. The first antigen may be CTLA4 and the second antigen may be CD221, or the second antigen may be CTLA4 and the first antigen may be CD221. The first antigen may be PD1 and the second antigen may be TRAILR2, or the second antigen may be PD1 and the first antigen may be TRAILR2. The first antigen may be PD1 and the second antigen may be PDL1, or the second antigen may be PD1 and the first antigen may be PDL1. The first antigen may be PD1 and the second antigen may be PDL1, or the second antigen may be PD1 and the first antigen may be PDL1. The multispecific antibody molecule may further comprise an IL-2 molecule. The multispecific antibody molecule may further comprise a CD40 agonist, e.g., a CD40L polypeptide or an agonistic anti-CD40 antibody molecule.

**Table 8. Sequences used to construct multispecific molecules.**

| Column 1: Construct | Column 2: heavy chain polypeptide 1 (HCP1) | Column 3: lambda light chain polypeptide (LLCP) | Column 4: heavy chain polypeptide 2 (HCP2) | Column 5: kappa light chain polypeptide (KLCP) |
|---|---|---|---|---|
| Multispecific molecule 1 | SEQ ID NO: 378 | SEQ ID NO: 345 | SEQ ID NO: 379 | SEQ ID NO: 318 |
| Multispecific molecule 2 | SEQ ID NO: 366 | SEQ ID NO: 367 | SEQ ID NO: 364 | SEQ ID NO: 365 |
| Multispecific molecule 3 | SEQ ID NO: 370 | SEQ ID NO: 363 | SEQ ID NO: 368 | SEQ ID NO: 306 |
| Multispecific molecule 4 | SEQ ID NO: 377 | SEQ ID NO: 348 | SEQ ID NO: 368 | SEQ ID NO: 306 |
| Multispecific molecule 5 | SEQ ID NO: 380 | SEQ ID NO: 336 | SEQ ID NO: 368 | SEQ ID NO: 306 |
| Multispecific molecule 6 | SEQ ID NO: 377 | SEQ ID NO: 348 | SEQ ID NO: 381 | SEQ ID NO: 382 |
| Multispecific molecule 7 | SEQ ID NO: 366 | SEQ ID NO: 367 | SEQ ID NO: 381 | SEQ ID NO: 382 |
| Multispecific molecule 8 | SEQ ID NO: 372 | SEQ ID NO: 373 | SEQ ID NO: 371 | SEQ ID NO: 306 |
| Multispecific molecule 9 | SEQ ID NO: 370 | SEQ ID NO: 373 | SEQ ID NO: 368 | SEQ ID NO: 306 |
| Multispecific molecule 10 | SEQ ID NO: 375 | SEQ ID NO: 373 | SEQ ID NO: 371 | SEQ ID NO: 306 |
| Multispecific molecule 11 | SEQ ID NO: 374 | SEQ ID NO: 373 | SEQ ID NO: 368 | SEQ ID NO: 306 |
| Multispecific molecule 12 | SEQ ID NO: 377 | SEQ ID NO: 348 | SEQ ID NO: 369 | SEQ ID NO: 376 |

**Table 9. Corresponding germline sequences of multispecific molecules.**

| Column 1: Construct | Column 2: heavy chain polypeptide 1 (HCP1) corresponding germline sequence | Column 3: lambda light chain polypeptide (LLCP) corresponding germline sequence | Column 4: heavy chain polypeptide 2 (HCP2) corresponding germline sequence | Column 5: kappa light chain polypeptide (KLCP) corresponding germline sequence |
|---|---|---|---|---|
| Multispecific molecule 1 | VH3-9*01 (SEQ ID NO: 196) | V13-19*01 (SEQ ID NO: 211) | VH3-23*01 (SEQ ID NO: 191) | Vk1-27*01 (SEQ ID NO: 200) |
| Multispecific molecule 2 | VH3-66*01 (SEQ ID NO: 194) | V12-14*01 (SEQ ID NO: 210) | VH4-31*01 (SEQ ID NO: 213) | Vk1-39*01 (SEQ ID NO: 201) |
| Multispecific molecule 3 | VH3-33*01 (SEQ ID NO: 193) | V11-44*01 (SEQ ID NO: 209) | VH3-30*01 (SEQ ID NO: 192) | Vk3-20*01 (SEQ ID NO: 205) |
| Multispecific molecule 4 | Vk3-20*01 (SEQ ID NO: 205) | V13-19*01 (SEQ ID NO: 211) | VH3-30*01 (SEQ ID NO: 192) | Vk3-20*01 (SEQ ID NO: 205) |
| Multispecific molecule 5 | VH1-69*01 (SEQ ID NO: 187) | V13-19*01 (SEQ ID NO: 211) | VH3-30*01 (SEQ ID NO: 192) | Vk3-20*01 (SEQ ID NO: 205) |
| Multispecific molecule 6 | Vk3-20*01 (SEQ ID NO: 205) | V13-19*01 (SEQ ID NO: 211) | VH3-33*01 (SEQ ID NO: 193) | Vk3-11*01 (SEQ ID NO: 204) |
| Multispecific molecule 7 | VH3-66*01 (SEQ ID NO: 194) | V12-14*01 (SEQ ID NO: 210) | VH3-33*01 (SEQ ID NO: 193) | Vk3-11*01 (SEQ ID NO: 204) |
| Multispecific molecule 8 | VH3-33*01 (SEQ ID NO: 193) | V11-44*01 (SEQ ID NO: 209) | VH3-30*01 (SEQ ID NO: 192) | Vk3-20*01 (SEQ ID NO: 205) |
| Multispecific molecule 9 | VH3-33*01 (SEQ ID NO: 193) | V11-44*01 (SEQ ID NO: 209) | VH3-30*01 (SEQ ID NO: 192) | Vk3-20*01 (SEQ ID NO: 205) |
| Multispecific molecule 10 | VH3-33*01 (SEQ ID NO: 193) | V11-44*01 (SEQ ID NO: 209) | VH3-30*01 (SEQ ID NO: 192) | Vk3-20*01 (SEQ ID NO: 205) |
| Multispecific molecule 11 | VH3-33*01 (SEQ ID NO: 193) | V11-44*01 (SEQ ID NO: 209) | VH3-30*01 (SEQ ID NO: 192) | Vk3-20*01 (SEQ ID NO: 205) |
| Multispecific molecule 12 | Vk3-20*01 (SEQ ID NO: 205) | V13-19*01 (SEQ ID NO: 211) | VH3-30*01 (SEQ ID NO: 192) | Vk3-20*01 (SEQ ID NO: 205) |

**Table 10. Amino acid sequences used to construct multispecific constructs.**

| SEQ ID NO | Amino Acid Sequence | Description | Germline |
|---|---|---|---|
| SEQ ID NO: 364 | QVQLQESGPGLVKPSQTLSLTCTVSGGSINNNNYYWTWIRQ HPGKGLEWIGYIYYSGSTFYNPSLKSRVTISVDTSKTQFSLKL SSVTAADTAVYYCAREDTMTGLDVWGQGTTVTVSSASTKG PSVFPLAPSSKSTSGGTAALGCLVKDYFPEPVTVSWNSGALT SGVHTFPAVLQSSGLYSLSSVVTVPSSSLGTQTYICNVNHKP SNTKVDKRVEPKSCDKTHTCPPCPAPELLGGPSVFLFPPKPK DTLMISRTPEVTCVVVDVSHEDPEVKFNWYVDGVEVHNAK TKPREEQYNSTYRVVSVLTVLHQDWLNGKEYKCKVSNKAL PAPIEKTISKAKGQPREPQVYTLPPCREEMTKNQVSLWCLV KGFYPSDIAVEWESNGQPENNYKTTPPVLDSDGSFFLYSKLT VDKSRWQQGNVFSCSVMHEALHNHYTQKSLSLSPGK | α-mesothelin AB237 heavy - hCHIg_Knob _Cys | VH4-31*01 (SEQ ID NO: 213) |
| SEQ ID NO: 365 | DIQMTQSPSSLSASVGDRVTITCRASQSINNYLNWYQQKPG KAPTLLIYAASSLQSGVPSRFSGSRSGTDFTLTISSLQPEDFA AYFCQQTYSNPTFGQGTKVEVKRTVAAPSVFIFPPSDEQLKS GTASVVCLLNNFYPREAKVQWKVDNALQSGNSQESVTEQD SKDSTYSLSSTLTLSKADYEKHKVYACEVTHQGLSSPVTKSF NRGEC | α-mesothelin AB237 light - hCLIg_vk | Vk1-39*01 (SEQ ID NO: 201) |
| SEQ ID NO: 366 | EVQLLESGGGLVQPGGSLRLSCAASGFTFSSYIMMWVRQAP GKGLEWVSSIYPSGGITFYADTVKGRFTISRDNSKNTLYLQM NSLRAEDTAVYYCARIKLGTVTTVDYWGQGTLVTVSSAST KGPSVFPLAPSSKSTSGGTAALGCLVKDYFPEPVTVSWNSG ALTSGVHTFPAVLQSSGLYSLSSVVTVPSSSLGTQTYICNVN HKPSNTKVDKRVEPKSCDKTHTCPPCPAPELLGGPSVFLFPP KPKDTLMISRTPEVTCVVVDVSHEDPEVKFNWYVDGVEVH NAKTKPREEQYNSTYRVVSVLTVLHQDWLNGKEYKCKVS NKALPAPIEKTISKAKGQPREPQVCTLPPSREEMTKNQVSLS | α-PDL1 heavy - hCHIg_Hole _Cys | VH3-66*01 (SEQ ID NO: 194) |
| | CAVKGFYPSDIAVEWESNGQPENNYKTTPPVLDSDGSFFLV SKLTVDKSRWQQGNVFSCSVMHEALHNHYTQKSLSLSPGK | | |
| SEQ ID NO: 367 | QSALTQPASVSGSPGQSITISCTGTSSDVGGYNYVSWYQQHP GKAPKLMIYDVSNRPSGVSNRFSGSKSGNTASLTISGLQAED EADYYCSSYTSSSTRVFGTGTKVTVLGQPKANPTVTLFPPSS EELQANKATLVCLISDFYPGAVTVAWKADGSPVKAGVETT KPSKQSNNKYAASSYLSLTPEQWKSHRSYSCQVTHEGSTVE KTVAPTECS | α-PDL1 light - hCLIg_vl | Vl2-14*01 (SEQ ID NO: 210) |
| SEQ ID NO: 368 | QVQLVESGGGVVQPGRSLRLSCAASGFTFSSYTMHWVRQA PGKGLEWVTFISYDGNNKYYADSVKGRFTISRDNSKNTLYL QMNSLRAEDTAIYYCARTGWLGPFDYWGQGTLVTVSSAST KGPSVFPLAPSSKSTSGGTAALGCLVKDYFPEPVTVSWNSG ALTSGVHTFPAVLQSSGLYSLSSVVTVPSSSLGTQTYICNVN HKPSNTKVDKRVEPKSCDKTHTCPPCPAPELLGGPSVFLFPP KPKDTLMISRTPEVTCVVVDVSHEDPEVKFNWYVDGVEVH NAKTKPREEQYNSTYRVVSVLTVLHQDWLNGKEYKCKVS NKALPAPIEKTISKAKGQPREPQVYTLPPCREEMTKNQVSL WCLVKGFYPSDIAVEWESNGQPENNYKTTPPVLDSDGSFFL YSKLTVDKSRWQQGNVFSCSVMHEALHNHYTQKSLSLSPG K | α-CTLA4 heavy - hCHIg_Knob _Cys | VH3-30*01 (SEQ ID NO: 192) |
| SEQ ID NO: 369 | QVQLVESGGGVVQPGRSLRLSCAASGFTFSSYTMHWVRQA PGKGLEWVTFISYDGNNKYYADSVKGRFTISRDNSKNTLYL QMNSLRAEDTAIYYCARTGWLGPFDYWGQGTLVTVSSAST KGPSVFPLAPSSKSTSGGTAALGCLVKDYFPEPVTVSWNSG ALTSGVHTFPAVLQSSGLYSLSSVVTVPSSSLGTQTYICNVN HKPSNTKVDKRVEPKSCDKTHTCPPCPAPELLGGPSVFLFPP KPKDTLMISRTPEVTCVVVDVSHEDPEVKFNWYVDGVEVH NAKTKPREEQYNSTYRVVSVLTVLHQDWLNGKEYKCKVS NKALPAPIEKTISKAKGQPREPQVYTLPPCREEMTKNQVSL WCLVKGFYPSDIAVEWESNGQPENNYKTTPPVLDSDGSFFL YSKLTVDKSRWQQGNVFSCSVMHEALHNHYTQKSLSLSPG KGGGGSGGGGSGGGGSEVQLVESGGGLVKPGGSLRLSCAA SGFTFSPYSVFWVRQAPGKGLEWVSSINTDSTYKYYADSVK GRFTISRDNAENSIFLQMNSLRAEDTAVYYCARDRSYYAFSS GSLSDYYYGLDVWGQGTLVTVSSGGGGSGGGGSGGGGSG GGGSDIVMTQSPLSLSVTPGEPASISCRSSQSLLHTNLYNYLD WYVQKPGQSPQLLIYLASNRASGVPDRFSGSGSGTDFTLKIS RVETEDVGVYYCMQALQIPRTFGQGTKLEIK | α-CTLA4 heavy - hCHIg_Knob _Cys - GH_scFv | VH3-30*01 (SEQ ID NO: 192) |
| SEQ ID NO: 370 | QVQLVESGGGVVQPGRSLRLSCAASGFTFSSYGMHWVRQA PGKGLEWVAFIRYDGSNKYYADSVKGRFTISRDNSKNTLYL QMNSLRAEDTAVYYCKTHGSHDNWGQGTMVTVSSASTKG PSVFPLAPSSKSTSGGTAALGCLVKDYFPEPVTVSWNSGALT SGVHTFPAVLQSSGLYSLSSVVTVPSSSLGTQTYICNVNHKP SNTKVDKRVEPKSCDKTHTCPPCPAPELLGGPSVFLFPPKPK DTLMISRTPEVTCVVVDVSHEDPEVKFNWYVDGVEVHNAK TKPREEQYNSTYRVVSVLTVLHQDWLNGKEYKCKVSNKAL PAPIEKTISKAKGQPREPQVCTLPPSREEMTKNQVSLSCAVK GFYPSDIAVEWESNGQPENNYKTTPPVLDSDGSFFLVSKLTV DKSRWQQGNVFSCSVMHEALHNHYTQKSLSLSPGK | α-IL12β heavy - hCHIg_Hole _Cys | VH3-33*01 (SEQ ID NO: 193) |
| SEQ ID NO: 371 | QVQLVESGGGVVQPGRSLRLSCAASGFTFSSYTMHWVRQA PGKGLEWVTFISYDGNNKYYADSVKGRFTISRDNSKNTLYL QMNSLRAEDTAIYYCARTGWLGPFDYWGQGTLVTVSSAST KGPSVFPLAPSSKSTSGGTAALGCLVKDYFPEPVTVSWNSG ALTSGVHTFPAVLQSSGLYSLSSVVTVPSSSLGTQTYICNVN HKPSNTKVDKRVEPKSCDKTHTCPPCPAPELLGGPSVFLFPP KPKDTLMISRTPEVTCVVVDVSHEDPEVKFNWYVDGVEVH NAKTKPREEQYNSTYRVVSVLTVLHQDWLNGKEYKCKVS NKALPAPIEKTISKAKGQPREPQVYTLPPSREEMTKNQVSLT CLVKGFYPSDIAVEWESNGQPENNYKTTPPVLDSDGSFFLYS KLTVDKSRWQQGNVFSCSVMHEALHNHYTQKSLSLSPGK | α-CTLA4 heavy - hCHIg | VH3-30*01 (SEQ ID NO: 192) |
| SEQ ID NO: 372 | QVQLVESGGGVVQPGRSLRLSCAASGFTFSSYGMHWVRQA PGKGLEWVAFIRYDGSNKYYADSVKGRFTISRDNSKNTLYL QMNSLRAEDTAVYYCKTHGSHDNWGQGTMVTVSSASTKG PSVFPLAPSSKSTSGGTAALGCLVKDYFPEPVTVSWNSGALT SGVHTFPAVLQSSGLYSLSSVVTVPSSSLGTQTYICNVNHKP SNTKVDKRVEPKSCDKTHTCPPCPAPELLGGPSVFLFPPKPK DTLMISRTPEVTCVVVDVSHEDPEVKFNWYVDGVEVHNAK TKPREEQYNSTYRVVSVLTVLHQDWLNGKEYKCKVSNKAL PAPIEKTISKAKGQPREPQVYTLPPSREEMTKNQVSLTCLVK GFYPSDIAVEWESNGQPENNYKTTPPVLDSDGSFFLYSKLTV DKSRWQQGNVFSCSVMHEALHNHYTQKSLSLSPGK | α-IL12β heavy - hCHIg | VH3-33*01 (SEQ ID NO: 193) |
| SEQ ID NO: 373 | QSVLTQPPSVSGAPGQRVTISCSGSRSNIGSNTVKWYQQLPG TAPKLLIYYNDQRPSGVPDRFSGSKSGTSASLAITGLQAEDE ADYYCQSYDRYTHPALLFGTGTKVTVLGQPKANPTVTLFPP SSEELQANKATLVCLISDFYPGAVTVAWKADGSPVKAGVET TKPSKQSNNKYAASSYLSLTPEQWKSHRSYSCQVTHEGSTV EKTVAPTECSGGGGSGGGGSGGGGSAPTSSSTKKTQLQLEH LLLDLQMILNGINNYKNPKLTRMLTAKFAMPKKATELKHL QCLEEELKPLEEVLNLAQSKNFHLRPRDLISNINVIVLELKGS ETTFMCEYADETATIVEFLNRWITFCQSIISTLT | α-IL12β light - hCLIg_vl - IL2 | Vl1-44*01 (SEQ ID NO: 209) |
| SEQ ID NO: 374 | QVQLVESGGGVVQPGRSLRLSCAASGFTFSSYGMHWVRQA PGKGLEWVAFIRYDGSNKYYADSVKGRFTISRDNSKNTLYL QMNSLRAEDTAVYYCKTHGSHDNWGQGTMVTVSSASTKG PSVFPLAPSSKSTSGGTAALGCLVKDYFPEPVTVSWNSGALT SGVHTFPAVLQSSGLYSLSSVVTVPSSSLGTQTYICNVNHKP SNTKVDKRVEPKSCDKTHTCPPCPAPELLGGPSVFLFPPKPK DTLMISRTPEVTCVVVDVSHEDPEVKFNWYVDGVEVHNAK TKPREEQYNSTYRVVSVLTVLHQDWLNGKEYKCKVSNKAL PAPIEKTISKAKGQPREPQVCTLPPSREEMTKNQVSLSCAVK GFYPSDIAVEWESNGQPENNYKTTPPVLDSDGSFFLVSKLTV DKSRWQQGNVFSCSVMHEALHNHYTQKSLSLSPGKGGGGS GGGGSGGGGSAPTSSSTKKTQLQLEHLLLDLQMILNGINNY KNPKLTRMLTAKFAMPKKATELKHLQCLEEELKPLEEVLNL AQSKNFHLRPRDLISNINVIVLELKGSETTFMCEYADETATIV EFLNRWITFCQSIISTLT | α-IL12β heavy - hCHIg_Hole _Cys | VH3-33*01 (SEQ ID NO: 193) |
| SEQ ID NO: 375 | QVQLVESGGGVVQPGRSLRLSCAASGFTFSSYGMHWVRQA PGKGLEWVAFIRYDGSNKYYADSVKGRFTISRDNSKNTLYL QMNSLRAEDTAVYYCKTHGSHDNWGQGTMVTVSSASTKG PSVFPLAPSSKSTSGGTAALGCLVKDYFPEPVTVSWNSGALT | α-IL12β heavy - hCHIg | VH3-33*01 (SEQ ID NO: 193) |
| | SGVHTFPAVLQSSGLYSLSSVVTVPSSSLGTQTYICNVNHKP SNTKVDKRVEPKSCDKTHTCPPCPAPELLGGPSVFLFPPKPK DTLMISRTPEVTCVVVDVSHEDPEVKFNWYVDGVEVHNAK TKPREEQYNSTYRVVSVLTVLHQDWLNGKEYKCKVSNKAL PAPIEKTISKAKGQPREPQVYTLPPSREEMTKNQVSLTCLVK GFYPSDIAVEWESNGQPENNYKTTPPVLDSDGSFFLYSKLTV DKSRWQQGNVFSCSVMHEALHNHYTQKSLSLSPGKGGGGS GGGGSGGGGSAPTSSSTKKTQLQLEHLLLDLQMILNGINNY KNPKLTRMLTAKFAMPKKATELKHLQCLEEELKPLEEVLNL AQSKNFHLRPRDLISNINVIVLELKGSETTFMCEYADETATIV EFLNRWITFCQSIISTLT | | |
| SEQ ID NO: 376 | EIVLTQSPGTLSLSPGERATLSCRASQSVGSSYLAWYQQKPG QAPRLLIYGAFSRATGIPDRFSGSGSGTDFTLTISRLEPEDFA VYYCQQYGSSPWTFGQGTKVEIKRTVAAPSVFIFPPSDEQLK SGTASVVCLLNNFYPREAKVQWKVDNALQSGNSQESVTEQ DSKDSTYSLSSTLTLSKADYEKHKVYACEVTHQGLSSPVTK SFNRGECGGGGSGGGGSGGGGSAPTSSSTKKTQLQLEHLLL DLQMILNGINNYKNPKLTRMLTAKFAMPKKATELKHLQCL EEELKPLEEVLNLAQSKNFHLRPRDLISNINVIVLELKGSETT FMCEYADETATIVEFLNRWITFCQSIISTLT | α-CTLA4 light - hCLIg_vk-IL2 | Vk3-20*01 (SEQ ID NO: 205) |
| SEQ ID NO: 377 | EVQLVQSGGGVERPGGSLRLSCAASGFTFDDYGMSWVRQA PGKGLEWVSGINWNGGSTGYADSVKGRVTISRDNAKNSLY LQMNSLRAEDTAVYYCAKILGAGRGWYFDLWGKGTTVTV SSASTKGPSVFPLAPSSKSTSGGTAALGCLVKDYFPEPVTVS WNSGALTSGVHTFPAVLQSSGLYSLSSVVTVPSSSLGTQTYI CNVNHKPSNTKVDKRVEPKSCDKTHTCPPCPAPELLGGPSV FLFPPKPKDTLMISRTPEVTCVVVDVSHEDPEVKFNWYVDG VEVHNAKTKPREEQYNSTYRVVSVLTVLHQDWLNGKEYK CKVSNKALPAPIEKTISKAKGQPREPQVCTLPPSREEMTKNQ VSLSCAVKGFYPSDIAVEWESNGQPENNYKTTPPVLDSDGS FFLVSKLTVDKSRWQQGNVFSCSVMHEALHNHYTQKSLSL SPGK | α-TNFR10β heavy - hCHIg_Hole _Cys | Vk3-20*01 (SEQ ID NO: 205) |
| SEQ ID NO: 378 | QVQLVQSGGGLVQPGGSLRLSCAASGFTFDDYAMHWVRQ APGKGLEWVAGISWDSGSTGYADSVKGRFTISRDNAKNSL YLQMNSLRAEDTALYYCARDLGAYQWVEGFDYWGQGTL VTVSSASTKGPSVFPLAPSSKSTSGGTAALGCLVKDYFPEPV TVSWNSGALTSGVHTFPAVLQSSGLYSLSSVVTVPSSSLGTQ TYICNVNHKPSNTKVDKRVEPKSCTIKPCPPCKCPAPNLLGG PSVFIFPPKIKDVLMISLSPIVTCVVVDVSEDDPDVQISWFVN NVEVHTAQTQTHREDYNSTLRVVSALPIQHQDWMSGKEFK CKVNNKDLPAPIERTISKPKGSVRAPQVYVLPPCEEEMTKK QVTLWCMVTDFMPEDIYVEWTNNGKTELNYKNTEPVLDSD GSYFMYSKLRVEKKNWVERNSYSCSVVHEGLHNHHTTKSF SRTPGK | α-HER3 heavy - mFc_Knob_ Cys | VH3-9*01 (SEQ ID NO: 196) |
| SEQ ID NO: 379 | EVQLLQSGGGLVQPGGSLRLSCAASGFMFSRYPMHWVRQA PGKGLEWVGSISGSGGATPYADSVKGRFTISRDNSKNTLYL QMNSLRAEDTAVYYCAKDFYQILTGNAFDYWGQGTTVTVS SASTKGPSVFPLAPSSKSTSGGTAALGCLVKDYFPEPVTVSW NSGALTSGVHTFPAVLQSSGLYSLSSVVTVPSSSLGTQTYIC NVNHKPSNTKVDKRVEPKSCTIKPCPPCKCPAPNLLGGPSVF | α-IGF1R heavy - mFc_Hole_C ys | VH3-23*01 (SEQ ID NO: 191) |
| | IFPPKIKDVLMISLSPIVTCVVVDVSEDDPDVQISWFVNNVEV HTAQTQTHREDYNSTLRVVSALPIQHQDWMSGKEFKCKVN NKDLPAPIERTISKPKGSVRAPQVCVLPPPEEEMTKKQVTLS CAVTDFMPEDIYVEWTNNGKTELNYKNTEPVLDSDGSYFM VSKLRVEKKNWVERNSYSCSVVHEGLHNHHTTKSFSRTPG K | | |
| SEQ ID NO: 380 | EVQLVQSGAEVKKPGSSVKVSCKASGGTFSSYAISWVRQAP GQGLEWMGGIIPIFGTANYAQKFQGRVTITADKSTSTAYME LSSLRSEDTAVYYCARAPLRFLEWSTQDHYYYYYMDVWG KGTTVTVSSASTKGPSVFPLAPSSKSTSGGTAALGCLVKDYF PEPVTVSWNSGALTSGVHTFPAVLQSSGLYSLSSVVTVPSSS LGTQTYICNVNHKPSNTKVDKRVEPKSCDKTHTCPPCPAPE LLGGPSVFLFPPKPKDTLMISRTPEVTCVVVDVSHEDPEVKF NWYVDGVEVHNAKTKPREEQYNSTYRVVSVLTVLHQDWL NGKEYKCKVSNKALPAPIEKTISKAKGQPREPQVCTLPPSRE EMTKNQVSLSCAVKGFYPSDIAVEWESNGQPENNYKTTPPV LDSDGSFFLVSKLTVDKSRWQQGNVFSCSVMHEALHNHYT QKSLSLSPGK | α-CD221 heavy - hCHIg_Hole _Cys | VH1-69*01 (SEQ ID NO: 187) |
| SEQ ID NO: 381 | QVQLVESGGGVVQPGRSLRLDCKASGITFSNSGMHWVRQA PGKGLEWVAVIWYDGSKRYYADSVKGRFTISRDNSKNTLF LQMNSLRAEDTAVYYCATNDDYWGQGTLVTVSSASTKGPS VFPLAPSSKSTSGGTAALGCLVKDYFPEPVTVSWNSGALTS GVHTFPAVLQSSGLYSLSSVVTVPSSSLGTQTYICNVNHKPS NTKVDKRVEPKSCDKTHTCPPCPAPELLGGPSVFLFPPKPKD TLMISRTPEVTCVVVDVSHEDPEVKFNWYVDGVEVHNAKT KPREEQYNSTYRVVSVLTVLHQDWLNGKEYKCKVSNKALP APIEKTISKAKGQPREPQVYTLPPCREEMTKNQVSLWCLVK GFYPSDIAVEWESNGQPENNYKTTPPVLDSDGSFFLYSKLTV DKSRWQQGNVFSCSVMHEALHNHYTQKSLSLSPGK | α-PD1 heavy - hCHIg_Knob _Cys | VH3-33*01 (SEQ ID NO: 193) |
| SEQ ID NO: 382 | EIVLTQSPATLSLSPGERATLSCRASQSVSSYLAWYQQKPGQ APRLLIYDASNRATGIPARFSGSGSGTDFTLTISSLEPEDFAV YYCQQSSNWPRTFGQGTKVEIKRTVAAPSVFIFPPSDEQLKS GTASVVCLLNNFYPREAKVQWKVDNALQSGNSQESVTEQD SKDSTYSLSSTLTLSKADYEKHKVYACEVTHQGLSSPVTKSF NRGEC | α-PD1 light - hCLIg_vk | Vk3-11*01 (SEQ ID NO: 204) |
| SEQ ID NO: 306 | EIVLTQSPGTLSLSPGERATLSCRASQSVGSSYLAWYQQKPG QAPRLLIYGAFSRATGIPDRFSGSGSGTDFTLTISRLEPEDFA VYYCQQYGSSPWTFGQGTKVEIKRTVAAPSVFIFPPSDEQLK SGTASVVCLLNNFYPREAKVQWKVDNALQSGNSQESVTEQ DSKDSTYSLSSTLTLSKADYEKHKVYACEVTHQGLSSPVTK SFNRGEC | α-CTLA4 light | Vk3-20*01 (SEQ ID NO: 205) |
| SEQ ID NO: 318 | DIQMTQSPSSLSASLGDRVTITCRASQGISSYLAWYQQKPGK APKLLIYAKSTLQSGVPSRFSGSGSGTDFTLTISSLQPEDSAT YYCQQYWTFPLTFGGGTKVEIKRTVAAPSVFIFPPSDEQLKS GTASVVCLLNNFYPREAKVQWKVDNALQSGNSQESVTEQD SKDSTYSLSSTLTLSKADYEKHKVYACEVTHQGLSSPVTKSF NRGEC | α-IGF1R light | Vk1-27*01 (SEQ ID NO: 200) |
| SEQ ID NO: 336 | SSELTQDPAVSVALGQTVRITCQGDSLRSYYATWYQQKPGQ APILVIYGENKRPSGIPDRFSGSSSGNTASLTITGAQAEDEAD YYCKSRDGSGQHLVFGGGTKLTVLGQPKANPTVTLFPPSSE | α-CD221 light | Vl3-19*01 |
| | ELQANKATLVCLISDFYPGAVTVAWKADGSPVKAGVETTK PSKQSNNKYAASSYLSLTPEQWKSHRSYSCQVTHEGSTVEK TVAPTECS | | (SEQ ID NO: 211) |
| SEQ ID NO: 345 | SYELTQDPAVSVALGQTVRITCQGDSLRSYYASWYQQKPG QAPVLVIYGKNNRPSGIPDRFSGSTSGNSASLTITGAQAEDE ADYYCNSRDSPGNQWVFGGGTKVTVLGGQPKANPTVTLFP PSSEELQANKATLVCLISDFYPGAVTVAWKADGSPVKAGVE TTKPSKQSNNKYAASSYLSLTPEQWKSHRSYSCQVTHEGST VEKTVAPTECS | α-HER3 light | Vl3-19*01 (SEQ ID NO: 211) |
| SEQ ID NO: 348 | SSELTQDPAVSVALGQTVRITCQGDSLRSYYASWYQQKPGQ APVLVIYGKNNRPSGIPDRFSGSSSGNTASLTITGAQAEDEA DYYCNSRDSSGNHVVFGGGTKLTVLGQPKANPTVTLFPPSS EELQANKATLVCLISDFYPGAVTVAWKADGSPVKAGVETT KPSKQSNNKYAASSYLSLTPEQWKSHRSYSCQVTHEGSTVE KTVAPTECS | α-TRAILR2 light | Vl3-19*01 (SEQ ID NO: 211) |
| SEQ ID NO: 363 | QSVLTQPPSVSGAPGQRVTISCSGSRSNIGSNTVKWYQQLPG TAPKLLIYYNDQRPSGVPDRFSGSKSGTSASLAITGLQAEDE ADYYCQSYDRYTHPALLFGTGTKVTVLGQPKANPTVTLFPP SSEELQANKATLVCLISDFYPGAVTVAWKADGSPVKAGVET TKPSKQSNNKYAASSYLSLTPEQWKSHRSYSCQVTHEGSTV EKTVAPTECS | α-IL12β light | Vl1-44*01 (SEQ ID NO: 209) |

**Table 11. Germline sequences**

| **SEQ ID NO** | **Descrip tion** | **Amino acid sequences** |
|---|---|---|
| 187 | VH1-69*01 | QVQLVQSGAEVKKPGSSVKVSCKASGGTFSSYAISWVRQAPGQGLEWMGGI IPIFGTANYAQKFQGRVTITADESTSTAYMELSSLRSEDTAVYYCAR |
| 191 | VH3-23*01 | EVQLLESGGGLVQPGGSLRLSCAASGFTFSSYAMSWVRQAPGKGLEWVSAI SGSGGSTYYADSVKGRFTISRDNSKNTLYLQMNSLRAEDTAVYYCAK |
| 192 | VH3-30*01 | QVQLVESGGGVVQPGRSLRLSCAASGFTFSSYAMHWVRQAPGKGLEWVAV ISYDGSNKYYADSVKGRFTISRDNSKNTLYLQMNSLRAEDTAVYYCAR |
| 194 | VH3-66*01 | EVQLVESGGGLVQPGGSLRLSCAASGFTVSSNYMSWVRQAPGKGLEWVSVI YSGGSTYYADSVKGRFTISRDNSKNTLYLQMNSLRAEDTAVYYCAR EVQLVESGGGLVQPGRSLRLSCAASGFTFDDYAMHWVRQAPGKGLEWVSG |
| 196 | VH3-9*01 | EVQLVESGGGLVQPGRSLRLSCAASGFTFDDYAMHWVRQAPGKGLEWVSG ISWNSGSIGYADSVKGRFTISRDNAKNSLYLQMNSLRAEDTALYYCAK |
| 200 | Vk1-27*01 | DIQMTQSPSSLSASVGDRVTITCRASQGISNYLAWYQQKPGKVPKLLIYAAST LQSGVPSRFSGSGSGTDFTLTISSLQPEDVATYYC |
| 201 | Vk1-39*01 | DIQMTQSPSSLSASVGDRVTITCRASQSISSYLNWYQQKPGKAPKLLIYAASS LQSGVPSRFSGSGSGTDFTLTISSLQPEDFATYYC |
| 204 | Vk3-11*01 | EIVLTQSPATLSLSPGERATLSCRASQSVSSYLAWYQQKPGQAPRLLIYDASN RATGIPARFSGSGSGTDFTLTISSLEPEDFAVYYC |
| 205 | Vk3-20*01 | EIVLTQSPGTLSLSPGERATLSCRASQSVSSSYLAWYQQKPGQAPRLLIYGAS SRATGIPDRFSGSGSGTDFTLTISRLEPEDFAVYYC |
| 209 | Vl1-44*01 | QSVLTQPPSASGTPGQRVTISCSGSSSNIGSNTVNWYQQLPGTAPKLLIYSNN ORPSGVPDRFSGSKSGTSASLAISGLOSEDEADYYC |
| 210 | Vl2-14*01 | QSALTQPASVSGSPGQSITISCTGTSSDVGGYNYVSWYQQHPGKAPKLMIYE VSNRPSGVSNRFSGSKSGNTASLTISGLQAEDEADYYC |
| 211 | Vl3-19*01 | SSELTQDPAVSVALGQTVRITCQGDSLRSYYASWYQQKPGQAPVLVIYGKN NRPSGIPDRFSGSSSGNTASLTITGAQAEDEADYYC |
| 213 | VH4-31*01 | QVQLQESGPGLVKPSQTLSLTCTVSGGSISSGGYYWSWIRQHPGKGLEWIGY IYYSGSTYYNPSLKSLVTISVDTSKNQFSLKLSSVTAADTAVYYCAR |

### Tumor Specific Targeting Moieties

The present disclosure provides, *inter alia,* multispecific (e.g., bi-, tri-, tetra- specific) molecules, that include, e.g., are engineered to contain, one or more tumor specific targeting moieties that direct the molecule to a tumor cell.

### Tumor-targeting moieties

The multispecific molecules disclosed herein may include a tumor-targeting moiety. The tumor targeting moiety can be chosen from an antibody molecule (e.g., an antigen binding domain as described herein), a receptor or a receptor fragment, or a ligand or a ligand fragment, or a combination thereof. The tumor targeting moiety may associate with, e.g., binds to, a tumor cell (e.g., a molecule, e.g., antigen, present on the surface of the tumor cell). The tumor targeting moiety may target, e.g., directs the multispecific molecules disclosed herein to a cancer (e.g., a cancer or tumor cells). The cancer may be chosen from a hematological cancer, a solid cancer, a metastatic cancer, or a combination thereof.

The multispecific molecule, e.g., the tumor-targeting moiety, may bind to a solid tumor antigen or a stromal antigen. The solid tumor antigen or stromal antigen can be present on a solid tumor, or a metastatic lesion thereof. The solid tumor may be chosen from one or more of pancreatic (*e.g.*, pancreatic adenocarcinoma), breast, colorectal, lung (*e.g.*, small or non-small cell lung cancer), skin, ovarian, or liver cancer. The solid tumor may be a fibrotic or desmoplastic solid tumor. For example, the solid tumor antigen or stromal antigen can be present on a tumor, e.g., a tumor of a class typified by having one or more of: limited tumor perfusion, compressed blood vessels, or fibrotic tumor interstitium.
The solid tumor antigen is chosen from one or more of: PDL1, mesothelin, CD47, gangloside 2 (GD2), prostate stem cell antigen (PSCA), prostate specific membrane antigen (PMSA), prostate-specific antigen (PSA), carcinoembryonic antigen (CEA), Ron Kinase, c-Met, Immature laminin receptor, TAG-72, BING-4, Calcium-activated chloride channel 2, Cyclin-B1, 9D7, Ep-CAM, EphA3, Her2/neu, Telomerase, SAP-1, Survivin, NY-ESO-1/LAGE-1, FRAME, SSX-2, Melan-A/MART-1, Gp100/pmel17, Tyrosinase, TRP-1/-2, MC1R, β-catenin, BRCA1/2, CDK4, CML66, Fibronectin, p53, Ras, TGF-B receptor, AFP, ETA, MAGE, MUC-1, CA-125, BAGE, GAGE, NY-ESO-1, β-catenin, CDK4, CDC27, CD47, α actinin-4, TRP1/gp75, TRP2, gp100, Melan-A/MART1, gangliosides, WT1, EphA3, Epidermal growth factor receptor (EGFR), CD20, MART-2, MART-1, MUC1, MLJC2, MUM1, NWM2, MUM3, NA88-1, NPM, OA1, OGT, RCC, RUI1, RUI2, SAGE, TRG, TRP1, TSTA, Folate receptor alpha, L1-CAM, CAIX, EGFRvIII, gpA33, GD3, GM2, VEGFR, Intergrins (Integrin alphaVbeta3, or Integrin alpha5Beta1), Carbohydrates (Le), IGF1R, EPHA3, TRAILR1, TRAILR2, or RANKL. The solid tumor antigen may be chosen from: Mesothelin, PDL1, GD2, PMSA, PSCA, CEA, Ron Kinase, or c-Met. The tumor-targeting moiety may be chosen from an antibody molecule to a cancer antigen chosen from mesothelin, PDL1, HER3, IGF1R, FAP, CD47 or CD123. In one embodiment, the tumor-targeting moiety includes an antibody molecule (e.g., Fab or scFv) that binds to mesothelin. In other embodiments, the multispecific molecule, e.g., the tumor-targeting moiety, binds to a stromal antigen. The stromal antigen is chosen from one or more of: fibroblast activating protease (FAP), TGF-beta, hyaluronic acid, collagen, e.g., collagen IV, tenascin C, or tenascin W.

The multispecific molecule, e.g., the tumor-targeting moiety, may bind to a molecule, e.g., antigen, present on the surface of a hematological cancer, e.g., a leukemia or a lymphoma. The hematological cancer may be a B-cell or T cell malignancy. The hematological cancer may be chosen from one or more of a Hodgkin's lymphoma, Non-Hodgkin's lymphoma (e.g., B cell lymphoma, diffuse large B cell lymphoma, follicular lymphoma, chronic lymphocytic leukemia, mantle cell lymphoma, marginal zone B-cell lymphoma, Burkitt lymphoma, lymphoplasmacytic lymphoma, hairy cell leukemia), acute myeloid leukemia (AML), chronic myeloid leukemia, myelodysplastic syndrome (MDS), multiple myeloma, or acute lymphocytic leukemia. The cancer may be other than acute myeloid leukemia (AML) or myelodysplastic syndrome (MDS). The hematological antigen is chosen from CD19, CD33, CD47, CD123, CD20, CD99, CD30, BCMA, CD38, CD22, SLAMF7, or NY-ESO1.

### Cytokine Molecules

Cytokines are proteinaceous signaling compounds that are mediators of the immune response. They control many different cellular functions including proliferation, differentiation and cell survival/apoptosis; cytokines are also involved in several pathophysiological processes including viral infections and autoimmune diseases. Cytokines are synthesized under various stimuli by a variety of cells of both the innate (monocytes, macrophages, dendritic cells) and adaptive (T- and B-cells) immune systems. Cytokines can be classified into two groups: pro- and anti-inflammatory. Pro-inflammatory cytokines, including e.g., IFNgamma, IL-1, IL-6 and TNF-alpha, are predominantly derived from the innate immune cells and Th1 cells. Anti-inflammatory cytokines, including e.g., IL-10, IL-4, IL-13 and IL-5, are synthesized from Th2 immune cells.

The present disclosure provides, *inter alia,* multi-specific (e.g., bi-, tri-, quad- specific) proteins, that include, e.g., are engineered to contain, one or more cytokine molecules, e.g., immunomodulatory (e.g., proinflammatory) cytokines and variants, e.g., functional variants, thereof. Accordingly, the cytokine molecule may be an interleukin or a variant, e.g., a functional variant thereof. The cytokine molecule may include a full length, a fragment or a variant of a cytokine; a cytokine receptor domain, e.g., a cytokine receptor dimerizing domain; or an agonist of a cytokine receptor, e.g., an antibody molecule (e.g., an agonistic antibody) to a cytokine receptor.

The cytokine molecule is chosen from IL-2, IL-7, IL-12, IL-15, IL-18, IL-21, or interferon gamma, or a fragment or variant thereof, or a combination of any of the aforesaid cytokines. The cytokine molecule can be a monomer or a dimer. The cytokine molecule may further include a cytokine receptor dimerizing domain. The cytokine molecule may be an agonist of a cytokine receptor, e.g., an antibody molecule (e.g., an agonistic antibody) to a cytokine receptor chosen from an IL-15Ra or IL-21R.

The cytokine molecule may be IL-15, e.g., human IL-15 (e.g., comprising the amino acid sequence:
NWVNVISDLKKIEDLIQSMHIDATLYTESDVHPSCKVTAMKCFLLELQVISLESGDASIH DTVENLIILANNSLSSNGNVTESGCKECEELEEKNIKEFLQSFVHIVQMFINTS (SEQ ID NO: 5), a fragment thereof, or an amino acid sequence substantially identical thereto (e.g., 95% to 99.9% identical thereto, or having at least one amino acid alteration, but not more than five, ten or fifteen alterations (*e.g*., substitutions, deletions, or insertions, *e.g.*, conservative substitutions) to the amino acid sequence of SEQ ID NO: 5.

The cytokine molecule may comprise a receptor dimerizing domain, e.g., an IL15Ralpha dimerizing domain. The IL15Ralpha dimerizing domain may comprise the amino acid sequence: MAPRRARGCRTLGLPALLLLLLLRPPATRGITCPPPMSVEHADIWVKSYSLYSRERYICN SGFKRKAGTSSLTECVL (SEQ ID NO: 6), a fragment thereof, or an amino acid sequence substantially identical thereto (e.g., 95% to 99.9% identical thereto, or having at least one amino acid alteration, but not more than five, ten or fifteen alterations (e.g., substitutions, deletions, or insertions, e.g., conservative substitutions) to the amino acid sequence of SEQ ID NO: 6. The cytokine molecule (e.g., IL-15) and the receptor dimerizing domain (e.g., an IL15Ralpha dimerizing domain) of the multispecific molecule may be covalently linked, e.g., via a linker (e.g., a Gly-Ser linker, e.g., a linker comprising the amino acid sequence SGGSGGGGSGGGSGGGGSLQ (SEQ ID NO: 7). The cytokine molecule (e.g., IL-15) and the receptor dimerizing domain (e.g., an IL15Ralpha dimerizing domain) of the multispecific molecule may not be covalently linked, e.g., are non-covalently associated.

The cytokine molecule may be IL-2, e.g., human IL-2 (e.g., comprising the amino acid sequence:
APTSSSTKKTQLQLEHLLLDLQMILNGINNYKNPKLTRMLTFKFYMPKKATELKHLQCL EEELKPLEEVLNLAQSKNFHLRPRDLISNINVIVLELKGSETTFMCEYADETATIVEFLNR WITFCQSIISTLT (SEQ ID NO: 8), a fragment thereof, or an amino acid sequence substantially identical thereto (e.g., 95% to 99.9% identical thereto, or having at least one amino acid alteration, but not more than five, ten or fifteen alterations (*e.g*., substitutions, deletions, or insertions, *e.g*., conservative substitutions) to the amino acid sequence of SEQ ID NO: 8).

The cytokine molecule may be IL-18, e.g., human IL-18 (e.g., comprising the amino acid sequence:
YFGKLESKLSVIRNLNDQVLFIDQGNRPLFEDMTDSDCRDNAPRTIFIISMYKDSQPRGM AVTISVKCEKISTLSCENKIISFKEMNPPDNIKDTKSDIIFFQRSVPGHDNKMQFESSSY EGYFLACEKERDLFKLILKKEDELGDRSΠVIFTVQNED (SEQ ID NO: 9), a fragment thereof, or an amino acid sequence substantially identical thereto (e.g., 95% to 99.9% identical thereto, or having at least one amino acid alteration, but not more than five, ten or fifteen alterations (*e.g*., substitutions, deletions, or insertions, *e.g*., conservative substitutions) to the amino acid sequence of SEQ ID NO: 9).

The cytokine molecule may be IL-21, e.g., human IL-21 (e.g., comprising the amino acid sequence:
QGQDRHMIRMRQLIDIVDQLKNYVNDLVPEFLPAPEDVETNCEWSAFSCFQKAQLKSA NTGNNERIINVSIKKLKRKPPSTNAGRRQKHRLTCPSCDSYEKKPPKEFLERFKSLLQKMI HQHLSSRTHGSEDS (SEQ ID NO: 10), a fragment thereof, or an amino acid sequence substantially identical thereto (e.g., 95% to 99.9% identical thereto, or having at least one amino acid alteration, but not more than five, ten or fifteen alterations (*e.g*., substitutions, deletions, or insertions, *e.g*., conservative substitutions) to the amino acid sequence of SEQ ID NO: 10).

The cytokine molecule may be interferon gamma, e.g., human interferon gamma (e*.*g., comprising the amino acid sequence:
QDPYVKEAENLKKYFNAGHSDVADNGTLFLGILKNWKEESDRKIMQSQIVSFYFKLFK NFKDDQSIQKSVETIKEDMNVKFFNSNKKKRDDFEKLTNYSVTDLNVQRKAIHELIQVM AELSPAAKTGKRKRSQMLFRG (SEQ ID NO: 11), a fragment thereof, or an amino acid sequence substantially identical thereto (e.g., 95% to 99.9% identical thereto, or having at least one amino acid alteration, but not more than five, ten or fifteen alterations (*e.g.*, substitutions, deletions, or insertions, *e.g.*, conservative substitutions) to the amino acid sequence of SEQ ID NO: 11).

### Immune Cell Engagers

The immune cell engagers of the multispecific molecules disclosed herein can mediate binding to, and/or activation of, an immune cell, e.g., an immune effector cell The immune cell may be chosen from an NK cell, a B cell, a dendritic cell, or a macrophage cell engager, or a combination thereof. The immune cell engager may be chosen from one, two, three, or all of an NK cell engager, a B cell engager, a dendritic cell engager, or a macrophage cell engager, or a combination thereof. The immune cell engager can be an agonist of the immune system. The immune cell engager may be an antibody molecule, a ligand molecule (e.g., a ligand that further comprises an immunoglobulin constant region, e.g., an Fc region), a small molecule, a nucleotide molecule.

### T Cell Engagers

The present disclosure provides, inter alia, multispecific (e.g., bi-, tri-, quad- specific) or multifunctional molecules, that are engineered to contain one or more T cell engagers that mediate binding to and/or activation of a T cell. Accordingly the T cell engager is selected from an antigen binding domain or ligand that binds to (e.g., and may activate) one or more of CD3, TCRα, TCRβ, TCRγ, TCRζ, ICOS, CD28, CD27, HVEM, LIGHT, CD40, 4-1BB, OX40, DR3, GITR, CD30, TIM1, SLAM, CD2, or CD226The T cell engager may be selected from an antigen binding domain or ligand that binds to and does not activate one or more of CD3, TCRα, TCRβ, TCRγ, TCRζ, ICOS, CD28, CD27, HVEM, LIGHT, CD40, 4-1BB, OX40, DR3, GITR, CD30, TIM1, SLAM, CD2, or CD226. The T cell engager may bind to CD3.

### Natural Killer Cell Engagers

Natural Killer (NK) cells recognize and destroy tumors and virus-infected cells in an antibody-independent manner. The regulation of NK cells is mediated by activating and inhibiting receptors on the NK cell surface. One family of activating receptors is the natural cytotoxicity receptors (NCRs) which include NKp30, NKp44 and NKp46. The NCRs initiate tumor targeting by recognition of heparan sulfate on cancer cells. NKG2D is a receptor that provides both stimulatory and costimulatory innate immune responses on activated killer (NK) cells, leading to cytotoxic activity. DNAM1 is a receptor involved in intercellular adhesion, lymphocyte signaling, cytotoxicity and lymphokine secretion mediated by cytotoxic T-lymphocyte (CTL) and NK cell. DAP10 (also known as HCST) is a transmembrane adapter protein which associates with KLRK1 to form an activation receptor KLRK1-HCST in lymphoid and myeloid cells; this receptor plays a major role in triggering cytotoxicity against target cells expressing cell surface ligands such as MHC class I chain-related MICA and MICB, and U(optionally L1)6-binding proteins (ULBPs); it KLRK1-HCST receptor plays a role in immune surveillance against tumors and is required for cytolysis of tumors cells; indeed, melanoma cells that do not express KLRK1 ligands escape from immune surveillance mediated by NK cells. CD16 is a receptor for the Fc region of IgG, which binds complexed or aggregated IgG and also monomeric IgG and thereby mediates antibody-dependent cellular cytotoxicity (ADCC) and other antibody-dependent responses, such as phagocytosis.

The NK cell engager may be a viral hemagglutinin (HA), HA is a glycoprotein found on the surface of influenza viruses. It is responsible for binding the virus to cells with sialic acid on the membranes, such as cells in the upper respiratory tract or erythrocytes. HA has at least 18 different antigens. These subtypes are named H1 through H18. NCRs can recognize viral proteins. NKp46 has been shown to be able to interact with the HA of influenza and the HA-NA of Paramyxovirus, including Sendai virus and Newcastle disease virus. Besides NKp46, NKp44 can also functionally interact with HA of different influenza subtypes.

The present disclosure provides, *inter alia,* multi-specific (e.g., bi-, tri-, quad- specific) proteins, that are engineered to contain one or more NK cell engager that mediate binding to and/or activation of an NK cell. Accordingly, in some embodiments, the NK cell engager is selected from an antigen binding domain or ligand that binds to (e.g., activates): NKp30, NKp40, NKp44, NKp46, NKG2D, DNAM1, DAP10, CD16 (e.g., CD16a, CD16b, or both), CRTAM, CD27, PSGL1, CD96, CD100 (SEMA4D), NKp80, CD244 (also known as SLAMF4 or 2B4), SLAMF6, SLAMF7, KIR2DS2, KIR2DS4, KIR3DS1, KIR2DS3, KIR2DS5, KIR2DS1, CD94, NKG2C, NKG2E, or CD160.

The NK cell engager may be a ligand of NKp44 or NKp46, which is a viral HA. Viral hemagglutinins (HA) are glyco proteins which are on the surface of viruses. HA proteins allow viruses to bind to the membrane of cells via sialic acid sugar moieties which contributes to the fusion of viral membranes with the cell membranes (see e.g., Eur J Immunol. 2001 Sep;31(9):2680-9 "Recognition of viral hemagglutinins by NKp44 but not by NKp30"; and Nature. 2001 Feb 22;409(6823):1055-60 "Recognition of haemagglutinins on virus-infected cells by NKp46 activates lysis by human NK cells").

The NK cell engager may be a ligand of DAP10, which is an adapter for NKG2D (see e.g., Proc Natl Acad Sci U S A. 2005 May 24; 102(21): 7641-7646; and Blood, 15 September 2011 Volume 118, Number 11).

The NK cell engager may be a ligand of CD16, which is a CD16a/b ligand, e.g., a CD16a/b ligand further comprising an antibody Fc region (see e.g., Front Immunol. 2013; 4: 76 discusses how antibodies use the Fc to trigger NK cells through CD16).

### B Cell, Macrophage & Dendritic Cell Engagers

Broadly, B cells, also known as B lymphocytes, are a type of white blood cell of the lymphocyte subtype. They function in the humoral immunity component of the adaptive immune system by secreting antibodies. Additionally, B cells present antigen (they are also classified as professional antigen-presenting cells (APCs)) and secrete cytokines. Macrophages are a type of white blood cell that engulfs and digests cellular debris, foreign substances, microbes, cancer cells via phagocytosis. Besides phagocytosis, they play important roles in nonspecific defense (innate immunity) and also help initiate specific defense mechanisms (adaptive immunity) by recruiting other immune cells such as lymphocytes. For example, they are important as antigen presenters to T cells. Beyond increasing inflammation and stimulating the immune system, macrophages also play an important anti-inflammatory role and can decrease immune reactions through the release of cytokines. Dendritic cells (DCs) are antigen-presenting cells that function in processing antigen material and present it on the cell surface to the T cells of the immune system.

The present disclosure provides, *inter alia,* multi-specific (e.g., bi-, tri-, quad- specific) proteins, that include, e.g., are engineered to contain, one or more B cell, macrophage, and/or dendritic cell engager that mediate binding to and/ or activation of a B cell, macrophage, and/or dendritic cell.

Accordingly, in some embodiments, the immune cell engager comprises a B cell, macrophage, and/or dendritic cell engager chosen from one or more of CD40 ligand (CD40L) or a CD70 ligand; an antibody molecule that binds to CD40 or CD70; an antibody molecule to OX40; an OX40 ligand (OX40L); an agonist of a Toll-like receptor (e.g., as described herein, e.g., a TLR4, e.g., a constitutively active TLR4 (caTLR4), or a TLR9 agonists); a 41BB; a CD2; a CD47; or a STING agonist, or a combination thereof.

In some embodiments, the B cell engager is a CD40L, an OX40L, or a CD70 ligand, or an antibody molecule that binds to OX40, CD40 or CD70.

In some embodiments, the macrophage engager is a CD2 agonist. In some embodiments, the macrophage engager is an antigen binding domain that binds to: CD40L or antigen binding domain or ligand that binds CD40, a Toll like receptor (TLR) agonist (e.g., as described herein), e.g., a TLR9 or TLR4 (e.g., caTLR4 (constitutively active TLR4), CD47, or a STING agonist. In some embodiments, the STING agonist is a cyclic dinucleotide, e.g., cyclic di-GMP (cdGMP) or cyclic di-AMP (cdAMP). In some embodiments, the STING agonist is biotinylated.

In some embodiments, the dendritic cell engager is a CD2 agonist. In some embodiments, the dendritic cell engager is a ligand, a receptor agonist, or an antibody molecule that binds to one or more of: OX40L, 41BB, a TLR agonist (e.g., as described herein) (e.g., TLR9 agonist, TLR4 (e.g., caTLR4 (constitutively active TLR4)), CD47, or and a STING agonist. The STING agonist may be a cyclic dinucleotide, e.g., cyclic di-GMP (cdGMP) or cyclic di-AMP (cdAMP). The STING agonist may be biotinylated.

The immune cell engager may mediate binding to, or activation of, one or more of a B cell, a macrophage, and/or a dendritic cell. Exemplary B cell, macrophage, and/or dendritic cell engagers can be chosen from one or more of CD40 ligand (CD40L) or a CD70 ligand; an antibody molecule that binds to CD40 or CD70; an antibody molecule to OX40; an OX40 ligand (OX40L); a Toll-like receptor agonist (e.g., a TLR4, e.g., a constitutively active TLR4 (caTLR4) or a TLR9 agonist); a 41BB agonist; a CD2; a CD47; or a STING agonist, or a combination thereof.

In some embodiments, the B cell engager is chosen from one or more of a CD40L, an OX40L, or a CD70 ligand, or an antibody molecule that binds to OX40, CD40 or CD70.

In other embodiments, the macrophage cell engager is chosen from one or more of a CD2 agonist; a CD40L; an OX40L; an antibody molecule that binds to OX40, CD40 or CD70; a Toll-like receptor agonist or a fragment thereof (e.g., a TLR4, e.g., a constitutively active TLR4 (caTLR4)); a CD47 agonist; or a STING agonist.

In other embodiments, the dendritic cell engager is chosen from one or more of a CD2 agonist, an OX40 antibody, an OX40L, 41BB agonist, a Toll-like receptor agonist or a fragment thereof (e.g., a TLR4, e.g., a constitutively active TLR4 (caTLR4)), CD47 agonist, or a STING agonist.

### Toll-Like Receptors

Toll-Like Receptors (TLRs) are evolutionarily conserved receptors are homologues of the Drosophila Toll protein, and recognize highly conserved structural motifs known as pathogenassociated microbial patterns (PAMPs), which are exclusively expressed by microbial pathogens, or danger-associated molecular patterns (DAMPs) that are endogenous molecules released from necrotic or dying cells. PAMPs include various bacterial cell wall components such as lipopolysaccharide (LPS), peptidoglycan (PGN) and lipopeptides, as well as flagellin, bacterial DNA and viral double-stranded RNA. DAMPs include intracellular proteins such as heat shock proteins as well as protein fragments from the extracellular matrix. Stimulation of TLRs by the corresponding PAMPs or DAMPs initiates signaling cascades leading to the activation of transcription factors, such as AP-1, NF-κB and interferon regulatory factors (IRFs). Signaling by TLRs results in a variety of cellular responses, including the production of interferons (IFNs), pro-inflammatory cytokines and effector cytokines that direct the adaptive immune response. TLRs are implicated in a number of inflammatory and immune disorders and play a role in cancer (Rakoff-Nahoum S. & Medzhitov R., 2009. Toll-like receptors and cancer. Nat Revs Cancer 9:57- 63.)

TLR3, TLR7, TLR8 and TLR9 recognize viral nucleic acids and induce type I IFNs. The signaling mechanisms leading to the induction of type I IFNs differ depending on the TLR activated. They involve the interferon regulatory factors, IRFs, a family of transcription factors known to play a critical role in antiviral defense, cell growth and immune regulation. Three IRFs (IRF3, IRF5 and IRF7) function as direct transducers of virus-mediated TLR signaling. TLR3 and TLR4 activate IRF3 and IRF7, while TLR7 and TLR8 activate IRF5 and IRF7 (Doyle S. et al., 2002. IRF3 mediates a TLR3/TLR4-specific antiviral gene program. Immunity. 17(3):251-63). Furthermore, type IIFN production stimulated by TLR9 ligand CpG-A has been shown to be mediated by PI(3)K and mTOR (Costa-Mattioli M. & Sonenberg N. 2008. RAPping production of type I interferon in pDCs through mTOR. Nature Immunol. 9: 1097-1099).

### TLR Agonists

A TLR agonist can agonize one or more TLR, e.g., one or more of human TLR- 1, 2, 3, 4, 5, 6, 7, 8, 9, or 10. An adjunctive agent described herein may be a TLR agonist. The TLR agonist may specifically agonize human TLR-9. The TLR-9 agonist may be a CpG moiety. As used herein, a CpG moiety, is a linear dinucleotide having the sequence: 5'-C-phosphate-G-3', that is, cytosine and guanine separated by only one phosphate.

The CpG moiety may comprise at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or more CpG dinucleotides. The CpG moiety may consist of 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 CpG dinucleotides. The CpG moiety may have 1-5, 1-10, 1-20, 1-30, 1-40, 1-50, 5-10, 5-20, 5-30, 10-20, 10-30, 10-40, or 10-50 CpG dinucleotides.

The TLR-9 agonist may be a synthetic ODN (oligodeoxynucleotides). CpG ODNs are short synthetic single-stranded DNA molecules containing unmethylated CpG dinucleotides in particular sequence contexts (CpG motifs). CpG ODNs possess a partially or completely phosphorothioated (PS) backbone, as opposed to the natural phosphodiester (PO) backbone found in genomic bacterial DNA. There are three major classes of CpG ODNs: classes A, B and C, which differ in their immunostimulatory activities. CpG-A ODNs are characterized by a PO central CpG-containing palindromic motif and a PS-modified 3' poly-G string. They induce high IFN-α production from pDCs but are weak stimulators of TLR9-dependent NF-κB signaling and pro-inflammatory cytokine (e.g. IL-6) production. CpG-B ODNs contain a full PS backbone with one or more CpG dinucleotides. They strongly activate B cells and TLR9-dependent NF-κB signaling but weakly stimulate IFN-α secretion. CpG-C ODNs combine features of both classes A and B. They contain a complete PS backbone and a CpG-containing palindromic motif. C-Class CpG ODNs induce strong IFN-α production from pDC as well as B cell stimulation.

### Exemplary Multispecific Molecules

Described below are exemplary multispecific molecules of the present disclosure illustrated through specific embodiments.

The multispecific molecule may comprise
(a) a first polypeptide chain having the following configuration from N-terminus to C-terminus: a first portion of a first antigen binding moiety (ABM) (e.g., wherein the first ABM comprises a VH-CH1 of a first Fab molecule, that binds to, e.g., a cancer antigen, connected, optionally via a linker to, a first subunit of a heterodimerization domain (e.g., an immunoglobulin CH2 connected to a TCRα constant domain);
(b) a second polypeptide chain having the following configuration from N-terminus to C-terminus: a first portion of a second ABM (e.g., wherein the second ABM comprises a VH-CH1 of a second Fab molecule, that binds to, e.g., a cancer antigen, connected, optionally via a linker to, a second subunit of a heterodimerization domain (e.g., an immunoglobulin CH2 connected to a TCRβ constant domain);
(c) a third polypeptide having the following configuration from N-terminus to C-terminus: a second portion of the first ABM (e.g., a VL-CL of the first Fab, where the VL is of kappa subtype and binds to, e.g., a cancer antigen (e.g., the same cancer antigen bound by the VH-CH1 of the first Fab molecule);
(d) a fourth polypeptide having the following configuration from N-terminus to C-terminus: a second portion of the second antigen domain (e.g. a VL-CL of the second Fab, where the VL is of lambda subtype and binds to, e.g., a cancer antigen, (e.g., the same cancer antigen bound by the VH-CH1 of the second Fab molecule).

The molecule may be comprised of a heterodimeric, human immunoglobulin IgG1 Fc-TCR domain with one heavy chain with variable domain from ipilimumab wherein the protein sequence from the N-terminus to the C-terminus of one of the heavy chains is comprised of an Ig kappa signal peptide (underlined), the ipilimumab heavy chain containing the CH2-TCRα core: METDTLLLWVLLLWVPGSTGQVQLVESGGGWQPGRSLRLSCAASGFTFSSYTMHWVRQAPGKG LEWVTFISYDGNNKYYADSVKGRFTISRDNSKNTLYLQMNSLRAEDTAIYYCARTGWLGPFDYW GQGTLVTVSSASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKRVEPKSCDKTHTCPPCPAPELL GGPSVFLFPPKPKDTLMISRTPEVTCVUVDVSHEDPEVKFNWYVDGVEVHNAKTKPREEQYNST YRVVSVLTVLHQDWLNGKEYKCKVSNKALPAPIEKTISKAKPDIQNPDPAVYQLRDSKSSDKSV CLFTDFDSQTNVSQSKDSDVYITDKCVLDMRSMDFKSNSAVAWSNKSDFACANAFNNSIIPEDT FFPSPE (SEQ ID NO: 12), while the other heavy chain from the N-terminus to the C-terminus is comprised of an Ig kappa signal peptide (underlined), and the briakinumab heavy chain sequence containing the CH2-TCRβ core:
METDTLLLWVLLLWVPGSTGQVQLVESGGGWQPGRSLRLSCAASGETFSSYGMHWVRQAPGKG LEWVAFIRYDGSNKYYADSVKGRFTISRDNSKNTLYLQMNSLRAEDTAVYYCKTHGSHDNWGQG TMVTVSSASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKRVEPKSCDKTHTCPPCPAPELLGGP SVFLFPPKPKDTLMISRTPEVTCVVVDVSHEDPEVKFNWYVDGVEVHNAKTKPREEQYNSTYRV VSVLTVLHQDWLNGKEYKCKVSNKALPAPIEKTISKAKEDLNKVFPPEVALFEPSEAEISHTQK ATLVCLATGFYPDHVELSWWVNGKEVHSGVCTDPQPLKEQPALNDSRYALSSRLRVSATFWQDP RNHFRCQVQFYGLSEADEWTQARAKPVTQIVSAEAWGRAD (SEQ ID NO: 13). One of the light chains has the protein sequence from the N-terminus to the C-terminus is comprised of an Ig kappa signal peptide (underlined), and the ipilimumab light chain sequence (kappa):
METDTLLLWVLLLWVPGSTGEIVLTQSPGTLSLSPGERATLSCRASQSVGSSYLAWYQQKPGQA PRLLIYGAFSRATGIPDRFSGSGSGTDFTLTISRLEPEDFAVYYCQQYGSSPWTFGQGTKVEIK RTVAAPSVFIFPPSDEQLKSGTASWCLLNNFYPREAKVQWKVDNALQSGNSQESVTEQDSKDS TYSLSSTLTLSKADYEKHKVYACEVTHQGLSSPVTKSFNRGEC (SEQ ID NO: 14), while the other light chain from the N-terminus to the C-terminus is comprised of an Ig kappa signal peptide (underlined), and the briakinumab light chain sequence (lambda):

METDTLLLWVLLLWVPGSTGQSVLTQPPSVSGAPGQRVTISCSGSRSNIGSNTVKWYQQLPGTA
PKLLIYYNDQRPSGVPDRFSGSKSGTSASLAITGLQAEDEADYYCQSYDRYTHPALLFGTGTKV

TVLGQPKANPTVTLFPPSSEELQANKATLVCLISDFYPGAVTVAWKADGSPVKAGVETTKPSKQ
SNNKYAASSYLSLTPEQWKSHRSYSCQVTHEGSTVEKTVAPTECS (SEQ ID NO: 15).

### Nucleic Acids

The invention also features nucleic acids comprising nucleotide sequences that encode heavy and light chain variable regions and CDRs or hypervariable loops of the antibody molecules, as described herein. For example, a first and second nucleic acid encoding heavy and light chain variable regions, respectively, of an antibody molecule chosen from one or more of the antibody molecules disclosed herein. The nucleic acid can comprise a nucleotide sequence as set forth in the tables herein, or a sequence substantially identical thereto (*e.g*., a sequence at least about 85%, 90%, 95%, 99% or more identical thereto, or which differs by no more than 3, 6, 15, 30, or 45 nucleotides from the sequences shown in the tables herein.

The nucleic acid may comprise a nucleotide sequence encoding at least one, two, or three CDRs or hypervariable loops from a heavy chain variable region having an amino acid sequence as set forth in the tables herein, or a sequence substantially homologous thereto (*e.g*., a sequence at least about 85%, 90%, 95%, 99% or more identical thereto, and/or having one or more substitutions, *e.g*., conserved substitutions). The nucleic acid may comprise a nucleotide sequence encoding at least one, two, or three CDRs or hypervariable loops from a light chain variable region having an amino acid sequence as set forth in the tables herein, or a sequence substantially homologous thereto (*e.g*., a sequence at least about 85%, 90%, 95%, 99% or more identical thereto, and/or having one or more substitutions, *e.g*., conserved substitutions). The nucleic acid may comprise a nucleotide sequence encoding at least one, two, three, four, five, or six CDRs or hypervariable loops from heavy and light chain variable regions having an amino acid sequence as set forth in the tables herein, or a sequence substantially homologous thereto (*e.g*., a sequence at least about 85%, 90%, 95%, 99% or more identical thereto, and/or having one or more substitutions, *e.g*., conserved substitutions).

The nucleic acid may comprise a nucleotide sequence encoding at least one, two, or three CDRs or hypervariable loops from a heavy chain variable region having the nucleotide sequence as set forth in the tables herein, a sequence substantially homologous thereto (*e.g*., a sequence at least about 85%, 90%, 95%, 99% or more identical thereto, and/or capable of hybridizing under the stringency conditions described herein). The nucleic acid may comprise a nucleotide sequence encoding at least one, two, or three CDRs or hypervariable loops from a light chain variable region having the nucleotide sequence as set forth in the tables herein, or a sequence substantially homologous thereto (*e.g*., a sequence at least about 85%, 90%, 95%, 99% or more identical thereto, and/or capable of hybridizing under the stringency conditions described herein). The nucleic acid may comprise a nucleotide sequence encoding at least one, two, three, four, five, or six CDRs or hypervariable loops from heavy and light chain variable regions having the nucleotide sequence as set forth in the tables herein, or a sequence substantially homologous thereto (*e.g*., a sequence at least about 85%, 90%, 95%, 99% or more identical thereto, and/or capable of hybridizing under the stringency conditions described herein).

In another aspect, the application features host cells and vectors containing the nucleic acids described herein. The nucleic acids may be present in a single vector or separate vectors present in the same host cell or separate host cell, as described in more detail herein below.

### Vectors

Further provided herein are vectors comprising the nucleotide sequences encoding an antibody molecule described herein. The vectors may comprise nucleotides encoding an antibody molecule described herein. The vectors of the invention comprise the nucleotide sequences described herein. The vectors include, but are not limited to, a virus, plasmid, cosmid, lambda phage or a yeast artificial chromosome (YAC).

Numerous vector systems can be employed. For example, one class of vectors utilizes DNA elements which are derived from animal viruses such as, for example, bovine papilloma virus, polyoma virus, adenovirus, vaccinia virus, baculovirus, retroviruses (Rous Sarcoma Virus, MMTV or MOMI,V) or SV40 virus. Another class of vectors utilizes RNA elements derived from RNA viruses such as Semliki Forest virus, Eastern Equine Encephalitis virus and Flaviviruses.

Additionally, cells which have stably integrated the DNA into their chromosomes may be selected by introducing one or more markers which allow for the selection of transfected host cells. The marker may provide, for example, prototropy to an auxotrophic host, biocide resistance (*e.g*., antibiotics), or resistance to heavy metals such as copper, or the like. The selectable marker gene can be either directly linked to the DNA sequences to be expressed, or introduced into the same cell by cotransformation. Additional elements may also be needed for optimal synthesis of mRNA. These elements may include splice signals, as well as transcriptional promoters, enhancers, and termination signals.

Once the expression vector or DNA sequence containing the constructs has been prepared for expression, the expression vectors may be transfected or introduced into an appropriate host cell. Various techniques may be employed to achieve this, such as, for example, protoplast fusion, calcium phosphate precipitation, electroporation, retroviral transduction, viral transfection, gene gun, lipid based transfection or other conventional techniques. In the case of protoplast fusion, the cells are grown in media and screened for the appropriate activity.

Methods and conditions for culturing the resulting transfected cells and for recovering the antibody molecule produced are known to those skilled in the art, and may be varied or optimized depending upon the specific expression vector and mammalian host cell employed, based upon the present description.

### Cells

In another aspect, the application features host cells and vectors containing the nucleic acids described herein. The nucleic acids may be present in a single vector or separate vectors present in the same host cell or separate host cell. The host cell can be a eukaryotic cell, *e.g.,* a mammalian cell, an insect cell, a yeast cell, or a prokaryotic cell, *e.g.*, *E. coli.* For example, the mammalian cell can be a cultured cell or a cell line. Exemplary mammalian cells include lymphocytic cell lines (*e.g*., NSO), Chinese hamster ovary cells (CHO), COS cells, oocyte cells, and cells from a transgenic animal, e.g., mammary epithelial cell.

The invention also provides host cells comprising a nucleic acid encoding an antibody molecule as described herein.

The host cells may be genetically engineered to comprise nucleic acids encoding the antibody molecule.

The host cells may be genetically engineered by using an expression cassette. The phrase "expression cassette," refers to nucleotide sequences, which are capable of affecting expression of a gene in hosts compatible with such sequences. Such cassettes may include a promoter, an open reading frame with or without introns, and a termination signal. Additional factors necessary or helpful in effecting expression may also be used, such as, for example, an inducible promoter.

The invention also provides host cells comprising the vectors described herein.

The cell can be, but is not limited to, a eukaryotic cell, a bacterial cell, an insect cell, or a human cell. Suitable eukaryotic cells include, but are not limited to, Vero cells, HeLa cells, COS cells, CHO cells, HEK293 cells, BHK cells and MDCKII cells. Suitable insect cells include, but are not limited to, Sf9 cells.

### Methods of Manufacturing Multispecific Molecules

Provided herein are, *inter alia,* methods of producing the multispecific molecules (e.g., multispecific (e.g., bispecific) antibody molecules) described herein. Accordingly, provided herein are methods of generating multispecific (e.g., bispecific) molecules comprising a non-immunoglobulin dimerization domain (e.g., a naturally occurring dimerization domain, e.g., a T cell receptor (TCR) constant domain) (e.g., as described herein).

The multispecific (e.g., bispecific) molecules may be produced in a single cell.

Disclosed herein are methods of making, e.g., producing, the multispecific molecule (e.g., multispecific antibody molecule) comprising an immunoglobulin CH2 domain and a TCR constant domain, comprising (a) generating a first antibody (e.g., a human antibody) comprising (i) a first heavy chain comprising a CH2 domain connected (optionally via a linker) to a first non-immunoglobulin dimerization domain (e.g., a naturally occurring dimerization domain, e.g., a TCRα constant domain) and (ii) a first light chain (e.g., a kappa light chain); (b) generating a second antibody (e.g., a human antibody) comprising a second heavy chain comprising a CH2 domain connected (optionally via a linker) to a second non-immunoglobulin dimerization domain (e.g., a naturally occurring dimerization domain, e.g., a TCRβ constant domain) and (ii) a second light chain (e.g., a lambda light chain), wherein the first and the second non-immunoglobulin dimerization domains are not the same; (c) transfecting a cell (or cells) with a nucleic acid encoding the amino acid sequence of the first antibody and the second antibody; (d) culturing the cell (or cells) under suitable conditions, e.g., conditions suitable for gene expression; (d) purifying the antibody (e.g., using Protein A); and (e) optionally determining the presence of the first and second heavy chain (e.g. via gel electrophoresis under reducing conditions); and (f) optionally determining the presence of correctly paired first and second heavy chains with the first and the second light chains, respectively (e.g., via mass spectrometry).

### Uses and Combination Therapies

The multispecific molecule described herein may be for use in a method of treating a cancer in a subject e.g., using a pharmaceutical composition described herein, the method comprising administering to the subject the multispecific molecule in an amount effective to treat the cancer. Also provided are methods for reducing or ameliorating a symptom of a cancer in a subject, as well as methods for inhibiting the growth of a cancer and/or killing one or more cancer cells. The methods described herein may decrease the size of a tumor and/or decrease the number of cancer cells in a subject administered with a described herein or a pharmaceutical composition described herein.

The cancer may be a hematological cancer. The hematological cancer may be a leukemia or a lymphoma. As used herein, a "hematologic cancer" refers to a tumor of the hematopoietic or lymphoid tissues, e.g., a tumor that affects blood, bone marrow, or lymph nodes. Exemplary hematologic malignancies include, but are not limited to, leukemia (*e.g*., acute lymphoblastic leukemia (ALL), acute myeloid leukemia (AML), chronic lymphocytic leukemia (CLL), chronic myelogenous leukemia (CML), hairy cell leukemia, acute monocytic leukemia (AMoL), chronic myelomonocytic leukemia (CMML), juvenile myelomonocytic leukemia (JMML), or large granular lymphocytic leukemia), lymphoma (*e.g.*, AIDS-related lymphoma, cutaneous T-cell lymphoma, Hodgkin lymphoma (*e.g*., classical Hodgkin lymphoma or nodular lymphocyte-predominant Hodgkin lymphoma), mycosis fungoides, non-Hodgkin lymphoma (*e.g*., B-cell non-Hodgkin lymphoma (*e.g*., Burkitt lymphoma, small lymphocytic lymphoma (CLL/SLL), diffuse large B-cell lymphoma, follicular lymphoma, immunoblastic large cell lymphoma, precursor B-lymphoblastic lymphoma, or mantle cell lymphoma) or T-cell non-Hodgkin lymphoma (mycosis fungoides, anaplastic large cell lymphoma, or precursor T-lymphoblastic lymphoma)), primary central nervous system lymphoma, Sézary syndrome, Waldenström macroglobulinemia), chronic myeloproliferative neoplasm, Langerhans cell histiocytosis, multiple myeloma/plasma cell neoplasm, myelodysplastic syndrome, or myelodysplastic/myeloproliferative neoplasm.

The cancer may be a solid cancer. Exemplary solid cancers include, but are not limited to, ovarian cancer, rectal cancer, stomach cancer, testicular cancer, cancer of the anal region, uterine cancer, colon cancer, rectal cancer, renal-cell carcinoma, liver cancer, non-small cell carcinoma of the lung, cancer of the small intestine, cancer of the esophagus, melanoma, Kaposi's sarcoma, cancer of the endocrine system, cancer of the thyroid gland, cancer of the parathyroid gland, cancer of the adrenal gland, bone cancer, pancreatic cancer, skin cancer, cancer of the head or neck, cutaneous or intraocular malignant melanoma, uterine cancer, brain stem glioma, pituitary adenoma, epidermoid cancer, carcinoma of the cervix squamous cell cancer, carcinoma of the fallopian tubes, carcinoma of the endometrium, carcinoma of the vagina, sarcoma of soft tissue, cancer of the urethra, carcinoma of the vulva, cancer of the penis, cancer of the bladder, cancer of the kidney or ureter, carcinoma of the renal pelvis, spinal axis tumor, neoplasm of the central nervous system (CNS), primary CNS lymphoma, tumor angiogenesis, metastatic lesions of said cancers, or combinations thereof.

The multispecific molecules (or pharmaceutical composition) may be administered in a manner appropriate to the disease to be treated or prevented. The quantity and frequency of administration will be determined by such factors as the condition of the patient, and the type and severity of the patient's disease. Appropriate dosages may be determined by clinical trials. For example, when "an effective amount" or "a therapeutic amount" is indicated, the precise amount of the pharmaceutical composition (or multispecific molecules) to be administered can be determined by a physician with consideration of individual differences in tumor size, extent of infection or metastasis, age, weight, and condition of the subject. The pharmaceutical composition described herein may be administered at a dosage of 10⁴ to 10⁹ cells/kg body weight, e.g., 10⁵ to 10⁶ cells/kg body weight, including all integer values within those ranges. The pharmaceutical composition described herein may be administered multiple times at these dosages. The pharmaceutical composition described herein may be administered using infusion techniques described in immunotherapy (see, *e.g*., Rosenberg et al., New Eng. J. of Med. 319:1676, 1988).

The multispecific molecules or pharmaceutical composition may be administered to the subject parenterally. The cells may be administered to the subject intravenously, subcutaneously, intratumorally, intranodally, intramuscularly, intradermally, or intraperitoneally. The cells may be administered, e.g., injected, directly into a tumor or lymph node. The cells are administered as an infusion (e.g., as described in Rosenberg et al., New Eng. J. of Med. 319:1676, 1988) or an intravenous push. The cells may be administered as an injectable depot formulation.
The subject may be a mammal. The subject may be a human, monkey, pig, dog, cat, cow, sheep, goat, rabbit, rat, or mouse. The subject may be a human. The subject may be a paediatric subject, *e.g*., less than 18 years of age, e.g., less than 17, 16, 15, 14, 13, 12, 11, 10, 9, 8, 7, 6, 5, 4, 3, 2, 1 or less years of age. The subject may be an adult, *e.g.*, at least 18 years of age, e.g., at least 19, 20, 21, 22, 23, 24, 25, 25-30, 30-35, 35-40, 40-50, 50-60, 60-70, 70-80, or 80-90 years of age.

### Combination Therapies

The multispecific molecules disclosed herein can be used in combination with a second therapeutic agent or procedure.

The multispecific molecule and the second therapeutic agent or procedure may be administered/performed after a subject has been diagnosed with a cancer, e.g., before the cancer has been eliminated from the subject. The multispecific molecule and the second therapeutic agent or procedure may be administered/performed simultaneously or concurrently. For example, the delivery of one treatment is still occurring when the delivery of the second commences, e.g., there is an overlap in administration of the treatments. The multispecific molecule and the second therapeutic agent or procedure may be administered/performed sequentially. For example, the delivery of one treatment ceases before the delivery of the other treatment begins.

Combination therapy may lead to more effective treatment than monotherapy with either agent alone. The combination of the first and second treatment may be more effective (e.g., leads to a greater reduction in symptoms and/or cancer cells) than the first or second treatment alone. The combination therapy may permit use of a lower dose of the first or the second treatment compared to the dose of the first or second treatment normally required to achieve similar effects when administered as a monotherapy. The combination therapy may have a partially additive effect, wholly additive effect, or greater than additive effect.

The multispecific molecule may be administered in combination with a therapy, *e.g*., a cancer therapy (*e.g*., one or more of anti-cancer agents, immunotherapy, photodynamic therapy (PDT), surgery and/or radiation). The terms "chemotherapeutic," "chemotherapeutic agent," and "anti-cancer agent" are used interchangeably herein. The administration of the multispecific molecule and the therapy, *e.g*., the cancer therapy, can be sequential (with or without overlap) or simultaneous. Administration of the multispecific molecule can be continuous or intermittent during the course of therapy (*e.g*., cancer therapy). Certain therapies described herein can be used to treat cancers and non-cancerous diseases. For example, PDT efficacy can be enhanced in cancerous and non-cancerous conditions (*e.g*., tuberculosis) using the methods and compositions described herein (reviewed in, *e.g.,* Agostinis, P. et al. (2011) CA CancerJ. Clin. 61:250-281).

### Anti-cancer therapies

The multispecific molecule may be administered in combination with a low or small molecular weight chemotherapeutic agent. Exemplary low or small molecular weight chemotherapeutic agents include, but not limited to, 13-cis-retinoic acid (isotretinoin, ACCUTANE^{®}), 2-CdA (2-chlorodeoxyadenosine, cladribine, LEUSTATIN^{™}), 5-azacitidine (azacitidine, VIDAZA^{®}), 5-fluorouracil (5-FU, fluorouracil, ADRUCIL^{®}), 6-mercaptopurine (6-MP, mercaptopurine, PURINETHOL^{®}), 6-TG (6-thioguanine, thioguanine, THIOGUANINE TABLOID^{®}), abraxane (paclitaxel protein-bound), actinomycin-D (dactinomycin, COSMEGEN^{®}), alitretinoin (PANRETIN^{®}), all-transretinoic acid (ATRA, tretinoin, VESANOID^{®}), altretamine (hexamethylmelamine, HMM, HFXALEN^{®}), amethopterin (methotrexate, methotrexate sodium, MTX, TREXALL^{™}, RHEUMATREX^{®}), amifostine (ETHYOL^{®}), arabinosylcytosine (Ara-C, cytarabine, CYTOSAR-U^{®}), arsenic trioxide (TRISENOX^{®}), asparaginase (Erwinia L-asparaginase, L-asparaginase, ELSPAR^{®}, KIDROLASE^{®}), BCNU (carmustine, BiCNU^{®}), bendamustine (TREANDA^{®}), bexarotene (TARGRETIN^{®}), bleomycin (BLENOXANE^{®}), busulfan (BUSULFEX^{®}, MYLERAN^{®}), calcium leucovorin (Citrovorum Factor, folinic acid, leucovorin), camptothecin-11 (CPT-11, irinotecan, CAMPTOSAR^{®}), capecitabine (XELODA^{®}), carboplatin (PARAPLATIN^{®}), carmustine wafer (prolifeprospan 20 with carmustine implant, GLIADEL^{®} wafer), CCI-779 (temsirolimus, TORISEL^{®}), CCNU (lomustine, CeeNU), CDDP (cisplatin, PLATINOL^{®}, PLATINOL-AQ^{®}), chlorambucil (leukeran), cyclophosphamide (CYTOXAN^{®}, NEOSAR^{®}), dacarbazine (DIC, DTIC, imidazole carboxamide, DTIC-DOME^{®}), daunomycin (daunorubicin, daunorubicin hydrochloride, rubidomycin hydrochloride, CERUBIDINE^{®}), decitabine (DACOGEN^{®}), dexrazoxane (ZINECARD^{®}), DHAD (mitoxantrone, NOVANTRONE^{®}), docetaxel (TAXOTERE^{®}), doxorubicin (ADRIAMYCIN^{®}, RUBEX^{®}), epirubicin (ELLENCE^{™}), estramustine (EMCYT^{®}), etoposide (VP-16, etoposide phosphate, TOPOSAR^{®}, VEPESID^{®}, ETOPOPHOS^{®}), floxuridine (FUDR^{®}), fludarabine (FLUDARA^{®}), fluorouracil (cream) (CARAC^{™}, EFUDEX^{®}, FLUOROPLEX^{®}), gemcitabine (GEMZAR^{®}), hydroxyurea (HYDREA^{®}, DROXIA^{™}, MYLOCEL^{™}), idarubicin (IDAMYCIN^{®}), ifosfamide (IFEX^{®}), ixabepilone (IXEMPRA^{™}), LCR (leurocristine, vincristine, VCR, ONCOVIN^{®}, VINCASARPFS^{®}), L-PAM (L-sarcolysin, melphalan, phenylalanine mustard, ALKERAN^{®}), mechlorethamine (mechlorethamine hydrochloride, mustine, nitrogen mustard, MUSTARGEN^{®}), mesna (MESNEX^{™}), mitomycin (mitomycin-C, MTC, MUTAMYCIN^{®}), nelarabine (ARRANON^{®}), oxaliplatin (ELOXATIN^{™}), paclitaxel (TAXOL^{®}, ONXAL^{™}), pegaspargase (PEG-L-asparaginase, ONCOSPAR^{®}), PEMETREXED (ALIMTA^{®}), pentostatin (NIPENT^{®}), procarbazine (MATULANE^{®}), streptozocin (ZANOSAR^{®}), temozolomide (TEMODAR^{®}), teniposide (VM-26, VUMON^{®}), TESPA (thiophosphoamide, thiotepa, TSPA, THIOPLEX^{®}), topotecan (HYCAMTIN^{®}), vinblastine (vinblastine sulfate, vincaleukoblastine, VLB, ALKABAN-AQ^{®}, VELBAN^{®}), vinorelbine (vinorelbine tartrate, NAVELBINE^{®}), and vorinostat (ZOLINZA^{®}).

The multispecific molecule may be administered in conjunction with a biologic. Biologics useful in the treatment of cancers are known in the art and a binding molecule of the invention may be administered, for example, in conjunction with such known biologics. For example, the FDA has approved the following biologics for the treatment of breast cancer: HERCEPTIN^{®} (trastuzumab, Genentech Inc., South San Francisco, Calif.; a humanized monoclonal antibody that has anti-tumor activity in HER2-positive breast cancer); FASLODEX^{®} (fulvestrant, AstraZeneca Pharmaceuticals, LP, Wilmington, Del.; an estrogenreceptor antagonist used to treat breast cancer); ARIMIDEX^{®} (anastrozole, AstraZeneca Pharmaceuticals, LP; a nonsteroidal aromatase inhibitor which blocks aromatase, an enzyme needed to make estrogen); Aromasin^{®} (exemestane, Pfizer Inc., New York, N.Y.; an irreversible, steroidal aromatase inactivator used in the treatment of breast cancer); FEMARA^{®} (letrozole, Novartis Pharmaceuticals, East Hanover, N.J.; a nonsteroidal aromatase inhibitor approved by the FDA to treat breast cancer); and NOLVADEX^{®} (tamoxifen, AstraZeneca Pharmaceuticals, LP; a nonsteroidal antiestrogen approved by the FDA to treat breast cancer). Other biologics with which the binding molecules of the invention may be combined include: AVASTIN^{®} (bevacizumab, Genentech Inc.; the first FDA-approved therapy designed to inhibit angiogenesis); and ZEVALIN^{®} (ibritumomab tiuxetan, Biogen Idee, Cambridge, Mass.; a radiolabeled monoclonal antibody currently approved for the treatment of B-cell lymphomas).

In addition, the FDA has approved the following biologics for the treatment of colorectal cancer: AVASTIN^{®}; ERBITUX^{®} (cetuximab, ImClone Systems Inc., New York, N.Y., and Bristol-Myers Squibb, New York, N.Y.; is a monoclonal antibody directed against the epidermal growth factor receptor (EGFR)); GLEEVEC^{®} (imatinib mesylate; a protein kinase inhibitor); and ERGAMISOL^{®} (levamisole hydrochloride, Janssen Pharmaceutica Products, LP, Titusville, N.J.; an immunomodulator approved by the FDA in 1990 as an adjuvant treatment in combination with 5-fluorouracil after surgical resection in patients with Dukes' Stage C colon cancer).

For the treatment of lung cancer, exemplary biologics include TARCEVA^{®} (erlotinib HCL, OSI Pharmaceuticals Inc., Melville, N.Y.; a small molecule designed to target the human epidermal growth factor receptor 1 (HER1) pathway).

For the treatment of multiple myeloma, exemplary biologics include VELCADE^{®} Velcade (bortezomib, Millennium Pharmaceuticals, Cambridge Mass.; a proteasome inhibitor). Additional biologics include THALIDOMID^{®} (thalidomide, Clegene Corporation, Warren, N.J.; an immunomodulatory agent and appears to have multiple actions, including the ability to inhibit the growth and survival of myeloma cells and anti-angiogenesis).

Additional exemplary cancer therapeutic antibodies include, but are not limited to, 3F8, abagovomab, adecatumumab, afutuzumab, alacizumab pegol, alemtuzumab (CAMPATH^{®}, MABCAMPATH^{®}), altumomab pentetate (HYBRI-CEAKER^{®}), anatumomab mafenatox, anrukinzumab (IMA-638), apolizumab, arcitumomab (CEA-SCAN^{®}), bavituximab, bectumomab (LYMPHOSCAN^{®}), belimumab (BENLYSTA^{®}, LYMPHOSTAT-B^{®}), besilesomab (SCINTIMUN^{®}), bevacizumab (AVASTIN^{®}), bivatuzumab mertansine, blinatumomab, brentuximab vedotin, cantuzumab mertansine, capromab pendetide (PROSTASCINT^{®}), catumaxomab (REMOVAB^{®}), CC49, cetuximab (C225, ERBITUX^{®}), citatuzumab bogatox, cixutumumab, clivatuzumab tetraxetan, conatumumab, dacetuzumab, denosumab (PROLIA^{®}), detumomab, ecromeximab, edrecolomab (PANOREX^{®}), elotuzumab, epitumomab cituxetan, epratuzumab, ertumaxomab (REXOMUN^{®}), etaracizumab, farletuzumab, figitumumab, fresolimumab, galiximab, gemtuzumab ozogamicin (MYLOTARG^{®}), girentuximab, glembatumumab vedotin, ibritumomab (ibritumomab tiuxetan, ZEVALIN^{®}), igovomab (INDIMACIS-125^{®}), intetumumab, inotuzumab ozogamicin, ipilimumab, iratumumab, labetuzumab (CEA-CIDE^{®}), lexatumumab, lintuzumab, lucatumumab, lumiliximab, mapatumumab, matuzumab, milatuzumab, minretumomab, mitumomab, nacolomab tafenatox, naptumomab estafenatox, necitumumab, nimotuzumab (THERACIM^{®}, THERALOC^{®}), nofetumomab merpentan (VERLUMA^{®}), ofatumumab (ARZERRA^{®}), olaratumab, oportuzumab monatox, oregovomab (OVAREX^{®}), panitumumab (VECTIBIX^{®}), pemtumomab (THERAGYN^{®}), pertuzumab (OMNITARG^{®}), pintumomab, pritumumab, ramucirumab, ranibizumab (LUCENTIS^{®}), rilotumumab, rituximab (MABTHERA^{®}, RITUXAN^{®}), robatumumab, satumomab pendetide, sibrotuzumab, siltuximab, sontuzumab, tacatuzumab tetraxetan (AFP-CIDE^{®}), taplitumomab paptox, tenatumomab, TGN1412, ticilimumab (tremelimumab), tigatuzumab, TNX-650, tositumomab (BEXXAR^{®}), trastuzumab (HERCEPTIN^{®}), tremelimumab, tucotuzumab celmoleukin, veltuzumab, volociximab, votumumab (HUMASPECT^{®}), zalutumumab (HUMAX-EGFR^{®}), and zanolimumab (HUMAX-CD4^{®}).

The multispecific molecule may be administered in combination with a viral cancer therapeutic agent. Exemplary viral cancer therapeutic agents include, but not limited to, vaccinia virus (wDD-CDSR), carcinoembryonic antigen-expressing measles virus, recombinant vaccinia virus (TK-deletion plus GM-CSF), Seneca Valley virus-001, Newcastle virus, coxsackie virus A21, GL-ONC1, EBNA1 C-terminal/LMP2 chimeric protein-expressing recombinant modified vaccinia Ankara vaccine, carcinoembryonic antigen-expressing measles virus, G207 oncolytic virus, modified vaccinia virus Ankara vaccine expressing p53, OncoVEX GM-CSF modified herpes-simplex 1 virus, fowlpox virus vaccine vector, recombinant vaccinia prostate-specific antigen vaccine, human papillomavirus 16/18 L1 virus-like particle/AS04 vaccine, MVA-EBNA1/LMP2 Inj. vaccine, quadrivalent HPV vaccine, quadrivalent human papillomavirus (types 6, 11, 16, 18) recombinant vaccine (GARDASIL^{®}), recombinant fowlpox-CEA(6D)/TRICOM vaccine; recombinant vaccinia-CEA(6D)-TRICOM vaccine, recombinant modified vaccinia Ankara-5T4 vaccine, recombinant fowlpox-TRICOM vaccine, oncolytic herpes virus NV1020, HPV L1 VLP vaccine V504, human papillomavirus bivalent (types 16 and 18) vaccine (CERVARIX^{®}), herpes simplex virus HF10, Ad5CMV-p53 gene, recombinant vaccinia DF3/MUC1 vaccine, recombinant vaccinia-MUC-1 vaccine, recombinant vaccinia-TRICOM vaccine, ALVAC MART-1 vaccine, replication-defective herpes simplex virus type I (HSV-1) vector expressing human Preproenkephalin (NP2), wild-type reovirus, reovirus type 3 Dearing (REOLYSIN^{®}), oncolytic virus HSV1716, recombinant modified vaccinia Ankara (MVA)-based vaccine encoding Epstein-Barr virus target antigens, recombinant fowlpoxprostate specific antigen vaccine, recombinant vaccinia prostate-specific antigen vaccine, recombinant vaccinia-B7.1 vaccine, rAd-p53 gene, Ad5-delta24RGD, HPV vaccine 580299, JX-594 (thymidine kinase-deleted vaccinia virus plus GM-CSF), HPV-16/18 L1/AS04, fowlpox virus vaccine vector, vaccinia-tyrosinase vaccine, MEDI-517 HPV-16/18 VLP AS04 vaccine, adenoviral vector containing the thymidine kinase of herpes simplex virus TK99UN, HspE7, FP253/Fludarabine, ALVAC(2) melanoma multi-antigen therapeutic vaccine, ALVAC-hB7.1, canarypox-hIL-12 melanoma vaccine, Ad-REIC/Dkk-3, rAd-IFN SCH 721015, TIL-Ad-INFg, Ad-ISF35, and coxsackievirus A21 (CVA21, CAVATAK^{®}).

The multispecific molecule may be administered in combination with a nanopharmaceutical. Exemplary cancer nanopharmaceuticals include, but not limited to, ABRAXANE^{®} (paclitaxel bound albumin nanoparticles), CRLX101 (CPT conjugated to a linear cyclodextrin-based polymer), CRLX288 (conjugating docetaxel to the biodegradable polymer poly (lactic-co-glycolic acid)), cytarabine liposomal (liposomal Ara-C, DEPOCYT^{™}), daunorubicin liposomal (DAUNOXOME^{®}), doxorubicin liposomal (DOXIL^{®}, CAELYX^{®}), encapsulated-daunorubicin citrate liposome (DAUNOXOME^{®}), and PEG anti-VEGF aptamer (MACUGEN^{®}).

The multispecific molecule may be administered in combination with paclitaxel or a paclitaxel formulation, e.g., TAXOL^{®}, protein-bound paclitaxel (e.g., ABRAXANE^{®}). Exemplary paclitaxel formulations include, but are not limited to, nanoparticle albumin-bound paclitaxel (ABRAXANE^{®}, marketed by Abraxis Bioscience), docosahexaenoic acid bound-paclitaxel (DHA-paclitaxel, Taxoprexin, marketed by Protarga), polyglutamate bound-paclitaxel (PG-paclitaxel, paclitaxel poliglumex, CT-2103, XYOTAX, marketed by Cell Therapeutic), the tumor-activated prodrug (TAP), ANG105 (Angiopep-2 bound to three molecules of paclitaxel, marketed by ImmunoGen), paclitaxel-EC-1 (paclitaxel bound to the erbB2-recognizing peptide EC-1; see Li et al., Biopolymers (2007) 87:225-230), and glucose-conjugated paclitaxel (*e.g*., 2'-paclitaxel methyl 2-glucopyranosyl succinate, see Liu et al., Bioorganic & Medicinal Chemistry Letters (2007) 17:617-620).

Exemplary RNAi and antisense RNA agents for treating cancer include, but not limited to, CALAA-01, siG12D LODER (Local Drug EluteR), and ALN-VSP02.

Other cancer therapeutic agents include, but not limited to, cytokines (e.g., aldesleukin (IL-2, Interleukin-2, PROLEUKIN^{®}), alpha Interferon (IFN-alpha, Interferon alfa, INTRON^{®} A (Interferon alfa-2b), ROFERON-A^{®} (Interferon alfa-2a)), Epoetin alfa (PROCRIT^{®}), filgrastim (G-CSF, Granulocyte - Colony Stimulating Factor, NEUPOGEN^{®}), GM-CSF (Granulocyte Macrophage Colony Stimulating Factor, sargramostim, LEUKINE^{™}), IL-11 (Interleukin-11, oprelvekin, NEUMEGA^{®}), Interferon alfa-2b (PEG conjugate) (PEG interferon, PEG-INTRON^{™}), and pegfilgrastim (NEULASTA^{™})), hormone therapy agents (e.g., aminoglutethimide (CYTADREN^{®}), anastrozole (ARIMIDEX^{®}), bicalutamide (CASODEX^{®}), exemestane (AROMASIN^{®}), fluoxymesterone (HALOTESTIN^{®}), flutamide (EULEXIN^{®}), fulvestrant (FASLODEX^{®}), goserelin (ZOLADEX^{®}), letrozole (FEMARA^{®}), leuprolide (ELIGARD^{™}, LUPRON^{®}, LUPRON DEPOT^{®}, VIADUR^{™}), megestrol (megestrol acetate, MEGACE^{®}), nilutamide (ANANDRON^{®}, NILANDRON^{®}), octreotide (octreotide acetate, SANDOSTATIN^{®}, SANDOSTATIN LAR^{®}), raloxifene (EVISTA^{®}), romiplostim (NPLATE^{®}), tamoxifen (NOVALDEX^{®}), and toremifene (FARESTON^{®})), phospholipase A2 inhibitors (e.g., anagrelide (AGRYLIN^{®})), biologic response modifiers (e.g., BCG (THERACYS^{®}, TICE^{®}), and Darbepoetin alfa (ARANESP^{®})), target therapy agents (e.g., bortezomib (VELCADE^{®}), dasatinib (SPRYCEL^{™}), denileukin diftitox (ONTAK^{®}), erlotinib (TARCEVA^{®}), everolimus (AFINITOR^{®}), gefitinib (IRESSA^{®}), imatinib mesylate (STI-571, GLEEVEC^{™}), lapatinib (TYKERB^{®}), sorafenib (NEXAVAR^{®}), and SU11248 (sunitinib, SUTENT^{®})), immunomodulatory and antiangiogenic agents (e.g., CC-5013 (lenalidomide, REVLIMID^{®}), and thalidomide (THALOMID^{®})), glucocorticosteroids (e.g., cortisone (hydrocortisone, hydrocortisone sodium phosphate, hydrocortisone sodium succinate, ALA-CORT^{®}, HYDROCORT ACETATE^{®}, hydrocortone phosphate LANACORT^{®}, SOLU-CORTEF^{®}), decadron (dexamethasone, dexamethasone acetate, dexamethasone sodium phosphate, DEXASONE^{®}, DIODEX^{®}, HEXADROL^{®}, MAXIDEX^{®}), methylprednisolone (6-methylprednisolone, methylprednisolone acetate, methylprednisolone sodium succinate, DURALONE^{®}, MEDRALONE^{®}, MEDROL^{®}, M-PREDNISOL^{®}, SOLU-MEDROL^{®}), prednisolone (DELTA-CORTEF^{®}, ORAPRED^{®}, PEDIAPRED^{®}, PRELONE^{®}), and prednisone (DELTASONE^{®}, LIQUID PRED^{®}, METICORTEN^{®}, ORASONE^{®})), and bisphosphonates (e.g., pamidronate (AREDIA^{®}), and zoledronic acid (ZOMETA^{®}))

The multispecific molecule may be used in combination with a tyrosine kinase inhibitor *(e.g.,* a receptor tyrosine kinase (RTK) inhibitor). Exemplary tyrosine kinase inhibitor include, but are not limited to, an epidermal growth factor (EGF) pathway inhibitor (e.g., an epidermal growth factor receptor (EGFR) inhibitor), a vascular endothelial growth factor (VEGF) pathway inhibitor (*e.g.,* an antibody against VEGF, a VEGF trap, a vascular endothelial growth factor receptor (VEGFR) inhibitor (*e.g.,* a VEGFR-1 inhibitor, a VEGFR-2 inhibitor, a VEGFR-3 inhibitor)), a platelet derived growth factor (PDGF) pathway inhibitor (*e.g.,* a platelet derived growth factor receptor (PDGFR) inhibitor (*e.g.,* a PDGFR-β inhibitor)), a RAF-1 inhibitor, a KIT inhibitor and a RET inhibitor. The anti-cancer agent used in combination with the AHCM agent may be selected from the group consisting of: axitinib (AG013736), bosutinib (SKI-606), cediranib (RECENTIN^{™}, AZD2171), dasatinib (SPRYCEL^{®}, BMS-354825), erlotinib (TARCEVA^{®}), gefitinib (IRESSA^{®}), imatinib (Gleevec^{®}, CGP57148B, STI-571), lapatinib (TYKERB^{®}, TYVERB^{®}), lestaurtinib (CEP-701), neratinib (HKI-272), nilotinib (TASIGNA^{®}), semaxanib (semaxinib, SU5416), sunitinib (SUTENT^{®}, SU11248), toceranib (PALLADIA^{®}), vandetanib (ZACTIMA^{®}, ZD6474), vatalanib (PTK787, PTK/ZK), trastuzumab (HERCEPTIN^{®}), bevacizumab (AVASTIN^{®}), rituximab (RITUXAN^{®}), cetuximab (ERBITUX^{®}), panitumumab (VECTIBIX^{®}), ranibizumab (Lucentis^{®}), nilotinib (TASIGNA^{®}), sorafenib (NEXAVAR^{®}), alemtuzumab (CAMPATH^{®}), gemtuzumab ozogamicin (MYLOTARG^{®}), ENMD-2076, PCI-32765, AC220, dovitinib lactate (TKI258, CHIR-258), BIBW 2992 (TOVOK^{™}), SGX523, PF-04217903, PF-02341066, PF-299804, BMS-777607, ABT-869, MP470, BIBF 1120 (VARGATEF^{®}), AP24534, JNJ-26483327, MGCD265, DCC-2036, BMS-690154, CEP-11981, tivozanib (AV-951), OSI-930, MM-121, XL-184, XL-647, XI,228, AEE788, AG-490, AST-6, BMS-599626, CUDC-101, PD153035, pelitinib (EKB-569), vandetanib (zactima), WZ3146, WZ4002, WZ8040, ABT-869 (linifanib), AEE788, AP24534 (ponatinib), AV-951(tivozanib), axitinib, BAY 73-4506 (regorafenib), brivanib alaninate (BMS-582664), brivanib (BMS-540215), cediranib (AZD2171), CHIR-258 (dovitinib), CP 673451, CYC116, E7080, Ki8751, masitinib (AB1010), MGCD-265, motesanib diphosphate (AMG-706), MP-470, OSI-930, Pazopanib Hydrochloride, PD173074,nSorafenib Tosylate(Bay 43-9006), SU 5402, TSU-68(SU6668), vatalanib, XI,880 (GSK1363089, EXEL-2880). Selected tyrosine kinase inhibitors are chosen from sunitinib, erlotinib, gefitinib, or sorafenib. The tyrosine kinase inhibitor may be sunitinib.

The multispecific molecule may be administered in combination with one of more of: an anti-angiogenic agent, or a vascular targeting agent or a vascular disrupting agent. Exemplary anti-angiogenic agents include, but are not limited to, VEGF inhibitors (*e.g*., anti-VEGF antibodies (*e.g.,* bevacizumab); VEGF receptor inhibitors (*e.g.,* itraconazole); inhibitors of cell proliferatin and/or migration of endothelial cells (*e.g.,* carboxyamidotriazole, TNP-470); inhibitors of angiogenesis stimulators (*e.g.,* suramin), among others. A vascular-targeting agent (VTA) or vascular disrupting agent (VDA) is designed to damage the vasculature (blood vessels) of cancer tumors causing central necrosis (reviewed in, *e.g.,* Thorpe, P.E. (2004) Clin. Cancer Res. Vol. 10:415-427). VTAs can be small-molecule. Exemplary small-molecule VTAs include, but are not limited to, microtubule destabilizing drugs (*e.g.,* combretastatin A-4 disodium phosphate (CA4P), ZD6126, AVE8062, Oxi 4503); and vadimezan (ASA404).

### Immune checkpoint inhibitors

Methods described herein may comprise use of an immune checkpoint inhibitor in combination with the multispecific molecule. The methods can be used in a therapeutic protocol *in vivo.*

An immune checkpoint inhibitor may inhibit a checkpoint molecule. Exemplary checkpoint molecules include but are not limited to CTLA4, PD1, PD-L1, PD-L2, TIM3, LAG3, CD160, 2B4, CD80, CD86, B7-H3 (CD276), B7-H4 (VTCN1), HVEM (TNFRSF14 or CD270), BTLA, KIR, MHC class I, MHC class II, GAL9, VISTA, BTLA, TIGIT, LAIR1, and A2aR. See, e.g., Pardoll. Nat. Rev. Cancer 12.4(2012):252-64.

The immune checkpoint inhibitor may be a PD-1 inhibitor, e.g., an anti-PD-1 antibody such as Nivolumab, Pembrolizumab or Pidilizumab. Nivolumab (also called MDX- 1106, MDX-1106-04, ONO-4538, or BMS-936558) is a fully human IgG4 monoclonal antibody that specifically inhibits PD1. See, e.g., US 8,008,449 and WO2006/121168. Pembrolizumab (also called Lambrolizumab, MK-3475, MK03475, SCH-900475 or KEYTRUDA^{®}; Merck) is a humanized IgG4 monoclonal antibody that binds to PD-1. See, e.g., Hamid, O. et al. (2013) New England Journal of Medicine 369 (2): 134-44, US 8,354,509 and WO2009/114335. Pidilizumab (also called CT-011 or Cure Tech) is a humanized IgGlk monoclonal antibody that binds to PD1. See, e.g., WO2009/101611. The inhibitor of PD-1 may be an antibody molecule having a sequence substantially identical or similar thereto, *e.g.,* a sequence at least 85%, 90%, 95% identical or higher to the sequence of Nivolumab, Pembrolizumab or Pidilizumab. Additional anti-PD1 antibodies, e.g., AMP 514 (Amplimmune), are described, e.g., in US 8,609,089, US 2010028330, and/or US 20120114649.

The PD-1 inhibitor may be an immunoadhesin, e.g., an immunoadhesin comprising an extracellular/PD-1 binding portion of a PD-1 ligand (e.g., PD-L1 or PD-L2) that is fused to a constant region *(e.g.,* an Fc region of an immunoglobulin). In embodiments, the PD-1 inhibitor is AMP-224 (B7-DCIg, *e.g.,* described in WO2011/066342 and WO2010/027827), a PD-L2 Fc fusion soluble receptor that blocks the interaction between B7-H1 and PD-1.

The immune checkpoint inhibitor may be a PD-L1 inhibitor, e.g., an antibody molecule. The PD-L1 inhibitor may be YW243.55.S70, MPDL3280A, MEDI-4736, MSB-0010718C, or MDX-1105. The anti-PD-L1 antibody may be MSB0010718C (also called A09-246-2; Merck Serono), which is a monoclonal antibody that binds to PD-L1. Exemplary humanized anti-PD-L1 antibodies are described, e.g., in WO2013/079174. The PD-L1 inhibitor may be an anti-PD-L1 antibody, e.g., YW243.55.S70. The YW243.55.S70 antibody is described, e.g., in WO 2010/077634. The PD-L1 inhibitor may be MDX-1105 (also called BMS-936559), which is described, e.g., in WO2007/005874. The PD-L1 inhibitor may be MDPL3280A (Genentech / Roche), which is a human Fc-optimized IgG1 monoclonal antibody against PD-L1. See, e.g., U.S. Patent No.: 7,943,743 and U.S Publication No.: 20120039906. The inhibitor of PD-L1 may be an antibody molecule having a sequence substantially identical or similar thereto, *e.g.,* a sequence at least 85%, 90%, 95% identical or higher to the sequence of YW243.55.S70, MPDL3280A, MEDI-4736, MSB-0010718C, or MDX-1105.

The immune checkpoint inhibitor may be a PD-L2 inhibitor, e.g., AMP-224 (which is a PD-L2 Fc fusion soluble receptor that blocks the interaction between PD1 and B7-H1. See, e.g., WO2010/027827 and WO2011/066342.

The immune checkpoint inhibitor may be a LAG-3 inhibitor, e.g., an anti-LAG-3 antibody molecule. In embodiments, the anti-LAG-3 antibody is BMS-986016 (also called BMS986016; Bristol-Myers Squibb). BMS-986016 and other humanized anti-LAG-3 antibodies are described, e.g., in US 2011/0150892, WO2010/019570, and WO2014/008218.

The immune checkpoint inhibitor may be a TIM-3 inhibitor, e.g., anti-TIM3 antibody molecule, e.g., described in U.S. Patent No.: 8,552,156, WO 2011/155607, EP 2581113 and U.S Publication No.: 2014/044728.

The immune checkpoint inhibitor may be a CTLA-4 inhibitor, e.g., anti-CTLA-4 antibody molecule. Exemplary anti-CTLA4 antibodies include Tremelimumab (IgG2 monoclonal antibody from Pfizer, formerly known as ticilimumab, CP-675,206); and Ipilimumab (also called MDX-010, CAS No. 477202-00-9). Other exemplary anti-CTLA-4 antibodies are described, *e.g.,* in U.S. Pat. No. 5,811,097.

### EXAMPLES

The following examples are intended to be illustrative, and are not meant in any way to be limiting.

### Example 1

### 1. Construction of the plasmids.

The DNA encoding the protein sequences was optimized for expression in *Cricetulus griseus,* synthesized, and cloned into the pcDNA3.4-TOPO (Life Technologies A14697) using Gateway cloning. All constructs contained an Ig Kappa leader sequence. The nucleic acid sequences used are shown in Table 1.

**Table 1. Nucleic acid sequences.**

| Sequence ID | Description | Nucleic Acid Sequence |
|---|---|---|
| SEQ ID NO: 16 | αCTLA4 ipilimumab VH | CAGGTGCAGCTGGTGGAATCTGGTGGCGGAGTTGTGCAGCCTGGCA GATCCCTGAGACTGTCTTGTGCCGCCTCCGGCTTCACCTTCTCCAG CTACACCATGCACTGGGTCCGACAGGCCCCTGGCAAAGGATTGGAG TGGGTCACCTTCATCTCTTACGACGGCAACAACAAGTACTACGCCG ACTCCGTGAAGGGCAGATTCACCATCTCTCGGGACAACTCCAAGAA CACCCTGTACCTGCAGATGAACTCCCTGAGAGCCGAGGACACCGCC ATCTACTACTGTGCTAGAACCGGCTGGCTGGGCCCCTTTGATTATT GGGGACAGGGCACCCTGGTCACCGTGTCCTCT |
| SEQ ID NO: 17 | αCTLA4 ipilimumab VL | GAGATCGTGCTGACCCAGTCTCCTGGCACACTGTCACTGTCTCCAG GCGAGAGAGCTACCCTGTCCTGTAGAGCCTCTCAGTCCGTGGGCTC CTCTTACCTGGCTTGGTATCAGCAGAAGCCCGGCCAGGCTCCTAGA CTGTTGATCTACGGCGCCTTCTCCAGAGCCACAGGCATCCCTGATA GATTCTCCGGCTCTGGCTCTGGCACCGACTTCACCCTGACCATCTC CAGACTGGAACCCGAGGACTTCGCCGTGTACTACTGTCAGCAGTAC GGCTCCTCTCCTTGGACCTTTGGCCAGGGCACCAAGGTGGAAATCA AG |
| SEQ ID NO: 18 | αIL12B briakinumab VH | CAGGTGCAGCTGGTGGAATCTGGTGGCGGAGTTGTGCAGCCTGGCA GATCCCTGAGACTGTCTTGTGCCGCCTCCGGCTTCACCTTCTCCTC TTACGGAATGCACTGGGTCCGACAGGCCCCTGGCAAAGGATTGGAG TGGGTCGCCTTCATCAGATACGACGGCTCCAACAAGTACTACGCCG ACTCCGTGAAGGGCAGATTCACCATCTCTCGGGACAACTCCAAGAA CACCCTGTACCTGCAGATGAACTCCCTGAGAGCCGAGGACACCGCC GTGTACTACTGCAAGACCCACGGCTCTCACGACAATTGGGGCCAGG GCACAATGGTCACCGTGTCCTCT |
| SEQ ID NO: 19 | αIL12B briakinumab VL | CAGTCCGTGTTGACCCAGCCTCCTTCTGTTTCTGGCGCTCCTGGCC AGAGAGTGACCATCTCTTGCTCCGGCTCTCGGTCCAACATCGGCTC CAATACCGTGAAGTGGTATCAGCAGCTGCCCGGCACAGCTCCCAAA CTGCTGATCTACTACAACGACCAGCGGCCTTCTGGCGTGCCCGATA GATTCTCTGGCTCCAAGTCTGGCACCTCTGCCAGCCTGGCTATTAC CGGACTGCAGGCTGAGGACGAGGCCGACTACTACTGCCAGTCTTAC GACCGGTACACCCATCCTGCTCTGCTGTTTGGCACCGGCACCAAAG TGACAGTGCTG |
| SEQ ID NO: 20 | hCL (kappa) | CGGACAGTGGCCGCTCCTTCCGTGTTCATCTTCCCACCTTCCGACG AGCAGCTGAAGTCCGGCACAGCTTCTGTCGTGTGCCTGCTGAACAA CTTCTACCCTCGGGAAGCCAAGGTGCAGTGGAAGGTGGACAATGCC CTGCAGTCCGGCAACTCCCAAGAGTCTGTGACCGAGCAGGACTCCA AGGACAGCACCTACAGCCTGTCCTCCACACTGACCCTGTCCAAGGC CGACTACGAGAAGCACAAGGTGTACGCCTGCGAAGTGACCCATCAG GGCCTGTCTAGCCCTGTGACCAAGTCTTTCAACCGGGGCGAGTGC |
| SEQ ID NO: 21 | hCL (lambda) | GGCCAGCCTAAGGCCAATCCTACCGTGACACTGTTCCCTCCATCCT CCGAAGAACTGCAGGCCAACAAGGCTACCCTCGTGTGCCTGATCTC CGACTTTTACCCTGGCGCTGTGACCGTGGCCTGGAAGGCTGATGGA TCTCCTGTGAAGGCTGGCGTGGAAACCACCAAGCCTTCCAAGCAGT CCAACAACAAATACGCCGCCTCCTCCTACCTGTCTCTGACCCCTGA ACAGTGGAAGTCCCACCGGTCCTACAGCTGCCAAGTGACCCATGAG GGCTCCACCGTGGAAAAGACCGTGGCTCCTACCGAGTGCTCC |
| SEQ ID NO: 22 | hCH1 | GCTTCCACCAAGGGACCCTCTGTGTTCCCTCTGGCTCCTTCCAGCA AGTCTACCTCTGGCGGAACAGCTGCTCTGGGCTGCCTGGTCAAGGA CTACTTTCCTGAGCCTGTGACCGTGTCTTGGAACTCTGGCGCTCTG ACATCCGGCGTGCACACCTTTCCAGCTGTGCTGCAATCCTCCGGCC TGTACTCTCTGTCCTCCGTCGTGACCGTGCCTTCTAGCTCTCTGGG CACCCAGACCTACATCTGCAATGTGAACCACAAGCCTTCCAACACC AAGGTGGACAAGAGAGTGGAACCCAAGTCCTGC |
| SEQ ID NO: 23 | hCH2 | GATAAGACCCACACCTGTCCACCATGTCCTGCTCCAGAACTGCTCG GCGGACCTTCCGTGTTCCTGTTTCCTCCAAAGCCTAAGGACACCCT GATGATCTCTCGGACCCCTGAAGTGACCTGCGTGGTGGTGGATGTG TCTCACGAGGATCCCGAAGTGAAGTTCAATTGGTACGTGGACGGCG TGGAAGTGCACAACGCCAAGACCAAGCCTAGAGAGGAACAGTACAA CTCCACCTACAGAGTGGTGTCCGTGCTGACCGTGCTGCACCAGGAT TGGCTGAACGGCAAAGAGTACAAGTGCAAGGTGTCCAACAAGGCCC TGCCTGCTCCTATCGAAAAGACCATCTCCAAGGCCAAA |
| SEQ ID NO: 24 | hTCRα | CCTGACATTCAGAACCCCGATCCTGCCGTGTACCAGCTGAGAGACT CCAAGTCCTCCGATAAGTCTGTGTGCCTGTTCACCGACTTCGACTC CCAGACCAACGTGTCCCAGTCCAAGGACTCCGACGTGTACATCACC GATAAGTGCGTGCTGGACATGCGGAGCATGGACTTCAAGTCTAACT CCGCCGTGGCCTGGTCTAACAAGTCCGATTTCGCCTGCGCCAACGC |
| | | CTTCAACAACAGCATTATCCCCGAGGACACATTCTTCCCATCACCT GAG |
| SEQ ID NO: 25 | hTCRβ | GAGGACCTGAACAAGGTTTTCCCACCTGAGGTGGCCCTGTTCGAGC CTTCTGAGGCTGAGATCTCTCACACCCAGAAAGCTACCCTCGTGTG TCTGGCCACCGGCTTCTACCCTGATCACGTGGAACTGTCTTGGTGG GTCAACGGAAAAGAGGTGCACTCCGGCGTCTGCACCGATCCTCAGC CTCTGAAAGAACAGCCCGCTCTGAACGACTCCAGATACGCCCTGTC CTCTCGGCTGAGAGTGTCTGCCACCTTCTGGCAGGACCCTCGGAAC CACTTTAGATGCCAGGTGCAGTTCTACGGCCTGTCCGAGGCTGATG AGTGGACCCAGGCTAGAGCCAAGCCAGTGACACAGATCGTGTCTGC CGAAGCCTGGGGCAGAGCCGAT |
| SEQ ID NO: 26 | G | GGT |
| SEQ ID NO: 27 | GG | GGTGGC |
| SEQ ID NO: 28 | GGG | GGTGGCGGA |
| SEQ ID NO: 29 | GGGG | GGTGGCGGAGGA |
| SEQ ID NO: 30 | GGGGS | GGTGGCGGAGGAAGC |
| SEQ ID NO: 31 | GGGGSG | GGTGGCGGAGGAAGCGGT |
| SEQ ID NO: 32 | GGGGSGG | GGTGGCGGAGGAAGCGGTGGC |
| SEQ ID NO: 33 | GGGGSGGG | GGTGGCGGAGGAAGCGGTGGCGGC |
| SEQ ID NO: 34 | GGGGSGGGG | GGTGGCGGAGGAAGCGGTGGCGGCGGA |
| SEQ ID NO: 35 | GGGGSGGGGS | GGTGGCGGAGGAAGCGGTGGCGGCGGATCT |
| SEQ ID NO: 36 | mCH2 | ACCATCAAGCCTTGTCCTCCATGCAAGTGCCCCGCTCCTAATC TGCTCGGAGGCCCTTCCGTGTTCATCTTCCCACCTAAGATCAA GGACGTGCTGATGATCTCCCTGTCTCCTATCGTGACCTGCGTG GTGGTGGACGTGTCCGAGGATGATCCTGACGTGCAGATCAGTT GGTTCGTGAACAACGTGGAAGTGCACACCGCTCAGACCCAGAC ACACAGAGAGGACTACAACAGCACCCTGAGAGTGGTGTCTGCC CTGCCTATCCAGCACCAGGATTGGATGTCCGGCAAAGAATTCA AGTGCAAAGTGAACAACAAGGACCTGCCTGCTCCAATCGAGCG GACCATCTCCAAGCCT |
| SEQ ID NO: 37 | mTCRα | AAGCCTAACATCCAGAATCCTGAGCCTGCCGTGTACCAGCTGA AGGACCCTAGATCTCAGGACTCTACCCTGTGCCTGTTCACCGA CTTCGACTCCCAGATCAACGTGCCCAAGACCATGGAATCCGGC ACCTTCATCACCGATAAGTGCGTGCTGGACATGAAGGCCATGG ACTCCAAGTCCAACGGCGCTATCGCCTGGTCCAACCAGACCAG CTTCACATGCCAGGACATCTTCAAAGAGACAAACGCCACCTAT CCTTCCAGCGACGTGCCCTGTGATGCTACCCTGACCGAGAAGT CCTTCGAGACAGACATGAACCTGAACTTCCAGAACCTGTCCTG ATGA |
| SEQ ID NO: 38 | mTCRβ | GTGGTGGAAGATCTGCGGAACGTGACCCCTCCTAAGGTGTCCC TGTTCGAGCCTTCCAAGGCCGAGATCGCCAACAAGCAGAAAGC TACCCTCGTGTGCCTGGCCAGAGGCTTCTTTCCTGACCACGTG GAACTGTCTTGGTGGGTCAACGGCAAAGAGGTGCACTCCGGCG TCTGTACCGATCCTCAGGCCTACAAAGAGTCCAACTACTCCTA CAGCCTGTCCTCTCGGCTGAGAGTGTCTGCCACCTTCTGGCAC AACCCTCGGAACCACTTCAGATGCCAGGTGCAGTTTCACGGCC TGTCCGAAGAGGACAAGTGGCCTGAGGGATCCCCTAAGCCTGT GACACAGAACATCTCTGCCGAGGCCTGGGGTAGAGCTGATGGT GGC |
| SEQ ID NO: 39 | mIL2 F56A Y59A | CTGGAACAGCTGCTGATGGACCTGCAAGAGCTGCTGTCCCGGA TGGAAAACTACCGGAACCTGAAGCTGCCCCGGATGCTGACCGC TAAGTTCGCTCTGCCTAAGCAGGCCACCGAGCTGAAGGATCTG CAGTGCCTGGAAGATGAGCTGGGCCCTCTGAGACACGTGCTGG ATCTGACCCAGTCCAAGTCCTTTCAGCTCGAGGACGCCGAGAA CTTCATCTCCAACATCAGAGTGACCGTGGTCAAGCTGAAGGGC TCCGACAACACCTTCGAGTGCCAGTTCGACGATGAGTCCGCTA CAGTGGTGGACTTCCTGCGGAGATGGATCGCCTTCTGCCAGTC CATCATCTCCACCTCTCCACAGTGATGA |
| SEQ ID NO: 40 | GGGGSGGGGSG GGGS | GGAGGATCTGGCGGAGGTGGAAGCGGCGGAGGCGGATCTTCTC AT |
| SEQ ID NO: 41 | hCH3_Knob | GGCCAGCCTAGGGAACCCCAGGTTTACACCCTGCCTCCATGCC GGGAAGAGATGACCAAGAACCAGGTGTCCCTGTGGTGCCTGGT TAAGGGCTTCTACCCCTCCGATATCGCCGTGGAATGGGAGTCT AATGGCCAGCCTGAGAACAACTACAAGACAACCCCTCCTGTGC TGGACTCCGACGGCTCATTCTTCCTGTACTCCAAGCTGACAGT GGACAAGTCCAGATGGCAGCAGGGCAACGTGTTCTCCTGCTCC GTGATGCACGAGGCCCTGCACAATCACTACACCCAGAAGTCCC TGTCTCTGAGCCCCGGCAAG |
| SEQ ID NO: 42 | hCH3_hole | GCCAGCCTCGGGAACCTCAAGTCTGTACCCTGCCTCCTAGCCG GGAAGAGATGACCAAGAACCAGGTGTCCCTGTCCTGCGCTGTG AAGGGCTTCTACCCTTCCGATATCGCCGTGGAATGGGAGAGCA ATGGCCAGCCTGAGAACAACTACAAGACCACACCTCCTGTGCT GGACTCCGACGGCTCATTCTTCCTGGTGTCCAAGCTGACAGTG GACAAGTCCAGATGGCAGCAGGGCAACGTGTTCTCCTGCTCCG TGATGCACGAGGCCCTGCACAATCACTACACCCAGAAGTCCCT GTCTCTGAGCCCCGGCAAG |

**Table 2. Sequences used to construct ORFs.**

| SEQ ID NO | Variable | Constant 1 | Constant 2 | Linker | Constant 3 | Linker | C-term |
|---|---|---|---|---|---|---|---|
| SEQ ID NO: 43 | SEQ ID NO: 16 | SEQ ID NO: 22 | SEQ ID NO: 23 | | SEQ ID NO: 24 | | |
| SEQ ID NO: 44 | SEQ ID NO: 16 | SEQ ID NO: 22 | SEQ ID NO: 23 | SEQ ID NO: 26 | SEQ ID NO: 24 | | |
| SEQ ID NO: 45 | SEQ ID NO: 16 | SEQ ID NO: 22 | SEQ ID NO: 23 | SEQ ID NO: 27 | SEQ ID NO: 24 | | |
| SEQ ID NO: 46 | SEQ ID NO: 16 | SEQ ID NO: 22 | SEQ ID NO: 23 | SEQ ID NO: 28 | SEQ ID NO: 24 | | |
| SEQ ID NO: 47 | SEQ ID NO: 16 | SEQ ID NO: 22 | SEQ ID NO: 23 | SEQ ID NO: 29 | SEQ ID NO: 24 | | |
| SEQ ID NO: 48 | SEQ ID NO: 16 | SEQ ID NO: 22 | SEQ ID NO: 23 | SEQ ID NO: 30 | SEQ ID NO: 24 | | |
| SEQ ID NO: 49 | SEQ ID NO: 16 | SEQ ID NO: 22 | SEQ ID NO: 23 | SEQ ID NO: 31 | SEQ ID NO: 24 | | |
| SEQ ID NO: 50 | SEQ ID NO: 16 | SEQ ID NO: 22 | SEQ ID NO: 23 | SEQ ID NO: 32 | SEQ ID NO: 24 | | |
| SEQ ID NO: 51 | SEQ ID NO: 16 | SEQ ID NO: 22 | SEQ ID NO: 23 | SEQ ID NO: 33 | SEQ ID NO: 24 | | |
| SEQ ID NO: 52 | SEQ ID NO: 16 | SEQ ID NO: 22 | SEQ ID NO: 23 | SEQ ID NO: 34 | SEQ ID NO: 24 | | |
| SEQ ID NO: 53 | SEQ ID NO: 16 | SEQ ID NO: 22 | SEQ ID NO: 23 | SEQ ID NO: 35 | SEQ ID NO: 24 | | |
| SEQ ID NO: 54 | SEQ ID NO: 17 | SEQ ID NO: 20 | | | | | |
| SEQ ID NO: 55 | SEQ ID NO: 18 | SEQ ID NO: 22 | SEQ ID NO: 23 | | SEQ ID NO: 25 | | |
| SEQ ID NO: 56 | SEQ ID NO: 18 | SEQ ID NO: 22 | SEQ ID NO: 23 | SEQ ID NO: 26 | SEQ ID NO: 25 | | |
| SEQ ID NO: 57 | SEQ ID NO: 18 | SEQ ID NO: 22 | SEQ ID NO: 23 | SEQ ID NO: 27 | SEQ ID NO: 25 | | |
| SEQ ID NO: 58 | SEQ ID NO: 18 | SEQ ID NO: 22 | SEQ ID NO: 23 | SEQ ID NO: 28 | SEQ ID NO: 25 | | |
| SEQ ID NO: 59 | SEQ ID NO: 18 | SEQ ID NO: 22 | SEQ ID NO: 23 | SEQ ID NO: 29 | SEQ ID NO: 25 | | |
| SEQ ID NO: 60 | SEQ ID NO: 18 | SEQ ID NO: 22 | SEQ ID NO: 23 | SEQ ID NO: 30 | SEQ ID NO: 25 | | |
| SEQ ID NO: 61 | SEQ ID NO: 18 | SEQ ID NO: 22 | SEQ ID NO: 23 | SEQ ID NO: 31 | SEQ ID NO: 25 | | |
| SEQ ID NO: 62 | SEQ ID NO: 18 | SEQ ID NO: 22 | SEQ ID NO: 23 | SEQ ID NO: 32 | SEQ ID NO: 25 | | |
| SEQ ID NO: 63 | SEQ ID NO: 18 | SEQ ID NO: 22 | SEQ ID NO: 23 | SEQ ID NO: 33 | SEQ ID NO: 25 | | |
| SEQ ID NO: 64 | SEQ ID NO: 18 | SEQ ID NO: 22 | SEQ ID NO: 23 | SEQ ID NO: 34 | SEQ ID NO: 25 | | |
| SEQ ID NO: 65 | SEQ ID NO: 18 | SEQ ID NO: 22 | SEQ ID NO: 23 | SEQ ID NO: 35 | SEQ ID NO: 25 | | |
| SEQ ID NO: 66 | SEQ ID NO: 19 | SEQ ID NO: 21 | | | | | |
| SEQ ID NO: 67 | | | SEQ ID NO: 36 | | SEQ ID NO: 37 | | |
| SEQ ID NO: 68 | | | SEQ ID NO: 36 | SEQ ID NO: 26 | SEQ ID NO: 37 | | |
| SEQ ID NO: 69 | | | SEQ ID NO: 36 | SEQ ID NO: 27 | SEQ ID NO: 22 | | |
| SEQ ID NO: 70 | | | SEQ ID NO: 36 | SEQ ID NO: 28 | SEQ ID NO: 37 | | |
| SEQ ID NO: 71 | | | SEQ ID NO: 36 | SEQ ID NO: 29 | SEQ ID NO: 37 | | |
| SEQ ID NO: 72 | | | SEQ ID NO: 36 | SEQ ID NO: 30 | SEQ ID NO: 37 | | |
| SEQ ID NO: 73 | | | SEQ ID NO: 36 | SEQ ID NO: 31 | SEQ ID NO: 37 | | |
| SEQ ID NO: 74 | | | SEQ ID NO: 36 | SEQ ID NO: 32 | SEQ ID NO: 37 | | |
| SEQ ID NO: 75 | | | SEQ ID NO: 36 | SEQ ID NO: 33 | SEQ ID NO: 37 | | |
| SEQ ID NO: 76 | | | SEQ ID NO: 36 | SEQ ID NO: 34 | SEQ ID NO: 37 | | |
| SEQ ID NO: 77 | | | SEQ ID NO: 36 | SEQ ID NO: 35 | SEQ ID NO: 37 | | |
| SEQ ID NO: 78 | | | SEQ ID NO: 36 | | SEQ ID NO: 38 | SEQ ID NO: 40 | SEQ ID NO: 39 |
| SEQ ID NO: 79 | | | SEQ ID NO: 36 | SEQ ID NO: 26 | SEQ ID NO: 38 | SEQ ID NO: 40 | SEQ ID NO: 39 |
| SEQ ID NO: 80 | | | SEQ ID NO: 36 | SEQ ID NO: 27 | SEQ ID NO: 38 | SEQ ID NO: 40 | SEQ ID NO: 39 |
| SEQ ID NO: 81 | | | SEQ ID NO: 36 | SEQ ID NO: 28 | SEQ ID NO: 38 | SEQ ID NO: 40 | SEQ ID NO: 39 |
| SEQ ID NO: 82 | | | SEQ ID NO: 36 | SEQ ID NO: 29 | SEQ ID NO: 38 | SEQ ID NO: 40 | SEQ ID NO: 39 |
| SEQ ID NO: 83 | | | SEQ ID NO: 36 | SEQ ID NO: 30 | SEQ ID NO: 38 | SEQ ID NO: 40 | SEQ ID NO: 39 |
| SEQ ID NO: 84 | | | SEQ ID NO: 36 | SEQ ID NO: 31 | SEQ ID NO: 38 | SEQ ID NO: 40 | SEQ ID NO: 39 |
| SEQ ID NO: 85 | | | SEQ ID NO: 36 | SEQ ID NO: 32 | SEQ ID NO: 38 | SEQ ID NO: 40 | SEQ ID NO: 39 |
| SEQ ID NO: 86 | | | SEQ ID NO: 36 | SEQ ID NO: 33 | SEQ ID NO: 38 | SEQ ID NO: 40 | SEQ ID NO: 39 |
| SEQ ID NO: 87 | | | SEQ ID NO: 36 | SEQ ID NO: 34 | SEQ ID NO: 38 | SEQ ID NO: 40 | SEQ ID NO: 39 |
| SEQ ID NO: 88 | | | SEQ ID NO: 36 | SEQ ID NO: 35 | SEQ ID NO: 38 | SEQ ID NO: 40 | SEQ ID NO: 39 |
| SEQ ID NO: 89 | SEQ ID NO: 16 | SEQ ID NO: 22 | SEQ ID NO: 23 | | SEQ ID NO: 41 | | |
| SEQ ID NO: 90 | SEQ ID NO: 18 | SEQ ID NO: 22 | SEQ ID NO: 23 | | SEQ ID NO: 42 | | |

**Table 3. Sequences used to construct ORFs.**

| SEQ ID NO | DNA Sequence |
|---|---|
| SEQ ID NO: 43 | CAGGTGCAGCTGGTGGAATCTGGTGGCGGAGTTGTGCAGCCTGGCAGATCCCTGAGA CTGTCTTGTGCCGCCTCCGGCTTCACCTTCTCCAGCTACACCATGCACTGGGTCCGA CAGGCCCCTGGCAAAGGATTGGAGTGGGTCACCTTCATCTCTTACGACGGCAACAAC AAGTACTACGCCGACTCCGTGAAGGGCAGATTCACCATCTCTCGGGACAACTCCAAG AACACCCTGTACCTGCAGATGAACTCCCTGAGAGCCGAGGACACCGCCATCTACTAC TGTGCTAGAACCGGCTGGCTGGGCCCCTTTGATTATTGGGGACAGGGCACCCTGGTC ACCGTGTCCTCTGCTTCCACCAAGGGACCCTCTGTGTTCCCTCTGGCTCCTTCCAGC AAGTCTACCTCTGGCGGAACAGCTGCTCTGGGCTGCCTGGTCAAGGACTACTTTCCT GAGCCTGTGACCGTGTCTTGGAACTCTGGCGCTCTGACATCCGGCGTGCACACCTTT CCAGCTGTGCTGCAATCCTCCGGCCTGTACTCTCTGTCCTCCGTCGTGACCGTGCCT TCTAGCTCTCTGGGCACCCAGACCTACATCTGCAATGTGAACCACAAGCCTTCCAAC ACCAAGGTGGACAAGAGAGTGGAACCCAAGTCCTGCGATAAGACCCACACCTGTCCA CCATGTCCTGCTCCAGAACTGCTCGGCGGACCTTCCGTGTTCCTGTTTCCTCCAAAG CCTAAGGACACCCTGATGATCTCTCGGACCCCTGAAGTGACCTGCGTGGTGGTGGAT GTGTCTCACGAGGATCCCGAAGTGAAGTTCAATTGGTACGTGGACGGCGTGGAAGTG CACAACGCCAAGACCAAGCCTAGAGAGGAACAGTACAACTCCACCTACAGAGTGGTG TCCGTGCTGACCGTGCTGCACCAGGATTGGCTGAACGGCAAAGAGTACAAGTGCAAG GTGTCCAACAAGGCCCTGCCTGCTCCTATCGAAAAGACCATCTCCAAGGCCAAACCT GACATTCAGAACCCCGATCCTGCCGTGTACCAGCTGAGAGACTCCAAGTCCTCCGAT AAGTCTGTGTGCCTGTTCACCGACTTCGACTCCCAGACCAACGTGTCCCAGTCCAAG GACTCCGACGTGTACATCACCGATAAGTGCGTGCTGGACATGCGGAGCATGGACTTC AAGTCTAACTCCGCCGTGGCCTGGTCTAACAAGTCCGATTTCGCCTGCGCCAACGCC TTCAACAACAGCATTATCCCCGAGGACACATTCTTCCCATCACCTGAG |
| SEQ ID NO: 44 | CAGGTGCAGCTGGTGGAATCTGGTGGCGGAGTTGTGCAGCCTGGCAGATCCCTGAGA CTGTCTTGTGCCGCCTCCGGCTTCACCTTCTCCAGCTACACCATGCACTGGGTCCGA CAGGCCCCTGGCAAAGGATTGGAGTGGGTCACCTTCATCTCTTACGACGGCAACAAC AAGTACTACGCCGACTCCGTGAAGGGCAGATTCACCATCTCTCGGGACAACTCCAAG AACACCCTGTACCTGCAGATGAACTCCCTGAGAGCCGAGGACACCGCCATCTACTAC TGTGCTAGAACCGGCTGGCTGGGCCCCTTTGATTATTGGGGACAGGGCACCCTGGTC ACCGTGTCCTCTGCTTCCACCAAGGGACCCTCTGTGTTCCCTCTGGCTCCTTCCAGC AAGTCTACCTCTGGCGGAACAGCTGCTCTGGGCTGCCTGGTCAAGGACTACTTTCCT GAGCCTGTGACCGTGTCTTGGAACTCTGGCGCTCTGACATCCGGCGTGCACACCTTT CCAGCTGTGCTGCAATCCTCCGGCCTGTACTCTCTGTCCTCCGTCGTGACCGTGCCT TCTAGCTCTCTGGGCACCCAGACCTACATCTGCAATGTGAACCACAAGCCTTCCAAC ACCAAGGTGGACAAGAGAGTGGAACCCAAGTCCTGCGATAAGACCCACACCTGTCCA CCATGTCCTGCTCCAGAACTGCTCGGCGGACCTTCCGTGTTCCTGTTTCCTCCAAAG CCTAAGGACACCCTGATGATCTCTCGGACCCCTGAAGTGACCTGCGTGGTGGTGGAT GTGTCTCACGAGGATCCCGAAGTGAAGTTCAATTGGTACGTGGACGGCGTGGAAGTG CACAACGCCAAGACCAAGCCTAGAGAGGAACAGTACAACTCCACCTACAGAGTGGTG TCCGTGCTGACCGTGCTGCACCAGGATTGGCTGAACGGCAAAGAGTACAAGTGCAAG |
| | GTGTCCAACAAGGCCCTGCCTGCTCCTATCGAAAAGACCATCTCCAAGGCCAAAGGT CCTGACATTCAGAACCCCGATCCTGCCGTGTACCAGCTGAGAGACTCCAAGTCCTCC GATAAGTCTGTGTGCCTGTTCACCGACTTCGACTCCCAGACCAACGTGTCCCAGTCC AAGGACTCCGACGTGTACATCACCGATAAGTGCGTGCTGGACATGCGGAGCATGGAC TTCAAGTCTAACTCCGCCGTGGCCTGGTCTAACAAGTCCGATTTCGCCTGCGCCAAC GCCTTCAACAACAGCATTATCCCCGAGGACACATTCTTCCCATCACCTGAG |
| SEQ ID NO: 45 | CAGGTGCAGCTGGTGGAATCTGGTGGCGGAGTTGTGCAGCCTGGCAGATCCCTGAGA CTGTCTTGTGCCGCCTCCGGCTTCACCTTCTCCAGCTACACCATGCACTGGGTCCGA CAGGCCCCTGGCAAAGGATTGGAGTGGGTCACCTTCATCTCTTACGACGGCAACAAC AAGTACTACGCCGACTCCGTGAAGGGCAGATTCACCATCTCTCGGGACAACTCCAAG AACACCCTGTACCTGCAGATGAACTCCCTGAGAGCCGAGGACACCGCCATCTACTAC TGTGCTAGAACCGGCTGGCTGGGCCCCTTTGATTATTGGGGACAGGGCACCCTGGTC ACCGTGTCCTCTGCTTCCACCAAGGGACCCTCTGTGTTCCCTCTGGCTCCTTCCAGC AAGTCTACCTCTGGCGGAACAGCTGCTCTGGGCTGCCTGGTCAAGGACTACTTTCCT GAGCCTGTGACCGTGTCTTGGAACTCTGGCGCTCTGACATCCGGCGTGCACACCTTT CCAGCTGTGCTGCAATCCTCCGGCCTGTACTCTCTGTCCTCCGTCGTGACCGTGCCT TCTAGCTCTCTGGGCACCCAGACCTACATCTGCAATGTGAACCACAAGCCTTCCAAC ACCAAGGTGGACAAGAGAGTGGAACCCAAGTCCTGCGATAAGACCCACACCTGTCCA CCATGTCCTGCTCCAGAACTGCTCGGCGGACCTTCCGTGTTCCTGTTTCCTCCAAAG CCTAAGGACACCCTGATGATCTCTCGGACCCCTGAAGTGACCTGCGTGGTGGTGGAT GTGTCTCACGAGGATCCCGAAGTGAAGTTCAATTGGTACGTGGACGGCGTGGAAGTG CACAACGCCAAGACCAAGCCTAGAGAGGAACAGTACAACTCCACCTACAGAGTGGTG TCCGTGCTGACCGTGCTGCACCAGGATTGGCTGAACGGCAAAGAGTACAAGTGCAAG GTGTCCAACAAGGCCCTGCCTGCTCCTATCGAAAAGACCATCTCCAAGGCCAAAGGT GGCCCTGACATTCAGAACCCCGATCCTGCCGTGTACCAGCTGAGAGACTCCAAGTCC TCCGATAAGTCTGTGTGCCTGTTCACCGACTTCGACTCCCAGACCAACGTGTCCCAG TCCAAGGACTCCGACGTGTACATCACCGATAAGTGCGTGCTGGACATGCGGAGCATG GACTTCAAGTCTAACTCCGCCGTGGCCTGGTCTAACAAGTCCGATTTCGCCTGCGCC AACGCCTTCAACAACAGCATTATCCCCGAGGACACATTCTTCCCATCACCTGAG |
| SEQ ID NO: 46 | CAGGTGCAGCTGGTGGAATCTGGTGGCGGAGTTGTGCAGCCTGGCAGATCCCTGAGA CTGTCTTGTGCCGCCTCCGGCTTCACCTTCTCCAGCTACACCATGCACTGGGTCCGA CAGGCCCCTGGCAAAGGATTGGAGTGGGTCACCTTCATCTCTTACGACGGCAACAAC AAGTACTACGCCGACTCCGTGAAGGGCAGATTCACCATCTCTCGGGACAACTCCAAG AACACCCTGTACCTGCAGATGAACTCCCTGAGAGCCGAGGACACCGCCATCTACTAC TGTGCTAGAACCGGCTGGCTGGGCCCCTTTGATTATTGGGGACAGGGCACCCTGGTC ACCGTGTCCTCTGCTTCCACCAAGGGACCCTCTGTGTTCCCTCTGGCTCCTTCCAGC AAGTCTACCTCTGGCGGAACAGCTGCTCTGGGCTGCCTGGTCAAGGACTACTTTCCT GAGCCTGTGACCGTGTCTTGGAACTCTGGCGCTCTGACATCCGGCGTGCACACCTTT CCAGCTGTGCTGCAATCCTCCGGCCTGTACTCTCTGTCCTCCGTCGTGACCGTGCCT TCTAGCTCTCTGGGCACCCAGACCTACATCTGCAATGTGAACCACAAGCCTTCCAAC ACCAAGGTGGACAAGAGAGTGGAACCCAAGTCCTGCGATAAGACCCACACCTGTCCA CCATGTCCTGCTCCAGAACTGCTCGGCGGACCTTCCGTGTTCCTGTTTCCTCCAAAG CCTAAGGACACCCTGATGATCTCTCGGACCCCTGAAGTGACCTGCGTGGTGGTGGAT GTGTCTCACGAGGATCCCGAAGTGAAGTTCAATTGGTACGTGGACGGCGTGGAAGTG CACAACGCCAAGACCAAGCCTAGAGAGGAACAGTACAACTCCACCTACAGAGTGGTG TCCGTGCTGACCGTGCTGCACCAGGATTGGCTGAACGGCAAAGAGTACAAGTGCAAG GTGTCCAACAAGGCCCTGCCTGCTCCTATCGAAAAGACCATCTCCAAGGCCAAAGGT |
| | GGCGGACCTGACATTCAGAACCCCGATCCTGCCGTGTACCAGCTGAGAGACTCCAAG TCCTCCGATAAGTCTGTGTGCCTGTTCACCGACTTCGACTCCCAGACCAACGTGTCC CAGTCCAAGGACTCCGACGTGTACATCACCGATAAGTGCGTGCTGGACATGCGGAGC ATGGACTTCAAGTCTAACTCCGCCGTGGCCTGGTCTAACAAGTCCGATTTCGCCTGC GCCAACGCCTTCAACAACAGCATTATCCCCGAGGACACATTCTTCCCATCACCTGAG |
| SEQ ID NO: 47 | CAGGTGCAGCTGGTGGAATCTGGTGGCGGAGTTGTGCAGCCTGGCAGATCCCTGAGA CTGTCTTGTGCCGCCTCCGGCTTCACCTTCTCCAGCTACACCATGCACTGGGTCCGA CAGGCCCCTGGCAAAGGATTGGAGTGGGTCACCTTCATCTCTTACGACGGCAACAAC AAGTACTACGCCGACTCCGTGAAGGGCAGATTCACCATCTCTCGGGACAACTCCAAG AACACCCTGTACCTGCAGATGAACTCCCTGAGAGCCGAGGACACCGCCATCTACTAC TGTGCTAGAACCGGCTGGCTGGGCCCCTTTGATTATTGGGGACAGGGCACCCTGGTC ACCGTGTCCTCTGCTTCCACCAAGGGACCCTCTGTGTTCCCTCTGGCTCCTTCCAGC AAGTCTACCTCTGGCGGAACAGCTGCTCTGGGCTGCCTGGTCAAGGACTACTTTCCT GAGCCTGTGACCGTGTCTTGGAACTCTGGCGCTCTGACATCCGGCGTGCACACCTTT CCAGCTGTGCTGCAATCCTCCGGCCTGTACTCTCTGTCCTCCGTCGTGACCGTGCCT TCTAGCTCTCTGGGCACCCAGACCTACATCTGCAATGTGAACCACAAGCCTTCCAAC ACCAAGGTGGACAAGAGAGTGGAACCCAAGTCCTGCGATAAGACCCACACCTGTCCA CCATGTCCTGCTCCAGAACTGCTCGGCGGACCTTCCGTGTTCCTGTTTCCTCCAAAG CCTAAGGACACCCTGATGATCTCTCGGACCCCTGAAGTGACCTGCGTGGTGGTGGAT GTGTCTCACGAGGATCCCGAAGTGAAGTTCAATTGGTACGTGGACGGCGTGGAAGTG CACAACGCCAAGACCAAGCCTAGAGAGGAACAGTACAACTCCACCTACAGAGTGGTG TCCGTGCTGACCGTGCTGCACCAGGATTGGCTGAACGGCAAAGAGTACAAGTGCAAG GTGTCCAACAAGGCCCTGCCTGCTCCTATCGAAAAGACCATCTCCAAGGCCAAAGGT GGCGGAGGACCTGACATTCAGAACCCCGATCCTGCCGTGTACCAGCTGAGAGACTCC AAGTCCTCCGATAAGTCTGTGTGCCTGTTCACCGACTTCGACTCCCAGACCAACGTG TCCCAGTCCAAGGACTCCGACGTGTACATCACCGATAAGTGCGTGCTGGACATGCGG AGCATGGACTTCAAGTCTAACTCCGCCGTGGCCTGGTCTAACAAGTCCGATTTCGCC TGCGCCAACGCCTTCAACAACAGCATTATCCCCGAGGACACATTCTTCCCATCACCT GAG |
| SEQ ID NO: 48 | CAGGTGCAGCTGGTGGAATCTGGTGGCGGAGTTGTGCAGCCTGGCAGATCCCTGAGA CTGTCTTGTGCCGCCTCCGGCTTCACCTTCTCCAGCTACACCATGCACTGGGTCCGA CAGGCCCCTGGCAAAGGATTGGAGTGGGTCACCTTCATCTCTTACGACGGCAACAAC AAGTACTACGCCGACTCCGTGAAGGGCAGATTCACCATCTCTCGGGACAACTCCAAG AACACCCTGTACCTGCAGATGAACTCCCTGAGAGCCGAGGACACCGCCATCTACTAC TGTGCTAGAACCGGCTGGCTGGGCCCCTTTGATTATTGGGGACAGGGCACCCTGGTC ACCGTGTCCTCTGCTTCCACCAAGGGACCCTCTGTGTTCCCTCTGGCTCCTTCCAGC AAGTCTACCTCTGGCGGAACAGCTGCTCTGGGCTGCCTGGTCAAGGACTACTTTCCT GAGCCTGTGACCGTGTCTTGGAACTCTGGCGCTCTGACATCCGGCGTGCACACCTTT CCAGCTGTGCTGCAATCCTCCGGCCTGTACTCTCTGTCCTCCGTCGTGACCGTGCCT TCTAGCTCTCTGGGCACCCAGACCTACATCTGCAATGTGAACCACAAGCCTTCCAAC ACCAAGGTGGACAAGAGAGTGGAACCCAAGTCCTGCGATAAGACCCACACCTGTCCA CCATGTCCTGCTCCAGAACTGCTCGGCGGACCTTCCGTGTTCCTGTTTCCTCCAAAG CCTAAGGACACCCTGATGATCTCTCGGACCCCTGAAGTGACCTGCGTGGTGGTGGAT GTGTCTCACGAGGATCCCGAAGTGAAGTTCAATTGGTACGTGGACGGCGTGGAAGTG CACAACGCCAAGACCAAGCCTAGAGAGGAACAGTACAACTCCACCTACAGAGTGGTG TCCGTGCTGACCGTGCTGCACCAGGATTGGCTGAACGGCAAAGAGTACAAGTGCAAG GTGTCCAACAAGGCCCTGCCTGCTCCTATCGAAAAGACCATCTCCAAGGCCAAAGGT |
| | GGCGGAGGAAGCCCTGACATTCAGAACCCCGATCCTGCCGTGTACCAGCTGAGAGAC TCCAAGTCCTCCGATAAGTCTGTGTGCCTGTTCACCGACTTCGACTCCCAGACCAAC GTGTCCCAGTCCAAGGACTCCGACGTGTACATCACCGATAAGTGCGTGCTGGACATG CGGAGCATGGACTTCAAGTCTAACTCCGCCGTGGCCTGGTCTAACAAGTCCGATTTC GCCTGCGCCAACGCCTTCAACAACAGCATTATCCCCGAGGACACATTCTTCCCATCA CCTGAG |
| SEQ ID NO: 49 | CAGGTGCAGCTGGTGGAATCTGGTGGCGGAGTTGTGCAGCCTGGCAGATCCCTGAGA CTGTCTTGTGCCGCCTCCGGCTTCACCTTCTCCAGCTACACCATGCACTGGGTCCGA CAGGCCCCTGGCAAAGGATTGGAGTGGGTCACCTTCATCTCTTACGACGGCAACAAC AAGTACTACGCCGACTCCGTGAAGGGCAGATTCACCATCTCTCGGGACAACTCCAAG AACACCCTGTACCTGCAGATGAACTCCCTGAGAGCCGAGGACACCGCCATCTACTAC TGTGCTAGAACCGGCTGGCTGGGCCCCTTTGATTATTGGGGACAGGGCACCCTGGTC ACCGTGTCCTCTGCTTCCACCAAGGGACCCTCTGTGTTCCCTCTGGCTCCTTCCAGC AAGTCTACCTCTGGCGGAACAGCTGCTCTGGGCTGCCTGGTCAAGGACTACTTTCCT GAGCCTGTGACCGTGTCTTGGAACTCTGGCGCTCTGACATCCGGCGTGCACACCTTT CCAGCTGTGCTGCAATCCTCCGGCCTGTACTCTCTGTCCTCCGTCGTGACCGTGCCT TCTAGCTCTCTGGGCACCCAGACCTACATCTGCAATGTGAACCACAAGCCTTCCAAC ACCAAGGTGGACAAGAGAGTGGAACCCAAGTCCTGCGATAAGACCCACACCTGTCCA CCATGTCCTGCTCCAGAACTGCTCGGCGGACCTTCCGTGTTCCTGTTTCCTCCAAAG CCTAAGGACACCCTGATGATCTCTCGGACCCCTGAAGTGACCTGCGTGGTGGTGGAT GTGTCTCACGAGGATCCCGAAGTGAAGTTCAATTGGTACGTGGACGGCGTGGAAGTG CACAACGCCAAGACCAAGCCTAGAGAGGAACAGTACAACTCCACCTACAGAGTGGTG TCCGTGCTGACCGTGCTGCACCAGGATTGGCTGAACGGCAAAGAGTACAAGTGCAAG GTGTCCAACAAGGCCCTGCCTGCTCCTATCGAAAAGACCATCTCCAAGGCCAAAGGT GGCGGAGGAAGCGGTCCTGACATTCAGAACCCCGATCCTGCCGTGTACCAGCTGAGA GACTCCAAGTCCTCCGATAAGTCTGTGTGCCTGTTCACCGACTTCGACTCCCAGACC AACGTGTCCCAGTCCAAGGACTCCGACGTGTACATCACCGATAAGTGCGTGCTGGAC ATGCGGAGCATGGACTTCAAGTCTAACTCCGCCGTGGCCTGGTCTAACAAGTCCGAT TTCGCCTGCGCCAACGCCTTCAACAACAGCATTATCCCCGAGGACACATTCTTCCCA TCACCTGAG |
| SEQ ID NO: 50 | CAGGTGCAGCTGGTGGAATCTGGTGGCGGAGTTGTGCAGCCTGGCAGATCCCTGAGA CTGTCTTGTGCCGCCTCCGGCTTCACCTTCTCCAGCTACACCATGCACTGGGTCCGA CAGGCCCCTGGCAAAGGATTGGAGTGGGTCACCTTCATCTCTTACGACGGCAACAAC AAGTACTACGCCGACTCCGTGAAGGGCAGATTCACCATCTCTCGGGACAACTCCAAG AACACCCTGTACCTGCAGATGAACTCCCTGAGAGCCGAGGACACCGCCATCTACTAC TGTGCTAGAACCGGCTGGCTGGGCCCCTTTGATTATTGGGGACAGGGCACCCTGGTC ACCGTGTCCTCTGCTTCCACCAAGGGACCCTCTGTGTTCCCTCTGGCTCCTTCCAGC AAGTCTACCTCTGGCGGAACAGCTGCTCTGGGCTGCCTGGTCAAGGACTACTTTCCT GAGCCTGTGACCGTGTCTTGGAACTCTGGCGCTCTGACATCCGGCGTGCACACCTTT CCAGCTGTGCTGCAATCCTCCGGCCTGTACTCTCTGTCCTCCGTCGTGACCGTGCCT TCTAGCTCTCTGGGCACCCAGACCTACATCTGCAATGTGAACCACAAGCCTTCCAAC ACCAAGGTGGACAAGAGAGTGGAACCCAAGTCCTGCGATAAGACCCACACCTGTCCA CCATGTCCTGCTCCAGAACTGCTCGGCGGACCTTCCGTGTTCCTGTTTCCTCCAAAG CCTAAGGACACCCTGATGATCTCTCGGACCCCTGAAGTGACCTGCGTGGTGGTGGAT GTGTCTCACGAGGATCCCGAAGTGAAGTTCAATTGGTACGTGGACGGCGTGGAAGTG CACAACGCCAAGACCAAGCCTAGAGAGGAACAGTACAACTCCACCTACAGAGTGGTG TCCGTGCTGACCGTGCTGCACCAGGATTGGCTGAACGGCAAAGAGTACAAGTGCAAG |
| | GTGTCCAACAAGGCCCTGCCTGCTCCTATCGAAAAGACCATCTCCAAGGCCAAAGGT GGCGGAGGAAGCGGTGGCCCTGACATTCAGAACCCCGATCCTGCCGTGTACCAGCTG AGAGACTCCAAGTCCTCCGATAAGTCTGTGTGCCTGTTCACCGACTTCGACTCCCAG ACCAACGTGTCCCAGTCCAAGGACTCCGACGTGTACATCACCGATAAGTGCGTGCTG GACATGCGGAGCATGGACTTCAAGTCTAACTCCGCCGTGGCCTGGTCTAACAAGTCC GATTTCGCCTGCGCCAACGCCTTCAACAACAGCATTATCCCCGAGGACACATTCTTC CCATCACCTGAG |
| SEQ ID NO: 51 | CAGGTGCAGCTGGTGGAATCTGGTGGCGGAGTTGTGCAGCCTGGCAGATCCCTGAGA CTGTCTTGTGCCGCCTCCGGCTTCACCTTCTCCAGCTACACCATGCACTGGGTCCGA CAGGCCCCTGGCAAAGGATTGGAGTGGGTCACCTTCATCTCTTACGACGGCAACAAC AAGTACTACGCCGACTCCGTGAAGGGCAGATTCACCATCTCTCGGGACAACTCCAAG AACACCCTGTACCTGCAGATGAACTCCCTGAGAGCCGAGGACACCGCCATCTACTAC TGTGCTAGAACCGGCTGGCTGGGCCCCTTTGATTATTGGGGACAGGGCACCCTGGTC ACCGTGTCCTCTGCTTCCACCAAGGGACCCTCTGTGTTCCCTCTGGCTCCTTCCAGC AAGTCTACCTCTGGCGGAACAGCTGCTCTGGGCTGCCTGGTCAAGGACTACTTTCCT GAGCCTGTGACCGTGTCTTGGAACTCTGGCGCTCTGACATCCGGCGTGCACACCTTT CCAGCTGTGCTGCAATCCTCCGGCCTGTACTCTCTGTCCTCCGTCGTGACCGTGCCT TCTAGCTCTCTGGGCACCCAGACCTACATCTGCAATGTGAACCACAAGCCTTCCAAC ACCAAGGTGGACAAGAGAGTGGAACCCAAGTCCTGCGATAAGACCCACACCTGTCCA CCATGTCCTGCTCCAGAACTGCTCGGCGGACCTTCCGTGTTCCTGTTTCCTCCAAAG CCTAAGGACACCCTGATGATCTCTCGGACCCCTGAAGTGACCTGCGTGGTGGTGGAT GTGTCTCACGAGGATCCCGAAGTGAAGTTCAATTGGTACGTGGACGGCGTGGAAGTG CACAACGCCAAGACCAAGCCTAGAGAGGAACAGTACAACTCCACCTACAGAGTGGTG TCCGTGCTGACCGTGCTGCACCAGGATTGGCTGAACGGCAAAGAGTACAAGTGCAAG GTGTCCAACAAGGCCCTGCCTGCTCCTATCGAAAAGACCATCTCCAAGGCCAAAGGT GGCGGAGGAAGCGGTGGCGGCCCTGACATTCAGAACCCCGATCCTGCCGTGTACCAG CTGAGAGACTCCAAGTCCTCCGATAAGTCTGTGTGCCTGTTCACCGACTTCGACTCC CAGACCAACGTGTCCCAGTCCAAGGACTCCGACGTGTACATCACCGATAAGTGCGTG CTGGACATGCGGAGCATGGACTTCAAGTCTAACTCCGCCGTGGCCTGGTCTAACAAG TCCGATTTCGCCTGCGCCAACGCCTTCAACAACAGCATTATCCCCGAGGACACATTC TTCCCATCACCTGAG |
| SEQ ID NO: 52 | CAGGTGCAGCTGGTGGAATCTGGTGGCGGAGTTGTGCAGCCTGGCAGATCCCTGAGA CTGTCTTGTGCCGCCTCCGGCTTCACCTTCTCCAGCTACACCATGCACTGGGTCCGA CAGGCCCCTGGCAAAGGATTGGAGTGGGTCACCTTCATCTCTTACGACGGCAACAAC AAGTACTACGCCGACTCCGTGAAGGGCAGATTCACCATCTCTCGGGACAACTCCAAG AACACCCTGTACCTGCAGATGAACTCCCTGAGAGCCGAGGACACCGCCATCTACTAC TGTGCTAGAACCGGCTGGCTGGGCCCCTTTGATTATTGGGGACAGGGCACCCTGGTC ACCGTGTCCTCTGCTTCCACCAAGGGACCCTCTGTGTTCCCTCTGGCTCCTTCCAGC AAGTCTACCTCTGGCGGAACAGCTGCTCTGGGCTGCCTGGTCAAGGACTACTTTCCT GAGCCTGTGACCGTGTCTTGGAACTCTGGCGCTCTGACATCCGGCGTGCACACCTTT CCAGCTGTGCTGCAATCCTCCGGCCTGTACTCTCTGTCCTCCGTCGTGACCGTGCCT TCTAGCTCTCTGGGCACCCAGACCTACATCTGCAATGTGAACCACAAGCCTTCCAAC ACCAAGGTGGACAAGAGAGTGGAACCCAAGTCCTGCGATAAGACCCACACCTGTCCA CCATGTCCTGCTCCAGAACTGCTCGGCGGACCTTCCGTGTTCCTGTTTCCTCCAAAG CCTAAGGACACCCTGATGATCTCTCGGACCCCTGAAGTGACCTGCGTGGTGGTGGAT GTGTCTCACGAGGATCCCGAAGTGAAGTTCAATTGGTACGTGGACGGCGTGGAAGTG CACAACGCCAAGACCAAGCCTAGAGAGGAACAGTACAACTCCACCTACAGAGTGGTG |
| | TCCGTGCTGACCGTGCTGCACCAGGATTGGCTGAACGGCAAAGAGTACAAGTGCAAG GTGTCCAACAAGGCCCTGCCTGCTCCTATCGAAAAGACCATCTCCAAGGCCAAAGGT GGCGGAGGAAGCGGTGGCGGCGGACCTGACATTCAGAACCCCGATCCTGCCGTGTAC CAGCTGAGAGACTCCAAGTCCTCCGATAAGTCTGTGTGCCTGTTCACCGACTTCGAC TCCCAGACCAACGTGTCCCAGTCCAAGGACTCCGACGTGTACATCACCGATAAGTGC GTGCTGGACATGCGGAGCATGGACTTCAAGTCTAACTCCGCCGTGGCCTGGTCTAAC AAGTCCGATTTCGCCTGCGCCAACGCCTTCAACAACAGCATTATCCCCGAGGACACA TTCTTCCCATCACCTGAG |
| SEQ ID NO: 53 | CAGGTGCAGCTGGTGGAATCTGGTGGCGGAGTTGTGCAGCCTGGCAGATCCCTGAGA CTGTCTTGTGCCGCCTCCGGCTTCACCTTCTCCAGCTACACCATGCACTGGGTCCGA CAGGCCCCTGGCAAAGGATTGGAGTGGGTCACCTTCATCTCTTACGACGGCAACAAC AAGTACTACGCCGACTCCGTGAAGGGCAGATTCACCATCTCTCGGGACAACTCCAAG AACACCCTGTACCTGCAGATGAACTCCCTGAGAGCCGAGGACACCGCCATCTACTAC TGTGCTAGAACCGGCTGGCTGGGCCCCTTTGATTATTGGGGACAGGGCACCCTGGTC ACCGTGTCCTCTGCTTCCACCAAGGGACCCTCTGTGTTCCCTCTGGCTCCTTCCAGC AAGTCTACCTCTGGCGGAACAGCTGCTCTGGGCTGCCTGGTCAAGGACTACTTTCCT GAGCCTGTGACCGTGTCTTGGAACTCTGGCGCTCTGACATCCGGCGTGCACACCTTT CCAGCTGTGCTGCAATCCTCCGGCCTGTACTCTCTGTCCTCCGTCGTGACCGTGCCT TCTAGCTCTCTGGGCACCCAGACCTACATCTGCAATGTGAACCACAAGCCTTCCAAC ACCAAGGTGGACAAGAGAGTGGAACCCAAGTCCTGCGATAAGACCCACACCTGTCCA CCATGTCCTGCTCCAGAACTGCTCGGCGGACCTTCCGTGTTCCTGTTTCCTCCAAAG CCTAAGGACACCCTGATGATCTCTCGGACCCCTGAAGTGACCTGCGTGGTGGTGGAT GTGTCTCACGAGGATCCCGAAGTGAAGTTCAATTGGTACGTGGACGGCGTGGAAGTG CACAACGCCAAGACCAAGCCTAGAGAGGAACAGTACAACTCCACCTACAGAGTGGTG TCCGTGCTGACCGTGCTGCACCAGGATTGGCTGAACGGCAAAGAGTACAAGTGCAAG GTGTCCAACAAGGCCCTGCCTGCTCCTATCGAAAAGACCATCTCCAAGGCCAAAGGT GGCGGAGGAAGCGGTGGCGGCGGATCTCCTGACATTCAGAACCCCGATCCTGCCGTG TACCAGCTGAGAGACTCCAAGTCCTCCGATAAGTCTGTGTGCCTGTTCACCGACTTC GACTCCCAGACCAACGTGTCCCAGTCCAAGGACTCCGACGTGTACATCACCGATAAG TGCGTGCTGGACATGCGGAGCATGGACTTCAAGTCTAACTCCGCCGTGGCCTGGTCT AACAAGTCCGATTTCGCCTGCGCCAACGCCTTCAACAACAGCATTATCCCCGAGGAC ACATTCTTCCCATCACCTGAG |
| SEQ ID NO: 54 | GAGATCGTGCTGACCCAGTCTCCTGGCACACTGTCACTGTCTCCAGGCGAGAGAGCT ACCCTGTCCTGTAGAGCCTCTCAGTCCGTGGGCTCCTCTTACCTGGCTTGGTATCAG CAGAAGCCCGGCCAGGCTCCTAGACTGTTGATCTACGGCGCCTTCTCCAGAGCCACA GGCATCCCTGATAGATTCTCCGGCTCTGGCTCTGGCACCGACTTCACCCTGACCATC TCCAGACTGGAACCCGAGGACTTCGCCGTGTACTACTGTCAGCAGTACGGCTCCTCT CCTTGGACCTTTGGCCAGGGCACCAAGGTGGAAATCAAGCGGACAGTGGCCGCTCCT TCCGTGTTCATCTTCCCACCTTCCGACGAGCAGCTGAAGTCCGGCACAGCTTCTGTC GTGTGCCTGCTGAACAACTTCTACCCTCGGGAAGCCAAGGTGCAGTGGAAGGTGGAC AATGCCCTGCAGTCCGGCAACTCCCAAGAGTCTGTGACCGAGCAGGACTCCAAGGAC AGCACCTACAGCCTGTCCTCCACACTGACCCTGTCCAAGGCCGACTACGAGAAGCAC AAGGTGTACGCCTGCGAAGTGACCCATCAGGGCCTGTCTAGCCCTGTGACCAAGTCT TTCAACCGGGGCGAGTGC |
| SEQ ID NO: 55 | CAGGTGCAGCTGGTGGAATCTGGTGGCGGAGTTGTGCAGCCTGGCAGATCCCTGAGA CTGTCTTGTGCCGCCTCCGGCTTCACCTTCTCCTCTTACGGAATGCACTGGGTCCGA CAGGCCCCTGGCAAAGGATTGGAGTGGGTCGCCTTCATCAGATACGACGGCTCCAAC |
| | AAGTACTACGCCGACTCCGTGAAGGGCAGATTCACCATCTCTCGGGACAACTCCAAG AACACCCTGTACCTGCAGATGAACTCCCTGAGAGCCGAGGACACCGCCGTGTACTAC TGCAAGACCCACGGCTCTCACGACAATTGGGGCCAGGGCACAATGGTCACCGTGTCC TCTGCTTCCACCAAGGGACCCTCTGTGTTCCCTCTGGCTCCTTCCAGCAAGTCTACC TCTGGCGGAACAGCTGCTCTGGGCTGCCTGGTCAAGGACTACTTTCCTGAGCCTGTG ACCGTGTCTTGGAACTCTGGCGCTCTGACATCCGGCGTGCACACCTTTCCAGCTGTG CTGCAATCCTCCGGCCTGTACTCTCTGTCCTCCGTCGTGACCGTGCCTTCTAGCTCT CTGGGCACCCAGACCTACATCTGCAATGTGAACCACAAGCCTTCCAACACCAAGGTG GACAAGAGAGTGGAACCCAAGTCCTGCGATAAGACCCACACCTGTCCACCATGTCCT GCTCCAGAACTGCTCGGCGGACCTTCCGTGTTCCTGTTTCCTCCAAAGCCTAAGGAC ACCCTGATGATCTCTCGGACCCCTGAAGTGACCTGCGTGGTGGTGGATGTGTCTCAC GAGGATCCCGAAGTGAAGTTCAATTGGTACGTGGACGGCGTGGAAGTGCACAACGCC AAGACCAAGCCTAGAGAGGAACAGTACAACTCCACCTACAGAGTGGTGTCCGTGCTG ACCGTGCTGCACCAGGATTGGCTGAACGGCAAAGAGTACAAGTGCAAGGTGTCCAAC AAGGCCCTGCCTGCTCCTATCGAAAAGACCATCTCCAAGGCCAAAGAGGACCTGAAC AAGGTTTTCCCACCTGAGGTGGCCCTGTTCGAGCCTTCTGAGGCTGAGATCTCTCAC ACCCAGAAAGCTACCCTCGTGTGTCTGGCCACCGGCTTCTACCCTGATCACGTGGAA CTGTCTTGGTGGGTCAACGGAAAAGAGGTGCACTCCGGCGTCTGCACCGATCCTCAG CCTCTGAAAGAACAGCCCGCTCTGAACGACTCCAGATACGCCCTGTCCTCTCGGCTG AGAGTGTCTGCCACCTTCTGGCAGGACCCTCGGAACCACTTTAGATGCCAGGTGCAG TTCTACGGCCTGTCCGAGGCTGATGAGTGGACCCAGGCTAGAGCCAAGCCAGTGACA CAGATCGTGTCTGCCGAAGCCTGGGGCAGAGCCGAT |
| SEQ ID NO: 56 | CAGGTGCAGCTGGTGGAATCTGGTGGCGGAGTTGTGCAGCCTGGCAGATCCCTGAGA CTGTCTTGTGCCGCCTCCGGCTTCACCTTCTCCTCTTACGGAATGCACTGGGTCCGA CAGGCCCCTGGCAAAGGATTGGAGTGGGTCGCCTTCATCAGATACGACGGCTCCAAC AAGTACTACGCCGACTCCGTGAAGGGCAGATTCACCATCTCTCGGGACAACTCCAAG AACACCCTGTACCTGCAGATGAACTCCCTGAGAGCCGAGGACACCGCCGTGTACTAC TGCAAGACCCACGGCTCTCACGACAATTGGGGCCAGGGCACAATGGTCACCGTGTCC TCTGCTTCCACCAAGGGACCCTCTGTGTTCCCTCTGGCTCCTTCCAGCAAGTCTACC TCTGGCGGAACAGCTGCTCTGGGCTGCCTGGTCAAGGACTACTTTCCTGAGCCTGTG ACCGTGTCTTGGAACTCTGGCGCTCTGACATCCGGCGTGCACACCTTTCCAGCTGTG CTGCAATCCTCCGGCCTGTACTCTCTGTCCTCCGTCGTGACCGTGCCTTCTAGCTCT CTGGGCACCCAGACCTACATCTGCAATGTGAACCACAAGCCTTCCAACACCAAGGTG GACAAGAGAGTGGAACCCAAGTCCTGCGATAAGACCCACACCTGTCCACCATGTCCT GCTCCAGAACTGCTCGGCGGACCTTCCGTGTTCCTGTTTCCTCCAAAGCCTAAGGAC ACCCTGATGATCTCTCGGACCCCTGAAGTGACCTGCGTGGTGGTGGATGTGTCTCAC GAGGATCCCGAAGTGAAGTTCAATTGGTACGTGGACGGCGTGGAAGTGCACAACGCC AAGACCAAGCCTAGAGAGGAACAGTACAACTCCACCTACAGAGTGGTGTCCGTGCTG ACCGTGCTGCACCAGGATTGGCTGAACGGCAAAGAGTACAAGTGCAAGGTGTCCAAC AAGGCCCTGCCTGCTCCTATCGAAAAGACCATCTCCAAGGCCAAAGGTGAGGACCTG AACAAGGTTTTCCCACCTGAGGTGGCCCTGTTCGAGCCTTCTGAGGCTGAGATCTCT CACACCCAGAAAGCTACCCTCGTGTGTCTGGCCACCGGCTTCTACCCTGATCACGTG GAACTGTCTTGGTGGGTCAACGGAAAAGAGGTGCACTCCGGCGTCTGCACCGATCCT CAGCCTCTGAAAGAACAGCCCGCTCTGAACGACTCCAGATACGCCCTGTCCTCTCGG CTGAGAGTGTCTGCCACCTTCTGGCAGGACCCTCGGAACCACTTTAGATGCCAGGTG CAGTTCTACGGCCTGTCCGAGGCTGATGAGTGGACCCAGGCTAGAGCCAAGCCAGTG ACACAGATCGTGTCTGCCGAAGCCTGGGGCAGAGCCGAT |
| SEQ ID NO: 57 | CAGGTGCAGCTGGTGGAATCTGGTGGCGGAGTTGTGCAGCCTGGCAGATCCCTGAGA CTGTCTTGTGCCGCCTCCGGCTTCACCTTCTCCTCTTACGGAATGCACTGGGTCCGA CAGGCCCCTGGCAAAGGATTGGAGTGGGTCGCCTTCATCAGATACGACGGCTCCAAC AAGTACTACGCCGACTCCGTGAAGGGCAGATTCACCATCTCTCGGGACAACTCCAAG AACACCCTGTACCTGCAGATGAACTCCCTGAGAGCCGAGGACACCGCCGTGTACTAC TGCAAGACCCACGGCTCTCACGACAATTGGGGCCAGGGCACAATGGTCACCGTGTCC TCTGCTTCCACCAAGGGACCCTCTGTGTTCCCTCTGGCTCCTTCCAGCAAGTCTACC TCTGGCGGAACAGCTGCTCTGGGCTGCCTGGTCAAGGACTACTTTCCTGAGCCTGTG ACCGTGTCTTGGAACTCTGGCGCTCTGACATCCGGCGTGCACACCTTTCCAGCTGTG CTGCAATCCTCCGGCCTGTACTCTCTGTCCTCCGTCGTGACCGTGCCTTCTAGCTCT CTGGGCACCCAGACCTACATCTGCAATGTGAACCACAAGCCTTCCAACACCAAGGTG GACAAGAGAGTGGAACCCAAGTCCTGCGATAAGACCCACACCTGTCCACCATGTCCT GCTCCAGAACTGCTCGGCGGACCTTCCGTGTTCCTGTTTCCTCCAAAGCCTAAGGAC ACCCTGATGATCTCTCGGACCCCTGAAGTGACCTGCGTGGTGGTGGATGTGTCTCAC GAGGATCCCGAAGTGAAGTTCAATTGGTACGTGGACGGCGTGGAAGTGCACAACGCC AAGACCAAGCCTAGAGAGGAACAGTACAACTCCACCTACAGAGTGGTGTCCGTGCTG ACCGTGCTGCACCAGGATTGGCTGAACGGCAAAGAGTACAAGTGCAAGGTGTCCAAC AAGGCCCTGCCTGCTCCTATCGAAAAGACCATCTCCAAGGCCAAAGGTGGCGAGGAC CTGAACAAGGTTTTCCCACCTGAGGTGGCCCTGTTCGAGCCTTCTGAGGCTGAGATC TCTCACACCCAGAAAGCTACCCTCGTGTGTCTGGCCACCGGCTTCTACCCTGATCAC GTGGAACTGTCTTGGTGGGTCAACGGAAAAGAGGTGCACTCCGGCGTCTGCACCGAT CCTCAGCCTCTGAAAGAACAGCCCGCTCTGAACGACTCCAGATACGCCCTGTCCTCT CGGCTGAGAGTGTCTGCCACCTTCTGGCAGGACCCTCGGAACCACTTTAGATGCCAG GTGCAGTTCTACGGCCTGTCCGAGGCTGATGAGTGGACCCAGGCTAGAGCCAAGCCA GTGACACAGATCGTGTCTGCCGAAGCCTGGGGCAGAGCCGAT |
| SEQ ID NO: 58 | CAGGTGCAGCTGGTGGAATCTGGTGGCGGAGTTGTGCAGCCTGGCAGATCCCTGAGA CTGTCTTGTGCCGCCTCCGGCTTCACCTTCTCCTCTTACGGAATGCACTGGGTCCGA CAGGCCCCTGGCAAAGGATTGGAGTGGGTCGCCTTCATCAGATACGACGGCTCCAAC AAGTACTACGCCGACTCCGTGAAGGGCAGATTCACCATCTCTCGGGACAACTCCAAG AACACCCTGTACCTGCAGATGAACTCCCTGAGAGCCGAGGACACCGCCGTGTACTAC TGCAAGACCCACGGCTCTCACGACAATTGGGGCCAGGGCACAATGGTCACCGTGTCC TCTGCTTCCACCAAGGGACCCTCTGTGTTCCCTCTGGCTCCTTCCAGCAAGTCTACC TCTGGCGGAACAGCTGCTCTGGGCTGCCTGGTCAAGGACTACTTTCCTGAGCCTGTG ACCGTGTCTTGGAACTCTGGCGCTCTGACATCCGGCGTGCACACCTTTCCAGCTGTG CTGCAATCCTCCGGCCTGTACTCTCTGTCCTCCGTCGTGACCGTGCCTTCTAGCTCT CTGGGCACCCAGACCTACATCTGCAATGTGAACCACAAGCCTTCCAACACCAAGGTG GACAAGAGAGTGGAACCCAAGTCCTGCGATAAGACCCACACCTGTCCACCATGTCCT GCTCCAGAACTGCTCGGCGGACCTTCCGTGTTCCTGTTTCCTCCAAAGCCTAAGGAC ACCCTGATGATCTCTCGGACCCCTGAAGTGACCTGCGTGGTGGTGGATGTGTCTCAC GAGGATCCCGAAGTGAAGTTCAATTGGTACGTGGACGGCGTGGAAGTGCACAACGCC AAGACCAAGCCTAGAGAGGAACAGTACAACTCCACCTACAGAGTGGTGTCCGTGCTG ACCGTGCTGCACCAGGATTGGCTGAACGGCAAAGAGTACAAGTGCAAGGTGTCCAAC AAGGCCCTGCCTGCTCCTATCGAAAAGACCATCTCCAAGGCCAAAGGTGGCGGAGAG GACCTGAACAAGGTTTTCCCACCTGAGGTGGCCCTGTTCGAGCCTTCTGAGGCTGAG ATCTCTCACACCCAGAAAGCTACCCTCGTGTGTCTGGCCACCGGCTTCTACCCTGAT CACGTGGAACTGTCTTGGTGGGTCAACGGAAAAGAGGTGCACTCCGGCGTCTGCACC GATCCTCAGCCTCTGAAAGAACAGCCCGCTCTGAACGACTCCAGATACGCCCTGTCC |
| | TCTCGGCTGAGAGTGTCTGCCACCTTCTGGCAGGACCCTCGGAACCACTTTAGATGC CAGGTGCAGTTCTACGGCCTGTCCGAGGCTGATGAGTGGACCCAGGCTAGAGCCAAG CCAGTGACACAGATCGTGTCTGCCGAAGCCTGGGGCAGAGCCGAT |
| SEQ ID NO: 59 | CAGGTGCAGCTGGTGGAATCTGGTGGCGGAGTTGTGCAGCCTGGCAGATCCCTGAGA CTGTCTTGTGCCGCCTCCGGCTTCACCTTCTCCTCTTACGGAATGCACTGGGTCCGA CAGGCCCCTGGCAAAGGATTGGAGTGGGTCGCCTTCATCAGATACGACGGCTCCAAC AAGTACTACGCCGACTCCGTGAAGGGCAGATTCACCATCTCTCGGGACAACTCCAAG AACACCCTGTACCTGCAGATGAACTCCCTGAGAGCCGAGGACACCGCCGTGTACTAC TGCAAGACCCACGGCTCTCACGACAATTGGGGCCAGGGCACAATGGTCACCGTGTCC TCTGCTTCCACCAAGGGACCCTCTGTGTTCCCTCTGGCTCCTTCCAGCAAGTCTACC TCTGGCGGAACAGCTGCTCTGGGCTGCCTGGTCAAGGACTACTTTCCTGAGCCTGTG ACCGTGTCTTGGAACTCTGGCGCTCTGACATCCGGCGTGCACACCTTTCCAGCTGTG CTGCAATCCTCCGGCCTGTACTCTCTGTCCTCCGTCGTGACCGTGCCTTCTAGCTCT CTGGGCACCCAGACCTACATCTGCAATGTGAACCACAAGCCTTCCAACACCAAGGTG GACAAGAGAGTGGAACCCAAGTCCTGCGATAAGACCCACACCTGTCCACCATGTCCT GCTCCAGAACTGCTCGGCGGACCTTCCGTGTTCCTGTTTCCTCCAAAGCCTAAGGAC ACCCTGATGATCTCTCGGACCCCTGAAGTGACCTGCGTGGTGGTGGATGTGTCTCAC GAGGATCCCGAAGTGAAGTTCAATTGGTACGTGGACGGCGTGGAAGTGCACAACGCC AAGACCAAGCCTAGAGAGGAACAGTACAACTCCACCTACAGAGTGGTGTCCGTGCTG ACCGTGCTGCACCAGGATTGGCTGAACGGCAAAGAGTACAAGTGCAAGGTGTCCAAC AAGGCCCTGCCTGCTCCTATCGAAAAGACCATCTCCAAGGCCAAAGGTGGCGGAGGA GAGGACCTGAACAAGGTTTTCCCACCTGAGGTGGCCCTGTTCGAGCCTTCTGAGGCT GAGATCTCTCACACCCAGAAAGCTACCCTCGTGTGTCTGGCCACCGGCTTCTACCCT GATCACGTGGAACTGTCTTGGTGGGTCAACGGAAAAGAGGTGCACTCCGGCGTCTGC ACCGATCCTCAGCCTCTGAAAGAACAGCCCGCTCTGAACGACTCCAGATACGCCCTG TCCTCTCGGCTGAGAGTGTCTGCCACCTTCTGGCAGGACCCTCGGAACCACTTTAGA TGCCAGGTGCAGTTCTACGGCCTGTCCGAGGCTGATGAGTGGACCCAGGCTAGAGCC AAGCCAGTGACACAGATCGTGTCTGCCGAAGCCTGGGGCAGAGCCGAT |
| SEQ ID NO: 60 | CAGGTGCAGCTGGTGGAATCTGGTGGCGGAGTTGTGCAGCCTGGCAGATCCCTGAGA CTGTCTTGTGCCGCCTCCGGCTTCACCTTCTCCTCTTACGGAATGCACTGGGTCCGA CAGGCCCCTGGCAAAGGATTGGAGTGGGTCGCCTTCATCAGATACGACGGCTCCAAC AAGTACTACGCCGACTCCGTGAAGGGCAGATTCACCATCTCTCGGGACAACTCCAAG AACACCCTGTACCTGCAGATGAACTCCCTGAGAGCCGAGGACACCGCCGTGTACTAC TGCAAGACCCACGGCTCTCACGACAATTGGGGCCAGGGCACAATGGTCACCGTGTCC TCTGCTTCCACCAAGGGACCCTCTGTGTTCCCTCTGGCTCCTTCCAGCAAGTCTACC TCTGGCGGAACAGCTGCTCTGGGCTGCCTGGTCAAGGACTACTTTCCTGAGCCTGTG ACCGTGTCTTGGAACTCTGGCGCTCTGACATCCGGCGTGCACACCTTTCCAGCTGTG CTGCAATCCTCCGGCCTGTACTCTCTGTCCTCCGTCGTGACCGTGCCTTCTAGCTCT CTGGGCACCCAGACCTACATCTGCAATGTGAACCACAAGCCTTCCAACACCAAGGTG GACAAGAGAGTGGAACCCAAGTCCTGCGATAAGACCCACACCTGTCCACCATGTCCT GCTCCAGAACTGCTCGGCGGACCTTCCGTGTTCCTGTTTCCTCCAAAGCCTAAGGAC ACCCTGATGATCTCTCGGACCCCTGAAGTGACCTGCGTGGTGGTGGATGTGTCTCAC GAGGATCCCGAAGTGAAGTTCAATTGGTACGTGGACGGCGTGGAAGTGCACAACGCC AAGACCAAGCCTAGAGAGGAACAGTACAACTCCACCTACAGAGTGGTGTCCGTGCTG ACCGTGCTGCACCAGGATTGGCTGAACGGCAAAGAGTACAAGTGCAAGGTGTCCAAC AAGGCCCTGCCTGCTCCTATCGAAAAGACCATCTCCAAGGCCAAAGGTGGCGGAGGA AGCGAGGACCTGAACAAGGTTTTCCCACCTGAGGTGGCCCTGTTCGAGCCTTCTGAG |
| | GCTGAGATCTCTCACACCCAGAAAGCTACCCTCGTGTGTCTGGCCACCGGCTTCTAC CCTGATCACGTGGAACTGTCTTGGTGGGTCAACGGAAAAGAGGTGCACTCCGGCGTC TGCACCGATCCTCAGCCTCTGAAAGAACAGCCCGCTCTGAACGACTCCAGATACGCC CTGTCCTCTCGGCTGAGAGTGTCTGCCACCTTCTGGCAGGACCCTCGGAACCACTTT AGATGCCAGGTGCAGTTCTACGGCCTGTCCGAGGCTGATGAGTGGACCCAGGCTAGA GCCAAGCCAGTGACACAGATCGTGTCTGCCGAAGCCTGGGGCAGAGCCGAT |
| SEQ ID NO: 61 | CAGGTGCAGCTGGTGGAATCTGGTGGCGGAGTTGTGCAGCCTGGCAGATCCCTGAGA CTGTCTTGTGCCGCCTCCGGCTTCACCTTCTCCTCTTACGGAATGCACTGGGTCCGA CAGGCCCCTGGCAAAGGATTGGAGTGGGTCGCCTTCATCAGATACGACGGCTCCAAC AAGTACTACGCCGACTCCGTGAAGGGCAGATTCACCATCTCTCGGGACAACTCCAAG AACACCCTGTACCTGCAGATGAACTCCCTGAGAGCCGAGGACACCGCCGTGTACTAC TGCAAGACCCACGGCTCTCACGACAATTGGGGCCAGGGCACAATGGTCACCGTGTCC TCTGCTTCCACCAAGGGACCCTCTGTGTTCCCTCTGGCTCCTTCCAGCAAGTCTACC TCTGGCGGAACAGCTGCTCTGGGCTGCCTGGTCAAGGACTACTTTCCTGAGCCTGTG ACCGTGTCTTGGAACTCTGGCGCTCTGACATCCGGCGTGCACACCTTTCCAGCTGTG CTGCAATCCTCCGGCCTGTACTCTCTGTCCTCCGTCGTGACCGTGCCTTCTAGCTCT CTGGGCACCCAGACCTACATCTGCAATGTGAACCACAAGCCTTCCAACACCAAGGTG GACAAGAGAGTGGAACCCAAGTCCTGCGATAAGACCCACACCTGTCCACCATGTCCT GCTCCAGAACTGCTCGGCGGACCTTCCGTGTTCCTGTTTCCTCCAAAGCCTAAGGAC ACCCTGATGATCTCTCGGACCCCTGAAGTGACCTGCGTGGTGGTGGATGTGTCTCAC GAGGATCCCGAAGTGAAGTTCAATTGGTACGTGGACGGCGTGGAAGTGCACAACGCC AAGACCAAGCCTAGAGAGGAACAGTACAACTCCACCTACAGAGTGGTGTCCGTGCTG ACCGTGCTGCACCAGGATTGGCTGAACGGCAAAGAGTACAAGTGCAAGGTGTCCAAC AAGGCCCTGCCTGCTCCTATCGAAAAGACCATCTCCAAGGCCAAAGGTGGCGGAGGA AGCGGTGAGGACCTGAACAAGGTTTTCCCACCTGAGGTGGCCCTGTTCGAGCCTTCT GAGGCTGAGATCTCTCACACCCAGAAAGCTACCCTCGTGTGTCTGGCCACCGGCTTC TACCCTGATCACGTGGAACTGTCTTGGTGGGTCAACGGAAAAGAGGTGCACTCCGGC GTCTGCACCGATCCTCAGCCTCTGAAAGAACAGCCCGCTCTGAACGACTCCAGATAC GCCCTGTCCTCTCGGCTGAGAGTGTCTGCCACCTTCTGGCAGGACCCTCGGAACCAC TTTAGATGCCAGGTGCAGTTCTACGGCCTGTCCGAGGCTGATGAGTGGACCCAGGCT AGAGCCAAGCCAGTGACACAGATCGTGTCTGCCGAAGCCTGGGGCAGAGCCGAT |
| SEQ ID NO: 62 | CAGGTGCAGCTGGTGGAATCTGGTGGCGGAGTTGTGCAGCCTGGCAGATCCCTGAGA CTGTCTTGTGCCGCCTCCGGCTTCACCTTCTCCTCTTACGGAATGCACTGGGTCCGA CAGGCCCCTGGCAAAGGATTGGAGTGGGTCGCCTTCATCAGATACGACGGCTCCAAC AAGTACTACGCCGACTCCGTGAAGGGCAGATTCACCATCTCTCGGGACAACTCCAAG AACACCCTGTACCTGCAGATGAACTCCCTGAGAGCCGAGGACACCGCCGTGTACTAC TGCAAGACCCACGGCTCTCACGACAATTGGGGCCAGGGCACAATGGTCACCGTGTCC TCTGCTTCCACCAAGGGACCCTCTGTGTTCCCTCTGGCTCCTTCCAGCAAGTCTACC TCTGGCGGAACAGCTGCTCTGGGCTGCCTGGTCAAGGACTACTTTCCTGAGCCTGTG ACCGTGTCTTGGAACTCTGGCGCTCTGACATCCGGCGTGCACACCTTTCCAGCTGTG CTGCAATCCTCCGGCCTGTACTCTCTGTCCTCCGTCGTGACCGTGCCTTCTAGCTCT CTGGGCACCCAGACCTACATCTGCAATGTGAACCACAAGCCTTCCAACACCAAGGTG GACAAGAGAGTGGAACCCAAGTCCTGCGATAAGACCCACACCTGTCCACCATGTCCT GCTCCAGAACTGCTCGGCGGACCTTCCGTGTTCCTGTTTCCTCCAAAGCCTAAGGAC ACCCTGATGATCTCTCGGACCCCTGAAGTGACCTGCGTGGTGGTGGATGTGTCTCAC GAGGATCCCGAAGTGAAGTTCAATTGGTACGTGGACGGCGTGGAAGTGCACAACGCC AAGACCAAGCCTAGAGAGGAACAGTACAACTCCACCTACAGAGTGGTGTCCGTGCTG |
| | ACCGTGCTGCACCAGGATTGGCTGAACGGCAAAGAGTACAAGTGCAAGGTGTCCAAC AAGGCCCTGCCTGCTCCTATCGAAAAGACCATCTCCAAGGCCAAAGGTGGCGGAGGA AGCGGTGGCGAGGACCTGAACAAGGTTTTCCCACCTGAGGTGGCCCTGTTCGAGCCT TCTGAGGCTGAGATCTCTCACACCCAGAAAGCTACCCTCGTGTGTCTGGCCACCGGC TTCTACCCTGATCACGTGGAACTGTCTTGGTGGGTCAACGGAAAAGAGGTGCACTCC GGCGTCTGCACCGATCCTCAGCCTCTGAAAGAACAGCCCGCTCTGAACGACTCCAGA TACGCCCTGTCCTCTCGGCTGAGAGTGTCTGCCACCTTCTGGCAGGACCCTCGGAAC CACTTTAGATGCCAGGTGCAGTTCTACGGCCTGTCCGAGGCTGATGAGTGGACCCAG GCTAGAGCCAAGCCAGTGACACAGATCGTGTCTGCCGAAGCCTGGGGCAGAGCCGAT |
| SEQ ID NO: 63 | CAGGTGCAGCTGGTGGAATCTGGTGGCGGAGTTGTGCAGCCTGGCAGATCCCTGAGA CTGTCTTGTGCCGCCTCCGGCTTCACCTTCTCCTCTTACGGAATGCACTGGGTCCGA CAGGCCCCTGGCAAAGGATTGGAGTGGGTCGCCTTCATCAGATACGACGGCTCCAAC AAGTACTACGCCGACTCCGTGAAGGGCAGATTCACCATCTCTCGGGACAACTCCAAG AACACCCTGTACCTGCAGATGAACTCCCTGAGAGCCGAGGACACCGCCGTGTACTAC TGCAAGACCCACGGCTCTCACGACAATTGGGGCCAGGGCACAATGGTCACCGTGTCC TCTGCTTCCACCAAGGGACCCTCTGTGTTCCCTCTGGCTCCTTCCAGCAAGTCTACC TCTGGCGGAACAGCTGCTCTGGGCTGCCTGGTCAAGGACTACTTTCCTGAGCCTGTG ACCGTGTCTTGGAACTCTGGCGCTCTGACATCCGGCGTGCACACCTTTCCAGCTGTG CTGCAATCCTCCGGCCTGTACTCTCTGTCCTCCGTCGTGACCGTGCCTTCTAGCTCT CTGGGCACCCAGACCTACATCTGCAATGTGAACCACAAGCCTTCCAACACCAAGGTG GACAAGAGAGTGGAACCCAAGTCCTGCGATAAGACCCACACCTGTCCACCATGTCCT GCTCCAGAACTGCTCGGCGGACCTTCCGTGTTCCTGTTTCCTCCAAAGCCTAAGGAC ACCCTGATGATCTCTCGGACCCCTGAAGTGACCTGCGTGGTGGTGGATGTGTCTCAC GAGGATCCCGAAGTGAAGTTCAATTGGTACGTGGACGGCGTGGAAGTGCACAACGCC AAGACCAAGCCTAGAGAGGAACAGTACAACTCCACCTACAGAGTGGTGTCCGTGCTG ACCGTGCTGCACCAGGATTGGCTGAACGGCAAAGAGTACAAGTGCAAGGTGTCCAAC AAGGCCCTGCCTGCTCCTATCGAAAAGACCATCTCCAAGGCCAAAGGTGGCGGAGGA AGCGGTGGCGGCGAGGACCTGAACAAGGTTTTCCCACCTGAGGTGGCCCTGTTCGAG CCTTCTGAGGCTGAGATCTCTCACACCCAGAAAGCTACCCTCGTGTGTCTGGCCACC GGCTTCTACCCTGATCACGTGGAACTGTCTTGGTGGGTCAACGGAAAAGAGGTGCAC TCCGGCGTCTGCACCGATCCTCAGCCTCTGAAAGAACAGCCCGCTCTGAACGACTCC AGATACGCCCTGTCCTCTCGGCTGAGAGTGTCTGCCACCTTCTGGCAGGACCCTCGG AACCACTTTAGATGCCAGGTGCAGTTCTACGGCCTGTCCGAGGCTGATGAGTGGACC CAGGCTAGAGCCAAGCCAGTGACACAGATCGTGTCTGCCGAAGCCTGGGGCAGAGCC GAT |
| SEQ ID NO: 64 | CAGGTGCAGCTGGTGGAATCTGGTGGCGGAGTTGTGCAGCCTGGCAGATCCCTGAGA CTGTCTTGTGCCGCCTCCGGCTTCACCTTCTCCTCTTACGGAATGCACTGGGTCCGA CAGGCCCCTGGCAAAGGATTGGAGTGGGTCGCCTTCATCAGATACGACGGCTCCAAC AAGTACTACGCCGACTCCGTGAAGGGCAGATTCACCATCTCTCGGGACAACTCCAAG AACACCCTGTACCTGCAGATGAACTCCCTGAGAGCCGAGGACACCGCCGTGTACTAC TGCAAGACCCACGGCTCTCACGACAATTGGGGCCAGGGCACAATGGTCACCGTGTCC TCTGCTTCCACCAAGGGACCCTCTGTGTTCCCTCTGGCTCCTTCCAGCAAGTCTACC TCTGGCGGAACAGCTGCTCTGGGCTGCCTGGTCAAGGACTACTTTCCTGAGCCTGTG ACCGTGTCTTGGAACTCTGGCGCTCTGACATCCGGCGTGCACACCTTTCCAGCTGTG CTGCAATCCTCCGGCCTGTACTCTCTGTCCTCCGTCGTGACCGTGCCTTCTAGCTCT CTGGGCACCCAGACCTACATCTGCAATGTGAACCACAAGCCTTCCAACACCAAGGTG GACAAGAGAGTGGAACCCAAGTCCTGCGATAAGACCCACACCTGTCCACCATGTCCT |
| | GCTCCAGAACTGCTCGGCGGACCTTCCGTGTTCCTGTTTCCTCCAAAGCCTAAGGAC ACCCTGATGATCTCTCGGACCCCTGAAGTGACCTGCGTGGTGGATGTGTCTCAC GAGGATCCCGAAGTGAAGTTCAATTGGTACGTGGACGGCGTGGAAGTGCACAACGCC AAGACCAAGCCTAGAGAGGAACAGTACAACTCCACCTACAGAGTGGTGTCCGTGCTG ACCGTGCTGCACCAGGATTGGCTGAACGGCAAAGAGTACAAGTGCAAGGTGTCCAAC AAGGCCCTGCCTGCTCCTATCGAAAAGACCATCTCCAAGGCCAAAGGTGGCGGAGGA AGCGGTGGCGGCGGAGAGGACCTGAACAAGGTTTTCCCACCTGAGGTGGCCCTGTTC GAGCCTTCTGAGGCTGAGATCTCTCACACCCAGAAAGCTACCCTCGTGTGTCTGGCC ACCGGCTTCTACCCTGATCACGTGGAACTGTCTTGGTGGGTCAACGGAAAAGAGGTG CACTCCGGCGTCTGCACCGATCCTCAGCCTCTGAAAGAACAGCCCGCTCTGAACGAC TCCAGATACGCCCTGTCCTCTCGGCTGAGAGTGTCTGCCACCTTCTGGCAGGACCCT CGGAACCACTTTAGATGCCAGGTGCAGTTCTACGGCCTGTCCGAGGCTGATGAGTGG ACCCAGGCTAGAGCCAAGCCAGTGACACAGATCGTGTCTGCCGAAGCCTGGGGCAGA GCCGAT |
| SEQ ID NO: 65 | CAGGTGCAGCTGGTGGAATCTGGTGGCGGAGTTGTGCAGCCTGGCAGATCCCTGAGA CTGTCTTGTGCCGCCTCCGGCTTCACCTTCTCCTCTTACGGAATGCACTGGGTCCGA CAGGCCCCTGGCAAAGGATTGGAGTGGGTCGCCTTCATCAGATACGACGGCTCCAAC AAGTACTACGCCGACTCCGTGAAGGGCAGATTCACCATCTCTCGGGACAACTCCAAG AACACCCTGTACCTGCAGATGAACTCCCTGAGAGCCGAGGACACCGCCGTGTACTAC TGCAAGACCCACGGCTCTCACGACAATTGGGGCCAGGGCACAATGGTCACCGTGTCC TCTGCTTCCACCAAGGGACCCTCTGTGTTCCCTCTGGCTCCTTCCAGCAAGTCTACC TCTGGCGGAACAGCTGCTCTGGGCTGCCTGGTCAAGGACTACTTTCCTGAGCCTGTG ACCGTGTCTTGGAACTCTGGCGCTCTGACATCCGGCGTGCACACCTTTCCAGCTGTG CTGCAATCCTCCGGCCTGTACTCTCTGTCCTCCGTCGTGACCGTGCCTTCTAGCTCT CTGGGCACCCAGACCTACATCTGCAATGTGAACCACAAGCCTTCCAACACCAAGGTG GACAAGAGAGTGGAACCCAAGTCCTGCGATAAGACCCACACCTGTCCACCATGTCCT GCTCCAGAACTGCTCGGCGGACCTTCCGTGTTCCTGTTTCCTCCAAAGCCTAAGGAC ACCCTGATGATCTCTCGGACCCCTGAAGTGACCTGCGTGGTGGTGGATGTGTCTCAC GAGGATCCCGAAGTGAAGTTCAATTGGTACGTGGACGGCGTGGAAGTGCACAACGCC AAGACCAAGCCTAGAGAGGAACAGTACAACTCCACCTACAGAGTGGTGTCCGTGCTG ACCGTGCTGCACCAGGATTGGCTGAACGGCAAAGAGTACAAGTGCAAGGTGTCCAAC AAGGCCCTGCCTGCTCCTATCGAAAAGACCATCTCCAAGGCCAAAGGTGGCGGAGGA AGCGGTGGCGGCGGATCTGAGGACCTGAACAAGGTTTTCCCACCTGAGGTGGCCCTG TTCGAGCCTTCTGAGGCTGAGATCTCTCACACCCAGAAAGCTACCCTCGTGTGTCTG GCCACCGGCTTCTACCCTGATCACGTGGAACTGTCTTGGTGGGTCAACGGAAAAGAG GTGCACTCCGGCGTCTGCACCGATCCTCAGCCTCTGAAAGAACAGCCCGCTCTGAAC GACTCCAGATACGCCCTGTCCTCTCGGCTGAGAGTGTCTGCCACCTTCTGGCAGGAC CCTCGGAACCACTTTAGATGCCAGGTGCAGTTCTACGGCCTGTCCGAGGCTGATGAG TGGACCCAGGCTAGAGCCAAGCCAGTGACACAGATCGTGTCTGCCGAAGCCTGGGGC AGAGCCGAT |
| SEQ ID NO: 66 | CAGTCCGTGTTGACCCAGCCTCCTTCTGTTTCTGGCGCTCCTGGCCAGAGAGTGACC ATCTCTTGCTCCGGCTCTCGGTCCAACATCGGCTCCAATACCGTGAAGTGGTATCAG CAGCTGCCCGGCACAGCTCCCAAACTGCTGATCTACTACAACGACCAGCGGCCTTCT GGCGTGCCCGATAGATTCTCTGGCTCCAAGTCTGGCACCTCTGCCAGCCTGGCTATT ACCGGACTGCAGGCTGAGGACGAGGCCGACTACTACTGCCAGTCTTACGACCGGTAC ACCCATCCTGCTCTGCTGTTTGGCACCGGCACCAAAGTGACAGTGCTGGGCCAGCCT AAGGCCAATCCTACCGTGACACTGTTCCCTCCATCCTCCGAAGAACTGCAGGCCAAC |
| | AAGGCTACCCTCGTGTGCCTGATCTCCGACTTTTACCCTGGCGCTGTGACCGTGGCC TGGAAGGCTGATGGATCTCCTGTGAAGGCTGGCGTGGAAACCACCAAGCCTTCCAAG CAGTCCAACAACAAATACGCCGCCTCCTCCTACCTGTCTCTGACCCCTGAACAGTGG AAGTCCCACCGGTCCTACAGCTGCCAAGTGACCCATGAGGGCTCCACCGTGGAAAAG ACCGTGGCTCCTACCGAGTGCTCC |
| SEQ ID NO: 67 | ACCATCAAGCCTTGTCCTCCATGCAAGTGCCCCGCTCCTAATCTGCTCGGAGGCCCT TCCGTGTTCATCTTCCCACCTAAGATCAAGGACGTGCTGATGATCTCCCTGTCTCCT ATCGTGACCTGCGTGGTGGTGGACGTGTCCGAGGATGATCCTGACGTGCAGATCAGT TGGTTCGTGAACAACGTGGAAGTGCACACCGCTCAGACCCAGACACACAGAGAGGAC TACAACAGCACCCTGAGAGTGGTGTCTGCCCTGCCTATCCAGCACCAGGATTGGATG TCCGGCAAAGAATTCAAGTGCAAAGTGAACAACAAGGACCTGCCTGCTCCAATCGAG CGGACCATCTCCAAGCCTAAGCCTAACATCCAGAATCCTGAGCCTGCCGTGTACCAG CTGAAGGACCCTAGATCTCAGGACTCTACCCTGTGCCTGTTCACCGACTTCGACTCC CAGATCAACGTGCCCAAGACCATGGAATCCGGCACCTTCATCACCGATAAGTGCGTG CTGGACATGAAGGCCATGGACTCCAAGTCCAACGGCGCTATCGCCTGGTCCAACCAG ACCAGCTTCACATGCCAGGACATCTTCAAAGAGACAAACGCCACCTATCCTTCCAGC GACGTGCCCTGTGATGCTACCCTGACCGAGAAGTCCTTCGAGACAGACATGAACCTG AACTTCCAGAACCTGTCCTGATGA |
| SEQ ID NO: 68 | ACCATCAAGCCTTGTCCTCCATGCAAGTGCCCCGCTCCTAATCTGCTCGGAGGCCCT TCCGTGTTCATCTTCCCACCTAAGATCAAGGACGTGCTGATGATCTCCCTGTCTCCT ATCGTGACCTGCGTGGTGGTGGACGTGTCCGAGGATGATCCTGACGTGCAGATCAGT TGGTTCGTGAACAACGTGGAAGTGCACACCGCTCAGACCCAGACACACAGAGAGGAC TACAACAGCACCCTGAGAGTGGTGTCTGCCCTGCCTATCCAGCACCAGGATTGGATG TCCGGCAAAGAATTCAAGTGCAAAGTGAACAACAAGGACCTGCCTGCTCCAATCGAG CGGACCATCTCCAAGCCTGGTAAGCCTAACATCCAGAATCCTGAGCCTGCCGTGTAC CAGCTGAAGGACCCTAGATCTCAGGACTCTACCCTGTGCCTGTTCACCGACTTCGAC TCCCAGATCAACGTGCCCAAGACCATGGAATCCGGCACCTTCATCACCGATAAGTGC GTGCTGGACATGAAGGCCATGGACTCCAAGTCCAACGGCGCTATCGCCTGGTCCAAC CAGACCAGCTTCACATGCCAGGACATCTTCAAAGAGACAAACGCCACCTATCCTTCC AGCGACGTGCCCTGTGATGCTACCCTGACCGAGAAGTCCTTCGAGACAGACATGAAC CTGAACTTCCAGAACCTGTCCTGATGA |
| SEQ ID NO: 69 | ACCATCAAGCCTTGTCCTCCATGCAAGTGCCCCGCTCCTAATCTGCTCGGAGGCCCT TCCGTGTTCATCTTCCCACCTAAGATCAAGGACGTGCTGATGATCTCCCTGTCTCCT ATCGTGACCTGCGTGGTGGTGGACGTGTCCGAGGATGATCCTGACGTGCAGATCAGT TGGTTCGTGAACAACGTGGAAGTGCACACCGCTCAGACCCAGACACACAGAGAGGAC TACAACAGCACCCTGAGAGTGGTGTCTGCCCTGCCTATCCAGCACCAGGATTGGATG TCCGGCAAAGAATTCAAGTGCAAAGTGAACAACAAGGACCTGCCTGCTCCAATCGAG CGGACCATCTCCAAGCCTGGTGGCGCTTCCACCAAGGGACCCTCTGTGTTCCCTCTG GCTCCTTCCAGCAAGTCTACCTCTGGCGGAACAGCTGCTCTGGGCTGCCTGGTCAAG GACTACTTTCCTGAGCCTGTGACCGTGTCTTGGAACTCTGGCGCTCTGACATCCGGC GTGCACACCTTTCCAGCTGTGCTGCAATCCTCCGGCCTGTACTCTCTGTCCTCCGTC GTGACCGTGCCTTCTAGCTCTCTGGGCACCCAGACCTACATCTGCAATGTGAACCAC AAGCCTTCCAACACCAAGGTGGACAAGAGAGTGGAACCCAAGTCCTGC |
| SEQ ID NO: 70 | ACCATCAAGCCTTGTCCTCCATGCAAGTGCCCCGCTCCTAATCTGCTCGGAGGCCCT TCCGTGTTCATCTTCCCACCTAAGATCAAGGACGTGCTGATGATCTCCCTGTCTCCT ATCGTGACCTGCGTGGTGGTGGACGTGTCCGAGGATGATCCTGACGTGCAGATCAGT TGGTTCGTGAACAACGTGGAAGTGCACACCGCTCAGACCCAGACACACAGAGAGGAC |
| | TACAACAGCACCCTGAGAGTGGTGTCTGCCCTGCCTATCCAGCACCAGGATTGGATG TCCGGCAAAGAATTCAAGTGCAAAGTGAACAACAAGGACCTGCCTGCTCCAATCGAG CGGACCATCTCCAAGCCTGGTGGCGGAAAGCCTAACATCCAGAATCCTGAGCCTGCC GTGTACCAGCTGAAGGACCCTAGATCTCAGGACTCTACCCTGTGCCTGTTCACCGAC TTCGACTCCCAGATCAACGTGCCCAAGACCATGGAATCCGGCACCTTCATCACCGAT AAGTGCGTGCTGGACATGAAGGCCATGGACTCCAAGTCCAACGGCGCTATCGCCTGG TCCAACCAGACCAGCTTCACATGCCAGGACATCTTCAAAGAGACAAACGCCACCTAT CCTTCCAGCGACGTGCCCTGTGATGCTACCCTGACCGAGAAGTCCTTCGAGACAGAC ATGAACCTGAACTTCCAGAACCTGTCCTGATGA |
| SEQ ID NO: 71 | ACCATCAAGCCTTGTCCTCCATGCAAGTGCCCCGCTCCTAATCTGCTCGGAGGCCCT TCCGTGTTCATCTTCCCACCTAAGATCAAGGACGTGCTGATGATCTCCCTGTCTCCT ATCGTGACCTGCGTGGTGGTGGACGTGTCCGAGGATGATCCTGACGTGCAGATCAGT TGGTTCGTGAACAACGTGGAAGTGCACACCGCTCAGACCCAGACACACAGAGAGGAC TACAACAGCACCCTGAGAGTGGTGTCTGCCCTGCCTATCCAGCACCAGGATTGGATG TCCGGCAAAGAATTCAAGTGCAAAGTGAACAACAAGGACCTGCCTGCTCCAATCGAG CGGACCATCTCCAAGCCTGGTGGCGGAGGAAAGCCTAACATCCAGAATCCTGAGCCT GCCGTGTACCAGCTGAAGGACCCTAGATCTCAGGACTCTACCCTGTGCCTGTTCACC GACTTCGACTCCCAGATCAACGTGCCCAAGACCATGGAATCCGGCACCTTCATCACC GATAAGTGCGTGCTGGACATGAAGGCCATGGACTCCAAGTCCAACGGCGCTATCGCC TGGTCCAACCAGACCAGCTTCACATGCCAGGACATCTTCAAAGAGACAAACGCCACC TATCCTTCCAGCGACGTGCCCTGTGATGCTACCCTGACCGAGAAGTCCTTCGAGACA GACATGAACCTGAACTTCCAGAACCTGTCCTGATGA |
| SEQ ID NO: 72 | ACCATCAAGCCTTGTCCTCCATGCAAGTGCCCCGCTCCTAATCTGCTCGGAGGCCCT TCCGTGTTCATCTTCCCACCTAAGATCAAGGACGTGCTGATGATCTCCCTGTCTCCT ATCGTGACCTGCGTGGTGGTGGACGTGTCCGAGGATGATCCTGACGTGCAGATCAGT TGGTTCGTGAACAACGTGGAAGTGCACACCGCTCAGACCCAGACACACAGAGAGGAC TACAACAGCACCCTGAGAGTGGTGTCTGCCCTGCCTATCCAGCACCAGGATTGGATG TCCGGCAAAGAATTCAAGTGCAAAGTGAACAACAAGGACCTGCCTGCTCCAATCGAG CGGACCATCTCCAAGCCTGGTGGCGGAGGAAGCAAGCCTAACATCCAGAATCCTGAG CCTGCCGTGTACCAGCTGAAGGACCCTAGATCTCAGGACTCTACCCTGTGCCTGTTC ACCGACTTCGACTCCCAGATCAACGTGCCCAAGACCATGGAATCCGGCACCTTCATC ACCGATAAGTGCGTGCTGGACATGAAGGCCATGGACTCCAAGTCCAACGGCGCTATC GCCTGGTCCAACCAGACCAGCTTCACATGCCAGGACATCTTCAAAGAGACAAACGCC ACCTATCCTTCCAGCGACGTGCCCTGTGATGCTACCCTGACCGAGAAGTCCTTCGAG ACAGACATGAACCTGAACTTCCAGAACCTGTCCTGATGA |
| SEQ ID NO: 73 | ACCATCAAGCCTTGTCCTCCATGCAAGTGCCCCGCTCCTAATCTGCTCGGAGGCCCT TCCGTGTTCATCTTCCCACCTAAGATCAAGGACGTGCTGATGATCTCCCTGTCTCCT ATCGTGACCTGCGTGGTGGTGGACGTGTCCGAGGATGATCCTGACGTGCAGATCAGT TGGTTCGTGAACAACGTGGAAGTGCACACCGCTCAGACCCAGACACACAGAGAGGAC TACAACAGCACCCTGAGAGTGGTGTCTGCCCTGCCTATCCAGCACCAGGATTGGATG TCCGGCAAAGAATTCAAGTGCAAAGTGAACAACAAGGACCTGCCTGCTCCAATCGAG CGGACCATCTCCAAGCCTGGTGGCGGAGGAAGCGGTAAGCCTAACATCCAGAATCCT GAGCCTGCCGTGTACCAGCTGAAGGACCCTAGATCTCAGGACTCTACCCTGTGCCTG TTCACCGACTTCGACTCCCAGATCAACGTGCCCAAGACCATGGAATCCGGCACCTTC ATCACCGATAAGTGCGTGCTGGACATGAAGGCCATGGACTCCAAGTCCAACGGCGCT ATCGCCTGGTCCAACCAGACCAGCTTCACATGCCAGGACATCTTCAAAGAGACAAAC |
| | GCCACCTATCCTTCCAGCGACGTGCCCTGTGATGCTACCCTGACCGAGAAGTCCTTC GAGACAGACATGAACCTGAACTTCCAGAACCTGTCCTGATGA |
| SEQ ID NO: 74 | ACCATCAAGCCTTGTCCTCCATGCAAGTGCCCCGCTCCTAATCTGCTCGGAGGCCCT TCCGTGTTCATCTTCCCACCTAAGATCAAGGACGTGCTGATGATCTCCCTGTCTCCT ATCGTGACCTGCGTGGTGGTGGACGTGTCCGAGGATGATCCTGACGTGCAGATCAGT TGGTTCGTGAACAACGTGGAAGTGCACACCGCTCAGACCCAGACACACAGAGAGGAC TACAACAGCACCCTGAGAGTGGTGTCTGCCCTGCCTATCCAGCACCAGGATTGGATG TCCGGCAAAGAATTCAAGTGCAAAGTGAACAACAAGGACCTGCCTGCTCCAATCGAG CGGACCATCTCCAAGCCTGGTGGCGGAGGAAGCGGTGGCAAGCCTAACATCCAGAAT CCTGAGCCTGCCGTGTACCAGCTGAAGGACCCTAGATCTCAGGACTCTACCCTGTGC CTGTTCACCGACTTCGACTCCCAGATCAACGTGCCCAAGACCATGGAATCCGGCACC TTCATCACCGATAAGTGCGTGCTGGACATGAAGGCCATGGACTCCAAGTCCAACGGC GCTATCGCCTGGTCCAACCAGACCAGCTTCACATGCCAGGACATCTTCAAAGAGACA AACGCCACCTATCCTTCCAGCGACGTGCCCTGTGATGCTACCCTGACCGAGAAGTCC TTCGAGACAGACATGAACCTGAACTTCCAGAACCTGTCCTGATGA |
| SEQ ID NO: 75 | ACCATCAAGCCTTGTCCTCCATGCAAGTGCCCCGCTCCTAATCTGCTCGGAGGCCCT TCCGTGTTCATCTTCCCACCTAAGATCAAGGACGTGCTGATGATCTCCCTGTCTCCT ATCGTGACCTGCGTGGTGGTGGACGTGTCCGAGGATGATCCTGACGTGCAGATCAGT TGGTTCGTGAACAACGTGGAAGTGCACACCGCTCAGACCCAGACACACAGAGAGGAC TACAACAGCACCCTGAGAGTGGTGTCTGCCCTGCCTATCCAGCACCAGGATTGGATG TCCGGCAAAGAATTCAAGTGCAAAGTGAACAACAAGGACCTGCCTGCTCCAATCGAG CGGACCATCTCCAAGCCTGGTGGCGGAGGAAGCGGTGGCGGCAAGCCTAACATCCAG AATCCTGAGCCTGCCGTGTACCAGCTGAAGGACCCTAGATCTCAGGACTCTACCCTG TGCCTGTTCACCGACTTCGACTCCCAGATCAACGTGCCCAAGACCATGGAATCCGGC ACCTTCATCACCGATAAGTGCGTGCTGGACATGAAGGCCATGGACTCCAAGTCCAAC GGCGCTATCGCCTGGTCCAACCAGACCAGCTTCACATGCCAGGACATCTTCAAAGAG ACAAACGCCACCTATCCTTCCAGCGACGTGCCCTGTGATGCTACCCTGACCGAGAAG TCCTTCGAGACAGACATGAACCTGAACTTCCAGAACCTGTCCTGATGA |
| SEQ ID NO: 76 | ACCATCAAGCCTTGTCCTCCATGCAAGTGCCCCGCTCCTAATCTGCTCGGAGGCCCT TCCGTGTTCATCTTCCCACCTAAGATCAAGGACGTGCTGATGATCTCCCTGTCTCCT ATCGTGACCTGCGTGGTGGTGGACGTGTCCGAGGATGATCCTGACGTGCAGATCAGT TGGTTCGTGAACAACGTGGAAGTGCACACCGCTCAGACCCAGACACACAGAGAGGAC TACAACAGCACCCTGAGAGTGGTGTCTGCCCTGCCTATCCAGCACCAGGATTGGATG TCCGGCAAAGAATTCAAGTGCAAAGTGAACAACAAGGACCTGCCTGCTCCAATCGAG CGGACCATCTCCAAGCCTGGTGGCGGAGGAAGCGGTGGCGGCGGAAAGCCTAACATC CAGAATCCTGAGCCTGCCGTGTACCAGCTGAAGGACCCTAGATCTCAGGACTCTACC CTGTGCCTGTTCACCGACTTCGACTCCCAGATCAACGTGCCCAAGACCATGGAATCC GGCACCTTCATCACCGATAAGTGCGTGCTGGACATGAAGGCCATGGACTCCAAGTCC AACGGCGCTATCGCCTGGTCCAACCAGACCAGCTTCACATGCCAGGACATCTTCAAA GAGACAAACGCCACCTATCCTTCCAGCGACGTGCCCTGTGATGCTACCCTGACCGAG AAGTCCTTCGAGACAGACATGAACCTGAACTTCCAGAACCTGTCCTGATGA |
| SEQ ID NO: 77 | ACCATCAAGCCTTGTCCTCCATGCAAGTGCCCCGCTCCTAATCTGCTCGGAGGCCCT TCCGTGTTCATCTTCCCACCTAAGATCAAGGACGTGCTGATGATCTCCCTGTCTCCT ATCGTGACCTGCGTGGTGGTGGACGTGTCCGAGGATGATCCTGACGTGCAGATCAGT TGGTTCGTGAACAACGTGGAAGTGCACACCGCTCAGACCCAGACACACAGAGAGGAC TACAACAGCACCCTGAGAGTGGTGTCTGCCCTGCCTATCCAGCACCAGGATTGGATG TCCGGCAAAGAATTCAAGTGCAAAGTGAACAACAAGGACCTGCCTGCTCCAATCGAG |
| | CGGACCATCTCCAAGCCTGGTGGCGGAGGAAGCGGTGGCGGCGGATCTAAGCCTAAC ATCCAGAATCCTGAGCCTGCCGTGTACCAGCTGAAGGACCCTAGATCTCAGGACTCT ACCCTGTGCCTGTTCACCGACTTCGACTCCCAGATCAACGTGCCCAAGACCATGGAA TCCGGCACCTTCATCACCGATAAGTGCGTGCTGGACATGAAGGCCATGGACTCCAAG TCCAACGGCGCTATCGCCTGGTCCAACCAGACCAGCTTCACATGCCAGGACATCTTC AAAGAGACAAACGCCACCTATCCTTCCAGCGACGTGCCCTGTGATGCTACCCTGACC GAGAAGTCCTTCGAGACAGACATGAACCTGAACTTCCAGAACCTGTCCTGATGA |
| SEQ ID NO: 78 | ACCATCAAGCCTTGTCCTCCATGCAAGTGCCCCGCTCCTAATCTGCTCGGAGGCCCT TCCGTGTTCATCTTCCCACCTAAGATCAAGGACGTGCTGATGATCTCCCTGTCTCCT ATCGTGACCTGCGTGGTGGTGGACGTGTCCGAGGATGATCCTGACGTGCAGATCAGT TGGTTCGTGAACAACGTGGAAGTGCACACCGCTCAGACCCAGACACACAGAGAGGAC TACAACAGCACCCTGAGAGTGGTGTCTGCCCTGCCTATCCAGCACCAGGATTGGATG TCCGGCAAAGAATTCAAGTGCAAAGTGAACAACAAGGACCTGCCTGCTCCAATCGAG CGGACCATCTCCAAGCCTGTGGTGGAAGATCTGCGGAACGTGACCCCTCCTAAGGTG TCCCTGTTCGAGCCTTCCAAGGCCGAGATCGCCAACAAGCAGAAAGCTACCCTCGTG TGCCTGGCCAGAGGCTTCTTTCCTGACCACGTGGAACTGTCTTGGTGGGTCAACGGC AAAGAGGTGCACTCCGGCGTCTGTACCGATCCTCAGGCCTACAAAGAGTCCAACTAC TCCTACAGCCTGTCCTCTCGGCTGAGAGTGTCTGCCACCTTCTGGCACAACCCTCGG AACCACTTCAGATGCCAGGTGCAGTTTCACGGCCTGTCCGAAGAGGACAAGTGGCCT GAGGGATCCCCTAAGCCTGTGACACAGAACATCTCTGCCGAGGCCTGGGGTAGAGCT GATGGTGGCGGAGGATCTGGCGGAGGTGGAAGCGGCGGAGGCGGATCTTCTCATCTG GAACAGCTGCTGATGGACCTGCAAGAGCTGCTGTCCCGGATGGAAAACTACCGGAAC CTGAAGCTGCCCCGGATGCTGACCGCTAAGTTCGCTCTGCCTAAGCAGGCCACCGAG CTGAAGGATCTGCAGTGCCTGGAAGATGAGCTGGGCCCTCTGAGACACGTGCTGGAT CTGACCCAGTCCAAGTCCTTTCAGCTCGAGGACGCCGAGAACTTCATCTCCAACATC AGAGTGACCGTGGTCAAGCTGAAGGGCTCCGACAACACCTTCGAGTGCCAGTTCGAC GATGAGTCCGCTACAGTGGTGGACTTCCTGCGGAGATGGATCGCCTTCTGCCAGTCC ATCATCTCCACCTCTCCACAGTGATGA |
| SEQ ID NO: 79 | ACCATCAAGCCTTGTCCTCCATGCAAGTGCCCCGCTCCTAATCTGCTCGGAGGCCCT TCCGTGTTCATCTTCCCACCTAAGATCAAGGACGTGCTGATGATCTCCCTGTCTCCT ATCGTGACCTGCGTGGTGGTGGACGTGTCCGAGGATGATCCTGACGTGCAGATCAGT TGGTTCGTGAACAACGTGGAAGTGCACACCGCTCAGACCCAGACACACAGAGAGGAC TACAACAGCACCCTGAGAGTGGTGTCTGCCCTGCCTATCCAGCACCAGGATTGGATG TCCGGCAAAGAATTCAAGTGCAAAGTGAACAACAAGGACCTGCCTGCTCCAATCGAG CGGACCATCTCCAAGCCTGGTGTGGTGGAAGATCTGCGGAACGTGACCCCTCCTAAG GTGTCCCTGTTCGAGCCTTCCAAGGCCGAGATCGCCAACAAGCAGAAAGCTACCCTC GTGTGCCTGGCCAGAGGCTTCTTTCCTGACCACGTGGAACTGTCTTGGTGGGTCAAC GGCAAAGAGGTGCACTCCGGCGTCTGTACCGATCCTCAGGCCTACAAAGAGTCCAAC TACTCCTACAGCCTGTCCTCTCGGCTGAGAGTGTCTGCCACCTTCTGGCACAACCCT CGGAACCACTTCAGATGCCAGGTGCAGTTTCACGGCCTGTCCGAAGAGGACAAGTGG CCTGAGGGATCCCCTAAGCCTGTGACACAGAACATCTCTGCCGAGGCCTGGGGTAGA GCTGATGGTGGCGGAGGATCTGGCGGAGGTGGAAGCGGCGGAGGCGGATCTTCTCAT CTGGAACAGCTGCTGATGGACCTGCAAGAGCTGCTGTCCCGGATGGAAAACTACCGG AACCTGAAGCTGCCCCGGATGCTGACCGCTAAGTTCGCTCTGCCTAAGCAGGCCACC GAGCTGAAGGATCTGCAGTGCCTGGAAGATGAGCTGGGCCCTCTGAGACACGTGCTG GATCTGACCCAGTCCAAGTCCTTTCAGCTCGAGGACGCCGAGAACTTCATCTCCAAC ATCAGAGTGACCGTGGTCAAGCTGAAGGGCTCCGACAACACCTTCGAGTGCCAGTTC |
| | GACGATGAGTCCGCTACAGTGGTGGACTTCCTGCGGAGATGGATCGCCTTCTGCCAG TCCATCATCTCCACCTCTCCACAGTGATGA |
| SEQ ID NO: 80 | ACCATCAAGCCTTGTCCTCCATGCAAGTGCCCCGCTCCTAATCTGCTCGGAGGCCCT TCCGTGTTCATCTTCCCACCTAAGATCAAGGACGTGCTGATGATCTCCCTGTCTCCT ATCGTGACCTGCGTGGTGGTGGACGTGTCCGAGGATGATCCTGACGTGCAGATCAGT TGGTTCGTGAACAACGTGGAAGTGCACACCGCTCAGACCCAGACACACAGAGAGGAC TACAACAGCACCCTGAGAGTGGTGTCTGCCCTGCCTATCCAGCACCAGGATTGGATG TCCGGCAAAGAATTCAAGTGCAAAGTGAACAACAAGGACCTGCCTGCTCCAATCGAG CGGACCATCTCCAAGCCTGGTGGCGTGGTGGAAGATCTGCGGAACGTGACCCCTCCT AAGGTGTCCCTGTTCGAGCCTTCCAAGGCCGAGATCGCCAACAAGCAGAAAGCTACC CTCGTGTGCCTGGCCAGAGGCTTCTTTCCTGACCACGTGGAACTGTCTTGGTGGGTC AACGGCAAAGAGGTGCACTCCGGCGTCTGTACCGATCCTCAGGCCTACAAAGAGTCC AACTACTCCTACAGCCTGTCCTCTCGGCTGAGAGTGTCTGCCACCTTCTGGCACAAC CCTCGGAACCACTTCAGATGCCAGGTGCAGTTTCACGGCCTGTCCGAAGAGGACAAG TGGCCTGAGGGATCCCCTAAGCCTGTGACACAGAACATCTCTGCCGAGGCCTGGGGT AGAGCTGATGGTGGCGGAGGATCTGGCGGAGGTGGAAGCGGCGGAGGCGGATCTTCT CATCTGGAACAGCTGCTGATGGACCTGCAAGAGCTGCTGTCCCGGATGGAAAACTAC CGGAACCTGAAGCTGCCCCGGATGCTGACCGCTAAGTTCGCTCTGCCTAAGCAGGCC ACCGAGCTGAAGGATCTGCAGTGCCTGGAAGATGAGCTGGGCCCTCTGAGACACGTG CTGGATCTGACCCAGTCCAAGTCCTTTCAGCTCGAGGACGCCGAGAACTTCATCTCC AACATCAGAGTGACCGTGGTCAAGCTGAAGGGCTCCGACAACACCTTCGAGTGCCAG TTCGACGATGAGTCCGCTACAGTGGTGGACTTCCTGCGGAGATGGATCGCCTTCTGC CAGTCCATCATCTCCACCTCTCCACAGTGATGA |
| SEQ ID NO: 81 | ACCATCAAGCCTTGTCCTCCATGCAAGTGCCCCGCTCCTAATCTGCTCGGAGGCCCT TCCGTGTTCATCTTCCCACCTAAGATCAAGGACGTGCTGATGATCTCCCTGTCTCCT ATCGTGACCTGCGTGGTGGTGGACGTGTCCGAGGATGATCCTGACGTGCAGATCAGT TGGTTCGTGAACAACGTGGAAGTGCACACCGCTCAGACCCAGACACACAGAGAGGAC TACAACAGCACCCTGAGAGTGGTGTCTGCCCTGCCTATCCAGCACCAGGATTGGATG TCCGGCAAAGAATTCAAGTGCAAAGTGAACAACAAGGACCTGCCTGCTCCAATCGAG CGGACCATCTCCAAGCCTGGTGGCGGAGTGGTGGAAGATCTGCGGAACGTGACCCCT CCTAAGGTGTCCCTGTTCGAGCCTTCCAAGGCCGAGATCGCCAACAAGCAGAAAGCT ACCCTCGTGTGCCTGGCCAGAGGCTTCTTTCCTGACCACGTGGAACTGTCTTGGTGG GTCAACGGCAAAGAGGTGCACTCCGGCGTCTGTACCGATCCTCAGGCCTACAAAGAG TCCAACTACTCCTACAGCCTGTCCTCTCGGCTGAGAGTGTCTGCCACCTTCTGGCAC AACCCTCGGAACCACTTCAGATGCCAGGTGCAGTTTCACGGCCTGTCCGAAGAGGAC AAGTGGCCTGAGGGATCCCCTAAGCCTGTGACACAGAACATCTCTGCCGAGGCCTGG GGTAGAGCTGATGGTGGCGGAGGATCTGGCGGAGGTGGAAGCGGCGGAGGCGGATCT TCTCATCTGGAACAGCTGCTGATGGACCTGCAAGAGCTGCTGTCCCGGATGGAAAAC TACCGGAACCTGAAGCTGCCCCGGATGCTGACCGCTAAGTTCGCTCTGCCTAAGCAG GCCACCGAGCTGAAGGATCTGCAGTGCCTGGAAGATGAGCTGGGCCCTCTGAGACAC GTGCTGGATCTGACCCAGTCCAAGTCCTTTCAGCTCGAGGACGCCGAGAACTTCATC TCCAACATCAGAGTGACCGTGGTCAAGCTGAAGGGCTCCGACAACACCTTCGAGTGC CAGTTCGACGATGAGTCCGCTACAGTGGTGGACTTCCTGCGGAGATGGATCGCCTTC TGCCAGTCCATCATCTCCACCTCTCCACAGTGATGA |
| SEQ ID NO: 82 | ACCATCAAGCCTTGTCCTCCATGCAAGTGCCCCGCTCCTAATCTGCTCGGAGGCCCT TCCGTGTTCATCTTCCCACCTAAGATCAAGGACGTGCTGATGATCTCCCTGTCTCCT ATCGTGACCTGCGTGGTGGTGGACGTGTCCGAGGATGATCCTGACGTGCAGATCAGT |
| | TGGTTCGTGAACAACGTGGAAGTGCACACCGCTCAGACCCAGACACACAGAGAGGAC TACAACAGCACCCTGAGAGTGGTGTCTGCCCTGCCTATCCAGCACCAGGATTGGATG TCCGGCAAAGAATTCAAGTGCAAAGTGAACAACAAGGACCTGCCTGCTCCAATCGAG CGGACCATCTCCAAGCCTGGTGGCGGAGGAGTGGTGGAAGATCTGCGGAACGTGACC CCTCCTAAGGTGTCCCTGTTCGAGCCTTCCAAGGCCGAGATCGCCAACAAGCAGAAA GCTACCCTCGTGTGCCTGGCCAGAGGCTTCTTTCCTGACCACGTGGAACTGTCTTGG TGGGTCAACGGCAAAGAGGTGCACTCCGGCGTCTGTACCGATCCTCAGGCCTACAAA GAGTCCAACTACTCCTACAGCCTGTCCTCTCGGCTGAGAGTGTCTGCCACCTTCTGG CACAACCCTCGGAACCACTTCAGATGCCAGGTGCAGTTTCACGGCCTGTCCGAAGAG GACAAGTGGCCTGAGGGATCCCCTAAGCCTGTGACACAGAACATCTCTGCCGAGGCC TGGGGTAGAGCTGATGGTGGCGGAGGATCTGGCGGAGGTGGAAGCGGCGGAGGCGGA TCTTCTCATCTGGAACAGCTGCTGATGGACCTGCAAGAGCTGCTGTCCCGGATGGAA AACTACCGGAACCTGAAGCTGCCCCGGATGCTGACCGCTAAGTTCGCTCTGCCTAAG CAGGCCACCGAGCTGAAGGATCTGCAGTGCCTGGAAGATGAGCTGGGCCCTCTGAGA CACGTGCTGGATCTGACCCAGTCCAAGTCCTTTCAGCTCGAGGACGCCGAGAACTTC ATCTCCAACATCAGAGTGACCGTGGTCAAGCTGAAGGGCTCCGACAACACCTTCGAG TGCCAGTTCGACGATGAGTCCGCTACAGTGGTGGACTTCCTGCGGAGATGGATCGCC TTCTGCCAGTCCATCATCTCCACCTCTCCACAGTGATGA |
| SEQ ID NO: 83 | ACCATCAAGCCTTGTCCTCCATGCAAGTGCCCCGCTCCTAATCTGCTCGGAGGCCCT TCCGTGTTCATCTTCCCACCTAAGATCAAGGACGTGCTGATGATCTCCCTGTCTCCT ATCGTGACCTGCGTGGTGGTGGACGTGTCCGAGGATGATCCTGACGTGCAGATCAGT TGGTTCGTGAACAACGTGGAAGTGCACACCGCTCAGACCCAGACACACAGAGAGGAC TACAACAGCACCCTGAGAGTGGTGTCTGCCCTGCCTATCCAGCACCAGGATTGGATG TCCGGCAAAGAATTCAAGTGCAAAGTGAACAACAAGGACCTGCCTGCTCCAATCGAG CGGACCATCTCCAAGCCTGGTGGCGGAGGAAGCGTGGTGGAAGATCTGCGGAACGTG ACCCCTCCTAAGGTGTCCCTGTTCGAGCCTTCCAAGGCCGAGATCGCCAACAAGCAG AAAGCTACCCTCGTGTGCCTGGCCAGAGGCTTCTTTCCTGACCACGTGGAACTGTCT TGGTGGGTCAACGGCAAAGAGGTGCACTCCGGCGTCTGTACCGATCCTCAGGCCTAC AAAGAGTCCAACTACTCCTACAGCCTGTCCTCTCGGCTGAGAGTGTCTGCCACCTTC TGGCACAACCCTCGGAACCACTTCAGATGCCAGGTGCAGTTTCACGGCCTGTCCGAA GAGGACAAGTGGCCTGAGGGATCCCCTAAGCCTGTGACACAGAACATCTCTGCCGAG GCCTGGGGTAGAGCTGATGGTGGCGGAGGATCTGGCGGAGGTGGAAGCGGCGGAGGC GGATCTTCTCATCTGGAACAGCTGCTGATGGACCTGCAAGAGCTGCTGTCCCGGATG GAAAACTACCGGAACCTGAAGCTGCCCCGGATGCTGACCGCTAAGTTCGCTCTGCCT AAGCAGGCCACCGAGCTGAAGGATCTGCAGTGCCTGGAAGATGAGCTGGGCCCTCTG AGACACGTGCTGGATCTGACCCAGTCCAAGTCCTTTCAGCTCGAGGACGCCGAGAAC TTCATCTCCAACATCAGAGTGACCGTGGTCAAGCTGAAGGGCTCCGACAACACCTTC GAGTGCCAGTTCGACGATGAGTCCGCTACAGTGGTGGACTTCCTGCGGAGATGGATC GCCTTCTGCCAGTCCATCATCTCCACCTCTCCACAGTGATGA |
| SEQ ID NO: 84 | ACCATCAAGCCTTGTCCTCCATGCAAGTGCCCCGCTCCTAATCTGCTCGGAGGCCCT TCCGTGTTCATCTTCCCACCTAAGATCAAGGACGTGCTGATGATCTCCCTGTCTCCT ATCGTGACCTGCGTGGTGGTGGACGTGTCCGAGGATGATCCTGACGTGCAGATCAGT TGGTTCGTGAACAACGTGGAAGTGCACACCGCTCAGACCCAGACACACAGAGAGGAC TACAACAGCACCCTGAGAGTGGTGTCTGCCCTGCCTATCCAGCACCAGGATTGGATG TCCGGCAAAGAATTCAAGTGCAAAGTGAACAACAAGGACCTGCCTGCTCCAATCGAG CGGACCATCTCCAAGCCTGGTGGCGGAGGAAGCGGTGTGGTGGAAGATCTGCGGAAC GTGACCCCTCCTAAGGTGTCCCTGTTCGAGCCTTCCAAGGCCGAGATCGCCAACAAG |
| | CAGAAAGCTACCCTCGTGTGCCTGGCCAGAGGCTTCTTTCCTGACCACGTGGAACTG TCTTGGTGGGTCAACGGCAAAGAGGTGCACTCCGGCGTCTGTACCGATCCTCAGGCC TACAAAGAGTCCAACTACTCCTACAGCCTGTCCTCTCGGCTGAGAGTGTCTGCCACC TTCTGGCACAACCCTCGGAACCACTTCAGATGCCAGGTGCAGTTTCACGGCCTGTCC GAAGAGGACAAGTGGCCTGAGGGATCCCCTAAGCCTGTGACACAGAACATCTCTGCC GAGGCCTGGGGTAGAGCTGATGGTGGCGGAGGATCTGGCGGAGGTGGAAGCGGCGGA GGCGGATCTTCTCATCTGGAACAGCTGCTGATGGACCTGCAAGAGCTGCTGTCCCGG ATGGAAAACTACCGGAACCTGAAGCTGCCCCGGATGCTGACCGCTAAGTTCGCTCTG CCTAAGCAGGCCACCGAGCTGAAGGATCTGCAGTGCCTGGAAGATGAGCTGGGCCCT CTGAGACACGTGCTGGATCTGACCCAGTCCAAGTCCTTTCAGCTCGAGGACGCCGAG AACTTCATCTCCAACATCAGAGTGACCGTGGTCAAGCTGAAGGGCTCCGACAACACC TTCGAGTGCCAGTTCGACGATGAGTCCGCTACAGTGGTGGACTTCCTGCGGAGATGG ATCGCCTTCTGCCAGTCCATCATCTCCACCTCTCCACAGTGATGA |
| SEQ ID NO: 85 | ACCATCAAGCCTTGTCCTCCATGCAAGTGCCCCGCTCCTAATCTGCTCGGAGGCCCT TCCGTGTTCATCTTCCCACCTAAGATCAAGGACGTGCTGATGATCTCCCTGTCTCCT ATCGTGACCTGCGTGGTGGTGGACGTGTCCGAGGATGATCCTGACGTGCAGATCAGT TGGTTCGTGAACAACGTGGAAGTGCACACCGCTCAGACCCAGACACACAGAGAGGAC TACAACAGCACCCTGAGAGTGGTGTCTGCCCTGCCTATCCAGCACCAGGATTGGATG TCCGGCAAAGAATTCAAGTGCAAAGTGAACAACAAGGACCTGCCTGCTCCAATCGAG CGGACCATCTCCAAGCCTGGTGGCGGAGGAAGCGGTGGCGTGGTGGAAGATCTGCGG AACGTGACCCCTCCTAAGGTGTCCCTGTTCGAGCCTTCCAAGGCCGAGATCGCCAAC AAGCAGAAAGCTACCCTCGTGTGCCTGGCCAGAGGCTTCTTTCCTGACCACGTGGAA CTGTCTTGGTGGGTCAACGGCAAAGAGGTGCACTCCGGCGTCTGTACCGATCCTCAG GCCTACAAAGAGTCCAACTACTCCTACAGCCTGTCCTCTCGGCTGAGAGTGTCTGCC ACCTTCTGGCACAACCCTCGGAACCACTTCAGATGCCAGGTGCAGTTTCACGGCCTG TCCGAAGAGGACAAGTGGCCTGAGGGATCCCCTAAGCCTGTGACACAGAACATCTCT GCCGAGGCCTGGGGTAGAGCTGATGGTGGCGGAGGATCTGGCGGAGGTGGAAGCGGC GGAGGCGGATCTTCTCATCTGGAACAGCTGCTGATGGACCTGCAAGAGCTGCTGTCC CGGATGGAAAACTACCGGAACCTGAAGCTGCCCCGGATGCTGACCGCTAAGTTCGCT CTGCCTAAGCAGGCCACCGAGCTGAAGGATCTGCAGTGCCTGGAAGATGAGCTGGGC CCTCTGAGACACGTGCTGGATCTGACCCAGTCCAAGTCCTTTCAGCTCGAGGACGCC GAGAACTTCATCTCCAACATCAGAGTGACCGTGGTCAAGCTGAAGGGCTCCGACAAC ACCTTCGAGTGCCAGTTCGACGATGAGTCCGCTACAGTGGTGGACTTCCTGCGGAGA TGGATCGCCTTCTGCCAGTCCATCATCTCCACCTCTCCACAGTGATGA |
| SEQ ID NO: 86 | ACCATCAAGCCTTGTCCTCCATGCAAGTGCCCCGCTCCTAATCTGCTCGGAGGCCCT TCCGTGTTCATCTTCCCACCTAAGATCAAGGACGTGCTGATGATCTCCCTGTCTCCT ATCGTGACCTGCGTGGTGGTGGACGTGTCCGAGGATGATCCTGACGTGCAGATCAGT TGGTTCGTGAACAACGTGGAAGTGCACACCGCTCAGACCCAGACACACAGAGAGGAC TACAACAGCACCCTGAGAGTGGTGTCTGCCCTGCCTATCCAGCACCAGGATTGGATG TCCGGCAAAGAATTCAAGTGCAAAGTGAACAACAAGGACCTGCCTGCTCCAATCGAG CGGACCATCTCCAAGCCTGGTGGCGGAGGAAGCGGTGGCGGCGTGGTGGAAGATCTG CGGAACGTGACCCCTCCTAAGGTGTCCCTGTTCGAGCCTTCCAAGGCCGAGATCGCC AACAAGCAGAAAGCTACCCTCGTGTGCCTGGCCAGAGGCTTCTTTCCTGACCACGTG GAACTGTCTTGGTGGGTCAACGGCAAAGAGGTGCACTCCGGCGTCTGTACCGATCCT CAGGCCTACAAAGAGTCCAACTACTCCTACAGCCTGTCCTCTCGGCTGAGAGTGTCT GCCACCTTCTGGCACAACCCTCGGAACCACTTCAGATGCCAGGTGCAGTTTCACGGC CTGTCCGAAGAGGACAAGTGGCCTGAGGGATCCCCTAAGCCTGTGACACAGAACATC |
| | TCTGCCGAGGCCTGGGGTAGAGCTGATGGTGGCGGAGGATCTGGCGGAGGTGGAAGC GGCGGAGGCGGATCTTCTCATCTGGAACAGCTGCTGATGGACCTGCAAGAGCTGCTG TCCCGGATGGAAAACTACCGGAACCTGAAGCTGCCCCGGATGCTGACCGCTAAGTTC GCTCTGCCTAAGCAGGCCACCGAGCTGAAGGATCTGCAGTGCCTGGAAGATGAGCTG GGCCCTCTGAGACACGTGCTGGATCTGACCCAGTCCAAGTCCTTTCAGCTCGAGGAC GCCGAGAACTTCATCTCCAACATCAGAGTGACCGTGGTCAAGCTGAAGGGCTCCGAC AACACCTTCGAGTGCCAGTTCGACGATGAGTCCGCTACAGTGGTGGACTTCCTGCGG AGATGGATCGCCTTCTGCCAGTCCATCATCTCCACCTCTCCACAGTGATGA |
| SEQ ID NO: 87 | ACCATCAAGCCTTGTCCTCCATGCAAGTGCCCCGCTCCTAATCTGCTCGGAGGCCCT TCCGTGTTCATCTTCCCACCTAAGATCAAGGACGTGCTGATGATCTCCCTGTCTCCT ATCGTGACCTGCGTGGTGGTGGACGTGTCCGAGGATGATCCTGACGTGCAGATCAGT TGGTTCGTGAACAACGTGGAAGTGCACACCGCTCAGACCCAGACACACAGAGAGGAC TACAACAGCACCCTGAGAGTGGTGTCTGCCCTGCCTATCCAGCACCAGGATTGGATG TCCGGCAAAGAATTCAAGTGCAAAGTGAACAACAAGGACCTGCCTGCTCCAATCGAG CGGACCATCTCCAAGCCTGGTGGCGGAGGAAGCGGTGGCGGCGGAGTGGTGGAAGAT CTGCGGAACGTGACCCCTCCTAAGGTGTCCCTGTTCGAGCCTTCCAAGGCCGAGATC GCCAACAAGCAGAAAGCTACCCTCGTGTGCCTGGCCAGAGGCTTCTTTCCTGACCAC GTGGAACTGTCTTGGTGGGTCAACGGCAAAGAGGTGCACTCCGGCGTCTGTACCGAT CCTCAGGCCTACAAAGAGTCCAACTACTCCTACAGCCTGTCCTCTCGGCTGAGAGTG TCTGCCACCTTCTGGCACAACCCTCGGAACCACTTCAGATGCCAGGTGCAGTTTCAC GGCCTGTCCGAAGAGGACAAGTGGCCTGAGGGATCCCCTAAGCCTGTGACACAGAAC ATCTCTGCCGAGGCCTGGGGTAGAGCTGATGGTGGCGGAGGATCTGGCGGAGGTGGA AGCGGCGGAGGCGGATCTTCTCATCTGGAACAGCTGCTGATGGACCTGCAAGAGCTG CTGTCCCGGATGGAAAACTACCGGAACCTGAAGCTGCCCCGGATGCTGACCGCTAAG TTCGCTCTGCCTAAGCAGGCCACCGAGCTGAAGGATCTGCAGTGCCTGGAAGATGAG CTGGGCCCTCTGAGACACGTGCTGGATCTGACCCAGTCCAAGTCCTTTCAGCTCGAG GACGCCGAGAACTTCATCTCCAACATCAGAGTGACCGTGGTCAAGCTGAAGGGCTCC GACAACACCTTCGAGTGCCAGTTCGACGATGAGTCCGCTACAGTGGTGGACTTCCTG CGGAGATGGATCGCCTTCTGCCAGTCCATCATCTCCACCTCTCCACAGTGATGA |
| SEQ ID NO: 88 | ACCATCAAGCCTTGTCCTCCATGCAAGTGCCCCGCTCCTAATCTGCTCGGAGGCCCT TCCGTGTTCATCTTCCCACCTAAGATCAAGGACGTGCTGATGATCTCCCTGTCTCCT ATCGTGACCTGCGTGGTGGTGGACGTGTCCGAGGATGATCCTGACGTGCAGATCAGT TGGTTCGTGAACAACGTGGAAGTGCACACCGCTCAGACCCAGACACACAGAGAGGAC TACAACAGCACCCTGAGAGTGGTGTCTGCCCTGCCTATCCAGCACCAGGATTGGATG TCCGGCAAAGAATTCAAGTGCAAAGTGAACAACAAGGACCTGCCTGCTCCAATCGAG CGGACCATCTCCAAGCCTGGTGGCGGAGGAAGCGGTGGCGGCGGATCTGTGGTGGAA GATCTGCGGAACGTGACCCCTCCTAAGGTGTCCCTGTTCGAGCCTTCCAAGGCCGAG ATCGCCAACAAGCAGAAAGCTACCCTCGTGTGCCTGGCCAGAGGCTTCTTTCCTGAC CACGTGGAACTGTCTTGGTGGGTCAACGGCAAAGAGGTGCACTCCGGCGTCTGTACC GATCCTCAGGCCTACAAAGAGTCCAACTACTCCTACAGCCTGTCCTCTCGGCTGAGA GTGTCTGCCACCTTCTGGCACAACCCTCGGAACCACTTCAGATGCCAGGTGCAGTTT CACGGCCTGTCCGAAGAGGACAAGTGGCCTGAGGGATCCCCTAAGCCTGTGACACAG AACATCTCTGCCGAGGCCTGGGGTAGAGCTGATGGTGGCGGAGGATCTGGCGGAGGT GGAAGCGGCGGAGGCGGATCTTCTCATCTGGAACAGCTGCTGATGGACCTGCAAGAG CTGCTGTCCCGGATGGAAAACTACCGGAACCTGAAGCTGCCCCGGATGCTGACCGCT AAGTTCGCTCTGCCTAAGCAGGCCACCGAGCTGAAGGATCTGCAGTGCCTGGAAGAT GAGCTGGGCCCTCTGAGACACGTGCTGGATCTGACCCAGTCCAAGTCCTTTCAGCTC |
| | GAGGACGCCGAGAACTTCATCTCCAACATCAGAGTGACCGTGGTCAAGCTGAAGGGC TCCGACAACACCTTCGAGTGCCAGTTCGACGATGAGTCCGCTACAGTGGTGGACTTC CTGCGGAGATGGATCGCCTTCTGCCAGTCCATCATCTCCACCTCTCCACAGTGATGA |
| SEQ ID NO: 89 | CAGGTGCAGCTGGTGGAATCTGGTGGCGGAGTTGTGCAGCCTGGCAGATCCCTGAGA CTGTCTTGTGCCGCCTCCGGCTTCACCTTCTCCAGCTACACCATGCACTGGGTCCGA CAGGCCCCTGGCAAAGGATTGGAGTGGGTCACCTTCATCTCTTACGACGGCAACAAC AAGTACTACGCCGACTCCGTGAAGGGCAGATTCACCATCTCTCGGGACAACTCCAAG AACACCCTGTACCTGCAGATGAACTCCCTGAGAGCCGAGGACACCGCCATCTACTAC TGTGCTAGAACCGGCTGGCTGGGCCCCTTTGATTATTGGGGACAGGGCACCCTGGTC ACCGTGTCCTCTGCTTCCACCAAGGGACCCTCTGTGTTCCCTCTGGCTCCTTCCAGC AAGTCTACCTCTGGCGGAACAGCTGCTCTGGGCTGCCTGGTCAAGGACTACTTTCCT GAGCCTGTGACCGTGTCTTGGAACTCTGGCGCTCTGACATCCGGCGTGCACACCTTT CCAGCTGTGCTGCAATCCTCCGGCCTGTACTCTCTGTCCTCCGTCGTGACCGTGCCT TCTAGCTCTCTGGGCACCCAGACCTACATCTGCAATGTGAACCACAAGCCTTCCAAC ACCAAGGTGGACAAGAGAGTGGAACCCAAGTCCTGCGATAAGACCCACACCTGTCCA CCATGTCCTGCTCCAGAACTGCTCGGCGGACCTTCCGTGTTCCTGTTTCCTCCAAAG CCTAAGGACACCCTGATGATCTCTCGGACCCCTGAAGTGACCTGCGTGGTGGTGGAT GTGTCTCACGAGGATCCCGAAGTGAAGTTCAATTGGTACGTGGACGGCGTGGAAGTG CACAACGCCAAGACCAAGCCTAGAGAGGAACAGTACAACTCCACCTACAGAGTGGTG TCCGTGCTGACCGTGCTGCACCAGGATTGGCTGAACGGCAAAGAGTACAAGTGCAAG GTGTCCAACAAGGCCCTGCCTGCTCCTATCGAAAAGACCATCTCCAAGGCCAAAGGC CAGCCTAGGGAACCCCAGGTTTACACCCTGCCTCCATGCCGGGAAGAGATGACCAAG AACCAGGTGTCCCTGTGGTGCCTGGTTAAGGGCTTCTACCCCTCCGATATCGCCGTG GAATGGGAGTCTAATGGCCAGCCTGAGAACAACTACAAGACAACCCCTCCTGTGCTG GACTCCGACGGCTCATTCTTCCTGTACTCCAAGCTGACAGTGGACAAGTCCAGATGG CAGCAGGGCAACGTGTTCTCCTGCTCCGTGATGCACGAGGCCCTGCACAATCACTAC ACCCAGAAGTCCCTGTCTCTGAGCCCCGGCAAG |
| SEQ ID NO: 90 | CAGGTGCAGCTGGTGGAATCTGGTGGCGGAGTTGTGCAGCCTGGCAGATCCCTGAGA CTGTCTTGTGCCGCCTCCGGCTTCACCTTCTCCTCTTACGGAATGCACTGGGTCCGA CAGGCCCCTGGCAAAGGATTGGAGTGGGTCGCCTTCATCAGATACGACGGCTCCAAC AAGTACTACGCCGACTCCGTGAAGGGCAGATTCACCATCTCTCGGGACAACTCCAAG AACACCCTGTACCTGCAGATGAACTCCCTGAGAGCCGAGGACACCGCCGTGTACTAC TGCAAGACCCACGGCTCTCACGACAATTGGGGCCAGGGCACAATGGTCACCGTGTCC TCTGCTTCCACCAAGGGACCCTCTGTGTTCCCTCTGGCTCCTTCCAGCAAGTCTACC TCTGGCGGAACAGCTGCTCTGGGCTGCCTGGTCAAGGACTACTTTCCTGAGCCTGTG ACCGTGTCTTGGAACTCTGGCGCTCTGACATCCGGCGTGCACACCTTTCCAGCTGTG CTGCAATCCTCCGGCCTGTACTCTCTGTCCTCCGTCGTGACCGTGCCTTCTAGCTCT CTGGGCACCCAGACCTACATCTGCAATGTGAACCACAAGCCTTCCAACACCAAGGTG GACAAGAGAGTGGAACCCAAGTCCTGCGATAAGACCCACACCTGTCCACCATGTCCT GCTCCAGAACTGCTCGGCGGACCTTCCGTGTTCCTGTTTCCTCCAAAGCCTAAGGAC ACCCTGATGATCTCTCGGACCCCTGAAGTGACCTGCGTGGTGGTGGATGTGTCTCAC GAGGATCCCGAAGTGAAGTTCAATTGGTACGTGGACGGCGTGGAAGTGCACAACGCC AAGACCAAGCCTAGAGAGGAACAGTACAACTCCACCTACAGAGTGGTGTCCGTGCTG ACCGTGCTGCACCAGGATTGGCTGAACGGCAAAGAGTACAAGTGCAAGGTGTCCAAC AAGGCCCTGCCTGCTCCTATCGAAAAGACCATCTCCAAGGCCAAAGCCAGCCTCGGG AACCTCAAGTCTGTACCCTGCCTCCTAGCCGGGAAGAGATGACCAAGAACCAGGTGT CCCTGTCCTGCGCTGTGAAGGGCTTCTACCCTTCCGATATCGCCGTGGAATGGGAGA |
| | GCAATGGCCAGCCTGAGAACAACTACAAGACCACACCTCCTGTGCTGGACTCCGACG GCTCATTCTTCCTGGTGTCCAAGCTGACAGTGGACAAGTCCAGATGGCAGCAGGGCA ACGTGTTCTCCTGCTCCGTGATGCACGAGGCCCTGCACAATCACTACACCCAGAAGT CCCTGTCTCTGAGCCCCGGCAAG |

### 2. Expression and Purification.

The plasmids were co-transfected into either Expi293 cells (Life Technologies A14527) or ExpiCHO cells (Life Technologies A29127). Transfections were performed using 1 mg of total DNA for a multispecific construct with a 1:1 heavy chain ratio and 3:2 light chain to heavy chain ratio. Transfection in Expi293 cells was done using linear 25,000 Da polyethylenimine (PEI, Polysciences Inc 23966) in a 3:1 ratio with the total DNA. The DNA and PEI were each added to 50 mL of OptiMem (Life Technologies 31985088) medium and sterile filtered. The DNA and PEI were combined for 10 minutes and added to the Expi293 cells with a density of 1.8-2.8 × 10⁶ cells/mL and a viability of at least 95 %. The ExpiCHO transfection was performed according to the manufacturer's instructions. Expi293 cells were grown in a humidified incubator at 37 °C with 8 % CO₂ for 5-7 days after transfection and ExpiCHO cells were grown for 14 days at 32 ⁰C with 5 % CO₂. The cells were pelleted by centrifugation at 4500 x g and the supernatant was filtered through a 0.2 µm membrane. Protein A resin (GE 17-1279-03) was added to the filtered supernatant and incubated for 1-3 hours at room temperature. The resin was packed into a column washed with 3 x 10 column volumes of Dulbecco's phosphate-buffered saline (DPBS, Life Technologies 14190-144). The bound protein was eluted from the column with 20 mM citrate, 100 mM NaCl, pH 2.9. The molecule was polished by size exclusion on a Superdex 200 column with a running buffer of DPBS. Fractions containing monomeric compound were pooled.

**Table 4. Amino Acid sequences.**

| SEQ ID NO | Description | Amino Acid Sequence |
|---|---|---|
| SEQ ID NO: 91 | αCTLA4 ipilimumab VH | QVQLVESGGGVVQPGRSLRLSCAASGFTFSSYTMHWVRQAPGKGLE WVTFISYDGNNKYYADSVKGRFTISRDNSKNTLYLQMNSLRAEDTA IYYCARTGWLGPFDYWGQGTLVTVSS |
| SEQ ID NO: 92 | αCTLA4 ipilimumab VL | EIVLTQSPGTLSLSPGERATLSCRASQSVGSSYLAWYQQKPGQAPR LLIYGAFSRATGIPDRFSGSGSGTDFTLTISRLEPEDFAVYYCQQY GSSPWTFGQGTKVEIK |
| SEQ ID NO: 93 | αIL12B briakinumab VH | |
| SEQ ID NO: 94 | αIL12B briakinumab VL | |
| SEQ ID NO: 95 | hCL (kappa) | |
| SEQ ID NO: 96 | hCL (lambda) | GQPKANPTVTLFPPSSEELQANKATLVCLISDFYPGAVTVAWKADG SPVKAGVETTKPSKQSNNKYAASSYLSLTPEQWKSHRSYSCQVTHE GSTVEKTVAPTECS |
| SEQ ID NO: 97 | hCH1 | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNT KVDKRVEPKSC |
| SEQ ID NO: 98 | hCH2 | DKTHTCPPCPAPELLGGPSVFLFPPKPKDTLMISRTPEVTCVVVDV SHEDPEVKFNWYVDGVEVHNAKTKPREEQYNSTYRVVSVLTVLHQD WLNGKEYKCKVSNKALPAPIEKTISKAK |
| SEQ ID NO: 99 | hTCRα | PDIQNPDPAVYQLRDSKSSDKSVCLFTDFDSQTNVSQSKDSDVYIT DKCVLDMRSMDFKSNSAVAWSNKSDFACANAFNNSIIPEDTFFPSP E |
| SEQ ID NO: 100 | hTCRβ | EDLNKVFPPEVALFEPSEAEISHTQKATLVCLATGFYPDHVELSWW VNGKEVHSGVCTDPQPLKEQPALNDSRYALSSRLRVSATFWQDPRN HFRCQVQFYGLSEADEWTQARAKPVTQIVSAEAWGRAD |
| SEQ ID NO: 101 | G | G |
| SEQ ID NO: 102 | GG | GG |
| SEQ ID NO: 103 | GGG | GGG |
| SEQ ID NO: 104 | GGGG | GGGG |
| SEQ ID NO: 105 | GGGGS | GGGGS |
| SEQ ID NO: 106 | GGGGSG | GGGGSG |
| SEQ ID NO: 107 | GGGGSGG | GGGGSGG |
| SEQ ID NO: 108 | GGGGSGGG | GGGGSGGG |
| SEQ ID NO: 109 | GGGGSGGGG | GGGGSGGGG |
| SEQ ID NO: 110 | GGGGSGGGGS | GGGGSGGGGS |
| SEQ ID NO: 111 | mCH2 | TIKPCPPCKCPAPNLLGGPSVFIFPPKIKDVLMISLSPIVTCV VVDVSEDDPDVQISWFVNNVEVHTAQTQTHREDYNSTLRVVSA LPIQHQDWMSGKEFKCKVNNKDLPAPIERTISKP |
| SEQ ID NO: 112 | mTCRα | KPNIQNPEPAVYQLKDPRSQDSTLCLFTDFDSQINVPKTMESG TFITDKCVLDMKAMDSKSNGAIAWSNQTSFTCQDIFKETNATY PSSDVPCDATLTEKSFETDMNLNFQNLS |
| SEQ ID NO: 113 | mTCRβ | VVEDLRNVTPPKVSLFEPSKAEIANKQKATLVCLARGFFPDHV ELSWWVNGKEVHSGVCTDPQAYKESNYSYSLSSRLRVSATFWH NPRNHFRCQVQFHGLSEEDKWPEGSPKPVTQNISAEAWGRAD |
| SEQ ID NO: 114 | mIL2 F56A Y59A | SHLEQLLMDLQELLSRMENYRNLKLPRMLTAKFALPKQATELK DLQCLEDELGPLRHVLDLTQSKSFQLEDAENFISNIRVTVVKL KGSDNTFECQFDDESATVVDFLRRWIAFCQSIISTSPQ |
| SEQ ID NO: 115 | GGGGSGGGGSG GGGS | GGGGSGGGGSGGGGS |
| SEQ ID NO: 116 | hCH3_Knob | GQPREPQVYTLPPCREEMTKNQVSLWCLVKGFYPSDIAVEWES NGQPENNYKTTPPVLDSDGSFFLYSKLTVDKSRWQQGNVFSCS VMHEALHNHYTQKSLSLSPGK |
| SEQ ID NO: 117 | hCH3_Hole | GQPREPQVCTLPPSREEMTKNQVSLSCAVKGFYPSDIAVEWES NGQPENNYKTTPPVLDSDGSFFLVSKLTVDKSRWQQGNVFSCS VMHEALHNHYTQKSLSLSPGK |

**Table 5. Sequences used to construct heavy and light chains.**

| Full length sequence | Variable | Constant 1 | Constant 2 | Linker | Constant 3 | Linker | C-term |
|---|---|---|---|---|---|---|---|
| SEQ ID NO: 118 | SEQ ID NO: 91 | SEQ ID NO: 97 | SEQ ID NO: 98 | | SEQ ID NO: 99 | | |
| SEQ ID NO: 119 | SEQ ID NO: 91 | SEQ ID NO: 97 | SEQ ID NO: 98 | SEQ ID NO: 101 | SEQ ID NO: 99 | | |
| SEQ ID NO: 120 | SEQ ID NO: 91 | SEQ ID NO: 97 | SEQ ID NO: 98 | SEQ ID NO: 102 | SEQ ID NO: 99 | | |
| SEQ ID NO: 121 | SEQ ID NO: 91 | SEQ ID NO: 97 | SEQ ID NO: 98 | SEQ ID NO: 103 | SEQ ID NO: 99 | | |
| SEQ ID NO: 122 | SEQ ID NO: 91 | SEQ ID NO: 97 | SEQ ID NO: 98 | SEQ ID NO: 104 | SEQ ID NO: 99 | | |
| SEQ ID NO: 123 | SEQ ID NO: 91 | SEQ ID NO: 97 | SEQ ID NO: 98 | SEQ ID NO: 105 | SEQ ID NO: 99 | | |
| SEQ ID NO: 124 | SEQ ID NO: 91 | SEQ ID NO: 97 | SEQ ID NO: 98 | SEQ ID NO: 106 | SEQ ID NO: 99 | | |
| SEQ ID NO: 125 | SEQ ID NO: 91 | SEQ ID NO: 97 | SEQ ID NO: 98 | SEQ ID NO: 107 | SEQ ID NO: 99 | | |
| SEQ ID NO: 126 | SEQ ID NO: 91 | SEQ ID NO: 97 | SEQ ID NO: 98 | SEQ ID NO: 108 | SEQ ID NO: 99 | | |
| SEQ ID NO: 127 | SEQ ID NO: 91 | SEQ ID NO: 97 | SEQ ID NO: 98 | SEQ ID NO: 109 | SEQ ID NO: 99 | | |
| SEQ ID NO: 128 | SEQ ID NO: 91 | SEQ ID NO: 97 | SEQ ID NO: 98 | SEQ ID NO: 110 | SEQ ID NO: 99 | | |
| SEQ ID NO: 129 | SEQ ID NO: 92 | SEQ ID NO: 95 | | | | | |
| SEQ ID NO: 130 | SEQ ID NO: 93 | SEQ ID NO: 97 | SEQ ID NO: 98 | | SEQ ID NO: 100 | | |
| SEQ ID NO: 131 | SEQ ID NO: 93 | SEQ ID NO: 97 | SEQ ID NO: 98 | SEQ ID NO: 101 | SEQ ID NO: 100 | | |
| SEQ ID NO: 132 | SEQ ID NO: 93 | SEQ ID NO: 97 | SEQ ID NO: 98 | SEQ ID NO: 102 | SEQ ID NO: 100 | | |
| SEQ ID NO: 133 | SEQ ID NO: 93 | SEQ ID NO: 97 | SEQ ID NO: 98 | SEQ ID NO: 103 | SEQ ID NO: 100 | | |
| SEQ ID NO: 134 | SEQ ID NO: 93 | SEQ ID NO: 97 | SEQ ID NO: 98 | SEQ ID NO: 104 | SEQ ID NO: 100 | | |
| SEQ ID NO: 135 | SEQ ID NO: 93 | SEQ ID NO: 97 | SEQ ID NO: 98 | SEQ ID NO: 105 | SEQ ID NO: 100 | | |
| SEQ ID NO: 136 | SEQ ID NO: 93 | SEQ ID NO: 97 | SEQ ID NO: 98 | SEQ ID NO: 106 | SEQ ID NO: 100 | | |
| SEQ ID NO: 137 | SEQ ID NO: 93 | SEQ ID NO: 97 | SEQ ID NO: 98 | SEQ ID NO: 107 | SEQ ID NO: 100 | | |
| SEQ ID NO: 138 | SEQ ID NO: 93 | SEQ ID NO: 97 | SEQ ID NO: 98 | SEQ ID NO: 108 | SEQ ID NO: 100 | | |
| SEQ ID NO: 139 | SEQ ID NO: 93 | SEQ ID NO: 97 | SEQ ID NO: 98 | SEQ ID NO: 109 | SEQ ID NO: 100 | | |
| SEQ ID NO: 140 | SEQ ID NO: 93 | SEQ ID NO: 97 | SEQ ID NO: 98 | SEQ ID NO: 110 | SEQ ID NO: 100 | | |
| SEQ ID NO: 141 | SEQ ID NO: 94 | SEQ ID NO: 96 | | | | | |
| SEQ ID NO: 142 | | | SEQ ID NO: 111 | | SEQ ID NO: 112 | | |
| SEQ ID NO: 143 | | | SEQ ID NO: 111 | SEQ ID NO: 101 | SEQ ID NO: 112 | | |
| SEQ ID NO: 144 | | | SEQ ID NO: 111 | SEQ ID NO: 102 | SEQ ID NO: 112 | | |
| SEQ ID NO: 145 | | | SEQ ID NO: 111 | SEQ ID NO: 103 | SEQ ID NO: 112 | | |
| SEQ ID NO: 146 | | | SEQ ID NO: 111 | SEQ ID NO: 104 | SEQ ID NO: 112 | | |
| SEQ ID NO: 147 | | | SEQ ID NO: 111 | SEQ ID NO: 105 | SEQ ID NO: 112 | | |
| SEQ ID NO: 148 | | | SEQ ID NO: 111 | SEQ ID NO: 106 | SEQ ID NO: 112 | | |
| SEQ ID NO: 149 | | | SEQ ID NO: 111 | SEQ ID NO: 107 | SEQ ID NO: 112 | | |
| SEQ ID NO: 150 | | | SEQ ID NO: 111 | SEQ ID NO: 108 | SEQ ID NO: 112 | | |
| SEQ ID NO: 151 | | | SEQ ID NO: 111 | SEQ ID NO: 109 | SEQ ID NO: 112 | | |
| SEQ ID NO: 152 | | | SEQ ID NO: 111 | SEQ ID NO: 110 | SEQ ID NO: 112 | | |
| SEQ ID NO: 153 | | | SEQ ID NO: 111 | | SEQ ID NO: 113 | SEQ ID NO: 115 | SEQ ID NO: 114 |
| SEQ ID NO: 154 | | | SEQ ID NO: 111 | SEQ ID NO: 101 | SEQ ID NO: 113 | SEQ ID NO: 115 | SEQ ID NO: 114 |
| SEQ ID NO: 155 | | | SEQ ID NO: 111 | SEQ ID NO: 102 | SEQ ID NO: 113 | SEQ ID NO: 115 | SEQ ID NO: 114 |
| SEQ ID NO: 156 | | | SEQ ID NO: 111 | SEQ ID NO: 103 | SEQ ID NO: 113 | SEQ ID NO: 115 | SEQ ID NO: 114 |
| SEQ ID NO: 157 | | | SEQ ID NO: 111 | SEQ ID NO: 104 | SEQ ID NO: 113 | SEQ ID NO: 115 | SEQ ID NO: 114 |
| SEQ ID NO: 166 | | | SEQ ID NO: 111 | SEQ ID NO: 105 | SEQ ID NO: 113 | SEQ ID NO: 115 | SEQ ID NO: 114 |
| SEQ ID NO: 167 | | | SEQ ID NO: 111 | SEQ ID NO: 106 | SEQ ID NO: 113 | SEQ ID NO: 115 | SEQ ID NO: 114 |
| SEQ ID NO: 160 | | | SEQ ID NO: 111 | SEQ ID NO: 107 | SEQ ID NO: 113 | SEQ ID NO: 115 | SEQ ID NO: 114 |
| SEQ ID NO: 161 | | | SEQ ID NO: 111 | SEQ ID NO: 108 | SEQ ID NO: 113 | SEQ ID NO: 115 | SEQ ID NO: 114 |
| SEQ ID NO: 162 | | | SEQ ID NO: 111 | SEQ ID NO: 109 | SEQ ID NO: 113 | SEQ ID NO: 115 | SEQ ID NO: 114 |
| SEQ ID NO: 163 | | | SEQ ID NO: 111 | SEQ ID NO: 110 | SEQ ID NO: 113 | SEQ ID NO: 115 | SEQ ID NO: 114 |
| SEQ ID NO: 164 | SEQ ID NO: 91 | SEQ ID NO: 97 | SEQ ID NO: 98 | | SEQ ID NO: 116 | | |
| SEQ ID NO: 165 | SEQ ID NO: 93 | SEQ ID NO: 97 | SEQ ID NO: 98 | | SEQ ID NO: 117 | | |

**Table 6. Sequences used to construct heavy and light chains.**

| SEQ ID NO | Sequence |
|---|---|
| SEQ ID NO: 118 | QVQLVESGGGVVQPGRSLRLSCAASGFTFSSYTMHWVRQAPGKGLEWVTFISY DGNNKYYADSVKGRFTISRDNSKNTLYLQMNSLRAEDTAIYYCARTGWLGPFD YWGQGTLVTVSSASTKGPSVFPLAPSSKSTSGGTAALGCLVKDYFPEPVTVSW NSGALTSGVHTFPAVLQSSGLYSLSSVVTVPSSSLGTQTYICNVNHKPSNTKV DKRVEPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDWLNGK EYKCKVSNKALPAPIEKTISKAKPDIQNPDPAVYQLRDSKSSDKSVCLFTDFD SQTNVSQSKDSDVYITDKCVLDMRSMDFKSNSAVAWSNKSDFACANAFNNSII PEDTFFPSPE |
| SEQ ID NO: 119 | QVQLVESGGGVVQPGRSLRLSCAASGFTFSSYTMHWVRQAPGKGLEWVTFISY DGNNKYYADSVKGRFTISRDNSKNTLYLQMNSLRAEDTAIYYCARTGWLGPFD YWGQGTLVTVSSASTKGPSVFPLAPSSKSTSGGTAALGCLVKDYFPEPVTVSW NSGALTSGVHTFPAVLQSSGLYSLSSVVTVPSSSLGTQTYICNVNHKPSNTKV DKRVEPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDWLNGK EYKCKVSNKALPAPIEKTISKAKGPDIQNPDPAVYQLRDSKSSDKSVCLFTDF DSQTNVSQSKDSDVYITDKCVLDMRSMDFKSNSAVAWSNKSDFACANAFNNSI IPEDTFFPSPE |
| SEQ ID NO: 120 | QVQLVESGGGVVQPGRSLRLSCAASGFTFSSYTMHWVRQAPGKGLEWVTFISY DGNNKYYADSVKGRFTISRDNSKNTLYLQMNSLRAEDTAIYYCARTGWLGPFD YWGQGTLVTVSSASTKGPSVFPLAPSSKSTSGGTAALGCLVKDYFPEPVTVSW NSGALTSGVHTFPAVLQSSGLYSLSSVVTVPSSSLGTQTYICNVNHKPSNTKV DKRVEPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDWLNGK EYKCKVSNKALPAPIEKTISKAKGGPDIQNPDPAVYQLRDSKSSDKSVCLFTD FDSQTNVSQSKDSDVYITDKCVLDMRSMDFKSNSAVAWSNKSDFACANAFNNS IIPEDTFFPSPE |
| SEQ ID NO: 121 | QVQLVESGGGVVQPGRSLRLSCAASGFTFSSYTMHWVRQAPGKGLEWVTFISY DGNNKYYADSVKGRFTISRDNSKNTLYLQMNSLRAEDTAIYYCARTGWLGPFD YWGQGTLVTVSSASTKGPSVFPLAPSSKSTSGGTAALGCLVKDYFPEPVTVSW NSGALTSGVHTFPAVLQSSGLYSLSSVVTVPSSSLGTQTYICNVNHKPSNTKV DKRVEPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDWLNGK EYKCKVSNKALPAPIEKTISKAKGGGPDIQNPDPAVYQLRDSKSSDKSVCLFT DFDSQTNVSQSKDSDVYITDKCVLDMRSMDFKSNSAVAWSNKSDFACANAFNN SIIPEDTFFPSPE |
| SEQ ID NO: 122 | QVQLVESGGGVVQPGRSLRLSCAASGFTFSSYTMHWVRQAPGKGLEWVTFISY DGNNKYYADSVKGRFTISRDNSKNTLYLQMNSLRAEDTAIYYCARTGWLGPFD YWGQGTLVTVSSASTKGPSVFPLAPSSKSTSGGTAALGCLVKDYFPEPVTVSW NSGALTSGVHTFPAVLQSSGLYSLSSVVTVPSSSLGTQTYICNVNHKPSNTKV DKRVEPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDWLNGK EYKCKVSNKALPAPIEKTISKAKGGGGPDIQNPDPAVYQLRDSKSSDKSVCLF TDFDSQTNVSQSKDSDVYITDKCVLDMRSMDFKSNSAVAWSNKSDFACANAFN NSIIPEDTFFPSPE |
| SEQ ID NO: 123 | QVQLVESGGGVVQPGRSLRLSCAASGFTFSSYTMHWVRQAPGKGLEWVTFISY DGNNKYYADSVKGRFTISRDNSKNTLYLQMNSLRAEDTAIYYCARTGWLGPFD YWGQGTLVTVSSASTKGPSVFPLAPSSKSTSGGTAALGCLVKDYFPEPVTVSW NSGALTSGVHTFPAVLQSSGLYSLSSVVTVPSSSLGTQTYICNVNHKPSNTKV DKRVEPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDWLNGK EYKCKVSNKALPAPIEKTISKAKGGGGSPDIQNPDPAVYQLRDSKSSDKSVCL FTDFDSQTNVSQSKDSDVYITDKCVLDMRSMDFKSNSAVAWSNKSDFACANAF NNSIIPEDTFFPSPE |
| SEQ ID NO: 124 | QVQLVESGGGVVQPGRSLRLSCAASGFTFSSYTMHWVRQAPGKGLEWVTFISY DGNNKYYADSVKGRFTISRDNSKNTLYLQMNSLRAEDTAIYYCARTGWLGPFD YWGQGTLVTVSSASTKGPSVFPLAPSSKSTSGGTAALGCLVKDYFPEPVTVSW NSGALTSGVHTFPAVLQSSGLYSLSSVVTVPSSSLGTQTYICNVNHKPSNTKV DKRVEPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDWLNGK EYKCKVSNKALPAPIEKTISKAKGGGGSGPDIQNPDPAVYQLRDSKSSDKSVC LFTDFDSQTNVSQSKDSDVYITDKCVLDMRSMDFKSNSAVAWSNKSDFACANA FNNSIIPEDTFFPSPE |
| SEQ ID NO: 125 | QVQLVESGGGVVQPGRSLRLSCAASGFTFSSYTMHWVRQAPGKGLEWVTFISY DGNNKYYADSVKGRFTISRDNSKNTLYLQMNSLRAEDTAIYYCARTGWLGPFD |
| | YWGQGTLVTVSSASTKGPSVFPLAPSSKSTSGGTAALGCLVKDYFPEPVTVSW NSGALTSGVHTFPAVLQSSGLYSLSSVVTVPSSSLGTQTYICNVNHKPSNTKV DKRVEPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDWLNGK EYKCKVSNKALPAPIEKTISKAKGGGGSGGPDIQNPDPAVYQLRDSKSSDKSV CLFTDFDSQTNVSQSKDSDVYITDKCVLDMRSMDFKSNSAVAWSNKSDFACAN AFNNSIIPEDTFFPSPE |
| SEQ ID NO: 126 | QVQLVESGGGVVQPGRSLRLSCAASGFTFSSYTMHWVRQAPGKGLEWVTFISY DGNNKYYADSVKGRFTISRDNSKNTLYLQMNSLRAEDTAIYYCARTGWLGPFD YWGQGTLVTVSSASTKGPSVFPLAPSSKSTSGGTAALGCLVKDYFPEPVTVSW NSGALTSGVHTFPAVLQSSGLYSLSSVVTVPSSSLGTQTYICNVNHKPSNTKV DKRVEPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDWLNGK EYKCKVSNKALPAPIEKTISKAKGGGGSGGGPDIQNPDPAVYQLRDSKSSDKS VCLFTDFDSQTNVSQSKDSDVYITDKCVLDMRSMDFKSNSAVAWSNKSDFACA NAFNNSIIPEDTFFPSPE |
| SEQ ID NO: 127 | QVQLVESGGGVVQPGRSLRLSCAASGFTFSSYTMHWVRQAPGKGLEWVTFISY DGNNKYYADSVKGRFTISRDNSKNTLYLQMNSLRAEDTAIYYCARTGWLGPFD YWGQGTLVTVSSASTKGPSVFPLAPSSKSTSGGTAALGCLVKDYFPEPVTVSW NSGALTSGVHTFPAVLQSSGLYSLSSVVTVPSSSLGTQTYICNVNHKPSNTKV DKRVEPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDWLNGK EYKCKVSNKALPAPIEKTISKAKGGGGSGGGGPDIQNPDPAVYQLRDSKSSDK SVCLFTDFDSQTNVSQSKDSDVYITDKCVLDMRSMDFKSNSAVAWSNKSDFAC ANAFNNSIIPEDTFFPSPE |
| SEQ ID NO: 128 | QVQLVESGGGVVQPGRSLRLSCAASGFTFSSYTMHWVRQAPGKGLEWVTFISY DGNNKYYADSVKGRFTISRDNSKNTLYLQMNSLRAEDTAIYYCARTGWLGPFD YWGQGTLVTVSSASTKGPSVFPLAPSSKSTSGGTAALGCLVKDYFPEPVTVSW NSGALTSGVHTFPAVLQSSGLYSLSSVVTVPSSSLGTQTYICNVNHKPSNTKV DKRVEPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDWLNGK EYKCKVSNKALPAPIEKTISKAKGGGGSGGGGSPDIQNPDPAVYQLRDSKSSD KSVCLFTDFDSQTNVSQSKDSDVYITDKCVLDMRSMDFKSNSAVAWSNKSDFA CANAFNNSIIPEDTFFPSPE |
| SEQ ID NO: 129 | EIVLTQSPGTLSLSPGERATLSCRASQSVGSSYLAWYQQKPGQAPRLLIYGAF SRATGIPDRFSGSGSGTDFTLTISRLEPEDFAVYYCQQYGSSPWTFGQGTKVE IK |
| SEQ ID NO: 130 | QVQLVESGGGVVQPGRSLRLSCAASGFTFSSYGMHWVRQAPGKGLEWVAFIRY DGSNKYYADSVKGRFTISRDNSKNTLYLQMNSLRAEDTAVYYCKTHGSHDNWG QGTMVTVSSASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKR VEPKSCDKTHTCPPCPAPELLGGPSVFLFPPKPKDTLMISRTPEVTCVVVDVS HEDPEVKFNWYVDGVEVHNAKTKPREEQYNSTYRVVSVLTVLHQDWLNGKEYK CKVSNKALPAPIEKTISKAKEDLNKVFPPEVALFEPSEAEISHTQKATLVCLA TGFYPDHVELSWWVNGKEVHSGVCTDPQPLKEQPALNDSRYALSSRLRVSATF WQDPRNHFRCQVQFYGLSEADEWTQARAKPVTQIVSAEAWGRAD |
| SEQ ID NO: 131 | QVQLVESGGGVVQPGRSLRLSCAASGFTFSSYGMHWVRQAPGKGLEWVAFIRY DGSNKYYADSVKGRFTISRDNSKNTLYLQMNSLRAEDTAVYYCKTHGSHDNWG QGTMVTVSSASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKR VEPKSCDKTHTCPPCPAPELLGGPSVFLFPPKPKDTLMISRTPEVTCVVVDVS HEDPEVKFNWYVDGVEVHNAKTKPREEQYNSTYRVVSVLTVLHQDWLNGKEYK CKVSNKALPAPIEKTISKAKGEDLNKVFPPEVALFEPSEAEISHTQKATLVCL ATGFYPDHVELSWWVNGKEVHSGVCTDPQPLKEQPALNDSRYALSSRLRVSAT FWQDPRNHFRCQVQFYGLSEADEWTQARAKPVTQIVSAEAWGRAD |
| SEQ ID NO: 132 | QVQLVESGGGVVQPGRSLRLSCAASGFTFSSYGMHWVRQAPGKGLEWVAFIRY DGSNKYYADSVKGRFTISRDNSKNTLYLQMNSLRAEDTAVYYCKTHGSHDNWG QGTMVTVSSASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKR VEPKSCDKTHTCPPCPAPELLGGPSVFLFPPKPKDTLMISRTPEVTCVVVDVS HEDPEVKFNWYVDGVEVHNAKTKPREEQYNSTYRVVSVLTVLHQDWLNGKEYK CKVSNKALPAPIEKTISKAKGGEDLNKVFPPEVALFEPSEAEISHTQKATLVC LATGFYPDHVELSWWVNGKEVHSGVCTDPQPLKEQPALNDSRYALSSRLRVSA TFWQDPRNHFRCQVQFYGLSEADEWTQARAKPVTQIVSAEAWGRAD |
| SEQ ID NO: 133 | QVQLVESGGGVVQPGRSLRLSCAASGFTFSSYGMHWVRQAPGKGLEWVAFIRY DGSNKYYADSVKGRFTISRDNSKNTLYLQMNSLRAEDTAVYYCKTHGSHDNWG QGTMVTVSSASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKR VEPKSCDKTHTCPPCPAPELLGGPSVFLFPPKPKDTLMISRTPEVTCVVVDVS HEDPEVKFNWYVDGVEVHNAKTKPREEQYNSTYRVVSVLTVLHQDWLNGKEYK CKVSNKALPAPIEKTISKAKGGGEDLNKVFPPEVALFEPSEAEISHTQKATLV CLATGFYPDHVELSWWVNGKEVHSGVCTDPQPLKEQPALNDSRYALSSRLRVS ATFWQDPRNHFRCQVQFYGLSEADEWTQARAKPVTQIVSAEAWGRAD |
| SEQ ID NO: 134 | QVQLVESGGGVVQPGRSLRLSCAASGFTFSSYGMHWVRQAPGKGLEWVAFIRY DGSNKYYADSVKGRFTISRDNSKNTLYLQMNSLRAEDTAVYYCKTHGSHDNWG QGTMVTVSSASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKR VEPKSCDKTHTCPPCPAPELLGGPSVFLFPPKPKDTLMISRTPEVTCVVVDVS HEDPEVKFNWYVDGVEVHNAKTKPREEQYNSTYRVVSVLTVLHQDWLNGKEYK CKVSNKALPAPIEKTISKAKGGGGEDLNKVFPPEVALFEPSEAEISHTQKATL VCLATGFYPDHVELSWWVNGKEVHSGVCTDPQPLKEQPALNDSRYALSSRLRV SATFWQDPRNHFRCQVQFYGLSEADEWTQARAKPVTQIVSAEAWGRAD |
| SEQ ID NO: 135 | QVQLVESGGGVVQPGRSLRLSCAASGFTFSSYGMHWVRQAPGKGLEWVAFIRY DGSNKYYADSVKGRFTISRDNSKNTLYLQMNSLRAEDTAVYYCKTHGSHDNWG QGTMVTVSSASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKR VEPKSCDKTHTCPPCPAPELLGGPSVFLFPPKPKDTLMISRTPEVTCVVVDVS HEDPEVKFNWYVDGVEVHNAKTKPREEQYNSTYRVVSVLTVLHQDWLNGKEYK CKVSNKALPAPIEKTISKAKGGGGSEDLNKVFPPEVALFEPSEAEISHTQKAT LVCLATGFYPDHVELSWWVNGKEVHSGVCTDPQPLKEQPALNDSRYALSSRLR VSATFWQDPRNHFRCQVQFYGLSEADEWTQARAKPVTQIVSAEAWGRAD |
| SEQ ID NO: 136 | QVQLVESGGGVVQPGRSLRLSCAASGFTFSSYGMHWVRQAPGKGLEWVAFIRY DGSNKYYADSVKGRFTISRDNSKNTLYLQMNSLRAEDTAVYYCKTHGSHDNWG |
| | QGTMVTVSSASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKR VEPKSCDKTHTCPPCPAPELLGGPSVFLFPPKPKDTLMISRTPEVTCVVVDVS HEDPEVKFNWYVDGVEVHNAKTKPREEQYNSTYRVVSVLTVLHQDWLNGKEYK CKVSNKALPAPIEKTISKAKGGGGSGEDLNKVFPPEVALFEPSEAEISHTQKA TLVCLATGFYPDHVELSWWVNGKEVHSGVCTDPQPLKEQPALNDSRYALSSRL RVSATFWQDPRNHFRCQVQFYGLSEADEWTQARAKPVTQIVSAEAWGRAD |
| SEQ ID NO: 137 | QVQLVESGGGVVQPGRSLRLSCAASGFTFSSYGMHWVRQAPGKGLEWVAFIRY DGSNKYYADSVKGRFTISRDNSKNTLYLQMNSLRAEDTAVYYCKTHGSHDNWG QGTMVTVSSASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKR VEPKSCDKTHTCPPCPAPELLGGPSVFLFPPKPKDTLMISRTPEVTCVVVDVS HEDPEVKFNWYVDGVEVHNAKTKPREEQYNSTYRVVSVLTVLHQDWLNGKEYK CKVSNKALPAPIEKTISKAKGGGGSGGEDLNKVFPPEVALFEPSEAEISHTQK ATLVCLATGFYPDHVELSWWVNGKEVHSGVCTDPQPLKEQPALNDSRYALSSR LRVSATFWQDPRNHFRCQVQFYGLSEADEWTQARAKPVTQIVSAEAWGRAD |
| SEQ ID NO: 138 | QVQLVESGGGVVQPGRSLRLSCAASGFTFSSYGMHWVRQAPGKGLEWVAFIRY DGSNKYYADSVKGRFTISRDNSKNTLYLQMNSLRAEDTAVYYCKTHGSHDNWG QGTMVTVSSASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKR VEPKSCDKTHTCPPCPAPELLGGPSVFLFPPKPKDTLMISRTPEVTCVVVDVS HEDPEVKFNWYVDGVEVHNAKTKPREEQYNSTYRVVSVLTVLHQDWLNGKEYK CKVSNKALPAPIEKTISKAKGGGGSGGGEDLNKVFPPEVALFEPSEAEISHTQ KATLVCLATGFYPDHVELSWWVNGKEVHSGVCTDPQPLKEQPALNDSRYALSS RLRVSATFWQDPRNHFRCQVQFYGLSEADEWTQARAKPVTQIVSAEAWGRAD |
| SEQ ID NO: 139 | QVQLVESGGGVVQPGRSLRLSCAASGFTFSSYGMHWVRQAPGKGLEWVAFIRY DGSNKYYADSVKGRFTISRDNSKNTLYLQMNSLRAEDTAVYYCKTHGSHDNWG QGTMVTVSSASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKR VEPKSCDKTHTCPPCPAPELLGGPSVFLFPPKPKDTLMISRTPEVTCVVVDVS HEDPEVKFNWYVDGVEVHNAKTKPREEQYNSTYRVVSVLTVLHQDWLNGKEYK CKVSNKALPAPIEKTISKAKGGGGSGGGGEDLNKVFPPEVALFEPSEAEISHT QKATLVCLATGFYPDHVELSWWVNGKEVHSGVCTDPQPLKEQPALNDSRYALS SRLRVSATFWQDPRNHFRCQVQFYGLSEADEWTQARAKPVTQIVSAEAWGRAD |
| SEQ ID NO: 140 | QVQLVESGGGVVQPGRSLRLSCAASGFTFSSYGMHWVRQAPGKGLEWVAFIRY DGSNKYYADSVKGRFTISRDNSKNTLYLQMNSLRAEDTAVYYCKTHGSHDNWG QGTMVTVSSASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKR VEPKSCDKTHTCPPCPAPELLGGPSVFLFPPKPKDTLMISRTPEVTCVVVDVS HEDPEVKFNWYVDGVEVHNAKTKPREEQYNSTYRVVSVLTVLHQDWLNGKEYK CKVSNKALPAPIEKTISKAKGGGGSGGGGSEDLNKVFPPEVALFEPSEAEISH TQKATLVCLATGFYPDHVELSWWVNGKEVHSGVCTDPQPLKEQPALNDSRYAL SSRLRVSATFWQDPRNHFRCQVQFYGLSEADEWTQARAKPVTQIVSAEAWGRA D |
| SEQ ID NO: 141 | QSVLTQPPSVSGAPGQRVTISCSGSRSNIGSNTVKWYQQLPGTAPKLLIYYND QRPSGVPDRFSGSKSGTSASLAITGLQAEDEADYYCQSYDRYTHPALLFGTGT KVTVLGQPKANPTVTLFPPSSEELQANKATLVCLISDFYPGAVTVAWKADGSP |
| | VKAGVETTKPSKQSNNKYAASSYLSLTPEQWKSHRSYSCQVTHEGSTVEKTVA PTECS |
| SEQ ID NO: 142 | TIKPCPPCKCPAPNLLGGPSVFIFPPKIKDVLMISLSPIVTCVVVDVSEDDPD VQISWFVNNVEVHTAQTQTHREDYNSTLRVVSALPIQHQDWMSGKEFKCKVNN KDLPAPIERTISKPKPNIQNPEPAVYQLKDPRSQDSTLCLFTDFDSQINVPKT MESGTFITDKCVLDMKAMDSKSNGAIAWSNQTSFTCQDIFKETNATYPSSDVP CDATLTEKSFETDMNLNFQNLS |
| SEQ ID NO: 143 | TIKPCPPCKCPAPNLLGGPSVFIFPPKIKDVLMISLSPIVTCVVVDVSEDDPD VQISWFVNNVEVHTAQTQTHREDYNSTLRVVSALPIQHQDWMSGKEFKCKVNN KDLPAPIERTISKPGKPNIQNPEPAVYQLKDPRSQDSTLCLFTDFDSQINVPK TMESGTFITDKCVLDMKAMDSKSNGAIAWSNQTSFTCQDIFKETNATYPSSDV PCDATLTEKSFETDMNLNFQNLS |
| SEQ ID NO: 144 | TIKPCPPCKCPAPNLLGGPSVFIFPPKIKDVLMISLSPIVTCVVVDVSEDDPD VQISWFVNNVEVHTAQTQTHREDYNSTLRVVSALPIQHQDWMSGKEFKCKVNN KDLPAPIERTISKPGGKPNIQNPEPAVYQLKDPRSQDSTLCLFTDFDSQINVP KTMESGTFITDKCVLDMKAMDSKSNGAIAWSNQTSFTCQDIFKETNATYPSSD VPCDATLTEKSFETDMNLNFQNLS |
| SEQ ID NO: 145 | TIKPCPPCKCPAPNLLGGPSVFIFPPKIKDVLMISLSPIVTCVVVDVSEDDPD VQISWFVNNVEVHTAQTQTHREDYNSTLRVVSALPIQHQDWMSGKEFKCKVNN KDLPAPIERTISKPGGGKPNIQNPEPAVYQLKDPRSQDSTLCLFTDFDSQINV PKTMESGTFITDKCVLDMKAMDSKSNGAIAWSNQTSFTCQDIFKETNATYPSS DVPCDATLTEKSFETDMNLNFQNLS |
| SEQ ID NO: 146 | TIKPCPPCKCPAPNLLGGPSVFIFPPKIKDVLMISLSPIVTCVVVDVSEDDPD VQISWFVNNVEVHTAQTQTHREDYNSTLRVVSALPIQHQDWMSGKEFKCKVNN KDLPAPIERTISKPGGGGKPNIQNPEPAVYQLKDPRSQDSTLCLFTDFDSQIN VPKTMESGTFITDKCVLDMKAMDSKSNGAIAWSNQTSFTCQDIFKETNATYPS SDVPCDATLTEKSFETDMNLNFQNLS |
| SEQ ID NO: 147 | TIKPCPPCKCPAPNLLGGPSVFIFPPKIKDVLMISLSPIVTCVVVDVSEDDPD VQISWFVNNVEVHTAQTQTHREDYNSTLRVVSALPIQHQDWMSGKEFKCKVNN KDLPAPIERTISKPGGGGSKPNIQNPEPAVYQLKDPRSQDSTLCLFTDFDSQI NVPKTMESGTFITDKCVLDMKAMDSKSNGAIAWSNQTSFTCQDIFKETNATYP SSDVPCDATLTEKSFETDMNLNFQNLS |
| SEQ ID NO: 148 | TIKPCPPCKCPAPNLLGGPSVFIFPPKIKDVLMISLSPIVTCVVVDVSEDDPD VQISWFVNNVEVHTAQTQTHREDYNSTLRVVSALPIQHQDWMSGKEFKCKVNN KDLPAPIERTISKPGGGGSGKPNIQNPEPAVYQLKDPRSQDSTLCLFTDFDSQ INVPKTMESGTFITDKCVLDMKAMDSKSNGAIAWSNQTSFTCQDIFKETNATY PSSDVPCDATLTEKSFETDMNLNFQNLS |
| SEQ ID NO: 149 | TIKPCPPCKCPAPNLLGGPSVFIFPPKIKDVLMISLSPIVTCVVVDVSEDDPD VQISWFVNNVEVHTAQTQTHREDYNSTLRVVSALPIQHQDWMSGKEFKCKVNN KDLPAPIERTISKPGGGGSGGKPNIQNPEPAVYQLKDPRSQDSTLCLFTDFDS QINVPKTMESGTFITDKCVLDMKAMDSKSNGAIAWSNQTSFTCQDIFKETNAT YPSSDVPCDATLTEKSFETDMNLNFQNLS |
| SEQ ID NO: 150 | TIKPCPPCKCPAPNLLGGPSVFIFPPKIKDVLMISLSPIVTCVVVDVSEDDPD VQISWFVNNVEVHTAQTQTHREDYNSTLRVVSALPIQHQDWMSGKEFKCKVNN KDLPAPIERTISKPGGGGSGGGKPNIQNPEPAVYQLKDPRSQDSTLCLFTDFD SQINVPKTMESGTFITDKCVLDMKAMDSKSNGAIAWSNQTSFTCQDIFKETNA TYPSSDVPCDATLTEKSFETDMNLNFQNLS |
| SEQ ID NO: 151 | TIKPCPPCKCPAPNLLGGPSVFIFPPKIKDVLMISLSPIVTCVVVDVSEDDPD VQISWFVNNVEVHTAQTQTHREDYNSTLRVVSALPIQHQDWMSGKEFKCKVNN KDLPAPIERTISKPGGGGSGGGGKPNIQNPEPAVYQLKDPRSQDSTLCLFTDF DSQINVPKTMESGTFITDKCVLDMKAMDSKSNGAIAWSNQTSFTCQDIFKETN ATYPSSDVPCDATLTEKSFETDMNLNFQNLS |
| SEQ ID NO: 152 | TIKPCPPCKCPAPNLLGGPSVFIFPPKIKDVLMISLSPIVTCVVVDVSEDDPD VQISWFVNNVEVHTAQTQTHREDYNSTLRVVSALPIQHQDWMSGKEFKCKVNN KDLPAPIERTISKPGGGGSGGGGSKPNIQNPEPAVYQLKDPRSQDSTLCLFTD FDSQINVPKTMESGTFITDKCVLDMKAMDSKSNGAIAWSNQTSFTCQDIFKET NATYPSSDVPCDATLTEKSFETDMNLNFQNLS |
| SEQ ID NO: 153 | TIKPCPPCKCPAPNLLGGPSVFIFPPKIKDVLMISLSPIVTCVVVDVSEDDPD VQISWFVNNVEVHTAQTQTHREDYNSTLRVVSALPIQHQDWMSGKEFKCKVNN KDLPAPIERTISKPVVEDLRNVTPPKVSLFEPSKAEIANKQKATLVCLARGFF PDHVELSWWVNGKEVHSGVCTDPQAYKESNYSYSLSSRLRVSATFWHNPRNHF RCQVQFHGLSEEDKWPEGSPKPVTQNISAEAWGRADGGGGSGGGGSGGGGSSH LEQLLMDLQELLSRMENYRNLKLPRMLTAKFALPKQATELKDLQCLEDELGPL RHVLDLTQSKSFQLEDAENFISNIRVTVVKLKGSDNTFECQFDDESATVVDFL RRWIAFCQSIISTSPQ |
| SEQ ID NO: 154 | TIKPCPPCKCPAPNLLGGPSVFIFPPKIKDVLMISLSPIVTCVVVDVSEDDPD VQISWFVNNVEVHTAQTQTHREDYNSTLRVVSALPIQHQDWMSGKEFKCKVNN KDLPAPIERTISKPGVVEDLRNVTPPKVSLFEPSKAEIANKQKATLVCLARGF FPDHVELSWWVNGKEVHSGVCTDPQAYKESNYSYSLSSRLRVSATFWHNPRNH FRCQVQFHGLSEEDKWPEGSPKPVTQNISAEAWGRADGGGGSGGGGSGGGGSS HLEQLLMDLQELLSRMENYRNLKLPRMLTAKFALPKQATELKDLQCLEDELGP LRHVLDLTQSKSFQLEDAENFISNIRVTVVKLKGSDNTFECQFDDESATVVDF LRRWIAFCQSIISTSPQ |
| SEQ ID NO: 155 | TIKPCPPCKCPAPNLLGGPSVFIFPPKIKDVLMISLSPIVTCVVVDVSEDDPD VQISWFVNNVEVHTAQTQTHREDYNSTLRVVSALPIQHQDWMSGKEFKCKVNN KDLPAPIERTISKPGGVVEDLRNVTPPKVSLFEPSKAEIANKQKATLVCLARG FFPDHVELSWWVNGKEVHSGVCTDPQAYKESNYSYSLSSRLRVSATFWHNPRN HFRCQVQFHGLSEEDKWPEGSPKPVTQNISAEAWGRADGGGGSGGGGSGGGGS SHLEQLLMDLQELLSRMENYRNLKLPRMLTAKFALPKQATELKDLQCLEDELG PLRHVLDLTQSKSFQLEDAENFISNIRVTVVKLKGSDNTFECQFDDESATVVD FLRRWIAFCQSIISTSPQ |
| SEQ ID NO: 156 | TIKPCPPCKCPAPNLLGGPSVFIFPPKIKDVLMISLSPIVTCVVVDVSEDDPD VQISWFVNNVEVHTAQTQTHREDYNSTLRVVSALPIQHQDWMSGKEFKCKVNN KDLPAPIERTISKPGGGVVEDLRNVTPPKVSLFEPSKAEIANKQKATLVCLAR GFFPDHVELSWWVNGKEVHSGVCTDPQAYKESNYSYSLSSRLRVSATFWHNPR NHFRCQVQFHGLSEEDKWPEGSPKPVTQNISAEAWGRADGGGGSGGGGSGGGG SSHLEQLLMDLQELLSRMENYRNLKLPRMLTAKFALPKQATELKDLQCLEDEL GPLRHVLDLTQSKSFQLEDAENFISNIRVTVVKLKGSDNTFECQFDDESATVV DFLRRWIAFCQSIISTSPQ |
| SEQ ID NO: 157 | TIKPCPPCKCPAPNLLGGPSVFIFPPKIKDVLMISLSPIVTCVVVDVSEDDPD VQISWFVNNVEVHTAQTQTHREDYNSTLRVVSALPIQHQDWMSGKEFKCKVNN KDLPAPIERTISKPGGGGVVEDLRNVTPPKVSLFEPSKAEIANKQKATLVCLA RGFFPDHVELSWWVNGKEVHSGVCTDPQAYKESNYSYSLSSRLRVSATFWHNP RNHFRCQVQFHGLSEEDKWPEGSPKPVTQNISAEAWGRADGGGGSGGGGSGGG |
| | GSSHLEQLLMDLQELLSRMENYRNLKLPRMLTAKFALPKQATELKDLQCLEDE LGPLRHVLDLTQSKSFQLEDAENFISNIRVTVVKLKGSDNTFECQFDDESATV VDFLRRWIAFCQSIISTSPQ |
| SEQ ID NO: 166 | TIKPCPPCKCPAPNLLGGPSVFIFPPKIKDVLMISLSPIVTCVVVDVSEDDPD VQISWFVNNVEVHTAQTQTHREDYNSTLRVVSALPIQHQDWMSGKEFKCKVNN KDLPAPIERTISKPGGGGSVVEDLRNVTPPKVSLFEPSKAEIANKQKATLVCL ARGFFPDHVELSWWVNGKEVHSGVCTDPQAYKESNYSYSLSSRLRVSATFWHN PRNHFRCQVQFHGLSEEDKWPEGSPKPVTQNISAEAWGRADGGGGSGGGGSGG GGSSHLEQLLMDLQELLSRMENYRNLKLPRMLTAKFALPKQATELKDLQCLED ELGPLRHVLDLTQSKSFQLEDAENFISNIRVTVVKLKGSDNTFECQFDDESAT VVDFLRRWIAFCQSIISTSPQ |
| SEQ ID NO: 167 | TIKPCPPCKCPAPNLLGGPSVFIFPPKIKDVLMISLSPIVTCVVVDVSEDDPD VQISWFVNNVEVHTAQTQTHREDYNSTLRVVSALPIQHQDWMSGKEFKCKVNN KDLPAPIERTISKPGGGGSGVVEDLRNVTPPKVSLFEPSKAEIANKQKATLVC LARGFFPDHVELSWWVNGKEVHSGVCTDPQAYKESNYSYSLSSRLRVSATFWH NPRNHFRCQVQFHGLSEEDKWPEGSPKPVTQNISAEAWGRADGGGGSGGGGSG GGGSSHLEQLLMDLQELLSRMENYRNLKLPRMLTAKFALPKQATELKDLQCLE DELGPLRHVLDLTQSKSFQLEDAENFISNIRVTVVKLKGSDNTFECQFDDESA TVVDFLRRWIAFCQSIISTSPQ |
| SEQ ID NO: 160 | TIKPCPPCKCPAPNLLGGPSVFIFPPKIKDVLMISLSPIVTCVVVDVSEDDPD VQISWFVNNVEVHTAQTQTHREDYNSTLRVVSALPIQHQDWMSGKEFKCKVNN KDLPAPIERTISKPGGGGSGGVVEDLRNVTPPKVSLFEPSKAEIANKQKATLV CLARGFFPDHVELSWWVNGKEVHSGVCTDPQAYKESNYSYSLSSRLRVSATFW HNPRNHFRCQVQFHGLSEEDKWPEGSPKPVTQNISAEAWGRADGGGGSGGGGS GGGGSSHLEQLLMDLQELLSRMENYRNLKLPRMLTAKFALPKQATELKDLQCL EDELGPLRHVLDLTQSKSFQLEDAENFISNIRVTVVKLKGSDNTFECQFDDES ATVVDFLRRWIAFCQSIISTSPQ |
| SEQ ID NO: 161 | TIKPCPPCKCPAPNLLGGPSVFIFPPKIKDVLMISLSPIVTCVVVDVSEDDPD VQISWFVNNVEVHTAQTQTHREDYNSTLRVVSALPIQHQDWMSGKEFKCKVNN KDLPAPIERTISKPGGGGSGGGVVEDLRNVTPPKVSLFEPSKAEIANKQKATL VCLARGFFPDHVELSWWVNGKEVHSGVCTDPQAYKESNYSYSLSSRLRVSATF WHNPRNHFRCQVQFHGLSEEDKWPEGSPKPVTQNISAEAWGRADGGGGSGGGG SGGGGSSHLEQLLMDLQELLSRMENYRNLKLPRMLTAKFALPKQATELKDLQC LEDELGPLRHVLDLTQSKSFQLEDAENFISNIRVTVVKLKGSDNTFECQFDDE SATVVDFLRRWIAFCQSIISTSPQ |
| SEQ ID NO: 162 | TIKPCPPCKCPAPNLLGGPSVFIFPPKIKDVLMISLSPIVTCVVVDVSEDDPD VQISWFVNNVEVHTAQTQTHREDYNSTLRVVSALPIQHQDWMSGKEFKCKVNN KDLPAPIERTISKPGGGGSGGGGVVEDLRNVTPPKVSLFEPSKAEIANKQKAT LVCLARGFFPDHVELSWWVNGKEVHSGVCTDPQAYKESNYSYSLSSRLRVSAT FWHNPRNHFRCQVQFHGLSEEDKWPEGSPKPVTQNISAEAWGRADGGGGSGGG GSGGGGSSHLEQLLMDLQELLSRMENYRNLKLPRMLTAKFALPKQATELKDLQ CLEDELGPLRHVLDLTQSKSFQLEDAENFISNIRVTVVKLKGSDNTFECQFDD ESATVVDFLRRWIAFCQSIISTSPQ |
| SEQ ID NO: 163 | TIKPCPPCKCPAPNLLGGPSVFIFPPKIKDVLMISLSPIVTCVVVDVSEDDPD VQISWFVNNVEVHTAQTQTHREDYNSTLRVVSALPIQHQDWMSGKEFKCKVNN KDLPAPIERTISKPGGGGSGGGGSVVEDLRNVTPPKVSLFEPSKAEIANKQKA TLVCLARGFFPDHVELSWWVNGKEVHSGVCTDPQAYKESNYSYSLSSRLRVSA |
| | TFWHNPRNHFRCQVQFHGLSEEDKWPEGSPKPVTQNISAEAWGRADGGGGSGG GGSGGGGSSHLEQLLMDLQELLSRMENYRNLKLPRMLTAKFALPKQATELKDL QCLEDELGPLRHVLDLTQSKSFQLEDAENFISNIRVTVVKLKGSDNTFECQFD DESATVVDFLRRWIAFCQSIISTSPQ |
| SEQ ID NO: 164 | QVQLVESGGGVVQPGRSLRLSCAASGFTFSSYTMHWVRQAPGKGLEWVTFISY DGNNKYYADSVKGRFTISRDNSKNTLYLQMNSLRAEDTAIYYCARTGWLGPFD YWGQGTLVTVSSASTKGPSVFPLAPSSKSTSGGTAALGCLVKDYFPEPVTVSW NSGALTSGVHTFPAVLQSSGLYSLSSVVTVPSSSLGTQTYICNVNHKPSNTKV DKRVEPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDWLNGK EYKCKVSNKALPAPIEKTISKAKGQPREPQVYTLPPCREEMTKNQVSLWCLVK GFYPSDIAVEWESNGQPENNYKTTPPVLDSDGSFFLYSKLTVDKSRWQQGNVF SCSVMHEALHNHYTQKSLSLSPGK |
| SEQ ID NO: 165 | QVQLVESGGGVVQPGRSLRLSCAASGFTFSSYGMHWVRQAPGKGLEWVAFIRY DGSNKYYADSVKGRFTISRDNSKNTLYLQMNSLRAEDTAVYYCKTHGSHDNWG QGTMVTVSSASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKR VEPKSCDKTHTCPPCPAPELLGGPSVFLFPPKPKDTLMISRTPEVTCVVVDVS HEDPEVKFNWYVDGVEVHNAKTKPREEQYNSTYRVVSVLTVLHQDWLNGKEYK CKVSNKALPAPIEKTISKAKGQPREPQVCTLPPSREEMTKNQVSLSCAVKGFY PSDIAVEWESNGQPENNYKTTPPVLDSDGSFFLVSKLTVDKSRWQQGNVFSCS VMHEALHNHYTQKSLSLSPGK |

**Table 7. Sequences used to construct multispecific molecules.**

| Multispecific molecule | Heavy Chain 1 | Light Chain 1 | Heavy Chain 2 | Light Chain 2 |
|---|---|---|---|---|
| Multispecific molecule 1 | SEQ ID NO: 164 | SEQ ID NO: 129 | SEQ ID NO: 165 | SEQ ID NO: 141 |
| Multispecific molecule 2 | SEQ ID NO: 118 | SEQ ID NO: 129 | SEQ ID NO: 130 | SEQ ID NO: 141 |

### 3. Kappa/Lambda select resin analysis of chain pairing.

The kappa and lambda light chain pairing of bispecific constructs was analyzed by incubating 1 mg of protein with 100 µL of either KappaSelect (GE 17-5458-01) or LambdaFabSelect (GE 17-5482-01) resin. After incubating for 1-3 hours, the resin was packed into a column, washed with 3 x 10 column volumes of Dulbecco's phosphate-buffered saline (DPBS, Life Technologies 14190-144). The bound protein was eluted from the column with 100 mM citrate, pH 2.5. The content of the load, flow-through, and elution fractions was analyzed using gels of samples non-reduced and reduced with 200 mM Bond-Breaker TCEP (Thermo Scientific 77720), allowing for the identification of the various chains. For quantitative assessment of the chain pairing, the amount of protein in the load and flow-through fractions was assessed using the absorbance at 280 nm with a NanoDrop.

### Example 2

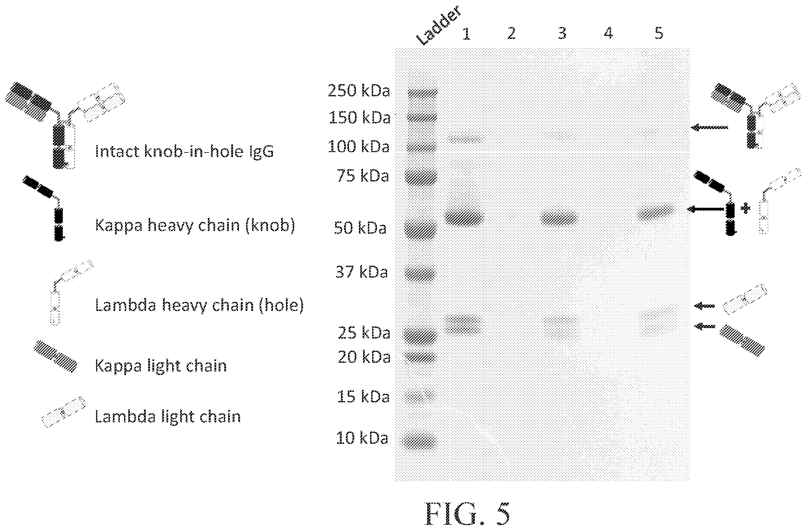

Multispecific molecule 1, represented by FIG. 4A, comprises an anti-CTLA4 targeting arm and an anti-IL12β targeting arm. As illustrated by FIG. 4A, one CH3 domain comprises knob mutations, and the other CH3 domain comprises hole mutations. Multispecific molecule 1 was expressed by co-transfecting cells with SEQ ID: 89, SEQ ID: 54, SEQ ID: 90, and SEQ ID: 66, to produce the four distinct chains: SEQ ID: 164, SEQ ID: 129, SEQ ID: 165, and SEQ ID: 141. A KappaSelect and LambdaFabSelect analysis was performed with multispecific molecule 1. The gel shows no protein in the flow-through of the KappaSelect or LambdaFabSelect columns (FIG. 5). The data suggest that multispecific molecule 1, which has the knob-into-holes IgG configuration shown in FIG. 4A, demonstrates correct heavy chain heterodimer formation and the two Fabs do not swap the kappa and lambda light chains with each other.

### Example 3

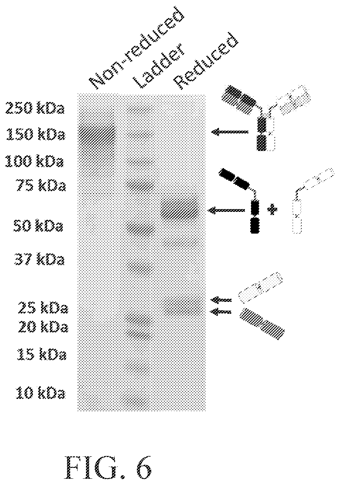

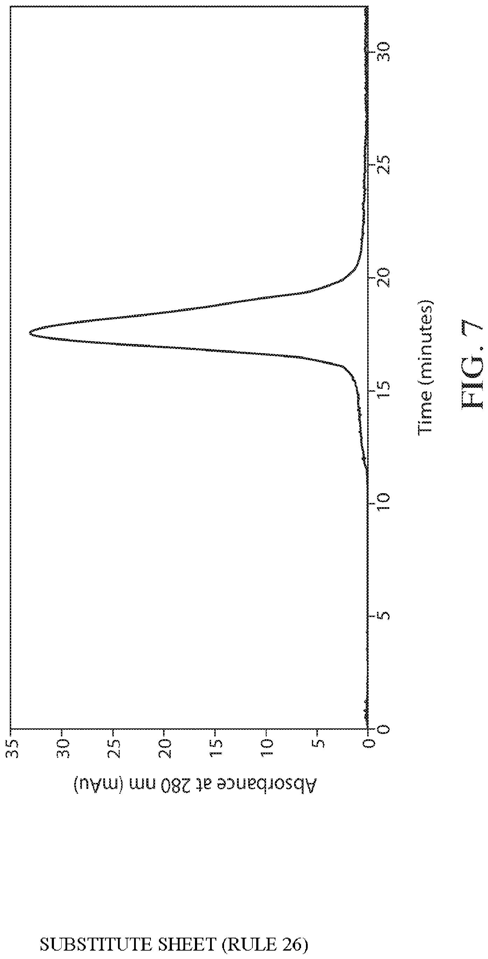

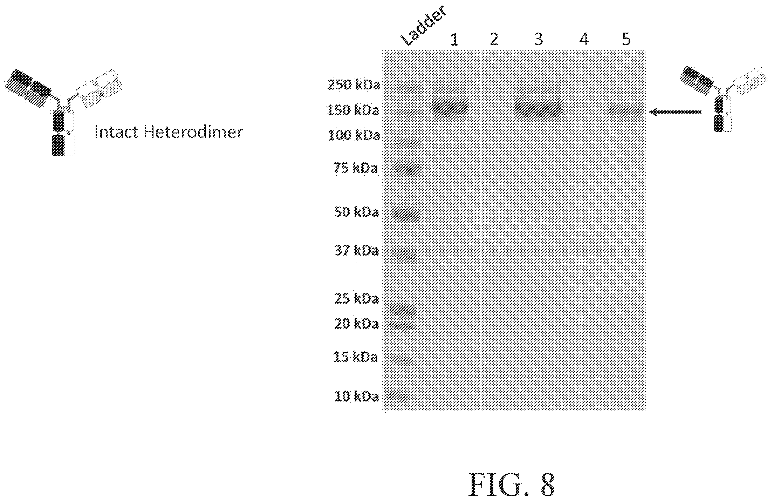

Multispecific molecule 2, represented by FIG. 4B, comprises an anti-CTLA4 targeting arm and an anti-IL12β targeting arm. As shown in FIG. 4B, one CH2 domain is linked to TCRα constant domain, and the other CH2 domain is linked to TCRβ constant domain. Multispecific molecule 2 was expressed by co-transfecting cells with SEQ ID: 43, SEQ ID: 54, SEQ ID: 55, and SEQ ID: 66, to produce the four distinct chains: SEQ ID: 118, SEQ ID: 129, SEQ ID: 130, and SEQ ID: 141. Multispecific molecule 2 was purified and a gel of the final molecule is shown in FIG. 6, displaying the intact protein in the non-reduced sample and all of the chains in the reduced sample. FIG. 6 suggests that multispecific molecule 2 behaves like a proper IgG like molecule. FIG. 7 shows the size exclusion chromatogram of multispecific molecule 2, indicating that it runs as heterodimer. A KappaSelect and LambdaFabSelect analysis was performed with multispecific molecule 2, shown in FIG. 8. Similar to multispecific molecule 1 tested in Example 2, the gel in FIG. 8 shows that there was no protein in the flow-through for either the KappaSelect or LambdaFabSelect columns. The data suggest that the two Fabs in multispecific molecule 2 do not swap light chains and the protein is an intact heterodimer, running at ~150 kDa.

Multispecific molecules 1 and 2 share the two Fab targeting arms and differ only in that multispecific molecule 1 comprises knob-into-holes CH3 domains whereas in multispecific molecule 2, the two CH3 domains in heavy chains are replaced by a TCRα constant domain and a TCRβ constant domain, respectively (see FIGs. 4A and 4B). The data described in Example 2 and Example 3 demonstrate that both molecules form stable heterodimers. Without wishing to be bound by theory, the TCRα constant domain and TCRβ constant domain can replace knob-into-holes CH3 domains to drive heterodimer formation.

## Claims

1. A multispecific molecule comprising at least two non-contiguous polypeptide chains and comprising:
(a) a first antigen binding moiety (ABM),
(b) a second ABM that does not bind to the same epitope as the first ABM, and
(c) a heterodimerization domain comprising:
- a first polypeptide chain comprising an immunoglobulin CH2 domain connected, optionally via a linker, to a TCRα constant domain or fragment thereof; and
- a second polypeptide chain comprising an immunoglobulin CH2 domain connected, optionally via a linker, to a TCRβ constant domain or fragment thereof; wherein:
(i) the first ABM is connected, optionally via a linker, to the first polypeptide chain;
(ii) the second ABM is connected, optionally via a linker, to the second polypeptide chain;
(iii) the TCRα constant domain or fragment thereof comprises or consists of at least 20, 30, 40, 50, 60, 70, 80, 90, or 100 contiguous amino acids of SEQ ID NO: 158 or at least 20, 30, 40, 50, 60, 70, or 80 contiguous amino acids of SEQ ID NO: 1; and/or
(iv) the TCRβ constant domain or fragment thereof comprises or consists of at least 20, 30, 40, 50, 60, 70, 80, 90, 100, 110, 120, 130, 140, 150, 160, or 170 contiguous amino acids of SEQ ID NO: 159 or at least 20, 30, 40, 50, 60, 70, 80, 90, 100, 110, 120, or 130 contiguous amino acids of SEQ ID NO: 2.

2. The multispecific molecule of claim 1,
(i) wherein:
the first ABM is connected, optionally via a linker, to the N-terminus or the C-terminus of the first polypeptide chain; and
the second ABM is connected, optionally via a linker, to the N-terminus or the C-terminus of the second polypeptide chain;
(ii) wherein:
the first polypeptide chain comprises the immunoglobulin CH2 domain connected, optionally via a linker, to the N-terminus or the C-terminus of the TCRα constant domain or fragment thereof, and/or
the second polypeptide chain comprises the immunoglobulin CH2 domain connected, optionally via a linker, to the N-terminus or the C-terminus of the TCRβ constant domain or fragment thereof; and/or
(iii) wherein:
(a) the first polypeptide chain of the heterodimerization domain does not comprise an immunoglobulin CH3 domain or a portion thereof;
(b) the second polypeptide chain of the heterodimerization domain does not comprise an immunoglobulin CH3 domain or a portion thereof;
(c) neither the first polypeptide chain nor the second polypeptide chain of the heterodimerization domain comprises an immunoglobulin CH3 domain or a portion thereof;
optionally, wherein the linker comprises or consists of the amino acid sequence of any one of SEQ ID NOs: 101-110.

3. The multispecific molecule of claim 1 or 2, wherein:
(i) the TCRα constant domain comprises or consists of the amino acid sequence of SEQ ID NO: 158, or a sequence having at least 75, 80, 85, 90, or 99% identity thereof, and/or
the TCRβ constant domain comprises or consists of the amino acid sequence of SEQ ID NO: 159, or a sequence having at least 75, 80, 85, 90, or 99% identity thereof,
optionally, wherein the TCRα constant domain comprises or consists of the amino acid sequence of SEQ ID NO: 158 and/or the TCRβ constant domain comprises or consists of the amino acid sequence of SEQ ID NO: 159;
(ii) the TCRα constant domain comprises or consists of the amino acid sequence of SEQ ID NO: 1, or a sequence having at least 75, 80, 85, 90, or 99% identity thereof, and/or
the TCRβ constant domain comprises or consists of the amino acid sequence of SEQ ID NO: 2, or a sequence having at least 75, 80, 85, 90, or 99% identity thereof,
optionally, wherein the TCRα constant domain comprises or consists of the amino acid sequence of SEQ ID NO: 1 and/or the TCRβ constant domain comprises or consists of the amino acid sequence of SEQ ID NO: 2;
(iii) the TCRα constant domain or fragment thereof comprises or consists of at least 30, 40, 50, 60, 70, 80, 90, or 100 contiguous amino acids of SEQ ID NO: 158 or at least 30, 40, 50, 60, 70, or 80 contiguous amino acids of SEQ ID NO: 1; and/or
the TCRβ constant domain or fragment thereof comprises or consists of at least 30, 40, 50, 60, 70, 80, 90, 100, 110, 120, 130, 140, 150, 160, or 170 contiguous amino acids of SEQ ID NO: 159 or at least 30, 40, 50, 60, 70, 80, 90, 100, 110, 120, or 130 contiguous amino acids of SEQ ID NO: 2; or
(iv) the TCRα constant domain or fragment thereof comprises amino acids 1-85 or 1-93 of SEQ ID NO: 158; and/or
the TCRβ constant domain or fragment thereof comprises amino acids 1-130 of SEQ ID NO: 159,
optionally, wherein the TCRα constant domain or fragment thereof comprises a T49C substitution, numbered according to SEQ ID NO: 158; and/or the TCRβ constant domain or fragment thereof comprises a S57C substitution, numbered according to SEQ ID NO: 159.

4. The multispecific molecule of any one of claims 1-3, wherein:
(i) the first ABM comprises a first antibody molecule, and the second ABM comprises a second antibody molecule, wherein:
(a) the first antibody molecule comprises a kappa light chain constant region or a fragment thereof, and the second antibody molecule comprises a lambda light chain constant region or a fragment thereof; or
(b) the first antibody molecule comprises a lambda light chain constant region or a fragment thereof, and the second antibody molecule comprises a kappa light chain constant region or a fragment thereof; or
(ii) the first ABM comprises a first heavy chain comprising a first heavy chain variable region and a first heavy chain constant region, and a lambda light chain comprising a lambda variable region and a lambda constant region, and the second ABM comprises a second heavy chain comprising a second heavy chain variable region and a second heavy chain constant region, and a kappa light chain comprising a kappa variable region and a kappa constant region.

5. The multispecific molecule of any one of claims 1-4, wherein the first ABM or the second ABM comprises a tumor-targeting moiety that binds to a tumor antigen, a stromal antigen, or a hematological antigen, wherein:
(i) the tumor antigen is present on one or more of pancreatic, breast, colorectal, lung, skin, ovarian, or liver cancer;
(ii) the tumor antigen is chosen from: PDL1, mesothelin, CD47, gangloside 2 (GD2), prostate stem cell antigen (PSCA), prostate specific membrane antigen (PMSA), prostatespecific antigen (PSA), carcinoembryonic antigen (CEA), Ron Kinase, c-Met, Immature laminin receptor, TAG-72, BING-4, Calcium-activated chloride channel 2, Cyclin-B1, 9D7, Ep-CAM, EphA3, Her2/neu, Telomerase, SAP-1, Survivin, NY-ESO-1/LAGE-1, PRAME, SSX-2, Melan-A/MART-1, Gp100/pmel17, Tyrosinase, MC1R, β-catenin, BRCA1/2, CDK4, CML66, Fibronectin, p53, Ras, TGF-B receptor, AFP, ETA, MAGE, CA-125, BAGE, GAGE,, CDC27, α actinin-4, TRP1/gp75, TRP2, gangliosides, WT1, Epidermal growth factor receptor (EGFR), CD20, MART-2, MUC1, MUC2, MUM1, MUM2, MUM3, NA88-1, NPM, OA1, OGT, RCC, RUI11, RU12, SAGE, TRG, TSTA, Folate receptor alpha, L1-CAM, CAIX, EGFRvIII, gpA33, GD3, GM2, VEGFR, Integrins, Carbohydrates (Le), IGF1R, TRAILR1, TRAILR2, or RANKL,
optionally, wherein Integrins are Integrin alphaVbeta3 or Integrin alpha5Beta1;
(iii) the stromal antigen is chosen from fibroblast activating protease (FAP), TGF-beta, hyaluronic acid, collagen, tenascin C, or tenascin W; or
(iv) the hematological antigen is chosen from CD19, CD33, CD47, CD123, CD20, CD99, CD30, BCMA, CD38, CD22, SLAMF7, or NY-ESO1.

6. The multispecific molecule of any one of claims 1-5, wherein the first ABM or the second ABM comprises an immune cell engager comprising a T cell engager, a natural killer (NK) cell engager, a B cell engager, a dendritic cell engager, or a macrophage cell engager, wherein:
(i) the T cell engager binds to CD3, TCRα, TCRβ, TCRγ, TCRζ, ICOS, CD28, CD27, HVEM, LIGHT, CD40, 4-1BB, OX40, DR3, GITR, CD30, TIM1, SLAM, CD2, or CD226;
(ii) the NK cell engager is chosen from an antibody molecule, or a ligand that binds to: NKp30, NKp40, NKp44, NKp46, NKG2D, DNAM1, DAP10, CD16, CRTAM, CD27, PSGL1, CD96, CD100 (SEMA4D), NKp80, CD244 (also known as SLAMF4 or 2B4), SLAMF6, SLAMF7, KIR2DS2, KIR2DS4, KIR3DS1, KIR2DS3, KIR2DS5, KIR2DS1, CD94, NKG2C, NKG2E, or CD160;
(iii) the B cell engager is a CD40L, an OX40L, or a CD70 ligand, or an antibody molecule that binds to OX40, CD40 or CD70;
(iv) the macrophage cell engager is a CD2 agonist; a CD40L; an OX40L; an antibody molecule that binds to OX40, CD40 or CD70; an agonist of a Toll-like receptor (TLR); CD47; or a STING agonist; or
(v) the dendritic cell engager is a CD2 agonist, an OX40 antibody, an OX40L, a 41BB agonist, a Toll-like receptor agonist or a fragment thereof, CD47 agonist, or a STING agonist.

7. The multispecific molecule of any one of claims 1-6, further comprising:
(i) a cytokine molecule chosen from interleukin-2 (IL-2), interleukin-7 (IL-7), interleukin-12 (IL-12), interleukin-15 (IL-15), interleukin-18 (IL-18), interleukin-21 (IL-21), or interferon gamma, or a fragment or variant thereof, or a combination thereof; or
(ii) a stromal modifying molecule comprising a hyaluronidase molecule, a collagenase molecule, a chondroitinase molecule, a matrix metalloproteinase, or a variant thereof.

8. The multispecific molecule of any one of claims 1-7, comprising:
(a) the first polypeptide chain having the following configuration from N-terminus to C-terminus: a first portion of the first ABM comprising a VH-CH1 of a first Fab molecule connected, optionally via a linker, to a first immunoglobulin CH2 connected, optionally via a linker, to the TCRα constant domain or fragment thereof;
(b) the second polypeptide chain having the following configuration from N-terminus to C-terminus: a first portion of the second ABM comprising a VH-CH1 of a second Fab molecule connected, optionally via a linker, to a second immunoglobulin CH2 connected, optionally via a linker, to the TCRβ constant domain or fragment thereof;
(c) a third polypeptide having the following configuration from N-terminus to C-terminus: a VL-CL of the first Fab; and
(d) a fourth polypeptide having the following configuration from N-terminus to C-terminus: a VL-CL of the second Fab,
wherein the VL-CL of the first Fab is of kappa subtype and the VL-CL of the second Fab is of lambda subtype, or the VL-CL of the first Fab is of lambda subtype and the VL-CL of the second Fab is of kappa subtype;
wherein the first and the third polypeptides form the first ABM that binds a first antigen, and the second and the fourth polypeptides form the second ABM that binds a second antigen,
wherein the first and the second polypeptides form a heterodimer, and,
optionally, wherein the TCRα constant domain comprises the amino acid sequence of SEQ ID NO: 1 or a sequence having at least 75, 80, 85, 90, or 99% identity thereof, and/or the TCRβ constant domain comprises the amino acid sequence of SEQ ID NO: 2 or a sequence having at least 75, 80, 85, 90, or 99% identity thereof.

9. An isolated nucleic acid molecule encoding the multispecific molecule of any one of claims 1-8.

10. A vector comprising the nucleic acid molecule of claim 9.

11. A host cell comprising the nucleic acid molecule of claim 9, or the vector of claim 10.

12. A pharmaceutical composition comprising the multispecific molecule of any one of claims 1-8 and a pharmaceutically acceptable carrier, excipient, or stabilizer.

13. A method of making the multispecific molecule of any one of claims 1-8, comprising culturing the host cell of claim 11, under conditions suitable for gene expression and/or heterodimerization.

14. The multispecific molecule of any one of claims 1-8 for use in a method of treating cancer in a subject in need thereof,the method comprising administering to the subject the multispecific molecule in an amount effective to treat the cancer.

## Patentansprüche

1. Multispezifisches Molekül, umfassend mindestens zwei nicht zusammenhängende Polypeptidketten und umfassend:
(a) eine erste antigenbindende Gruppe (antigen binding moiety - ABM),
(b) eine zweite ABM, die nicht an das gleiche Epitop wie die erste ABM bindet, und
(c) einen Heterodimerisationsbereich, umfassend:
- eine erste Polypeptidkette, umfassend einen Immunglobulin-CH2-Bereich, der, gegebenenfalls über einen Linker, mit einem konstanten TCRα-Bereich oder einem Fragment davon verbunden ist; und
- eine zweite Polypeptidkette, umfassend einen Immunglobulin-CH2-Bereich, der, gegebenenfalls über einen Linker, mit einem konstanten TCRβ-Bereich oder einem Fragment davon verbunden ist; wobei:
(i) die erste ABM, gegebenenfalls über einen Linker, mit der ersten Polypeptidkette verbunden ist;
(ii) die zweite ABM, gegebenenfalls über einen Linker, mit der zweiten Polypeptidkette verbunden ist;
(iii) der konstante TCRα-Bereich oder das Fragment davon mindestens 20, 30, 40, 50, 60, 70, 80, 90 oder 100 zusammenhängende Aminosäuren der SEQ-ID-NR: 158 oder mindestens 20, 30, 40, 50, 60, 70 oder 80 zusammenhängende Aminosäuren der SEQ-ID-NR: 1 umfasst oder daraus besteht; und/oder
(iv) der konstante TCRβ-Bereich oder das Fragment davon mindestens 20, 30, 40, 50, 60, 70, 80, 90, 100, 110, 120, 130, 140, 150, 160 oder 170 zusammenhängende Aminosäuren der SEQ-ID-NR: 159 oder mindestens 20, 30, 40, 50, 60, 70, 80, 90, 100, 110, 120 oder 130 zusammenhängende Aminosäuren der SEQ-ID-NR: 2 umfasst oder daraus besteht.

2. Multispezifisches Molekül nach Anspruch 1,
(i) wobei:
die erste ABM, gegebenenfalls über einen Linker, mit dem N-Terminus oder dem C-Terminus der ersten Polypeptidkette verbunden ist; und
die zweite ABM, gegebenenfalls über einen Linker, mit dem N-Terminus oder dem C-Terminus der zweiten Polypeptidkette verbunden ist;
(ii) wobei:
die erste Polypeptidkette den Immunglobulin-CH2-Bereich umfasst, der, gegebenenfalls über einen Linker, mit dem N-Terminus oder dem C-Terminus des konstanten TCRα-Bereichs oder dem Fragment davon verbunden ist, und/oder
die zweite Polypeptidkette den Immunglobulin-CH2-Bereich umfasst, der, gegebenenfalls über einen Linker, mit dem N-Terminus oder dem C-Terminus des konstanten TCRβ-Bereichs oder dem Fragment davon verbunden ist; und/oder
(iii) wobei:
(a) die erste Polypeptidkette des Heterodimerisationsbereichs keinen Immunglobulin-CH3-Bereich oder einen Abschnitt davon umfasst;
(b) die zweite Polypeptidkette des Heterodimerisationsbereichs keinen Immunglobulin-CH3-Bereich oder einen Abschnitt davon umfasst;
(c) weder die erste Polypeptidkette noch die zweite Polypeptidkette des Heterodimerisationsbereichs einen Immunglobulin-CH3-Bereich oder einen Abschnitt davon umfasst;
wobei optional der Linker die Aminosäuresequenz einer der SEQ-ID-NRs: 101-110 umfasst oder daraus besteht.

3. Multispezifisches Molekül nach Anspruch 1 oder 2, wobei:
(i) der konstante TCRα-Bereich die Aminosäuresequenz der SEQ-ID-NR: 158 oder eine Sequenz mit mindestens 75, 80, 85, 90 oder 99 % Identität davon umfasst oder daraus besteht und/oder der konstante TCRβ-Bereich die Aminosäuresequenz der SEQ-ID-NR: 159 oder eine Sequenz mit mindestens 75, 80, 85, 90 oder 99 % Identität davon umfasst oder daraus besteht,
wobei optional der konstante TCRα-Bereich die Aminosäuresequenz der SEQ-ID-NR: 158 umfasst oder daraus besteht und/oder der konstante TCRβ-Bereich die Aminosäuresequenz von SEQ-ID-NR: 159 umfasst oder daraus besteht;
(ii) der konstante TCRα-Bereich die Aminosäuresequenz der SEQ-ID-NR: 1 oder eine Sequenz mit mindestens 75, 80, 85, 90 oder 99 % Identität davon umfasst oder daraus besteht und/oder der konstante TCRβ-Bereich die Aminosäuresequenz der SEQ-ID-NR: 2 oder eine Sequenz mit mindestens 75, 80, 85, 90 oder 99 % Identität davon umfasst oder daraus besteht,
wobei optional der konstante TCRα-Bereich die Aminosäuresequenz der SEQ-ID-NR: 1 umfasst oder daraus besteht und/oder der konstante TCRβ-Bereich die Aminosäuresequenz von SEQ-ID-NR: 2 umfasst oder daraus besteht;
(iii) der konstante TCRα-Bereich oder das Fragment davon mindestens 30, 40, 50, 60, 70, 80, 90 oder 100 zusammenhängende Aminosäuren der SEQ-ID-NR: 158 oder mindestens 30, 40, 50, 60, 70 oder 80 zusammenhängende Aminosäuren der SEQ-ID-NR: 1 umfasst oder daraus besteht; und/oder
der konstante TCRβ-Bereich oder das Fragment davon mindestens 30, 40, 50, 60, 70, 80, 90, 100, 110, 120, 130, 140, 150, 160 oder 170 zusammenhängende Aminosäuren der SEQ-ID-NR: 159 oder mindestens 30, 40, 50, 60, 70, 80, 90, 100, 110, 120 oder 130 zusammenhängende Aminosäuren der SEQ-ID-NR: 2 umfasst oder daraus besteht; oder
(iv) der konstante TCRα-Bereich oder das Fragment davon die Aminosäuren 1-85 oder 1-93 von SEQ-ID-NR: 158 umfasst; und/oder
der konstante TCRβ-Bereich oder das Fragment davon die Aminosäuren 1-130 der SEQ-ID-NR: 159 umfasst,
wobei optional der konstante TCRα-Bereich oder das Fragment davon eine T49C-Substitution umfasst, die gemäß SEQ-ID-NR: 158 nummeriert ist; und/oder der konstante TCRβ-Bereich oder das Fragment davon eine S57C-Substitution umfasst, die gemäß SEQ-ID-NR: 159 nummeriert ist.

4. Multispezifisches Molekül nach einem der Ansprüche 1-3, wobei:
(i) die erste ABM ein erstes Antikörpermolekül umfasst und die zweite ABM ein zweites Antikörpermolekül umfasst, wobei:
(a) das erste Antikörpermolekül eine konstante Region der Kappa-Leichtkette oder ein Fragment davon umfasst und das zweite Antikörpermolekül eine konstante Region der Lambda-Leichtkette oder ein Fragment davon umfasst; oder
(b) das erste Antikörpermolekül eine konstante Region der Lambda-Leichtkette oder ein Fragment davon umfasst und das zweite Antikörpermolekül eine konstante Region der Kappa-Leichtkette oder ein Fragment davon umfasst; oder
(ii) die erste ABM eine erste Schwerkette, die eine erste variable Region der Schwerkette und eine erste konstante Region der Schwerkette umfasst, und eine Lambda-Leichtkette, die eine variable Region der Lambda-Kette und eine konstante Region der Lambda-Kette umfasst, umfasst, und die zweite ABM eine zweite Schwerkette, die eine zweite variable Region der Schwerkette und eine zweite konstante Region der Schwerkette umfasst, und eine Kappa-Leichtkette, die eine variable Region der Kappa-Kette und eine konstante Region der Kappa-Kette umfasst, umfasst.

5. Multispezifisches Molekül nach einem der Ansprüche 1-4, wobei die erste ABM oder die zweite ABM eine tumorgerichtete Gruppe umfasst, die an ein Tumorantigen, ein stromales Antigen oder ein hämatologisches Antigen bindet, wobei:
(i) das Tumorantigen bei einem oder mehreren von Pankreas-, Brust-, Kolorektal-, Lungen-, Haut-, Eierstock- oder Leberkrebs vorhanden ist;
(ii) das Tumorantigen ausgewählt ist aus: PDL1, Mesothelin, CD47, Ganglosid 2 (GD2), Prostata-Stammzell-Antigen (PSCA), prostataspezifisches Membran-Antigen (PMSA), prostataspezifisches Antigen (PSA), carcinoembryonales Antigen (CEA), Ron-Kinase, c-Met, unreifer Laminin-Rezeptor, TAG-72, BING-4, Calcium-aktivierter Chloridkanal 2, Cyclin-B1, 9D7, Ep-CAM, EphA3, Her2/neu, Telomerase, SAP-1, Survivin, NY-ESO-1/LAGE-1, PRAME, SSX-2, Melan-A/MART-1, Gp100/pmel17, Tyrosinase, MC1R, β-Catenin, BRCA 1/2, CDK4, CML66, Fibronectin, p53, Ras, TGF-B-Rezeptor, AFP, ETA, MAGE, CA-125, BAGE, GAGE, CDC27, α-Actinin-4, TRP1/gp75, TRP2, Ganglioside, WT1, Epidermaler Wachstumsfaktor-Rezeptor (EGFR), CD20, MART-2, MUC1, MUC2, MUM1, MUM2, MUM3, NA88-1, NPM, OA1, OGT, RCC, RUI11, RU12, SAGE, TRG, TSTA, Folatrezeptor alpha, L1-CAM, CAIX, EGFRvIII, gpA33, GD3, GM2, VEGFR, Integrine, Kohlenhydrate (Le), IGF1R, TRAILR1, TRAILR2 oder RANKL,
wobei optional die Integrine Integrin alphaVbeta3 oder Integrin alpha5beta1 sind;
(iii) das stromale Antigen ausgewählt ist aus Fibroblastenaktivierender Protease (FAP), TGF-beta, Hyaluronsäure, Kollagen, Tenascin C oder Tenascin W; oder
(iv) das hämatologische Antigen ausgewählt ist aus CD19, CD33, CD47, CD123, CD20, CD99, CD30, BCMA, CD38, CD22, SLAMF7 oder NY-ESO1.

6. Multispezifisches Molekül nach einem der Ansprüche 1-5, wobei die erste ABM oder die zweite ABM einen Immunzellen-Engager umfasst, der einen T-Zellen-Engager, einen Natürliche-Killer(NK)-Zellen-Engager, einen B-Zellen-Engager, einen Dendritische-Zellen-Engager oder einen Makrophagen-Zellen-Engager umfasst, wobei:
(i) der T-Zellen-Engager an CD3, TCRα, TCRβ, TCRγ, TCRζ, ICOS, CD28, CD27, HVEM, LIGHT, CD40, 4-1BB, OX40, DR3, GITR, CD30, TIM1, SLAM, CD2 oder CD226 bindet;
(ii) der NK-Zellen-Engager ausgewählt ist aus einem Antikörpermolekül oder einem Liganden, der an Folgende bindet: NKp30, NKp40, NKp44, NKp46, NKG2D, DNAM1, DAP10, CD16, CRTAM, CD27, PSGL1, CD96, CD100 (SEMA4D), NKp80, CD244 (auch bekannt als SLAMF4 oder 2B4), SLAMF6, SLAMF7, KIR2DS2, KIR2DS4, KIR3DS1, KIR2DS3, KIR2DS5, KIR2DS1, CD94, NKG2C, NKG2E oder CD160;
(iii) der B-Zellen-Engager ein CD40L, ein OX40L oder ein CD70-Ligand oder ein Antikörpermolekül, das an OX40, CD40 oder CD70 bindet, ist;
(iv) der Makrophagen-Zellen-Engager ein CD2-Agonist; ein CD40L; ein OX40L; ein Antikörpermolekül, das an OX40, CD40 oder CD70 bindet; ein Agonist eines Toll-like-Rezeptors (TLR); CD47; oder ein STING-Agonist ist; oder
(v) der Dendritische-Zellen-Engager ein CD2-Agonist, ein OX40-Antikörper, ein OX40L, ein 41BB-Agonist, ein Toll-like-Rezeptor-Agonist oder ein Fragment davon, ein CD47-Agonist oder ein STING-Agonist ist.

7. Multispezifisches Molekül nach einem der Ansprüche 1-6, ferner umfassend:
(i) ein Zytokinmolekül, ausgewählt aus Interleukin-2 (IL-2), Interleukin-7 (IL-7), Interleukin-12 (IL-12), Interleukin-15 (IL-15), Interleukin-18 (IL-18), Interleukin-21 (IL-21) oder Interferon gamma, oder einem Fragment oder einer Variante davon, oder einer Kombination davon; oder
(ii) ein stromal modifizierendes Molekül, das ein Hyaluronidase-Molekül, ein Collagenase-Molekül, ein Chondroitinase-Molekül, eine Matrix-Metalloproteinase oder eine Variante davon umfasst.

8. Multispezifisches Molekül nach einem der Ansprüche 1-7, umfassend:
(a) die erste Polypeptidkette, die vom N-Terminus zum C-Terminus die folgende Konfiguration aufweist: einen ersten Abschnitt der ersten ABM, die eine VH-CH1 eines ersten Fab-Moleküls umfasst und, gegebenenfalls über einen Linker, mit einem ersten Immunglobulin CH2 verbunden ist, das, gegebenenfalls über einen Linker, mit dem konstanten TCRα-Bereich oder einem Fragment davon verbunden ist;
(b) die zweite Polypeptidkette, die vom N-Terminus zum C-Terminus die folgende Konfiguration aufweist: einen ersten Abschnitt der zweiten ABM, die eine VH-CH1 eines zweiten Fab-Moleküls umfasst und, gegebenenfalls über einen Linker, mit einem zweiten Immunglobulin CH2 verbunden ist, das, gegebenenfalls über einen Linker, mit dem konstanten TCRβ-Bereich oder einem Fragment davon verbunden ist;
(c) ein drittes Polypeptid, das die folgende Konfiguration vom N-Terminus zum C-Terminus aufweist: eine VL-CL des ersten Fab; und
(d) ein viertes Polypeptid, das die folgende Konfiguration vom N-Terminus zum C-Terminus aufweist: eine VL-CL des zweiten Fab,
wobei die VL-CL des ersten Fab vom Kappa-Subtyp ist und die VL-CL des zweiten Fab vom Lambda-Subtyp ist oder die VL-CL des ersten Fab vom Lambda-Subtyp ist und die VL-CL des zweiten Fab vom Kappa-Subtyp ist;
wobei das erste und das dritte Polypeptid die erste ABM bilden, die ein erstes Antigen bindet, und das zweite und das vierte Polypeptid die zweite ABM bilden, die ein zweites Antigen bindet,
wobei das erste und das zweite Polypeptid ein Heterodimer bilden, und
wobei optional der konstante TCRα-Bereich die Aminosäuresequenz von SEQ-ID-NR: 1 oder eine Sequenz mit mindestens 75, 80, 85, 90 oder 99 % Identität davon umfasst und/oder der konstante TCRβ-Bereich die Aminosäuresequenz von SEQ-ID-NR: 2 oder eine Sequenz mit mindestens 75, 80, 85, 90 oder 99 % Identität davon umfasst.

9. Isoliertes Nukleinsäuremolekül, welches das multispezifische Molekül nach einem der Ansprüche 1-8 codiert.

10. Vektor, umfassend das Nukleinsäuremolekül nach Anspruch 9.

11. Wirtszelle, umfassend das Nukleinsäuremolekül nach Anspruch 9 oder den Vektor nach Anspruch 10.

12. Pharmazeutische Zusammensetzung, umfassend das multispezifische Molekül nach einem der Ansprüche 1-8 und einen pharmazeutisch annehmbaren Träger, Hilfsstoff oder Stabilisator.

13. Verfahren zur Herstellung des multispezifischen Moleküls nach einem der Ansprüche 1-8, umfassend die Kultivierung der Wirtszelle nach Anspruch 11 unter Bedingungen, die für die Genexpression und/oder Heterodimerisation geeignet sind.

14. Multispezifisches Molekül nach einem der Ansprüche 1-8 zur Verwendung in einem Verfahren zur Behandlung von Krebs bei einem Subjekt, das dessen bedarf, wobei das Verfahren das Verabreichen des multispezifischen Moleküls an das Subjekt in einer zur Behandlung des Krebses wirksamen Menge umfasst.

## Revendications

1. Molécule multispécifique comprenant au moins deux chaînes polypeptidiques non contiguës et comprenant :
(a) une première fraction de liaison à un antigène (ABM),
(b) une seconde ABM qui ne se lie pas au même épitope que la première ABM, et
(c) un domaine d'hétérodimérisation comprenant :
- une première chaîne polypeptidique comprenant un domaine CH2 d'immunoglobuline relié, éventuellement par l'intermédiaire d'un lieur, à un domaine constant TCRα ou un fragment de celui-ci ; et
- une seconde chaîne polypeptidique comprenant un domaine CH2 d'immunoglobuline relié, éventuellement par l'intermédiaire d'un lieur, à un domaine constant TCRβ ou un fragment de celui-ci ; dans lequel :
(i) la première ABM est reliée, éventuellement par l'intermédiaire d'un lieur, à la première chaîne polypeptidique ;
(ii) la seconde ABM est reliée, éventuellement par l'intermédiaire d'un lieur, à la seconde chaîne polypeptidique ;
(iii) le domaine constant TCRα ou un fragment de celui-ci comprend ou est constitué d'au moins 20, 30, 40, 50, 60, 70, 80, 90 ou 100 acides aminés contigus de SEQ ID NO: 158 ou d'au moins 20, 30, 40, 50, 60, 70 ou 80 acides aminés contigus de SEQ ID NO: 1 ; et/ou
(iv) le domaine constant TCRβ ou un fragment de celui-ci comprend ou est constitué d'au moins 20, 30, 40, 50, 60, 70, 80, 90, 100, 110, 120, 130, 140, 150, 160 ou 170 acides aminés contigus de SEQ ID NO: 159 ou d'au moins 20, 30, 40, 50, 60, 70, 80, 90, 100, 110, 120 ou 130 acides aminés contigus de SEQ ID NO: 2.

2. Molécule multispécifique selon la revendication 1,
(i) dans laquelle :
la première ABM est reliée, éventuellement par l'intermédiaire d'un lieur, à la terminaison N ou à la terminaison C de la première chaîne polypeptidique ; et
la seconde ABM est reliée, éventuellement par l'intermédiaire d'un lieur, à la terminaison N ou à la terminaison C de la seconde chaîne polypeptidique ;
(ii) dans laquelle :
la première chaîne polypeptidique comprend le domaine CH2 d'immunoglobuline relié, éventuellement par l'intermédiaire d'un lieur, à la terminaison N ou à la terminaison C du domaine constant TCRα ou un fragment de celui-ci, et/ou
la seconde chaîne polypeptidique comprend le domaine CH2 d'immunoglobuline relié, éventuellement par l'intermédiaire d'un lieur, à la terminaison N ou la terminaison C du domaine constant TCRβ ou un fragment de celui-ci ; et/ou
(iii) dans laquelle :
(a) la première chaîne polypeptidique du domaine d'hétérodimérisation ne comprend pas de domaine CH3 d'immunoglobuline ou une partie de celui-ci ;
(b) la seconde chaîne polypeptidique du domaine d'hétérodimérisation ne comprend pas de domaine CH3 d'immunoglobuline ou une partie de celui-ci ;
(c) ni la première chaîne polypeptidique ni la seconde chaîne polypeptidique du domaine d'hétérodimérisation ne comprend un domaine CH3 d'immunoglobuline ou une partie de celui-ci ;
éventuellement, dans laquelle le lieur comprend ou est constitué de la séquence d'acides aminés de l'une quelconque des SEQ ID NO: 101-110.

3. Molécule multispécifique selon la revendication 1 ou 2, dans laquelle :
(i) le domaine constant TCRα comprend ou est constitué de la séquence d'acides aminés de SEQ ID NO: 158, ou d'une séquence ayant au moins 75, 80, 85, 90, ou 99 % d'identité de celle-ci, et/ou
le domaine constant TCRβ comprend ou est constitué de la séquence d'acides aminés de SEQ ID NO: 159, ou d'une séquence ayant au moins 75, 80, 85, 90, ou 99 % d'identité de celle-ci, éventuellement, dans laquelle le domaine constant TCRα comprend ou est constitué de la séquence d'acides aminés de SEQ ID NO: 158 et/ou le domaine constant TCRβ comprend ou est constitué de la séquence d'acides aminés de SEQ ID NO: 159 ;
(ii) le domaine constant TCRα comprend ou est constitué de la séquence d'acides aminés de SEQ ID NO: 1, ou d'une séquence ayant au moins 75, 80, 85, 90, ou 99 % d'identité de celle-ci, et/ou
le domaine constant TCRβ comprend ou est constitué de la séquence d'acides aminés de SEQ ID NO: 2, ou d'une séquence ayant au moins 75, 80, 85, 90, ou 99 % d'identité de celle-ci, éventuellement, dans laquelle le domaine constant TCRα comprend ou est constitué de la séquence d'acides aminés de SEQ ID NO: 1 et/ou le domaine constant TCRβ comprend ou est constitué de la séquence d'acides aminés de SEQ ID NO: 2 ;
(iii) le domaine constant TCRα ou un fragment de celui-ci comprend ou est constitué d'au moins 30, 40, 50, 60, 70, 80, 90 ou 100 acides aminés contigus de SEQ ID NO: 158 ou d'au moins 30, 40, 50, 60, 70 ou 80 acides aminés contigus de SEQ ID NO: 1 ; et/ou
le domaine constant TCRβ ou un fragment de celui-ci comprend ou est constitué d'au moins 30, 40, 50, 60, 70, 80, 90, 100, 110, 120, 130, 140, 150, 160 ou 170 acides aminés contigus de SEQ ID NO: 159 ou d'au moins 30, 40, 50, 60, 70, 80, 90, 100, 110, 120 ou 130 acides aminés contigus de SEQ ID NO: 2 ; ou (iv) le domaine constant TCRα ou un fragment de celui-ci comprend les acides aminés 1-85 ou 1-93 de SEQ ID NO: 158 ; et/ou
le domaine constant TCRβ ou un fragment de celui-ci comprend les acides aminés 1-130 de SEQ ID NO: 159,
éventuellement, dans laquelle le domaine constant TCRα ou un fragment de celui-ci comprend une substitution T49C, numérotée selon SEQ ID NO: 158 ; et/ou le domaine constant TCRβ ou un fragment de celui-ci comprend une substitution S57C, numérotée selon SEQ ID NO: 159.

4. Molécule multispécifique selon l'une quelconque des revendications 1 à 3, dans laquelle :
(i) la première ABM comprend une première molécule d'anticorps, et la seconde ABM comprend une seconde molécule d'anticorps, dans laquelle :
(a) la première molécule d'anticorps comprend une région constante de chaîne légère kappa ou un fragment de celle-ci, et la seconde molécule d'anticorps comprend une région constante de chaîne légère lambda ou un fragment de celle-ci ; ou
(b) la première molécule d'anticorps comprend une région constante de chaîne légère lambda ou un fragment de celle-ci, et la seconde molécule d'anticorps comprend une région constante de chaîne légère kappa ou un fragment de celle-ci ; ou
(ii) la première ABM comprend une première chaîne lourde comprenant une première région variable de chaîne lourde et une première région constante de chaîne lourde, et une chaîne légère lambda comprenant une région variable lambda et une région constante lambda, et la seconde ABM comprend une seconde chaîne lourde comprenant une seconde région variable de chaîne lourde et une seconde région constante de chaîne lourde, et une chaîne légère kappa comprenant une région variable kappa et une région constante kappa.

5. Molécule multispécifique selon l'une quelconque des revendications 1 à 4, dans laquelle la première ABM ou la seconde ABM comprend une fraction de ciblage tumoral qui se lie à un antigène tumoral, un antigène stromal ou un antigène hématologique, dans laquelle :
(i) l'antigène tumoral est présent sur un ou plusieurs parmi le cancer du pancréas, du sein, colorectal, du poumon, de la peau, des ovaires ou du foie ;
(ii) l'antigène tumoral est choisi parmi les éléments suivants : PDL1, mésothéline, CD47, gangloside 2 (GD2), antigène de cellules souche de la prostate (PSCA), antigène membranaire spécifique de la prostate (PMSA), antigène spécifique de la prostate (PSA), antigène carcinoembryonnaire (CEA), Ron Kinase, c-Met, récepteur de laminine immature, TAG-72, BING-4, canal chlorure activé par le calcium 2, Cyclin-B1, 9D7, Ep-CAM, EphA3, Her2/neu, télomérase, SAP-1, Survivine, NY-ESO-1/LAGE-1, PRAME, SSX-2, Melan-A/MART-1, Gp100/pmel17, Tyrosinase, MC1R, β-caténine, BRCA1/2, CDK4, CML66, Fibronectine, p53, Ras, récepteur TGF-B, AFP, ETA, MAGE, CA-125, BAGE, GAGE, CDC27, α actinin-4, TRP1/gp75, TRP2, gangliosides, WT1, récepteur du facteur de croissance épidermique (EGFR), CD20, MART-2, MUC1, MUC2, MUM1, MUM2, MUM3, NA88-1, NPM, OA1, OGT, RCC, RUI11, RU12, SAGE, TRG, TSTA, récepteur alpha du folate, L1-CAM, CAIX, EGFRvIII, gpA33, GD3, GM2, VEGFR, intégrines, glucides (Le), IGF1R, TRAILR1, TRAILR2, ou RANKL,
éventuellement, dans laquelle les intégrines sont l'intégrine alphaVbeta3 ou l'intégrine alpha5Beta1 ;
(iii) l'antigène stromal est choisi parmi la protéase d'activation de fibroblaste (FAP), le TGF-bêta, l'acide hyaluronique, le collagène, la ténascine C, ou la ténascine W ; ou
(iv) l'antigène hématologique est choisi parmi CD19, CD33, CD47, CD123, CD20, CD99, CD30, BCMA, CD38, CD22, SLAMF7, NY-ESO1.

6. Molécule multispécifique selon l'une quelconque des revendications 1 à 5, dans laquelle la première ABM ou la seconde ABM comprend un introducteur de cellules immunitaires comprenant un introducteur de cellules T, un introducteur de cellules tueuses naturelles (NK), un introducteur de cellules B, un introducteur de cellules dendritiques, ou un introducteur de cellules macrophages, dans laquelle :
(i) l'introducteur de cellules T se lie à CD3, TCRα, TCRβ, TCRγ, TCRζ, ICOS, CD28, CD27, HVEM, LIGHT, CD40, 4-1BB, OX40, DR3, GITR, CD30, TIM1, SLAM, CD2 ou CD226 ;
(ii) l'introducteur de cellules NK est choisi parmi une molécule d'anticorps, ou un ligand qui se lie à : NKp30, NKp40, NKp44, NKp46, NKG2D, DNAM1, DAP10, CD16, CRTAM, CD27, PSGL1, CD96, CD100 (SEMA4D), NKp80, CD244 (également connu sous le nom de SLAMF4 ou 2B4), SLAMF6, SLAMF7, KIR2DS2, KIR2DS4, KIR3DS1, KIR2DS3, KIR2DS5, KIR2DS1, CD94, NKG2C, NKG2E ou CD160 ;
(iii) l'introducteur de cellules B est un CD40L, un OX40L, ou un ligand de CD70, ou une molécule d'anticorps qui se lie à OX40, CD40 ou CD70 ;
(iv) l'introducteur de cellule macrophagique est un agoniste de CD2 ; un CD40L ; un OX40L ; une molécule d'anticorps qui se lie à OX40, CD40 ou CD70 ; un agoniste d'un récepteur de type Toll (TLR) ; CD47 ; ou un agoniste de STING ; ou
(v) l'introducteur de cellule dendritique est un agoniste de CD2, un anticorps OX40, un OX40L, un agoniste de 41BB, un agoniste de récepteur de type Toll ou un fragment de celui-ci, un agoniste de CD47, ou un agoniste de STING.

7. Molécule multispécifique selon l'une quelconque des revendications 1 à 6, comprenant en outre :
(i) une molécule de cytokine choisie parmi l'interleukine-2 (IL-2), l'interleukine-7 (IL-7), l'interleukine-12 (IL-12), l'interleukine-15 (IL-15), l'interleukine-18 (IL-18), l'interleukine-21 (IL-21), ou l'interféron gamma, ou un fragment ou variant de ceux-ci, ou une combinaison de ceux-ci ; ou
(ii) une molécule de modification de stroma comprenant une molécule de hyaluronidase, une molécule de collagénase, une molécule de chondroïtinase, une métalloprotéinase matricielle, ou un variant de celle-ci.

8. Molécule multispécifique selon l'une quelconque des revendications 1 à 7, comprenant :
(a) la première chaîne polypeptidique ayant la configuration suivante de la terminaison N à la terminaison C : une première partie de la première ABM comprenant un VH-CH1 d'une première molécule Fab reliée, éventuellement par l'intermédiaire d'un lieur, à une première immunoglobuline CH2 reliée, éventuellement par l'intermédiaire d'un lieur, au domaine constant TCRα ou un fragment de celui-ci ;
(b) la seconde chaîne polypeptidique ayant la configuration suivante de la terminaison N à la terminaison C : une première partie de la seconde ABM comprenant un VH-CH1 d'une seconde molécule Fab reliée, éventuellement par l'intermédiaire d'un lieur, à une seconde immunoglobuline CH2 reliée, éventuellement par l'intermédiaire d'un lieur, au domaine constant TCRβ ou un fragment de celui-ci ;
(c) un troisième polypeptide ayant la configuration suivante de la terminaison N à la terminaison C : un VL-CL du premier Fab ; et
(d) un quatrième polypeptide ayant la configuration suivante de la terminaison N à la terminaison C : un VL-CL du second Fab,
dans laquelle le VL-CL du premier Fab est de sous-type kappa et le VL-CL du second Fab est de sous-type lambda, ou le VL-CL du premier Fab est de sous-type lambda et le VL-CL du second Fab est de sous-type kappa ;
dans laquelle les premier et troisième polypeptides forment la première ABM qui se lie à un premier antigène, et les deuxième et quatrième polypeptides formant la seconde ABM qui se lie à un second antigène,
dans laquelle les premier et deuxième polypeptides forment un hétérodimère, et,
éventuellement, dans laquelle le domaine constant TCRα comprend la séquence d'acides aminés de SEQ ID NO: 1 ou une séquence ayant au moins 75, 80, 85, 90, ou 99 % d'identité de celle-ci, et/ou le domaine constant TCRβ comprend la séquence d'acides aminés de SEQ ID NO: 2 ou une séquence ayant au moins 75, 80, 85, 90, ou 99 % d'identité de celle-ci.

9. Molécule d'acide nucléique isolée codant pour la molécule multispécifique selon l'une quelconque des revendications 1 à 8.

10. Vecteur comprenant la molécule d'acide nucléique selon la revendication 9.

11. Cellule hôte comprenant la molécule d'acide nucléique selon la revendication 9 ou le vecteur selon la revendication 10.

12. Composition pharmaceutique comprenant la molécule multispécifique selon l'une quelconque des revendications 1 à 8, et un support, excipient ou stabilisant pharmaceutiquement acceptable.

13. Procédé de fabrication de la molécule multispécifique selon l'une quelconque des revendications 1 à 8, comprenant la culture de la cellule hôte selon la revendication 11, dans des conditions adaptées à l'expression génique et/ou à l'hétérodimérisation.

14. Molécule multispécifique selon l'une quelconque des revendications 1 à 8 destinée à être utilisée dans un procédé de traitement du cancer chez un sujet en ayant besoin, le procédé comprenant l'administration au sujet de la molécule multispécifique en une quantité efficace pour traiter le cancer.
